# EUROPEAN PATENT SPECIFICATION

(11) **EP 3 434 788 B1**
(45) Date of publication and mention of the grant of the patent: **23.08.2023**
(21) Application number: 18194185.7
(22) Date of filing: 14.03.2014
(51) Int. Cl.: C12Q 1/6886

(54) **CLASSIFICATION AND ACTIONABILITY INDICES FOR LUNG CANCER**
KLASSIFIZIERUNGS- UND AKTIONABILITÄTSINDIZES FÜR LUNGENKREBS
INDICE DE CLASSIFICATION ET D'APTITUDE AU TRAITEMENT POUR LE CANCER DU POUMON

(30) Priority: 15.03.2013 US 201361799399 P
(43) Date of publication of application: 30.01.2019
(62) Divisional of application: 14717365.2
(73) Proprietor: Life Technologies Corporation, Carlsbad, CA 92008 (US)
(72) Inventor: BANDLA, Santhoshi, Carlsbad, CA 92008 (US); ROSS, Douglas, Carlsbad, CA 92008 (US); SADIS, Seth, Carlsbad, CA 92008 (US); WYNGAARD, Peter, Carlsbad, CA 92008 (US)
(74) Representative: Thermo Fisher Scientific- Life Sciences Solutions Group

(56) References cited:
- WO-A1-2012/170715
- WO-A1-2013/033169
- WO-A2-2011/056688
- WO-A2-2012/023138
- US-A1- 2010 286 143
- LAMONTANARA ALLAN JOAQUIM ET AL: "Mechanisms of resistance to BCR-ABL and other kinase inhibitors", BIOCHIMICA ET BIOPHYSICA ACTA (BBA) - PROTEINS & PROTEOMICS, ELSEVIER, NETHERLANDS, vol. 1834, no. 7, 28 December 2012 (2012-12-28), pages 1449-1459, XP028562761, ISSN: 1570-9639, DOI: 10.1016/J.BBAPAP.2012.12.009
- HSIEH M H ET AL: "Complex mutation patterns of epidermal growth factor receptor gene associated with variable responses to gefitinib treatment in patients with non-small cell lung cancer", LUNG CANCER, ELSEVIER, AMSTERDAM, NL, vol. 53, no. 3, 1 September 2006 (2006-09-01), pages 311-322, XP024893994, ISSN: 0169-5002, DOI: 10.1016/J.LUNGCAN.2006.06.005 [retrieved on 2006-09-01]

## Description

### BACKGROUND

Lung cancer is the leading cause of cancer deaths among both men and women. It is a fast growing and highly fatal disease. Nearly 60% of people diagnosed with lung cancer die within one year of diagnosis and approximately 75% die within 2 years. There are two major types of lung cancer: small cell lung cancer (SCLC) and non-small cell lung cancer (NSCLC). Approximately 85% of lung cancers are NSCLC. There are 3 sub-types of NSCLC, which differ in size, shape, and biochemical make-up. Approximately 25-30% of all lung cancers are squamous cell carcinomas. Adenocarcinomas (e.g., bronchioloalveolar carcinoma) account for approximately 40% of lung cancers, and are usually found in the outer region of the lung. Large-cell undifferentiated carcinoma accounts for approximately 10-15% of all lung cancers.

SCLC and NSCLC are treated very differently. SCLC is mainly treated with chemotherapy, either alone or in combination with radiation. In contrast with treatment for SCLC, surgery is the only reliable method to cure NSCLC. Lymph nodes are also removed to assess the spread of cancer. In addition to surgery, chemotherapy can be used to treat NSCLC.

A growing number of treatment regimens are becoming available for lung adenocarcinomas. However, the treatment regimes in many cases are each only effective against lung cancers that have a particular genetic variation. Therefore, a test that could detect many different specific actionable genetic variations would have significant value to lung cancer patients. However, the tissue required for currently available multiple genetic variance assays far exceeds what is available from a typical tumor biopsy or resection. Furthermore, tests are not available for many of the genetic variations, and there is no tool that can recommend a treatment based on a comprehensive scan of many known, actionable genetic variations. WO2012170715 provided methods and systems of molecular profiling of diseases, such as cancer. The molecular profiling can be used to identify treatments for a disease, such as treatments that were not initially identified as a treatment for the disease or not expected to be a treatment for a particular disease. US2010286143 discloses amethod of providing a genetic profile of a tumor, that specifically determins the identity of certain alleles for each of EGFR and KRAS plus one or more alleles from one or more of AKT1, APC, BRAF, CTNNB1, FLT3, IDH1, JAK2, KIT, MAP2K1, NOTCH1, NRAS, PIK3CA, PTEN, and TP53 in the genomic DNA. WO2011056688 discloses a method of identifying a candidate treatment by determining a molecular profile using immunohistochemistry (IHC) analysis. WO2012023138 employs volatile organic compounds for detecting cell dysplasia and genetic alterations associated with lung cancer while WO2013033169 discloses methods of identifying genomic translocations associated with cancer.

The disclosed compositions, kits and methods provide comprehensive genetic variance screening of a lung cancer in a single panel utilizing a single lung cancer sample. The genetic variants screened have actionable treatments or are known to not respond well to certain treatments. This forms the basis of an actionable treatment recommendation framework provided herein.

### BRIEF SUMMARY

The disclosure provides methods, compositions and kits. In one embodiment, a method to determine an actionable treatment recommendation for a subject diagnosed with lung cancer is provided. The method comprises: obtaining a biological sample from the subject; using a set of amplification primer pairs that hybridize to and amplify ALK, ROS1, KRAS, BRAF, ERBB2, MET, RET, FGFR1 and KIT/PDGFRA genes to generate amplicons in a single assay, wherein the set of amplification primer pairs can amplify nucleic acid of one or more lung cancerassociated allelic variants of said genes; and detecting by nucleic acid sequencing at least one variant among , ALK, ROS1, KRAS, BRAF, ERBB2, MET, RET, FGFR1, and KIT/PDGFRA, genes from the sample, andKIT/PDGFRA; determining, based on the at least one variant detected, an actionable treatment recommendation for the subject.

The method may additionally comprise detecting a variant of EGFR ERRBB4, FGFR2, FGFR3, DDR2, NRAS, PTEN, MAP2K1, TP53, STK11, CTNNB1, SMAD4, FBXW7, PIK3CA, AKT1, HRAS and NOTCH 1 genesKIT/PDGFRA.

The disclosure provides a method to determine an actionable treatment recommendation for a subject diagnosed with lung cancer, comprising: detecting in a sample from a subject, at least one variant using a set of probes that hybridize to and amplify ALK, ROS1, KRAS, BRAF, ERBB2, MET, RET, FGFR1, and KIT/PDGFRA genes to detect at least one variant, and determining, based on the at least one variant detected, an actionable treatment recommendation for the subject.

A method to determine the likelihood of a response to a treatment in an individual afflicted with lung cancer is provided. The method comprises: determining the presence or absence of at least one gene variant in a sample obtained from the individual, wherein the at least one variant is in EGFR, ALK, ROS1, KRAS, BRAF, ERBB2, ERRBB4, MET, RET, FGFR1, FGFR2, FGFR3, DDR2, NRAS, PTEN, MAP2K1, TP53, STK11, CTNNB1, SMAD4, FBXW7, NOTCH 1, KIT/PDGFRA, PIK3CA, AKT1, and HRAS genes, wherein the presence of at least one variant indicates the individual is likely or unlikely to respond to the treatment, wherein the treatment is selected from: crizotinib when the variant detected is an ALK fusion; ROS 1 fusion (EZR, SLC34A2, CD74, and/or SDC4); MET gene amplification; EGFR tyrosine kinase inhibitor (TKI) when the variant detected is EGFR (L858R, Exon 19 del, and/or G719X); a non-EGFR TKI treatment when the variant detected is EGFR T790M; a MEK inhibitor when the variant detected is KRAS G12C/V/D/A/S/R/F, G13C, G13D and/or G12F; vermurafenib when the variant detected is BRAF V600E; an irreversible pan-erb inhibitor when the variant detected is ERBB2 exon 20 ins; and a PIC3CA inhibitor when the variant detected is PIK3CA (E545K, E545G, E545a, H1047R, E542K and/or H1047L).

In another embodiment, the disclosure provides a method of detecting a nucleic acid variant in a sample, comprising obtaining a biological sample, amplifying at least one gene selected from EGFR, ALK, ROS 1, KRAS, BRAF, ERBB2, ERRBB4, MET, RET, FGFR1, FGFR2, FGFR3, DDR2, NRAS, PTEN, MAP2K1, TP53, STK11, CTNNB1, SMAD4, FBXW7, NOTCH 1, KIT/PDGFRA, PIK3CA, AKT1, and HRAS genes, using primers that (a) amplifying at least one variant selected from EGFR (L858R, Exon 19 del, G719X and/or T790M), KRAS (G12C/V/D/A/S/R/F, G13C, G13D and/or G12F), BRAF (L597R, D594H/N, V600E), ERBB2 exon 20 ins, PIK3CA (E545K, E545G, E545a, H1047R, and/or H1047L); and (b) detecting at least one nucleic acid variant present in the sample.

A method of treating lung adenocarcinoma in a patient is disclosed. The method comprises: testing for the presence of variants in at least one of ALK, ROS 1, KRAS, BRAF, ERBB2, MET, RET, FGFR1, and KIT/PDGFRA genes in a lung tumor sample from the patient and administering a therapeutically effective amount a treatment to the patient, wherein the treatment is: Crizotinib when the variant detected is an ALK fusion, ROS1 fusion (EZR, SLC34A2, CD74, and/or SDC4), or MET gene amplification; EGFR tyrosine kinase inhibitor (TKI) when the variant detected is EGFR (L858R, Exon 19 del, and/or G719X); a MEK inhibitor when the variant detected is KRAS G12C/V/D/A/S/R/F, G13C, G13D and/or G12F; Vermurafenib when the variant detected is BRAF V600E; and an irreversible pan-erb inhibitor when the variant detected is ERBB2 exon 20 ins.

In yet another embodiment, the disclosure provides a method of identifying patients with lung cancer eligible for treatment with crizotnib, an EGFR TKI, or a treatment other than an EGFR TKI, a MEK inhibitor, vermurafenib, or an irreversible pan-erb inhibitor, comprising testing a lung tumor sample from the patient in a single assay for the presence of a variant comprising an ALK fusion, ROS1 fusion (EZR, SLC34A2, CD74, and/or SDC4), EGFR (L858R, Exon 19 del, and/or T790M), KRAS (G12C/V/D/A), wherein the presence of at least one of said variants indicates the patient is eligible for treatment with at least one of said treatments.

The disclosure, in certain embodiments, also provides a kit comprising a set of probes, wherein the set of probes specifically recognize the genes EGFR, ALK, ROS1, KRAS, BRAF, ERBB2, ERRBB4, MET, RET, FGFR1, FGFR2, FGFR3, DDR2, NRAS, PTEN, MAP2K1, TP53, STK11, CTNNB1, SMAD4, FBXW7, NOTCH 1, KIT/PDGFRA, PIK3CA, AKT1, and HRAS, and wherein the set of probes can recognize and distinguish one or more allelic variants of the genes EGFR, ALK, ROS1, KRAS, BRAF, ERBB2, ERRBB4, MET, RET, FGFR1, FGFR2, FGFR3, DDR2, NRAS, PTEN, MAP2K1, TP53, STK11, CTNNB1, SMAD4, FBXW7, NOTCH 1, KIT/PDGFRA, PIK3CA, AKT1, and HRAS.

Certain embodiments of the disclosure further provide a composition comprising a set of probes, wherein the set of probes specifically recognize the genes EGFR, ALK, ROS1, KRAS, BRAF, ERBB2, ERRBB4, MET, RET, FGFR1, FGFR2, FGFR3, DDR2, NRAS, PTEN, MAP2K1, TP53, STK11, CTNNB1, SMAD4, FBXW7, NOTCH 1, KIT/PDGFRA, PIK3CA, AKT1, and HRAS, and wherein the set of probes can recognize and distinguish one or more allelic variants of the genes EGFR, ALK, ROS1, KRAS, BRAF, ERBB2, ERRBB4, MET, RET, FGFR1, FGFR2, FGFR3, DDR2, NRAS, PTEN, MAP2K1, TP53, STK11, CTNNB1, SMAD4, FBXW7, NOTCH 1, KIT/PDGFRA, PIK3CA, AKT1, and HRAS.

### DETAILED DESCRIPTION

The disclosure provides compositions, kits, and methods for detecting a plurality of genes and associated variants in a subject with lung cancer. The compositions, kits, and methods include a set of oligonucleotides, typically primers and/or probes that can hybridize to identify a gene variant. The methods disclosed herein provide for a mutation status of a tumor to be determined and subsequently associated with an actionable treatment recommendation. In certain embodiments, methods for determining a treatment and treating a subject with lung cancer are provided.

An advantage of the disclosed compositions, kits, and methods is the ability to recommend an actionable treatment for a subject diagnosed with lung cancer, by comprehensively screening a tumor sample for a plurality of high and/or optionally low prevalence genetic variances that are most likely to have an impact on the appropriate clinical course of action for the subject. In certain embodiments, by determining the mutation status of the disclosed combination of gene variations, the methods provide an actionable treatment recommendation for greater than 50% of lung adenocarcinoma subjects. This comprehensive screening is performed in a single panel and therefore can be performed utilizing a single biological sample, thus preserving valuable sample.

### Definitions

"Lung cancer" refers generally to two main types of lung cancer categorized by the size and appearance of the malignant cells: non-small cell (approximately 80% of cases) and small-cell (roughly 20% of cases) lung cancer. Lung adenocarcinoma is the most common subtype of non-small cell lung cancer (NSCLC); other subtypes include squamous cell lung carcinoma, bronchioloalveolar carcinoma, large cell carcinoma, carcinoid, adenoid cystic carcinoma, cylindroma, and mucoepidermoid carcinoma. In one embodiment, lung cancers are staged according to stages I-IV, with I being an early stage and IV being the most advanced.

"Prognosis" refers, e.g., to overall survival, long term mortality, and disease free survival. In one embodiment, long term mortality refers to death within 5 years after diagnosis of lung cancer. Although prognosis within 1, 2, or 3 years is also contemplated as is a prognosis beyond 5 years.

Other forms of cancer include carcinomas, sarcomas, adenocarcinomas, lymphomas, leukemias, etc., including solid and lymphoid cancers, head and neck cancer, e.g., oral cavity, pharyngeal and tongue cancer, kidney, breast, kidney, bladder, colon, ovarian, prostate, pancreas, stomach, brain, head and neck, skin, uterine, testicular, esophagus, and liver cancer, including hepatocarcinoma, lymphoma, including non-Hodgkin's lymphomas *(e.g*., Burkitt's, Small Cell, and Large Cell lymphomas) and Hodgkin's lymphoma, leukemia, and multiple myeloma.

The term "marker" or "biomarker" refers to a molecule (typically protein, nucleic acid, carbohydrate, or lipid) that is expressed in the cell, expressed on the surface of a cancer cell or secreted by a cancer cell in comparison to a non-cancer cell, and which is useful for the diagnosis of cancer, for providing a prognosis, and for preferential targeting of a pharmacological agent to the cancer cell. Oftentimes, such markers are molecules that are overexpressed in a lung cancer or other cancer cell in comparison to a non-cancer cell, for instance, 1-fold overexpression, 2-fold overexpression, 3-fold overexpression or more in comparison to a normal cell. Further, a marker can be a molecule that is inappropriately synthesized in the cancer cell, for instance, a molecule that contains deletions, additions or mutations in comparison to the molecule expressed on a normal cell. Alternatively, such biomarkers are molecules that are underexpressed in a cancer cell in comparison to a non-cancer cell, for instance, 1-fold underexpression, 2-fold underexpression, 3-fold underexpression, or more. Further, a marker can be a molecule that is inappropriately synthesized in cancer, for instance, a molecule that contains deletions, additions or mutations in comparison to the molecule expressed on a normal cell.

It will be understood by the skilled artisan that markers may be used in combination with other markers or tests for any of the uses, *e.g*., prediction, diagnosis, or prognosis of cancer, disclosed herein.

"Biological sample" includes sections of tissues such as biopsy and autopsy samples, and frozen sections taken for histologic purposes. Such samples include blood and blood fractions or products (*e.g*., serum, platelets, red blood cells, and the like), sputum, bronchoalveolar lavage, cultured cells, *e.g*., primary cultures, explants, and transformed cells, stool, urine, etc. A biological sample is typically obtained from a eukaryotic organism, most preferably a mammal such as a primate *e.g*., chimpanzee or human; cow; dog; cat; a rodent, *e.g*., guinea pig, rat, Mouse; rabbit; or a bird; reptile; or fish.

A "biopsy" refers to the process of removing a tissue sample for diagnostic or prognostic evaluation, and to the tissue specimen itself. Any biopsy technique known in the art can be applied to the diagnostic and prognostic methods of the present invention. The biopsy technique applied will depend on the tissue type to be evaluated *(e.g*., lung *etc.),* the size and type of the tumor, among other factors. Representative biopsy techniques include, but are not limited to, excisional biopsy, incisional biopsy, needle biopsy, surgical biopsy, and bone marrow biopsy. An "excisional biopsy" refers to the removal of an entire tumor mass with a small margin of normal tissue surrounding it. An "incisional biopsy" refers to the removal of a wedge of tissue from within the tumor. A diagnosis or prognosis made by endoscopy or radiographic guidance can require a "core-needle biopsy", or a "fine-needle aspiration biopsy" which generally obtains a suspension of cells from within a target tissue. Biopsy techniques are discussed, for example, in Harrison's Principles of Internal Medicine, Kasper, et al., eds., 16th ed., 2005, Chapter 70, and throughout Part V.

The terms "overexpress," "overexpression," or "overexpressed" interchangeably refer to a protein or nucleic acid (RNA) that is translated or transcribed at a detectably greater level, usually in a cancer cell, in comparison to a normal cell. The term includes overexpression due to transcription, post transcriptional processing, translation, post-translational processing, cellular localization *(*e.g., organelle, cytoplasm, nucleus, cell surface), and RNA and protein stability, as compared to a normal cell. Overexpression can be detected using conventional techniques for detecting mRNA (*i.e.*, RT-PCR, PCR, hybridization) or proteins (*i.e*., ELISA, immunohistochemical techniques). Overexpression can be 10%, 20%, 30%, 40%, 50%, 60%, 70%, 80%, 90% or more in comparison to a normal cell. In certain instances, overexpression is 1-fold, 2-fold, 3-fold, 4-fold or more higher levels of transcription or translation in comparison to a normal cell.

The terms "underexpress," "underexpression," or "underexpressed" or "downregulated" interchangeably refer to a protein or nucleic acid that is translated or transcribed at a detectably lower level in a cancer cell, in comparison to a normal cell. The term includes underexpression due to transcription, post transcriptional processing, translation, post-translational processing, cellular localization (*e.g*., organelle, cytoplasm, nucleus, cell surface), and RNA and protein stability, as compared to a control. Underexpression can be detected using conventional techniques for detecting mRNA (*i.e*., RT-PCR, PCR, hybridization) or proteins (*i.e*., ELISA, immunohistochemical techniques). Underexpression can be 10%, 20%, 30%, 40%, 50%, 60%, 70%, 80%, 90% or less in comparison to a control. In certain instances, underexpression is 1-fold, 2-fold, 3-fold, 4-fold or more lower levels of transcription or translation in comparison to a control.

The term "differentially expressed" or "differentially regulated" refers generally to a protein or nucleic acid that is overexpressed (upregulated) or underexpressed (downregulated) in one sample compared to at least one other sample, generally in a cancer patient compared to a sample of non-cancerous tissue in the context of the present invention.

"Therapeutic treatment" and "cancer therapies" refers to chemotherapy, hormonal therapy, radiotherapy, immunotherapy, and biologic and small molecule targeted therapy.

By "therapeutically effective amount or dose" or "sufficient amount or dose" herein is meant a dose that produces effects for which it is administered. The exact dose will depend on the purpose of the treatment, and will be ascertainable by one skilled in the art using known techniques (*see, e.g.,* Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that arc later modified, e.g., hydroxyproline, γ-carboxyglutamate, and O-phosphoserine. Amino acid analogs refers to compounds that have the same basic chemical structure as a naturally occurring amino acid, *i.e.,* an a carbon that is bound to a hydrogen, a carboxyl group, an amino group, and an R group, *e.g.*, homoserine, norleucine, methionine sulfoxide, methionine methyl sulfonium. Such analogs have modified R groups (*e.g*., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention.

The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serino (S), Threonine (T); and 8) Cysteine (C), Methionine (M). *See, e.g.,* Creighton, *Proteins* (1984).

The phrase "specifically (or selectively) binds" when referring to a protein, nucleic acid, antibody, or small molecule compound refers to a binding reaction that is determinative of the presence of the protein or nucleic acid, such as the differentially expressed genes of the present invention, often in a heterogeneous population of proteins or nucleic acids and other biologics. In the case of antibodies, under designated immunoassay conditions, a specified antibody may bind to a particular protein at least two times the background and more typically more than 10 to 100 times background. Specific binding to an antibody under such conditions requires an antibody that is selected for its specificity for a particular protein. For example, polyclonal antibodies can be selected to obtain only those polyclonal antibodies that are specifically immunoreactive with the selected antigen and not with other proteins. This selection may be achieved by subtracting out antibodies that cross-react with other molecules. A variety of immunoassay formats may be used to select antibodies specifically immunoreactive with a particular protein. For example, solid-phase ELISA immunoassays are routinely used to select antibodies specifically immunoreactive with a protein (*see, e.g.,* Harlow & Lane, Antibodies, A Laboratory Manual (1988) for a description of immunoassay formats and conditions that can be used to determine specific immunoreactivity).

The phrase "functional effects" in the context of assays for testing compounds that modulate a marker protein includes the determination of a parameter that is indirectly or directly under the influence of a biomarker of the invention, e.g., a chemical or phenotypic. A functional effect therefore includes ligand binding activity, transcriptional activation or repression, the ability of cells to proliferate, the ability to migrate, among others. "Functional effects" include *in vitro, in vivo,* and *ex vivo* activities.

By "determining the functional effect" is meant assaying for a compound that increases or decreases a parameter that is indirectly or directly under the influence of a biomarker of the invention, *e.g*., measuring physical and chemical or phenotypic effects. Such functional effects can be measured by any means known to those skilled in the art, *e.g.,* changes in spectroscopic characteristics (*e.g.,* fluorescence, absorbance, refractive index); hydrodynamic (*e.g*., shape), chromatographic; or solubility properties for the protein; ligand binding assays, *e.g.*, binding to antibodies; measuring inducible markers or transcriptional activation of the marker; measuring changes in enzymatic activity; the ability to increase or decrease cellular proliferation, apoptosis, cell cycle arrest, measuring changes in cell surface markers. The functional effects can be evaluated by many means known to those skilled in the art, *e.g*., microscopy for quantitative or qualitative measures of alterations in morphological features, measurement of changes in RNA or protein levels for other genes expressed in placental tissue, measurement of RNA stability, identification of downstream or reporter gene expression (CAT, luciferase, β-gal, GFP and the like), *e.g.,* via chemiluminescence, fluorescence, colorimetric reactions, antibody binding, inducible markers, etc.

"Inhibitors," "activators," and "modulators" of the markers are used to refer to activating, inhibitory, or modulating molecules identified using *in vitro* and *in vivo* assays of cancer biomarkers. Inhibitors are compounds that, *e.g*., bind to, partially or totally block activity, decrease, prevent, delay activation, inactivate, desensitize, or down regulate the activity or expression of cancer biomarkers. "Activators" are compounds that increase, open, activate, facilitate, enhance activation, sensitize, agonize, or up regulate activity of cancer biomarkers, *e.g*., agonists. Inhibitors, activators, or modulators also include genetically modified versions of cancer biomarkers, *e.g*., versions with altered activity, as well as naturally occurring and synthetic ligands, antagonists, agonists, antibodies, peptides, cyclic peptides, nucleic acids, antisense molecules, ribozymes, RNAi and siRNA molecules, small organic molecules and the like. Such assays for inhibitors and activators include, *e.g.,* expressing cancer biomarkers *in vitro,* in cells, or cell extracts, applying putative modulator compounds, and then determining the functional effects on activity, as described above.

Samples or assays comprising cancer biomarkers that are treated with a potential activator, inhibitor, or modulator are compared to control samples without the inhibitor, activator, or modulator to examine the extent of inhibition. Control samples (untreated with inhibitors) are assigned a relative protein activity value of 100%. Inhibition of cancer biomarkers is achieved when the activity value relative to the control is about 80%, preferably 50%, more preferably 25-0%. Activation of cancer biomarkers is achieved when the activity value relative to the control (untreated with activators) is 110%, more preferably 150%, more preferably 200-500% (*i.e*., two to five fold higher relative to the control), more preferably 1000-3000% higher.

The term "test compound" or "drug candidate" or "modulator" or grammatical equivalents as used herein describes any molecule, either naturally occurring or synthetic, *e.g.,* protein, oligopeptide (*e.g.,* from about 5 to about 25 amino acids in length, preferably from about 10 to 20 or 12 to 18 amino acids in length, preferably 12, 15, or 18 amino acids in length), small organic molecule, polysaccharide, peptide, circular peptide, lipid, fatty acid, siRNA, polynucleotide, oligonucleotide, etc., to be tested for the capacity to directly or indirectly modulate cancer biomarkers. The test compound can be in the form of a library of test compounds, such as a combinatorial or randomized library that provides a sufficient range of diversity. Test compounds are optionally linked to a fusion partner, *e.g*., targeting compounds, rescue compounds, dimerization compounds, stabilizing compounds, addressable compounds, and other functional moieties. Conventionally, new chemical entities with useful properties are generated by identifying a test compound (called a "lead compound") with some desirable property or activity, *e.g*., inhibiting activity, creating variants of the lead compound, and evaluating the property and activity of those variant compounds. Often, high throughput screening (HTS) methods are employed for such an analysis.

In some embodiments are provided a kit that includes a set of probes. A "probe" or "probes" refers to a polynucleotide that is at least eight (8) nucleotides in length and which forms a hybrid structure with a target sequence, due to complementarity of at least one sequence in the probe with a sequence in the target region. The polynucleotide can be composed of DNA and/or RNA. Probes in certain embodiments, are detectably labeled, as discussed in more detail herein. Probes can vary significantly in size. Generally, probes are, for example, at least 8 to 15 nucleotides in length. Other probes are, for example, at least 20, 30 or 40 nucleotides long. Still other probes are somewhat longer, being at least, for example, 50, 60, 70, 80, 90 nucleotides long. Yet other probes are longer still, and are at least, for example, 100, 150, 200 or more nucleotides long. Probes can be of any specific length that falls within the foregoing ranges as well. Preferably, the probe does not contain a sequence complementary to the sequence(s) used to prime for a target sequence during the polymerase chain reaction.

The terms "complementary" or "complementarity" are used in reference to polynucleotides (that is, a sequence of nucleotides) related by the base-pairing rules. For example, the sequence "A-G-T," is complementary to the sequence "T-C-A." Complementarity may be "partial," in which only some of the nucleic acids' bases are matched according to the base pairing rules. Alternatively, there may be "complete" or "total" complementarity between the nucleic acids. The degree of complementarity between nucleic acid strands has significant effects on the efficiency and strength of hybridization between nucleic acid strands.

"Oligonucleotide" or "polynucleotide" refers to a polymer of a single-stranded or double-stranded deoxyribonucleotide or ribonucleotide, which may be unmodified RNA or DNA or modified RNA or DNA.

"Amplification detection assay" refers to a primer pair and matched probe wherein the primer pair flanks a region of a target nucleic acid, typically a target gene, that defines an amplicon, and wherein the probe binds to the amplicon.

A set of probes typically refers to a set of primers, usually primer pairs, and/or detectably-labeled probes that are used to detect the target genetic variations used in the actionable treatment recommendations of the disclosure. As a non-limiting example, a set of primers that are used to detect variants of EGFR, ALK, ROS1, KRAS, BRAF, ERBB2, ERRBB4, MET, RET, FGFR1, FGFR2, FGFR3, DDR2, NRAS, PTEN, MAP2K1, TP53, STK11, CTNNB1, SMAD4, FBXW7, NOTCH 1, KIT/PDGFRA, PIK3CA, AKT1, and HRAS, include at least one primer and typically a pair of amplification primers for each of the aforementioned genes, that are used to amplify a nucleic acid region that spans a particular genetic variant region in the aforementioned genes. As another non-limiting example, a set of amplification detection assays for EGFR, ALK, ROS1, KRAS, BRAF, ERBB2, ERRBB4, MET, RET, FGFR1, FGFR2, FGFR3, DDR2, NRAS, PTEN, MAP2K1, TP53, STK11, CTNNB1, SMAD4, FBXW7, NOTCH 1, KIT/PDGFRA, PIK3CA, AKT1, and HRAS genes includes a set of primer pairs and matched probes for each of the aforementioned genes. The primer pairs are used in an amplification reaction to define an amplicon that spans a region for a target genetic variation for each of the aforementioned genes. The set of amplicons are detected by a set of matched probes. In an exemplary embodiment, the invention is a set of TaqMan^{™} (Roche Molecular Systems, Pleasanton, CA) assays that are used to detect a set of target genetic variations used in the methods of the invention. For example, in one embodiment, the invention is a set of Taqman assays that detect the detect EGFR, ALK, ROS1, KRAS, BRAF, ERBB2, ERRBB4, MET, RET, FGFR1, FGFR2, FGFR3, DDR2, NRAS, PTEN, MAP2K1, TP53, STK11, CTNNB1, SMAD4, FBXW7, NOTCH 1, KIT/PDGFRA, PIK3CA, AKT1, and HRAS genes.

In one embodiment, the set of probes are a set of primers used to generate amplicons that are detected by a nucleic acid sequencing reaction, such as a next generation sequencing reaction. In these embodiments, for example, AmpliSEQ^{™} (Life Technologies/Ion Torrent, Carlsbad, CA) or TruSEQ^{™} (Illumina, San Diego, CA) technology can be employed.

A modified ribonucleotide or deoxyribonucleotide refer to molecules that can be used in place of naturally occurring bases in nucleic acid and includes, but is not limited to, modified purines and pyrimidines, minor bases, convertible nucleosides, structural analogs of purines and pyrimidines, labeled, derivatized and modified nucleosides and nucleotides, conjugated nucleosides and nucleotides, sequence modifiers, terminus modifiers, spacer modifiers, and nucleotides with backbone modifications, including, but not limited to, ribose- modified nucleotides, phosphoramidates, phosphorothioates, phosphonamidites, methyl phosphonates, methyl phosphoramidites, methyl phosphonamidites, 5'-β- cyanoethyl phosphoramidites, methylenephosphonates, phosphorodithioates, peptide nucleic acids, achiral and neutral internucleotidic linkages.

In some embodiments are provided a kit that includes a set of probes provided wherein the set of probes specifically hybridize with polynucleotides encoding EGFR, ALK, ROS1, KRAS, BRAF, ERBB2, ERRBB4, MET, RET, FGFR1, FGFR2, FGFR3, DDR2, NRAS, PTEN, MAP2K1, TP53, STK11, CTNNB1, SMAD4, FBXW7, NOTCH 1, KIT/PDGFRA, PIK3CA, AKT1, and HRAS or muteins thereof.

"Hybridize" or "hybridization" refers to the binding between nucleic acids. The conditions for hybridization can be varied according to the sequence homology of the nucleic acids to be bound. Thus, if the sequence homology between the subject nucleic acids is high, stringent conditions are used. If the sequence homology is low, mild conditions are used. When the hybridization conditions are stringent, the hybridization specificity increases, and this increase of the hybridization specificity leads to a decrease in the yield of non-specific hybridization products. However, under mild hybridization conditions, the hybridization specificity decreases, and this decrease in the hybridization specificity leads to an increase in the yield of non-specific hybridization products.

"Stringent conditions" refers to conditions under which a probe will hybridize to its target subsequence, typically in a complex mixture of nucleic acids, but to no other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, stringent conditions are selected to be about 5-10°C lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions may also be achieved with the addition of destabilizing agents such as formamide. For selective or specific hybridization, a positive signal is at least two times background, preferably 10 times background hybridization. Exemplary stringent hybridization conditions can be as following: 50% formamide, 5x SSC, and 1% SDS, incubating at 42°C, or, 5x SSC, 1% SDS, incubating at 65°C, with wash in 0.2x SSC, and 0.1% SDS at 65°C.

Nucleic acids that do not hybridize to each other under stringent conditions are still substantially identical if the polypeptides which they encode are substantially identical. This occurs, for example, when a copy of a nucleic acid is created using the maximum codon degeneracy permitted by the genetic code. In such cases, the nucleic acids typically hybridize under moderately stringent hybridization conditions. Exemplary "moderately stringent hybridization conditions" include a hybridization in a buffer of 40% formamide, 1 M NaCl, 1% SDS at 37°C, and a wash in 1X SSC at 45^{°}C. A positive hybridization is at least twice background. Those of ordinary skill will readily recognize that alternative hybridization and wash conditions can be utilized to provide conditions of similar stringency. Additional guidelines for determining hybridization parameters are provided in numerous reference, *e.g.,* and *Current Protocols in Molecular Biology,* ed.

Hybridization between nucleic acids can occur between a DNA molecule and a DNA molecule, hybridization between a DNA molecule and a RNA molecule, and hybridization between a RNA molecule and a RNA molecule.

"AKT1" or "AKT" refers to human v-akt murine thymoma viral oncogene homolog 1, transcript variant 1; a polynucleotide encoding a RAC-alpha serine/threonine-protein kinase and appears as GenBank accession NM_005163.2, as updated on 30 April 2011.

"ALK" refers to anaplastic lymphoma receptor tyrosine kinase, also known as anaplastic lymphoma kinase, is a gene that encodes a receptor tyrosine kinase, which belongs to the insulin receptor superfamily. This gene has been found to be rearranged, mutated, or amplified in a series of tumors including anaplastic large cell lymphomas, neuroblastoma, and non-small cell lung cancer. The chromosomal rearrangements are the most common genetic alterations in this gene, which result in creation of multiple fusion genes in tumorigenesis, including ALK (chromosome 2)/EML4 (chromosome 2), ALK/RANBP2 (chromosome 2), ALK/ATIC (chromosome 2), ALK/TFG (chromosome 3), ALK/NPM1 (chromosome 5), ALK/SQSTM1 (chromosome 5), ALK/KIF5B (chromosome 10), ALK/CLTC (chromosome 17), ALK/TPM4 (chromosome 19), and ALK/MSN (chromosome X). The translocation of ALK and EML4 results in a fusion protein. One polynucleotide encoding the fusion protein appears as GenBank accession AB274722.1, as updated on 11 January 2008. Soda et al. "Identification of the transforming EML4-ALK fusion gene in non-small-cell lung cancer" (2007) Nature 448(7153):561-566. "EML" refers to "echinoderm microtubule associated protein like 4."

"BRAF" refers to the proto-oncogene B-Raf and v-Raf, also referred to as serine/threonine-protein kinase B-Raf; a polynucleotide encoding a serine/threonine protein kinase and appears as GenBank accession NM_004333.4, as updated on 24 April 2011. Variants of BRAF include polynucleotides encoding amino acid substitutions at amino acid positions 594 and 600. By "amino acid substitution" or " amino acid substitutions" is meant the replacement of an amino acid at a particular position in a parent polypeptide sequence with another amino acid. For example, the substitution D594H refers to a variant polypeptide, in which the aspartic acid at position 594 is replaced with histidine. Other variant polypeptides of BRAF include D594N and V600E.

"EGFR" or "Epidermal growth factor receptor" or "EGFR" refers to a tyrosine kinase cell surface receptor and is encoded by one of four alternative transcripts appearing as GenBank accession NM_005228.3, NM201282.1, NM201283.1 and NM201284.1. Variants of EGFR include a deletion in exon 19, an insertion in exon 20, and amino acid substitutions T790M and L858R. Complex mutation patterns of epidermal growth factor receptor gene are associated with variable responses to gefitinib treatment in patients with non-small cell lung cancer (HSIEH M H et al, LUNG CANCER, vol. 53, pages 311 - 322.

"ERBB2" also referred to as v-erb-b2 erythroblastic leukemia viral oncogene homolog 2, is a member of the EGFR/ErbB family and appears as GenBank accession NM_004448.2, as updated on 1 May 2011. Variants of ERBB2 include an insertion in Exon 20.

"FGFR1" or "fibroblast growth factor receptor 1" is also referred to as fms-related tyrosine kinase-2 and CD331. The nine alternative transcripts encoding FGFR1 protein appear as GenBank accession NM_023110.2, NM_001174063.1, NM_001174064.1, NM_001174065.1, NM_001174066.1, NM 001174067.1, NM_015850.3, NM_023105.2 and NM_023106.2 all as updated as on 30 April 2011.

"HRAS" or "Harvey rat sarcoma viral oncogene homolog" is encoded by a polynucleotide appearing as GenBank accession NM_005343.2, as updated 17 April 2011. Variants of HRAS include the amino acid substitutions Q61L and Q61R.

"KRAS" or "Kirsten rat sarcoma viral oncogene homolog" is encoded by two alternative transcripts appearing as GenBank accession NM_004985.3 and NM_033360.2. Variants of KRAS include the amino acid substitutions G12A/C/D/F/R/V.

"MET" or "MNNG HOS transforming gene" encodes a protein referred to as hepatocyte growth factor receptor and is encoded by a polynucleotide appearing as GenBank accession NM_000245.2 and NM_001127500.1.

"PIK3CA" or "phosphatidylinositol-4,5-bisphosphate 3-kinase, catalytic subunit alpha" is encoded by a polynucleotide appearing as NM_006218.2, as updated on 1 May 2011. Variants of PIK3CA include the amino acid substitutions E545A/G/K and H1047L/R.

"RET" or "rearranged during transfection" encodes a receptor tyrosine kinase. The chromosomal rearrangements are the most common genetic alterations in this gene, which result in creation of multiple fusion genes in tumorigenesis, including kinesin family member 5B ("KIF5B")/RET, coiled-coil domain containing 6 ("CCDC6")/RET and nuclear receptor coactivator 4 ("NCOA4")/RET. A representative of the polynucleotide encoded by RET appears as NM_020630.4.

"ROS1" or "c-Ros receptor tyrosine kinase" belongs to the sevenless subfamily of tyrosine kinase insulin receptor genes. A representative of the polynucleotide encoded by ROS1 appears as NM_002944.2, as last updated on 28-January 2013.

"KIT/PDGFRA" refers to two genes. "KIT," also referred to as "proto-oncogene c-Kit" or "tyrosine-protein kinase Kit" encodes a cytokine receptor. A representative of the polynucleotide encoded by PDGFA appears as NM_000222.2. "PDGFA" is the gene encoding "alpha-type platelet-derived growth factor receptor." A representative of the polynucleotide encoded by PDGFA appears as NM_006206.4.

A "mutein" or "variant" refers to a polynucleotide or polypeptide that differs relative to a wild-type or the most prevalent form in a population of individuals by the exchange, deletion, or insertion of one or more nucleotides or amino acids, respectively. The number of nucleotides or amino acids exchanged, deleted, or inserted can be 1, 2, 3, 4, 5, 6, 7, 8, 9, 10, 11, 12, 13, 14, 15, 16, 17, 18, 19, 20 or more such as 25, 30, 35, 40, 45 or 50. The term mutein can also encompass a translocation, for example the fusion of genes encoding the polypeptides EML4 and ALK. In some embodiments there is provided a kit encompassing a set of probes provided wherein the set of probes specifically hybridize with polynucleotides encoding AKT1, ALK, BRAF, ERBB2, EGFR, FGFR1, HRAS, KIT, KRAS, MET, PIK3CA, RET and ROS or muteins thereof, wherein the set of probes distinguish between the muteins and the muteins include one or more of the polynucleotides encoding AKT1 (E17K), BRAF (L597R, D594H/N, V600E), EGFR (L858R, G719X, T790M), HRAS (Q61L/K/R, G12C/D), KRAS G12A/C/D/F/R/V) and PIK3CA (E545A/G/K, H1047L/R).

"Copy number" or "copy number variation" refers to alterations of the DNA of a genome that result in a cell having an abnormal number of copies of one or more sections of DNA. Copy number variations correspond to relatively large regions of the genome that have been deleted (copy number loss) or duplicated (copy number gain) on certain chromosomes.

"Single nucleotide polymorphism" or "SNP" refers to a DNA sequence variation that occurs when a single nucleotide (A, T, G, or C) in the genome differs between members of a biological species or paired chromosomes in a human.

In other embodiments, the two or more probes are primer pairs.

A "primer" or "primer sequence" refers to an oligonucleotide that hybridizes to a target nucleic acid sequence (for example, a DNA template to be amplified) to prime a nucleic acid synthesis reaction. The primer may be a DNA oligonucleotide, a RNA oligonucleotide, or a chimeric sequence. The primer may contain natural, synthetic, or modified nucleotides. Both the upper and lower limits of the length of the primer are empirically determined. The lower limit on primer length is the minimum length that is required to form a stable duplex upon hybridization with the target nucleic acid under nucleic acid amplification reaction conditions. Very short primers (usually less than 3-4 nucleotides long) do not form thermodynamically stable duplexes with target nucleic acid under such hybridization conditions. The upper limit is often determined by the possibility of having a duplex formation in a region other than the pre-determined nucleic acid sequence in the target nucleic acid. Generally, suitable primer lengths are in the range of about 10 to about 40 nucleotides long. In certain embodiments, for example, a primer can be 10-40, 15-30, or 10-20 nucleotides long. A primer is capable of acting as a point of initiation of synthesis on a polynucleotide sequence when placed under appropriate conditions.

The primer will be completely or substantially complementary to a region of the target polynucleotide sequence to be copied. Therefore, under conditions conducive to hybridization, the primer will anneal to the complementary region of the target sequence. Upon addition of suitable reactants, including, but not limited to, a polymerase, nucleotide triphosphates, etc., the primer is extended by the polymerizing agent to form a copy of the target sequence. The primer may be single-stranded or alternatively may be partially double-stranded.

In some embodiments there is provided a kit encompassing at least 4 primer pairs and 4 detectably labeled probes, wherein the at least 4 primer pairs and the at least 4 detectably labeled probes are not any one of the four primer pairs. In these non-limiting embodiments, the 4 primer pairs and 4 detectably labeled probes form 4 amplification detection assays.

"Detection," "detectable" and grammatical equivalents thereof refers to ways of determining the presence and/or quantity and/or identity of a target nucleic acid sequence. In some embodiments, detection occurs amplifying the target nucleic acid sequence. In other embodiments, sequencing of the target nucleic acid can be characterized as "detecting" the target nucleic acid. A label attached to the probe can include any of a variety of different labels known in the art that can be detected by, for example, chemical or physical means. Labels that can be attached to probes may include, for example, fluorescent and luminescence materials.

"Amplifying," "amplification," and grammatical equivalents thereof refers to any method by which at least a part of a target nucleic acid sequence is reproduced in a template-dependent manner, including without limitation, a broad range of techniques for amplifying nucleic acid sequences, either linearly or exponentially. Exemplary means for performing an amplifying step include ligase chain reaction (LCR), ligase detection reaction (LDR), ligation followed by Q-replicase amplification, PCR, primer extension, strand displacement amplification (SDA), hyperbranched strand displacement amplification, multiple displacement amplification (MDA), nucleic acid strand-based amplification (NASBA), two-step multiplexed amplifications, rolling circle amplification (RCA), recombinase-polymerase amplification (RPA)(TwistDx, Cambridg, UK), and self-sustained sequence replication (3SR), including multiplex versions or combinations thereof, for example but not limited to, OLA/PCR, PCR/OLA, LDR/PCR, PCR/PCR/LDR, PCR/LDR, LCR/PCR, PCR/LCR (also known as combined chain reaction-CCR), and the like. Descriptions of such techniques can be found in, among other places, Sambrook et al. Molecular Cloning, 3rd Edition; Ausbel et al.; PCR Primer: A Laboratory Manual, Diffenbach, Ed., Cold Spring Harbor Press (1995); The Electronic Protocol Book, Chang Bioscience (2002), Msuih et al., J. Clin. Micro. 34:501-07 (1996); The Nucleic Acid Protocols Handbook, R. Rapley, ed., Humana Press, Totowa, N.J. (2002).

Analysis of nucleic acid markers can be performed using techniques known in the art including, without limitation, sequence analysis, and electrophoretic analysis. Non-limiting examples of sequence analysis include Maxam-Gilbert sequencing, Sanger sequencing, capillary array DNA sequencing, thermal cycle sequencing (Sears et al., Biotechniques, 13:626-633 (1992)), solid-phase sequencing (Zimmerman et al., Methods Mol. Cell Biol., 3:39-42 (1992)), sequencing with mass spectrometry such as matrix-assisted laser desorption/ionization time-of-flight mass spectrometry (MALDI-TOF/MS; Fu et al., Nat. Biotechnol., 16:381-384 (1998)), and sequencing by hybridization. Chee et al., Science, 274:610-614 (1996); Drmanac et al., Science, 260:1649-1652 (1993); Drmanac et al., Nat. Biotechnol., 16:54-58 (1998). Non-limiting examples of electrophoretic analysis include slab gel electrophoresis such as agarose or polyacrylamide gel electrophoresis, capillary electrophoresis, and denaturing gradient gel electrophoresis. Additionally, next generation sequencing methods can be performed using commercially available kits and instruments from companies such as the Life Technologies/Ion Torrent PGM or Proton, the Illumina HiSEQ or MiSEQ, and the Roche/454 next generation sequencing system.

In some embodiments, the amount of probe that gives a fluorescent signal in response to an excited light typically relates to the amount of nucleic acid produced in the amplification reaction. Thus, in some embodiments, the amount of fluorescent signal is related to the amount of product created in the amplification reaction. In such embodiments, one can therefore measure the amount of amplification product by measuring the intensity of the fluorescent signal from the fluorescent indicator.

"Detectably labeled probe" refers to a molecule used in an amplification reaction, typically for quantitative or real-time PCR analysis, as well as end-point analysis. Such detector probes can be used to monitor the amplification of the target nucleic acid sequence. In some embodiments, detector probes present in an amplification reaction are suitable for monitoring the amount of amplicon(s) produced as a function of time. Such detector probes include, but are not limited to, the 5'-exonuclease assay (TAQMAN^{®} probes described herein (see also U.S. Pat. No. 5,538,848) various stem-loop molecular beacons (see for example, U.S. Pat. Nos. 6,103,476 and 5,925,517 and Tyagi and Kramer, 1996, Nature Biotechnology 14:303-308), stemless or linear beacons (see, e.g., WO 99/21881), PNA Molecular Beacons^{™} (see, e.g., U.S. Pat. Nos. 6,355,421 and 6,593,091), linear PNA beacons (see, for example, Kubista et al., 2001, SPIE 4264:53-58), non-FRET probes (see, for example, U.S. Pat. No. 6,150,097), Sunrise^{®}/Amplifluor^{™} probes (U.S. Pat. No. 6,548,250), stem-loop and duplex Scorpion probes (Solinas et al., 2001, Nucleic Acids Research 29:E96 and U.S. Pat. No. 6,589,743), bulge loop probes (U.S. Pat. No. 6,590,091), pseudo knot probes (U.S. Pat. No. 6,589,250), cyclicons (U.S. Pat. No. 6,383,752), MGB Eclipse^{™} probe (Epoch Biosciences), hairpin probes (U.S. Pat. No. 6,596,490), peptide nucleic acid (PNA) light-up probes, self-assembled nanoparticle probes, and ferrocene-modified probes described, for example, in U.S. Pat. No. 6,485,901; Mhlanga et al., 2001, Methods 25:463-471; Whitcombe et al., 1999, Nature Biotechnology. 17:804-807; Isacsson et al., 2000, Molecular Cell Probes. 14:321-328; Svanvik et al., 2000, Anal Biochem. 281:26-35; Wolffs et al., 2001, Biotechniques 766:769-771; Tsourkas et al., 2002, Nucleic Acids Research. 30:4208-4215; Riccelli et al., 2002, Nucleic Acids Research 30:4088-4093; Zhang et al., 2002 Shanghai. 34:329-332; Maxwell et al., 2002, J. Am. Chem. Soc. 124:9606-9612; Broude et al., 2002, Trends Biotechnol. 20:249-56; Huang et al., 2002, Chem. Res. Toxicol. 15:118-126; and Yu et al., 2001, J. Am. Chem. Soc 14:11155-11161.

Detector probes can also include quenchers, including without limitation black hole quenchers (Biosearch), Iowa Black (IDT), QSY quencher (Molecular Probes), and Dabsyl and Dabcel sulfonate/carboxylate Quenchers (Epoch).

Detector probes can also include two probes, wherein for example a fluor is on one probe, and a quencher is on the other probe, wherein hybridization of the two probes together on a target quenches the signal, or wherein hybridization on the target alters the signal signature via a change in fluorescence. Detector probes can also comprise sulfonate derivatives of fluorescenin dyes with SO₃ instead of the carboxylate group, phosphoramidite forms of fluorescein, phosphoramidite forms of CY 5 (commercially available for example from Amersham). In some embodiments, interchelating labels are used such as ethidium bromide, SYBR^{®} Green I (Molecular Probes), and PicoGreen^{®} (Molecular Probes), thereby allowing visualization in real-time, or end point, of an amplification product in the absence of a detector probe. In some embodiments, real-time visualization can comprise both an intercalating detector probe and a sequence-based detector probe can be employed. In some embodiments, the detector probe is at least partially quenched when not hybridized to a complementary sequence in the amplification reaction, and is at least partially unquenched when hybridized to a complementary sequence in the amplification reaction. In some embodiments, the detector probes of the present teachings have a Tm of 63-69° C., though it will be appreciated that guided by the present teachings routine experimentation can result in detector probes with other Tms. In some embodiments, probes can further comprise various modifications such as a minor groove binder (see for example U.S. Pat. No. 6,486,308) to further provide desirable thermodynamic characteristics.

In some embodiments, detection can occur through any of a variety of mobility dependent analytical techniques based on differential rates of migration between different analyte species. Exemplary mobility-dependent analysis techniques include electrophoresis, chromatography, mass spectroscopy, sedimentation, for example, gradient centrifugation, field-flow fractionation, multi-stage extraction techniques, and the like. In some embodiments, mobility probes can be hybridized to amplification products, and the identity of the target nucleic acid sequence determined via a mobility dependent analysis technique of the eluted mobility probes, as described for example in Published P.C.T. Application WO04/46344 to Rosenblum et al., and WO01/92579 to Wenz et al. In some embodiments, detection can be achieved by various microarrays and related software such as the Applied Biosystems Array System with the Applied Biosystems 1700 Chemiluminescent Microarray Analyzer and other commercially available array systems available from Affymetrix, Agilent, IIlumina, and Amersham Biosciences, among others (see also Gerry et al., J. Mol. Biol. 292:251-62, 1999; De Bellis et al., Minerva Biotec 14:247-52, 2002; and Stears et al., Nat. Med. 9: 14045, including supplements, 2003). It will also be appreciated that detection can comprise reporter groups that are incorporated into the reaction products, either as part of labeled primers or due to the incorporation of labeled dNTPs during an amplification, or attached to reaction products, for example but not limited to, via hybridization tag complements comprising reporter groups or via linker arms that are integral or attached to reaction products. Detection of unlabeled reaction products, for example using mass spectrometry, is also within the scope of the current teachings.

The kits of the present invention may also comprise instructions for performing one or more methods described herein and/or a description of one or more compositions or reagents described herein. Instructions and/or descriptions may be in printed form and may be included in a kit insert. A kit also may include a written description of an Internet location that provides such instructions or descriptions.

In some embodiments is provided a composition comprising a set of probes and a sample, wherein the set of probes specifically recognize the genes AKT1, ALK, BRAF, ERBB2, EGFR, FGFR1, HRAS, KIT, KRAS, MET, PIK3CA, RET and ROS, and wherein the set of probes can recognize and distinguish one or more allelic variants of the genes AKT1, ALK, BRAF, ERBB2, EGFR, HRAS, KRAS, MET, PIK3CA, RET and ROS.

Any combination of the disclosed genes and variants can be included in the kits and compositions. For instance, the genes and variants can be selected from a combination of actionability index (AI) categories and variant prevalence, as described in more detail herein. In this regard, in varying embodiments of the disclosed compositions and kits, the gene variants can be selected from an actionability index and percentage prevalence selected from AI1 + Prevalence > 1%, AI2 + Prevalence > 1%, AI3 + Prevalence > 1%, AI1 + Prevalence 0.1%-1%, AI2 + Prevalence 0.1%-1%, AI3 + Prevalence 0.1%-1%, and combinations thereof.

In certain embodiments, methods to determine an actionable treatment recommendation for a subject diagnosed with lung cancer are provided. Other embodiments include methods to determine the likelihood of a response to a treatment in a subject afflicted with lung cancer and methods for treating a patient with lung cancer

In one embodiment of the methods, the lung cancer sub type is lung adenocarcinoma. In certain embodiments, the lung cancer subtype is squamous cell lung carcinoma.

The methods comprise the steps of obtaining a sample from a patient, detecting at least one variant in a gene of interest, and determining an AI or treatment for the patient based on the gene variant detected.

The patient sample can be any bodily tissue or fluid that includes nucleic acids from the lung cancer in the subject. In certain embodiments, the sample will be a blood sample comprising circulating tumor cells or cell free DNA. In other embodiments, the sample can be a tissue, such as a lung tissue. The lung tissue can be from a tumor tissue and may be fresh frozen or formalin-fixed, paraffin-embedded (FFPE). In certain embodiments, a lung tumor FFPE sample is obtained.

Five categories of AIs are provided herein. AI1 represents a category for which there is clinical consensus on a treatment recommendation based on the genetic variant status. The data source for AI1 is the National Comprehensive Cancer Network Practice Guidelines in Oncology (NCCN Guidelines) for non-small cell lung cancer (NSCLC) (Version 2.2013). This index is assigned if the NCCN Guidelines specifically recommends a therapy based on gene and variant type.

AI2 represents a category for which there exists a clinical trial or clinical case report evidence for treatment response in patients based on genetic variant status.

AI3 is a category in which one or more clinical trials are in progress in which genetic variant status is used as an enrollment criteria, that is particular genes and variants are required as part of the clinical trial enrollment criteria (for inclusion or exclusion).

AI4 is a category for which there is preclinical evidence for treatment response based on genetic variant status. The index contains genes and events reported to show an association with preclinical treatment response.

AI5 is a category in which a targeted therapy is available for the gene that is aberrant. This index is based on the requirement for a gene and associated variant in order for the therapy to be considered actionable.

Lung cancer variants are prioritized based on prevalence of greater than 0.1%. Prevalence was determined from reference datasets of lung cancer by counting all of the clinical specimens tested that were found to contain one of the gene variants described in this invention, and expressing that value as a percentage relative to all of the clinical specimens tested. For example, the prevalence of 0.1% to 1% and prevalence of greater than 1% of gene variants in adenocarcinoma and squamous cell carcinoma are shown herein (see Tables 1 and 3), however any subset of the percentage range, or below or above the percentage range, can be used to represent additional genetic variants associated with an AI. The variants include but are not limited to SNPs, insertions, deletions, translocations, and copy number variation (e.g., gain or loss).

**TABLE 1**

| **Lung Adenocarcinoma** | | |
|---|---|---|
| **Actionability Index** | **Prevalence > 1%** | **Prevalence 0.1%-1%** |
| **AI1** | EGFR (L858R, Exon 19 del, T790M, exon 20 ins) | EGFR (G719X) |
| | ALK translocation/fusion (EML4-ALK) | KRAS (G12S, G13C, G13D, G12R, G12F) |
| | ROS1 (EZR-ROS1, SLC34A2-ROS1, CD74-ROS1, SDC4-ROS1) | |
| | KRAS (G12C, G12V, G12D, G12A) | |
| **AI2** | BRAF (V600E) | PIK3CA (E545K, E545G, E545A, H1047R, H1047L) |
| | ERBB2 (Exon 20 ins) | |
| | MET CN gain | |
| **AI3** | RET translocation | AKT1 (E17K) |
| | EGFR CN gain | BRAF (L597R, D594H/N) |
| | ERBB2 CN gain | HRAS (Q61L/K/R, G12C/D, G13C/S/R/V) |
| | FGFR1 CN gain | |
| | KIT/PDGFRA amplification | PIK3CA (E542K) |

As shown in Table 1, the genetic variants disclosed herein and associated AIs, provide treatment options for over 50% of all primary lung adenocarcinomas. This type of comprehensive screening of lung cancer gene variants and treatment recommendations for over 50% of the lung adenocarcinoma patient population has been heretofore unavailable. The disclosure provides a method of gene variant determination that can be performed in a single assay or panel, which allows greater variant detection using the precious little sample obtained from a typical lung tumor biopsy or surgical resection. It should be understood that the genes and variants identified herein are non-limiting examples and genes and variants can be readily added or removed identify valuable patient variants and treatment options. Further, any combination of AI and prevalence can be detected in the methods provided herein. For example, in one embodiment, all AI categories and variants can be determined. In another embodiment, AI1 + Prevalence > 1%, AI2 + Prevalence > 1%, AI3 + Prevalence > 1%, AI1 + Prevalence 0.1%-1%, AI2 + Prevalence 0.1%-1%, AI3 + Prevalence 0.1%-1% and any combination thereof can be determined in the methods disclosed herein.

The disclosure provides treatment options for numerous subsets of the adenocarcinoma and squamous cell carcinoma population depending on the combination of the percentage prevalence of the markers chosen and the AI categories. As shown in Tables 4-10, by choosing different combinations of AI+ % prevalence, treatment options can be provided for varying percentages of the afflicted population (See Example II).

The disclosure further provides actionable treatment recommendations for a subject with lung cancer based on the subject's tumor's genetic variant status. The actionable treatment recommendations can include pharmaceutical therapeutics, surgery, photodynamic therapy (PTD), laser therapy, radiation, dietary guidance, clinical trial suggestions, etc. The actionable treatment recommendations provided herein (see Tables 2 and 3) are exemplary. Additional actionable treatment recommendations can be added or removed as additional data, publications, clinical reports, treatments, and clinical trials become available. Further, additional information can be used to provide actionable treatment recommendations, including, but not limited to, age, gender, family history, lifestyle, dietary, as well as other relevant factors.

In certain embodiments, the method comprises performing the actionable treatment recommendation. Accordingly, performing the actionable treatment recommendation can include, without limitation, administering a therapeutically effective amount of one or more therapeutic agents (chemotherapeutics, targeted therapeutics, antiangiogenics, etc), implementing a dietary regimen, administering radiation and/or enrolling in one or more clinical trials.

Examples of chemotherapeutics to treat lung cancer include: Cisplatin or carboplatin, gemcitabine, paclitaxel, docetaxel, etoposide, and/or vinorelbine. Targeted therapeutics (drugs that specifically block the growth and spread of cancer) include monoclonal antibodies such as, but not limited to, bevacizumab (AVASTIN^{™}) and cetuximab; and tyrosine kinase inhibitors (TKIs) such as, but not limited to, gefitinib (IRESSA^{™}.), erlotinib (TARCEVA^{™}) crizotinib and/or vemurafenib.

Additional chemotherapeutics to treat lung cancer include, but are not limited to, TKIs: vandetanib, tofacitinib, sunitinib malate, sorafenib, ruxolitinib, regorafenib, ponatinib, pazopanib, nilotinib, leflunomide, lapatinib ditosylate, imatinib mesilate, gefitinib, erlotinib, dasatinib, crizotinib, cabozantinib, bosutinib, axitinib, radotinib, tivozanib, masitinib, afatinib, XL-647, trebananib, tivantinib, SAR-302503, rilotumumab, ramucirumab, plitidepsin, pacritinib, orantinib, nintedanib, neratinib, nelipepimut-S, motesanib diphosphate, midostaurin, linifanib, lenvatinib, ibrutinib, fostamatinib disodium, elpamotide, dovitinib lactate, dacomitinib, cediranib, baricitinib, apatinib, Angiozyme, X-82, WBI-1001, VX-509, varlitinib, TSR-011, tovetumab, telatinib, RG-7853, RAF-265, R-343, R-333, quizartinib dihydrochloride, PR-610, poziotinib, PLX-3397, PF-04554878, Pablocan, NS-018, momelotinib, MK-1775, milciclib maleate, MGCD-265, linsitinib, LDK-378, KX2-391, KD-020, JNJ-40346527, JI-101, INCB-028060, icrucumab, golvatinib, GLPG-0634, gandotinib, foretinib, famitinib, ENMD-2076, danusertib, CT-327, crenolanib, BMS-911543, BMS-777607, BMS-754807, BMS-690514, bafetinib, AZD-8931, AZD-4547, AVX-901, AVL-301, AT-9283, ASP-015K, AP-26113, AL-39324, AKN-028, AE-37, AC-480, 2586184, X-396, volitinib, VM-206, U3-1565, theliatinib, TAS-115, sulfatinib, SB-1317, SAR-125844, S-49076, rebastinib, R84 antibody, Peregrine, R-548, R-348, PRT-062607, P-2745, ONO-4059, NRC-AN-019, LY-2801653, KB-004, JTE-052, JTE-051, IMC-3C5, ilorasertib, IDN-6439, HM-71224, HM-61713, henatinib, GSK-2256098, epitinib, EMD-1214063, E-3810, EOS, CUDC-101, CT-1578, cipatinib, CDX-301, CC-292, BI-853520, BGJ-398, ASP-3026, ARRY-614, ARRY-382, AMG-780, AMG-337, AMG-208, AL-3818, AC-430, 4SC-203, Z-650, X-379, WEE-1/CSN5, Tekmira Pharmaceuticals, Wee-1 kinase inhibitors, Tekmira Pharmaceuticals, VS-4718, VEGFR2 inhibitor, AB Science, VEGF/rGel, Clayton Biotechnologies, VEGF inhibitors, Interprotein, UR-67767, tyrosine kinase inhibitors, Bristol-Myers Squibb, tyrosine kinase inhibitor, Aurigene Discovery Technologies, tyrosine kinase 2 inhibitors, Sareum, TrkA ZFP TF, TrkA inhibitor, Proximagen, TP-0903, TP-0413, TKI, Allergan, Sym-013, syk kinase inhibitors, Almirall, Syk kinase inhibitors, AbbVie, SYK inhibitor programme, Ziarco, SUN-K706, SN-34003, SN-29966, SIM-930, SIM-6802, SIM-010603, SGI-7079, SEL-24-1, SCIB-2, SAR-397769, RET kinase inhibitor, Bionomics, R-256, PRT-062070, PRT-060318, PRS-110, PLX-7486, ORS-1006, ORB-0006, ORB-0004, ORB-0003, ONO-WG-307, ON-044580, NVP-BSK805, NNI-351, NMS-P948, NMS-E628, NMS-173, MT-062, MRLB-11055, MG-516, KX2-361, KIT816 inhibitor, AB Science, janus kinase inhibitor, Celgene, JAK3-inhibitor, Principia BioPharma, Jak1 inhibitor, Genentech, JAK inhibitors, Almirall, INCB-16562, hR1-derivatives, Immunomedics, HMPL-281, HM-018, GTX-186, GSK-143, GS-9973, GFB-204, gastrointestinal stromal tumour therapy, Clovis Oncology, G-801, FX-007, FLT4 kinase inhibitors, Sareum, FLT3/cKit inhibitor, Johnson & Johnson, flt-4 kinase inhibitors, Sareum, flt-3 kinase inhibitors, Sareum, FAK inhibitors, Takeda, FAK inhibitor, Verastem, EN-3351, DNX-04040, DNX-02079, DLX-521, deuterated tofacitinib, Auspex Pharmaceuticals, DCC-2721, DCC-2701, DCC-2618, CTX-0294945, CTx-0294886, CT-340, CT-053, CST-102, CS-510, CPL-407-22, CH-5451098, CG-206481, CG-026828, CFAK-C4, CCT-137690, CC-509, c-Met kinase inhibitors, Rhizen, BXL-1H5, BTK inhibitors, Mannkind, Btk inhibitor, Pharmacyclics-3, Btk inhibitor, Aurigene Discovery Technologies, BGB-324, BGB-001, Bcr-Abl/Lyn inhibitor, AB Science, aurora kinase + FLT3 kinase inhibitor, Sareum, aurora kinase + ALK inhibitor, Sareum, aurora kinase + ALK inhibitor, AstraZeneca, ASP-502D, ASP-08112, ARYY-111, AR-523, anticancer, leukemia, Critical, anticancer therapy, Agios-1, ANG-3070, ALK inhibitors, AstraZeneca, Alk inhibitor, Cephalon-3, ALK inhibitor, Aurigene Discovery Technologies, AL-2846, TrkB modulators, Hermo Pharma, TLK-60596, TLK-60404, CYC-116, ARRY-380, ZD-4190, Yissum Project No. B-1146, XL-999, XL-820, XL-228, VX-667, vatalanib, tyrosine protein kinase inhibs, tyrosine kinase inhibs, Yissum, tyrosine kinase inhibs, CSL, tyrosine kinase antags, ICRT, tozasertib lactate, TG-100-13, tandutinib, TAK-593, TAK-285, Symadex, Syk kinase inhibitor, SGX, SU-5271, SU-14813, SGX-523, semaxanib, saracatinib, RP 53801, RG-14620, RG-13291, RG-13022, R-112, PLX-647, PKI-166, Pharmaprojects No. 6085, Pharmaprojects No. 4960, Pharmaprojects No. 4923, Pharmaprojects No. 4863, Pharmaprojects No. 3624, Pharmaprojects No. 3292, Pharmaprojects No. 3054, PF-562271, PF-4217903, NVP-TAE226, mubritinib, MEDI-547, lestaurtinib, KW-2449, KSB-102, KRN-633, IMC-EB10, GW-282974, Flt3-kinase inhibitor,Lilly, FCE-26806, EphA2 vaccine, MedImmune, EMD-55900, EMD-1204831, desmal, degrasyns, CNF-201 series, CGP-57148, CEP-7055, CEP-5214, CEP-075, CE-245677, CDP-860, canertinib dihydrochloride, cancer vaccine, Ajinomoto, bscEphA2xCD3, MedImmune, brivanib alaninate, breast cancer therapy,Galapago, BIBX-1382, AZD-9935, AZD-6918, AZD-4769, AZD-1480, AVE-0950, Argos, AP-23464, AP-23451, AP-22408, anti-HER2/neu mimetic,Cyclacel, anti-HER-2/neu antisense, Tekm, amuvatinib, AG-490, AG-18, AG-13958, AEG-41174, ZM-254530, ZK-CDK, ZK-261991, ZD-1838, ZAP70 kinase inhibitors, Kinex, ZAP-70 inhibitors, Cellzome, ZAP inhibitors, Ariad, ZAP 70 inhibitors, Galapagos, ZAP 70 inhibitors, Celgene, YW327.6S2, YM-359445, YM-231146, YM-193306, XV-615, XL-019, XC-441, XB-387, Wee-1 kinase inhibitor, Banyu, VX-322, VRT-124894, VEGFR2 kinase inhibitors, Takeda, VEGFR/EGFR inhib, Amphora, VEGFR-2 kinase inhibitors, Hanmi, VEGFR-2 antagonist, Affymax, VEGF/rGel, Targa, VEGF-TK inhibitors,AstraZeneca, VEGF-R inhibitors, Novartis, VEGF modulators, 3-D, VEGF inhibitors, Onconova, VEGF inhibitor, Chugai, V-930, U3-1800, U3-1784, tyrphostins, Yissum, tyrosine kinase inhibs,Novar-2, tyrosine kinase inhibs, Sanofi, tyrosine kinase inhib,Abbott-2, tyrosine kinase inhib, Pfizer, tyrosine kinase inhib, IQB, tyrosine kinase inhib, Abbott, tyrosine kinase inhi,Abbott-3, trkB inhibitors, Amphora, TrkA inhibitors, Telik, TrkA blocker, Pfizer, TLN-232, TKM-0150, Tie-2 kinase inhibitors, GSK, TIE-2 inhibitors, Ontogen, Tie-2 inhibitors, AstraZeneca, Tie-2 inhibitors, Amgen-3, Tie-2 inhibitors, Amgen-2, Tie-2 inhibitors, Amgen, Tie-2 antagonists, Semaia, Tie-1R IFP, Receptor BioLogix, TG-101-223, TG-101-209, TG-100948, TG-100435, TG-100-96, TG-100-801, TG-100-598, TAE-684, T3-106, T-cell kinase inhibitors, Cell, syk kinase inhibitor, Bayer, Syk inhibitors, CrystalGenomics, Syk inhibitors, Astellas-2, Syk inhibitors, Amphora, SU-11657, SU-0879, SSR-106462, SRN-004, Src/Abl inhibitors, Ariad, Src non-RTK antagonists, SUGEN, Src inhibitors, Amphora, spiroindolines, Pfizer, SP-5.2, sorafenib bead, Biocompatibles, SMi-11958, SH2 inhibitors, NIH, SH-268, SGX-393, SGX-126, SGI-1252, SC-102380, SC-101080, SB-238039, SAR-131675, RWJ-64777, RWJ-540973, RPR-127963E, RP-1776, Ro-4383596, RNAi cancer therapy, Benitec Biopharma, RM-6427, rheumatoid arthritis therapy, SRI International, RET inhibitors, Cell T, RB-200h, R545, Rigel, R3Mab, R-723, R-507, R-499, R-1530, QPM5-986, QPAB-1556, PX-104.1, PS-608504, prostate cancer ther, Sequenom, prodigiosin, PRI-105, PP1, Scripps, PN-355, phenylalanine derivatives, NIH, Pharmaprojects No. 6492, Pharmaprojects No. 6291, Pharmaprojects No. 6271, Pharmaprojects No. 6267, Pharmaprojects No. 6140, Pharmaprojects No. 6138, Pharmaprojects No. 6083, Pharmaprojects No. 6059, Pharmaprojects No. 6013, Pharmaprojects No. 5330, Pharmaprojects No. 4855, Pharmaprojects No. 4597, Pharmaprojects No. 4368, Pharmaprojects No. 4164, Pharmaprojects No. 3985, Pharmaprojects No. 3495, Pharmaprojects No. 3135, PF-371989, PF-337210, PF-00120130, pelitinib, pegdinetanib, PDGFR-alpha inhibitors, Deciphera, PDGFR inhibitor, Pulmokine, PDGFR inhibitor, Array, PDGF receptor inhibitor,Kyowa, PDGF receptor inhibitor, Array, PDGF kinase inhibitors, Kinex, PD-180970, PD-173956, PD-171026, PD-169540, PD-166285, PD-154233, PD-153035, PD-0166285, PCI-31523, pazopanib hydrochloride (ophthalmic), pan-HER kinase inhib, Ambit-2, pan-HER inhibitor, SUGEN, pan-HER ACL, p561ck inhibitors, BI, OSI-930, OSI-817, OSI-632, OSI-296, ONC-101, ON-88210, ON-045270, NVP-AEW541, NVP-AAK980-NX, NV-50, NSC-242557, NNC-47-0011, NMS-P626, NL-0031, nilotinib, once-daily, nicotinamide derivatives, Bristol-Myers Squibb, neuT MAb, Philadelphia, multikinase inhibitors, Amphor, mullerian inhibiting subst, Ma, MS therapy, Critical Outcome Technologies, MP-371, MLN-608, MK-8033, MK-2461, Met/Ron kinase inhibs, SGX, Met/Gab1 antagonist, Semaia, Met RTK antagonists, SUGEN, Met receptor inhibs, Ontogen, Met kinase inhibitor, BMS, Met inhibitors, Amphora, MEDI-548, MED-A300, ME-103, MC-2002, Lyn kinase inhibitor, CRT, Lyn B inhibitors, Onconova, lymphostin, LP-590, leflunomide, SUGEN, lck/Btk kinase inhibitors, AEgera, lck kinase inhibitors, Kinex, lck kinase inhibitors, Celgene, Lck inhibitors, Green Cross, lck inhibitors, Amphora, lck inhibitors, Amgen, lck inhibitors, Abbott, lavendustin A analogues, NIH, LAT inhibitors, NIH, L-000021649, KX-2-377, KST-638, KRX-211, KRX-123, KRN-383, KM-2550, kit inhibitor, Amphora, kinase inhibitors, SGX-2, kinase inhibitors, SGX-1, kinase inhibitors, MethylGene, kinase inhibitors, Amgen, kinase inhibitor, Cephalon, KIN-4104, Ki-8751, Ki-20227, Ki-11502, KF-250706, KDR kinase inhibs, Celltech, KDR kinase inhibitors, Merck & Co-2, KDR kinase inhibitors, Merck & Co-1, Kdr kinase inhibitors, Amgen, KDR inhibitors, Abbott, KDR inhibitor, LGLS, K252a, JNJ-38877605, JNJ-26483327, JNJ-17029259, JNJ-141, Janex-1, JAK3 inhibitors,Pharmacopeia-2, Jak3 inhibitors, Portola, JAK2 inhibitors, Merck & Co, JAK2 inhibitors, Deciphera, JAK2 inhibitors, Amgen, JAK2 inhibitors, Abbott, JAK2 inhibitor, CV, Cytopia, JAK2 inhibitor, cancer, Cytopia, JAK2 inhibitor, Astex, JAK-3 inhibitors, Cellzome, JAK inhibitors, Genentech, JAK inhibitors, BioCryst, JAK inhibitor, Pulmokine, JAK 1/3 inhibitor, Rigel, ITK inhibitors, GlaxoSmithKline, ISU-101, interleukin-2 inducible T-cell kinase inhibitors, Vertex, INSM-18, inherbins, Enkam, IMC-1C11, imatinib, sublingual, Kedem Pharmaceuticals, IGF-1R inhibitor, Allostera, IGF-1 inhibitors, Ontogen, HMPL-010, HM-95091, HM-60781, HM-30XXX series, Her2/neu & EGFR Ab, Fulcrum, HER2 vaccine, ImmunoFrontier, HER-2 binder, Borean, Her-1/Her-2 dual inhibitor, Hanmi, Her inhibitors, Deciphera, HEM-80322, HDAC multi-target inhibitors, Curis, GW-771806, GW-654652, GSK-1838705A, GNE-A, glioblastoma gene therapy, Biogen Idec, genistein, gene therapy, UCSD, focal adhesion kinase inhibitor, Kinex, FMS kinase inhibitors, Cytopia, FLT-3 MAb, ImClone, Flt-3 inhibitor, Elan, Fit 3/4 anticancer, Sentinel, FAK/JAK2 inhibitors, Cephalon, FAK inhibitors, Ontogen, FAK inhibitors, Novartis, FAK inhibitors, GlaxoSmithKline, FAK inhibitors, Cytopia, EXEL-6309, Etk/BMX kinase inhibitors, SuperGen, erbstatin, erbB-2 PNV, UAB, erbB-2 inhibitors, Cengent, ER-068224, ephrin-B4 sol receptor,VasGene, ephrin-B4 RTK inhib, VasGene, EphA2 receptor tyrosine kinase inhibitor, Pfizer, ENMD-981693, EHT-102, EHT-0101, EGFR/Her-2 kinase inhibitors, Shionogi, EGFR-CA, EGFR kinase inhibitors, Kinex, EGF-genistein, Wayne, EGF-593A, EG-3306, DX-2240, DP-4577, DP-4157, DP-2629, DP-2514, doramapimod, DNX-5000 series, DN-30 Fab, dianilinophthalimide, deuterated erlotinib, CoNCERT, dendritic cell modulators, Antisoma, DD-2, Jak inhibitors, DD-2, dual Jak3/Syk, DCC-2909, DCC-2157, D-69491, CYT-977, CYT-645, CX-4715, curcumin analogues, Onconova, CUDC-107, CT-100, CT-052923, CS-230, CP-724714, CP-673451, CP-564959, CP-292597, CP-127374, Cmpd-1, CL-387785, CKD-712, CHIR-200131, CH-330331, CGP-53716, CGP-52411, CGI-1746, CGEN-B2, CGEN-241, CFAK-Y15, CEP-37440, CEP-33779, CEP-28122, CEP-2563 dihydrochloride, CEP-18050, CEP-17940, celastrol, CDP-791, CB-173, cancer vaccine, bcr-abl, Mologen, cancer therapeutics, Cephalon, CAB-051, c-Src kinase inhibs, AstraZene, c-Met/Her inhibitors, Decipher, c-Met kinase inhibitor, Cephalon, c-Met inhibitors, Roche, c-Met inhibitor, Merck, c-kit inhibitors, Deciphera, c-kit inhibitors, Cell, c-Abl inhibitors, Plexxikon, c-Abl inhibitors, Onconova, BVB-808, Btk inhibitors, Bristol-Myers Squibb, Btk inhibitor, Pharmacyclics-2, BSF-466895, Brk/PTK6 inhibitors, Merck & Co, BreMel/rGel, BPI-703010, BPI-702001, BP-100-2.01, BMX kinase inhibitors, Amphora, BMS-817378, BMS-754807 back-up, BMS-743816, BMS-577098, BLZ-945, BIW-8556, BIO-106, Behcet's disease therapy, Cr, BAY-85-3474, AZM-475271, AZD-0424, AZ-Tak1, AZ-23, Axl kinase inhibitors, SuperGen, Axl inhibitors, Deciphera, Axl inhibitors, CRT, AVL-101, AV-412, aurora/FLT3 kinase inhibs, Im, AST-6, AST-487, ARRY-872, ARRY-768, ARRY-470, ARRY-333786, apricoxib + EGFR-TKI, Tragara, AP-23994, AP-23485, anticancers, CoNCERT, anticancers, Bracco, anticancers, Avila-4, anticancers, Avila-3, anticancers, Avila-2, anticancer ZFPs, ToolGen, anticancer therapy, Ariad, anticancer MAbs, Xencor-2, anticancer MAbs, Kolltan, antiangiogenic ther, Deciphera, anti-Tie-1 MAb, Dyax, anti-PDGF-B MAbs, Mill, anti-inflammatory, Kinex, anti-inflammatory, Avila, anti-inflammatory ther, Vitae, anti-HER2neu scFv, Micromet, anti-HER2/Flt3 ligand, Symbi, anti-HER2 MAb, Abiogen, anti-Flt-1 MAbs, ImClone, anti-fak oligonucleotides, anti-ErbB-2 MAbs, Enzon, anti-EphA4 MAb, MedImmune, anti-EGFRvIII MAbs, Amgen, anti-EGFR MAb, Xencor, anti-EGFR immunotoxin, IVAX, anti-CD20/Flt3 ligand, Symbi, Anti-Cancer Ligands, Enchira, anti-ALK MAb, MedImmune, angiopoietins, Regeneron, AMG-Jak2-01, AMG-458, AMG-191, ALK inhibitors, PharmaDesign, ALK inhibitors, Lilly, ALK inhibitors, Cephalon -2, AI-1008, AHNP, Fulcrum, AGN-211745, AGN-199659, AG-957, AG-1295, AEE-788, and ADL-681. Mechanisms of resistance to BCR-ABL and other kinase inhibitors are already known, e.g. from Lamontanara, et al.,Biochimica et Biophysica Acta (BBA) - Proteins and Proteomics,Vol. 1834, Pages 1449-1459.

ErbB tyrosine kinase inhibitor (ERbB) include but are not limited to; vandetanib, lapatinib ditosylate, gefitinib, erlotinib, afatinib, XL-647, neratinib, nelipepimut-S, dovitinib lactate, dacomitinib, varlitinib, RAF-265, PR-610, poziotinib, KD-020, BMS-690514, AZD-8931, AVX-901, AVL-301, AE-37, AC-480, VM-206, theliatinib, IDN-6439, HM-61713, epitinib, CUDC-101, cipatinib, Z-650, SN-34003, SN-29966, MT-062, CST-102, ARRY-380, XL-999, vatalanib, TAK-285, SU-5271, PKI-166, Pharmaprojects No. 4960, Pharmaprojects No. 3624, mubritinib, KSB-102, GW-282974, EMD-55900, CNF-201 series, canertinib dihydrochloride, cancer vaccine, Ajinomoto, breast cancer therapy,Galapago, BIBX-1382, AZD-4769, Argos, AP-23464, anti-HER2/neu mimetic,Cyclacel, anti-HER-2/neu antisense, Tekm, AG-18, ZM-254530, ZD-1838, VEGFR/EGFR inhib, Amphora, VEGF-TK inhibitors,AstraZeneca, V-930, RNAi cancer therapy, Benitec Biopharma, RM-6427, RB-200h, PX-104.1, Pharmaprojects No. 6291, Pharmaprojects No. 6271, Pharmaprojects No. 4164, Pharmaprojects No. 3985, Pharmaprojects No. 3495, pelitinib, PD-169540, PD-166285, PD-154233, PD-153035, pan-HER kinase inhib, Ambit-2, pan-HER inhibitor, SUGEN, pan-HER ACL, ON-045270, NSC-242557, NL-0031, mullerian inhibiting subst, Ma, ME-103, kinase inhibitors, Amgen, JNJ-26483327, ISU-101, INSM-18, inherbins, Enkam, HM-60781, HM-30XXX series, Her2/neu & EGFR Ab, Fulcrum, HER2 vaccine, ImmunoFrontier, HER-2 binder, Borean, Her-1/Her-2 dual inhibitor, Hanmi, Her inhibitors, Deciphera, HEM-80322, gene therapy, UCSD, erbB-2 PNV, UAB, erbB-2 inhibitors, Cengent, EHT-102, EGFR/Her-2 kinase inhibitors, Shionogi, EGFR-CA, EGFR kinase inhibitors, Kinex, EGF-593A, dianilinophthalimide, deuterated erlotinib, CoNCERT, D-69491, curcumin analogues, Onconova, CUDC-107, CP-724714, CP-292597, CL-387785, CGEN-B2, CAB-051, c-Met/Her inhibitors, Decipher, BreMel/rGel, BIO-106, AV-412, AST-6, ARRY-333786, apricoxib + EGFR-TKI, Tragara, anticancers, CoNCERT, anticancer MAbs, Xencor-2, anti-HER2neu scFv, Micromet, anti-HER2 MAb, Abiogen, anti-ErbB-2 MAbs, Enzon, anti-EGFRvIII MAbs, Amgen, anti-EGFR MAb, Xencor, anti-EGFR immunotoxin, IVAX, Anti-Cancer Ligands, Enchira, AHNP, Fulcrum, AEE-788, and ADL-681.

MEK1 or MEK2 (MEK) include, but are not limited to: Trametinib, ARRY-438162, WX-554, Selumetinib, Pimasertib, E-6201, BAY-86-9766, TAK-733, PD-0325901, GDC-0623, BI-847325, AS-703988, ARRY-704, Antroquinonol, CI-1040, SMK-17, RO-5068760, PD-98059, and ER-803064.

PIK3CA related treatments include, but are not limited to: perifosine, BKM-120, ZSTK-474, XL-765, XL-147, PX-866, PKI-587, pictilisib, PF-04691502, BYL-719, BEZ-235, BAY-80-6946, PWT-33597, PI3 kinase/mTOR inhibitor, Lilly, INK-1117, GSK-2126458, GDC-0084, GDC-0032, DS-7423, CUDC-907, BAY-1082439, WX-037, SB-2343, PI3/mTOR kinase inhibitors, Amgen, mTOR inhibitor/PI3 kinase inhibitor, Lilly-1, LOR-220, HMPL-518, HM-032, GNE-317, CUDC908, CLR-1401, anticancers, Progenics, anticancer therapy, Sphaera Pharma-1, AMG-511, AEZS-136, AEZS-132, AEZS-131, AEZS-129, pictilisib, companion diagnostic, GDC-0980, companion diagnostic, GDC-0032, companion diagnostic, AZD-8055, VEL-015, SF-2523, SF-2506, SF-1126, PX-2000, PKI-179, PI3K p110alpha inhibitors, Ast, PI3K inhibitors, Semafore-2, PI3K inhibitors, Invitrogen, PI3K inhibitor conjugate,Semaf, PI3K conjugates,Semafore, PI3-irreversible alpha inhibitors, Pathway, PI3-alpha/delta inhibitors, Pathway Therapeutics, PI3-alpha inhibitors, Pathway Therapeutics, PI3 kinase inhibitors, Wyeth, PI3 kinase inhibitors, Telik, PI3 kinase alpha selective inhibitors, Xcovery, PI-620, PF-4989216, PF-04979064, PF-00271897, PDK1 inhibitors, GlaxoSmithKline, ONC-201, KN-309, isoform-selective PI3a/β kinase inhibitors, Sanofi, inositol kinase inhibs, ICRT, HM-5016699, hepatocellular carcinoma therapy, Sonitu, GSK-1059615, glioblastoma therapy, Hoffinann-La Roche, EZN-4150, CU-906, CU-903, CNX-1351, antithrombotic, Cerylid, 4-methylpteridinones.

Tretaments directed to ALK include, but are not limited to: crizotinib, companion diagnostic, AbbVie, crizotinib, TSR-011, RG-7853, LDK-378, AP-26113, X-396, ASP-3026, NMS-E628, DLX-521, aurora kinase + ALK inhibitor, Sareum, aurora kinase + ALK inhibitor, AstraZeneca, ALK inhibitors, AstraZeneca, Alk inhibitor, Cephalon-3, ALK inhibitor, Aurigene Discovery Technologies, LDK-378, companion diagnostic, crizotinib, companion diagnostic, Roche, TAE-684, kinase inhibitor, Cephalon, GSK-1838705A, EXEL-6309, Cmpd-1, CEP-37440, CEP-28122, CEP-18050, cancer therapeutics, Cephalon, anti-ALK MAb, MedImmune, ALK inhibitors, PharmaDesign, ALK inhibitors, Lilly, ALK inhibitors, and Cephalon -2.

Treatments directed to RET include, but are not limited to: vandetanib, sunitinib malate, sorafenib, regorafenib, cabozantinib, SAR-302503, motesanib diphosphate, apatinib, RET kinase inhibitor, Bionomics, NMS-173, MG-516, sorafenib bead, Biocompatibles, RET inhibitors, Cell T, MP-371, kinase inhibitors, MethylGene, JNJ-26483327, DCC-2157, and AST-487 .

Accordingly, these and other agents can be used alone or in combination to treat NSCLC and can be included as an actionable treatment recommendation as disclosed herein.

Methods directed to determining a likelihood of a positive or negative response to a treatment and/or treating a subject based on the gene variant detected in the subject's sample are also provided herein. Referring to Tables 2 and 3, in certain embodiments, an actionable treatment recommendation refers to a particular treatment. For example, an EML4-ALK fusion present in a tumor sample leads to a recommendation of treatment with crizotinib. In contrast, the presence of an EGFR T790M mutation indicates that an EGFR tyrosine kinase inhibitor (TKI) would not be an appropriate treatment as this variant renders the tumor cell resistant to TKIs. The actionable treatment recommendation can be used to administer a treatment or withhold a treatment, depending on the variant status of a subject's tumor.

**TABLE 2**

| **Lung Adenocarcinoma** | | | |
|---|---|---|---|
| **AI Category** | **Genetic** | **Variant** | **Actionable treatment recommendation** |
| AI1 | ALK | EML4-ALK, KIF5B-ALK, KLC1-ALK, TGF-ALK fusions | Crizotinib |
| AI1 | EGFR | L858R, Exon 19 deletion | EGFR TKIs |
| AI1 | EGFR | Exon 20 insertion (in frame, 3-18 base pairs) | Resistant to EGFR TKIs |
| AI1 | EGFR | T790M | Resistant to EGFR TKIs |
| AI1/AI2 | KRAS | G12C, G12V, G12D, G12A, G12S, G13C, G13D, G12R, G12F | Resistant to EGFR TKI (AI1) |
| | | | Sensitive to MEK inhibitors (AI2) |
| AI1 | ROS1 | EZR-ROS1, SLC34A2-ROS1, CD74-ROS1, SDC4-ROS1 | Crizotinib |
| AI2 | BRAF | V600E | Vemurafenib |
| AI2 | ERBB2 | Exon 20 insertion | Irreversible pan-erb inhibitors (e.g., afatinib, neratinib) |
| AI2 | MET | CN gain | Resistant to EGFR TKIs |
| | | | Sensitive to Crizotinib |
| AI2 | PIK3CA | E545K, E545G, E545A, H1047R, H1047L | PIK3CA inhibitors (e.g.,BKM120) |
| AI3 | AKT1 | E17K | 1 Open Phase II Trial (Lung cancer, AKT mutation) |
| AI3 | BRAF | L597R | 3 Open Phase I trials (solid cancer), 1 Open Phase II trial (lung cancer, BRAF mutation) |
| AI3 | BRAF | G469R, D594H/N | 3 Open Phase I trials (solid cancer), 1 Open Phase II trial (lung cancer, BRAF mutation) |
| AI3 | EGFR | G719X | 1 Open Phase I (NSCLC), 1 Open Phase 1 (solid cancer), 1 open Phase II (NSCLC) |
| AI3 | HRAS | Q61L/K/R, G12C/D, G13C/S/R/V | 1 Open Phase II (lung cancer, HRAS mutations) |
| AI3 | PIK3CA | E542K | 2 Open Phase I (solid cancer), 1 Open Phase II trial (NSCLC, PIK3CA mutation) |

**TABLE 3**

| **Squamous Cell Lung Carcinoma** | | | |
|---|---|---|---|
| **AI Category** | **Prevalence > 1%** | **Prevalence 0.1%-1%** | **Actionable treatment recommendation** |
| AI1 | EGFR (L858R, Exon 19 del) | EGFR (G719X) | EGFR TKIs |
| AI1/AI2 | KRAS (G12C, G12D) | KRAS (G12A, G12V) | Resistant to TKIs (All); Sensitive to MEK Inhibitors (AI2) |
| AI2 | MET CN gain | | Resistant to TKIs; Sensitive to Crizotinib |
| AI2 | PIK3CA (E545K, E542K, H1047R) | | PIK3CA Inhibitors (e.g., BKM120) |
| AI3 | AKT1 (E17K) | | 1 Open Phase II Trial (Lung cancer, AKT mutation) |
| AI3 | | HRAS (Q61,/K/R, G12C/D) | 1 Open Phase II (Lung cancer; HRAS mutation) |
| AI3 | EGFR CN gain | | 1 Open Phase II (lung cancer; EGFR amplification) |
| AI3 | ERBB2 CN gain | | 2 Open Phase II ( Lung cancer; ERBB2 amplification) |
| AI3 | FGFR1 CN gain | | 2 Open Phase I; Phase II (Solid cancer; FGFR1 amplification) |
| AI3 | KIT/PDGFRA CN gain | | 1 Open Phase II (Lung cancer; PDGFRA amplification) |
| AI3 | PTEN Del | | 4 Open Phase I/II (NSCLC, PTEN alterations) |

**TABLE 4**

| **Adenocarcinoma** | | |
|---|---|---|
| **AI1-AI2-AI3-Gene-Event** | **No.** | **Percentage** |
| ALK- Fusion | 2 | 1% |
| BRAF-Mutation | 3 | 2% |
| BRAF-Mutation; PIK3CA-mutation* | 1 | 1% |
| EGFR-CN Amp | 3 | 2% |
| EGFR-Mutation | 13 | 8% |
| EGFR-Mutation; EGFR-CN Amp* | 3 | 2% |
| ERBB2-CN Amp | 3 | 2% |
| ERBB2-mutation | 3 | 2% |
| Amp | 2 | 1% |
| HRAS-Mutation | 1 | 1% |
| KIT- CN Amp | 1 | 1% |
| KRAS-Mutation; PIK3CA-Mutation* | 2 | 1% |
| KRAS-Mutation | 39 | 24% |
| KRAS-Mutation; EGFR-CN Amp* | 1 | 1% |
| MET-CN Amp | 3 | 2% |
| PIK3CA-mutation | 3 | 2% |
| RET- Fusion | 1 | 1% |
| ROS1- Fusion | 2 | 1% |
| WT | 79 | 48% |

**TABLE 5**

| **Adenocarcinoma** | | |
|---|---|---|
| **AI1-AI2-AI3-Gene-Variant** | **No** | **Percentage** |
| BRAF-D594H; PIK3CA-E542K* | 1 | 1% |
| BRAF-D594N | 1 | 1% |
| BRAF-V600E | 2 | 1% |
| CCDC6-RET Fusion | 1 | 1% |
| CD74-ROS1 Fusion | 1 | 1% |
| EGFR-CN Amp | 3 | 2% |
| EGFR-E19Del | 4 | 2% |
| EGFR-E19Del; EGFR-CN Amp* | 3 | 2% |
| EGFR-G719A | 1 | 1% |
| EGFR-L858R | 7 | 4% |
| EGFR-L858R; EGFR-T790M* | 1 | 1% |
| EML4-ALK Fusion | 2 | 1% |
| ERBB2-CN Amp | 3 | 2% |
| ERBB2-E20Ins | 3 | 2% |
| FGFRl-CN Amp | 2 | 1% |
| HRAS-Q61L | 1 | 1% |
| KIT- CN Amp | 1 | 1% |
| KRAS-G12A | 4 | 2% |
| KRAS-G12C | 21 | 13% |
| KRAS-G12C; EGFR-CN Amp* | 1 | 1% |
| KRAS-G12C; PIK3CA-E545K* | 2 | 1% |
| KRAS-G12D | 2 | 1% |
| KRAS-G12V | 11 | 7% |
| KRAS-G13D | 1 | 1% |
| MET-CN Amp | 3 | 2% |
| PIK3CA-E545K | 2 | 1% |
| PIK3CA-H1047R | 1 | 1% |
| SLC34A2-ROS1 Fusion | 1 | 1% |
| WT | 79 | 48% |

| | | |
|---|---|---|
| ^{∗}Double mutant genotypes | | |

**TABLE 6**

| **Adenocarcinoma** | | |
|---|---|---|
| **AI1, AI2 Gene event** | **No.** | **Percentage** |
| MET-CN Gain | 1 | 1% |
| PIK3CA-Mutation | 14 | 8% |
| PIK3CA-Mutation; MET-CN Gain* | 1 | 1% |
| WT | 161 | 91% |

| | | |
|---|---|---|
| *Double mutant genotypes | | |

**TABLE 7**

| **Adenocarcinoma** | | |
|---|---|---|
| **AI1, AI2 Gene event** | **No.** | **Percentage** |
| MET-CN Gain | 1 | 1% |
| PIK3CA-Mutation | 14 | 8% |
| PIK3CA-Mutation; MET-CN Gain* | 1 | 1% |
| WT | 161 | 91% |

| | | |
|---|---|---|
| ^{∗}Double mutant genotypes | | |

**TABLE 8**

| **Adenocarcinoma** | | |
|---|---|---|
| **AI1, AI2 Gene event** | **No.** | **Percentage** |
| MET-CN Gain | 1 | 1% |
| PIK3CA-Mutation | 14 | 8% |
| PIK3CA-Mutation; MET-CN Gain* | 1 | 1% |
| WT | 161 | 91% |

| | | |
|---|---|---|
| *Double mutant genotypes | | |

**TABLE 9**

| **Squamous Cell Carcinoma** | | |
|---|---|---|
| **AI1, A12, AI3-Gene event** | **No.** | **Percentage** |
| EGFR- CN Gain | 12 | 7% |
| ERBB2-CN Gain | 1 | 1% |
| FGFRl- CN Gain | 23 | 13% |
| KIT-CN Gain | 1 | 1% |
| MET-CN Gain | 1 | 1% |
| PIK3CA-Mutation | 11 | 6% |
| PIK3CA-Mutation; EGFR- CN Gain* | 1 | 1% |
| PIK3CA-Mutation; FGFR1- CN Gain* | 2 | 1% |
| PIK3CA-Mutation; MET-CN Gain* | 1 | 1% |
| PTEN- CN Loss | 2 | 1% |
| WT | 122 | 69% |

| | | |
|---|---|---|
| ^{∗}Double mutant genotypes | | |

**TABLE 10**

| **Squamous Cell Carcinoma** | | |
|---|---|---|
| **AI1, AI2 Gene Events** | **No.** | **Percentage** |
| AI2 | 16 | 9% |
| WT | 161 | 91% |

**TABLE 11**

| Highly Actionable Molecular Targets in NSCLC | | |
|---|---|---|
| Source | Type | Gene Target |
| DNA | Oncogenes | EGFR, ERBB2, ERBB4, MET, FGFR1, FGFR2, FGFR3, DDR2, ALK |
| | EGFR Pathway | KRAS, NRAS, PIK3CA, BRAF, MAP2K1, AKT1 |
| | Tumor Suppressor Genes | PTEN, TP53, CTNNB1, NOTCH1, STK11, SMED4, FBXW7 |
| RNA | Fusion Genes | ALK, RET, ROS |

**TABLE 13**

| Gene Symbol | Variant Type | Level of evidence | Approved targeted agent | Indications and uses | Limitations of usage |
|---|---|---|---|---|---|
| ALK | Fusion | 1 | crizotinib | Xalkori- kinase inhibitor indicated for treatment of patients with metastatic NSCLC whose tumors are ALK-positive as detected by an FDA-approved test | |
| ALK | Fusion | 1 | | | |
| RET | Fusion | 2 | None | None | None |
| RET | Fusion | 2 | | | |
| ROS1 | Fusion | 1 | None | None | None |

### Reports

In another aspect, the invention features a report indicating a prognosis or treatment response prediction of a subject with cancer. The report can, for example, be in electronic or paper form. The report can include basic patient information, including a subject identifier (*e.g*., the subject's name, a social security number, a medical insurance number, or a randomly generated number), physical characteristics of the subject (*e.g*., age, weight, or sex), the requesting physician's name, the date the prognosis was generated, and the date of sample collection. The reported prognosis can relate to likelihood of survival for a certain period of time, likelihood of response to certain treatments within a certain period of time (*e.g*., chemotherapeutic or surgical treatments), and/or likelihood of recurrence of cancer. The reported prognosis can be in the form of a percentage chance of survival for a certain period of time, percentage chance of favorable response to treatment (favorable response can be defined, e.g., tumor shrinkage or slowing of tumor growth), or recurrence over a defined period of time (*e.g*., 20% chance of survival over a five year period). In another embodiment, the reported prognosis can be a general description of the likelihood of survival, response to treatment, or recurrence over a period of time. In another embodiment, the reported prognosis can be in the form of a graph. In addition to the gene expression levels and gene variants/mutations, the reported prognosis may also take into account additional characteristics of the subject (*e.g*., age, stage of cancer, gender, previous treatment, fitness, cardiovascular health, and mental health).

In addition to a prognosis, the report can optionally include raw data concerning the expression level or mutation status of genes of interest.

### EXAMPLES

### Example I

Genomic and gene variant data was obtained from Life Technologies and Compendia Bioscience's ONCOMINE^{™} Concepts Edition and ONCOMINE^{™} Power Tools, a suite of web applications and web browsers that integrates and unifies high-throughput cancer profiling data by systematic collection, curation, ontologization and analysis. In addition, mutation gene variant data was also obtained from Life Technologies and Compendia Bioscience's curation and analysis of next generation sequencing data available from The Cancer Genome Atlas (TCGA) Portal.

Data obtained from the TCGA contains mutation results from datasets processed and annotated by different genome sequencing centers. All of the mutation data characterized in TCGA was somatic mutation data containing mutation variants specific to the tumor specimen and not observed in the normal tissue specimen obtained from the same individual. To obtain consistent variant annotation, the mutations obtained from TCGA were re-annotated based on a single set of transcripts and variant classification rules. A standard annotation pipeline ensured that mutations were evaluated consistently and were subject to common interpretation during the identification of lung cancer gene variants. In the Mutation Annotation step, the mutations obtained from TCGA were re-annotated against a standard transcript set. This transcript set included RefGene transcripts from hg 18 and hg 19 genome builds, obtained from UCSC on February 19, 2012.

Mutation data incorporated into ONCOMINE Power Tools was derived from multiple sources including the Sanger Institute's Catalogue of Somatic Mutations in Cancer (COSMIC). Mutation data sourced from COSMIC retained its original annotation.

Recurrent gene mutations in multiple clinical samples were identified based on the position of the variant in the gene coding sequence. Missense mutation variants were inferred if the mutation was a single nucleotide polymorphism (SNP) in a coding exon that changed the encoded amino acid. Such missense mutation gene variants were recurrent if the same gene contained the same SNP in multiple samples. Hotspot in frame insertion/deletion mutation variants were inferred if the nucleotide mutation was an insertion or deletion divisible by 3 nucleotides.

The frequency of recurrent hotspot missense mutation and/or hotspot in frame insertion/deletion mutation in different genes in lung cancer was characterized by counting all of the clinical specimens tested that were found to contain the gene variants and expressing that value as a percentage relative to all of the clinical specimens tested. A list of all the genes with prevalent hotspot missense mutations in lung cancer was derived.

Gene copy number data for lung cancer was obtained from the ONCOMINE DNA Copy PowerTool. A minimal common region analysis was performed to identify chromosomal regions of focal amplification in lung cancer. Contiguous chromosomal regions (common regions) containing copy gain (≥0.9 log2 copy number) in 2 or more samples were identified. Within each common region, the genes that were aberrant in the highest number of samples (n) and also those that were aberrant in one less the highest number (n-1) were identified. Alternatively, genes aberrant in 95% of the highest number of samples (n) were identified. The frequency of these peak regions was determined by calculating the number of samples with copy gain relative to the total number of samples analyzed and expressing this value as a percentage. The most prevalent peak regions in lung cancer typically contained known cancer genes such as MET, FGFR1, EGFR, ERBB2, KIT/PDGFRA.

Gene variants with prevalent hotspot missense mutations, focal amplification, or gene fusion were investigated further to determine whether they had actionability evidence associated with actionability index levels 1-3.

Gene variants associated with AI1 were identified in the National Comprehensive Cancer Network Practice Guidelines in Oncology (NCCN Guidelines) for non-small cell lung cancer (NSCLC) (Version 2.2013). Such gene variants were those that the Guidelines provided specific treatment recommendations. For example, patients with lung adenocarcinoma whose tumor specimen was found to contain EGFR L858R variants were recommended to consider treatment with an EGFR inhibitor such as erlotinib or gefitnib.

Gene variants associated with AI2 were identified in public literature sources such as the National Center for Biotechnology Information (NCBI) PubMed, a web browser containing citations for biomedical literature.

Gene variants associated with AI3 were identified by searching databases of clinical trial information such as ClinicalTrials.Gov and citelineⒸ TrialTrove for matching gene and variant type annotation in the enrollment criteria of ongoing clinical trials.

Referring to Tables 4-5, the methods disclosed herein provide an actionable treatment recommendation for 50% of adenocarcinoma subjects. A cohort of 165 patients with primary lung adenocarcinoma was characterized by next generation sequencing methods. The gene variants were mapped onto this population. Most patients were observed to have only a single aberration out of the entire panel. Collectively, approximately 52% of subjects were positive for at least one genetic variance. The prevalence of gene variants in combinations of the AI1, AI2, and AI3 categories are shown in Tables 4-8.

### Example II

A 177 cohort of patients with lung squamous cell carcinoma were characterized by next generation sequencing methods and gene variants were mapped onto this population, according to the methods of Example I. The prevalence of gene variants in AI1, AI2, and AI3 categories in the TCGA squamous cell carcinoma 177 patient cohort are shown in Tables 9-10.

Additional genes and their levels of evidence and corresponding actionabilities are shown in Tables 11-14

### Example III

A patient presents with late stage NSCLC. A test is conducted to determine the mutation status of highly actionable NSCLC biomarkers in Table 11 in one panel to preserve limited tumor biopsy sample. A report is generated outlining the mutation status of the sample and corresponding actionability indices. A course of treatment is determined based on the mutation status of the patient's tumor.

It is understood that the examples and embodiments described herein are for illustrative purposes only and that various modifications or changes in light thereof will be suggested to persons skilled in the art.

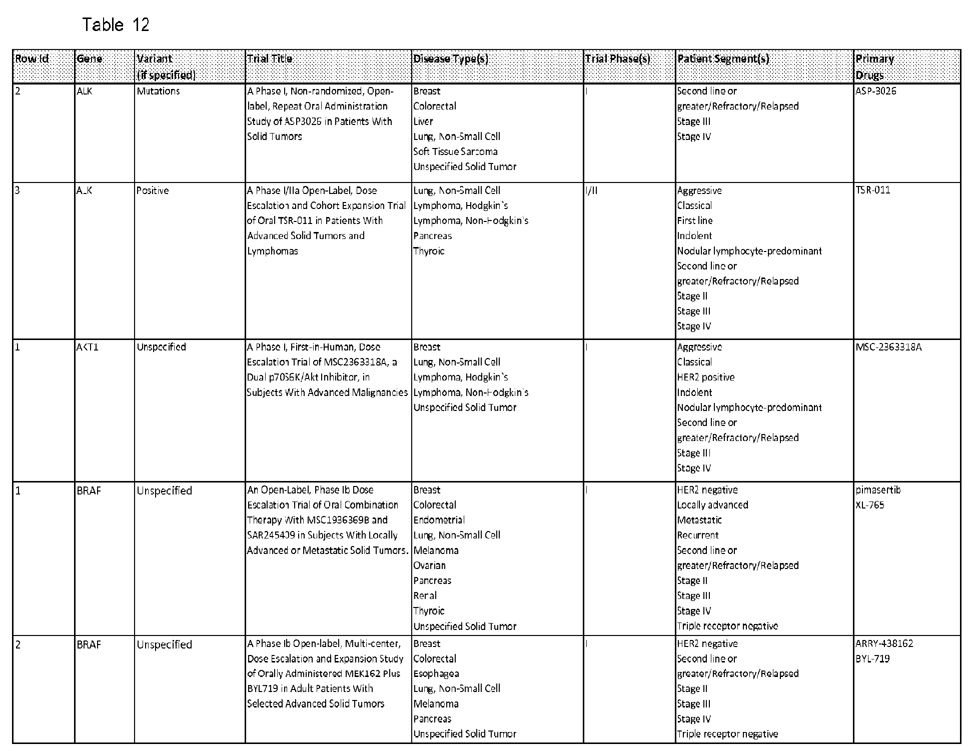

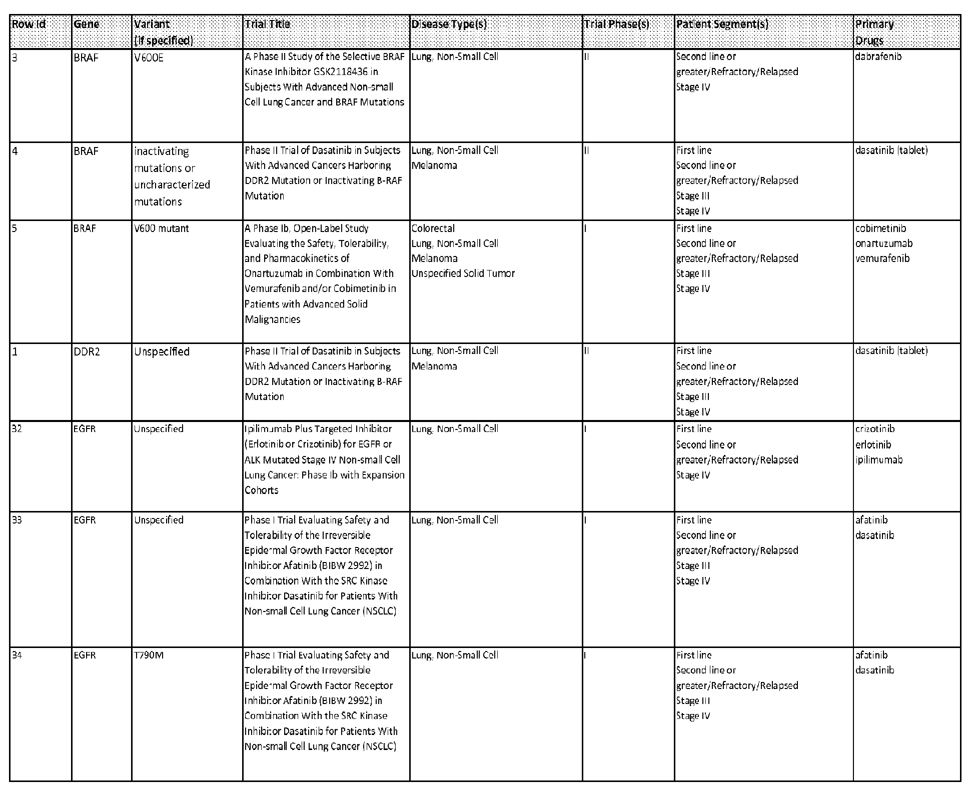

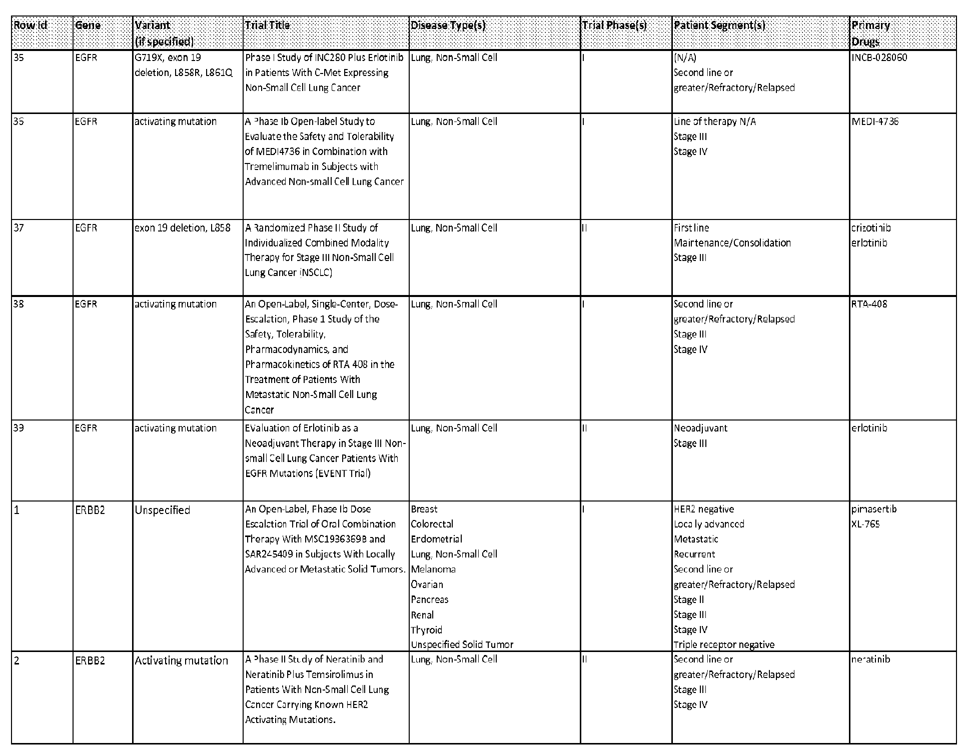

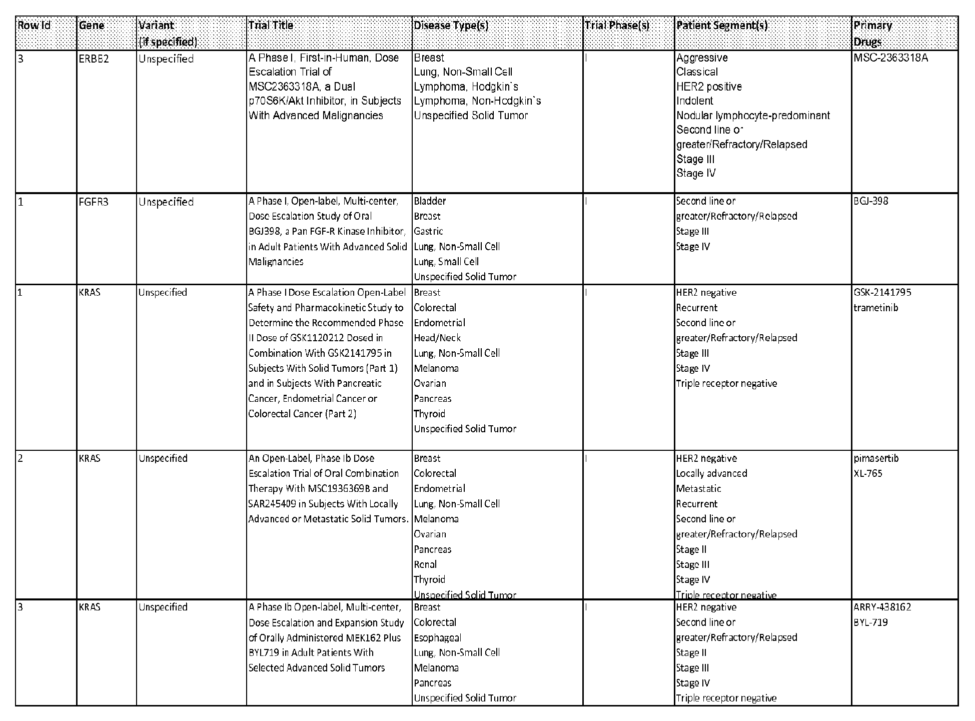

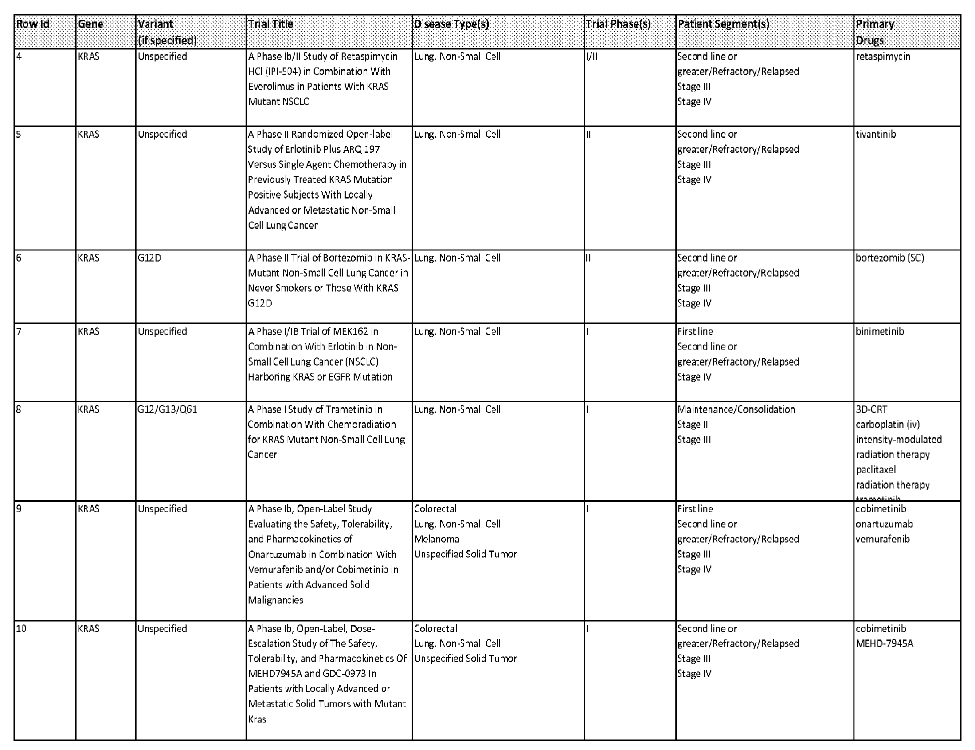

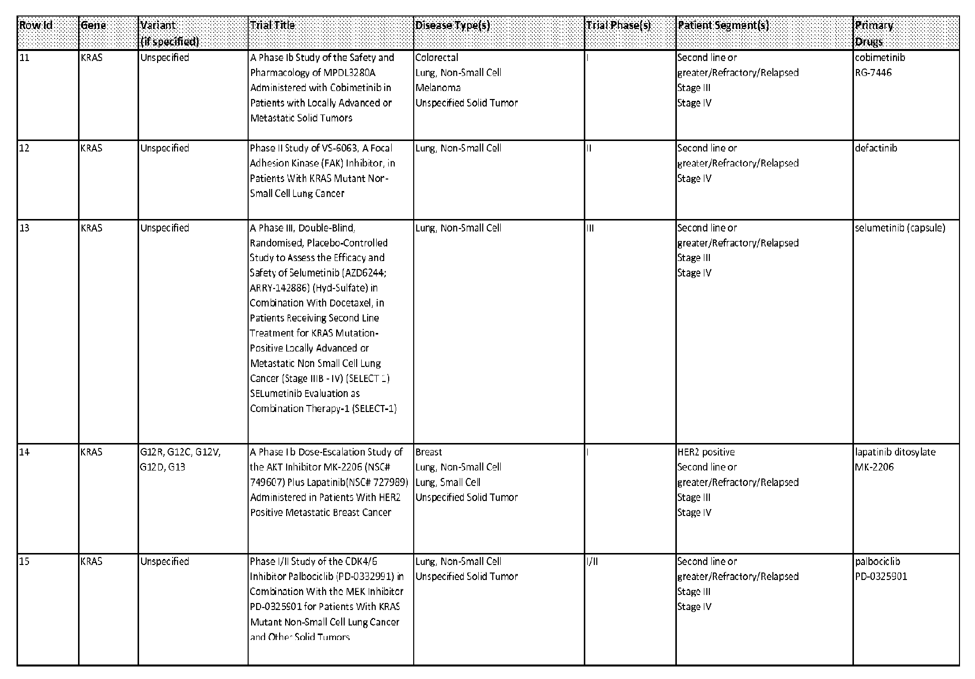

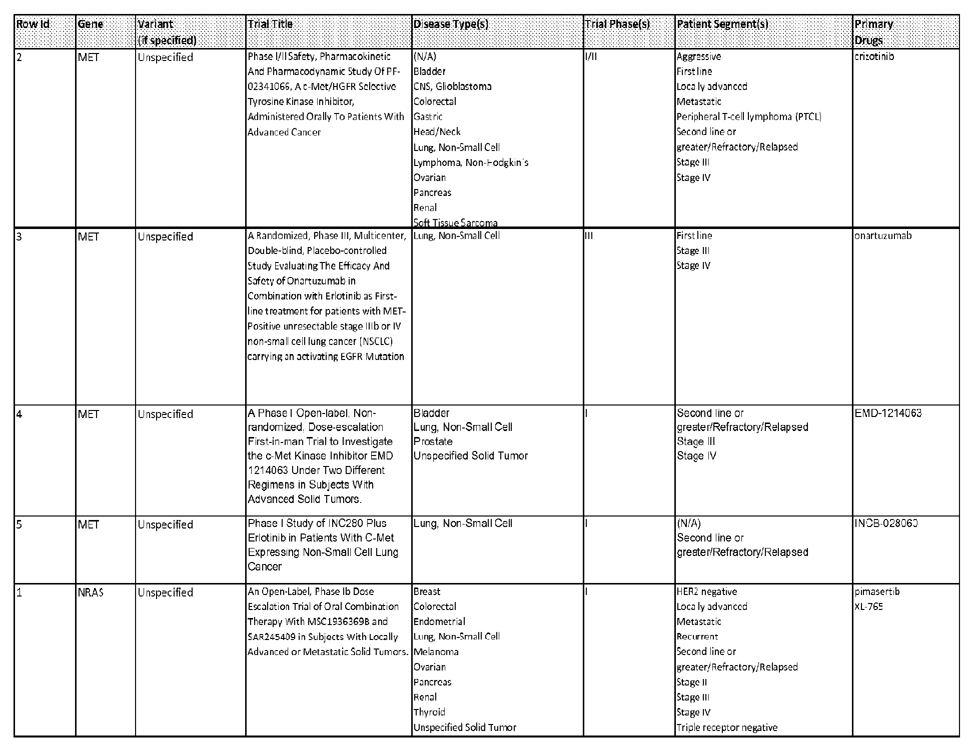

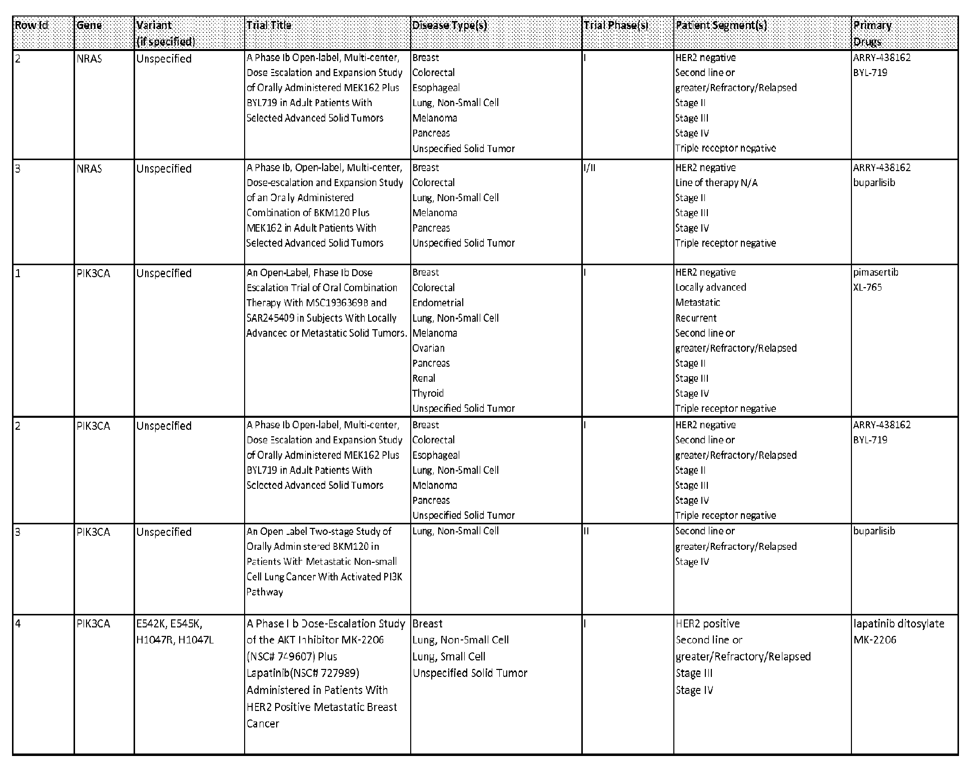

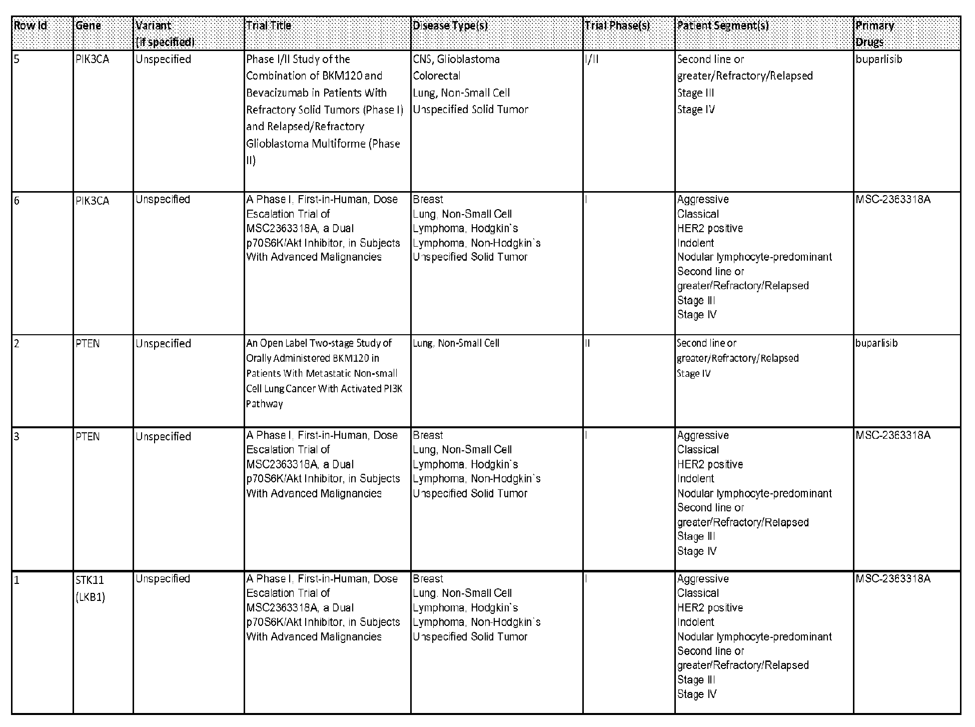

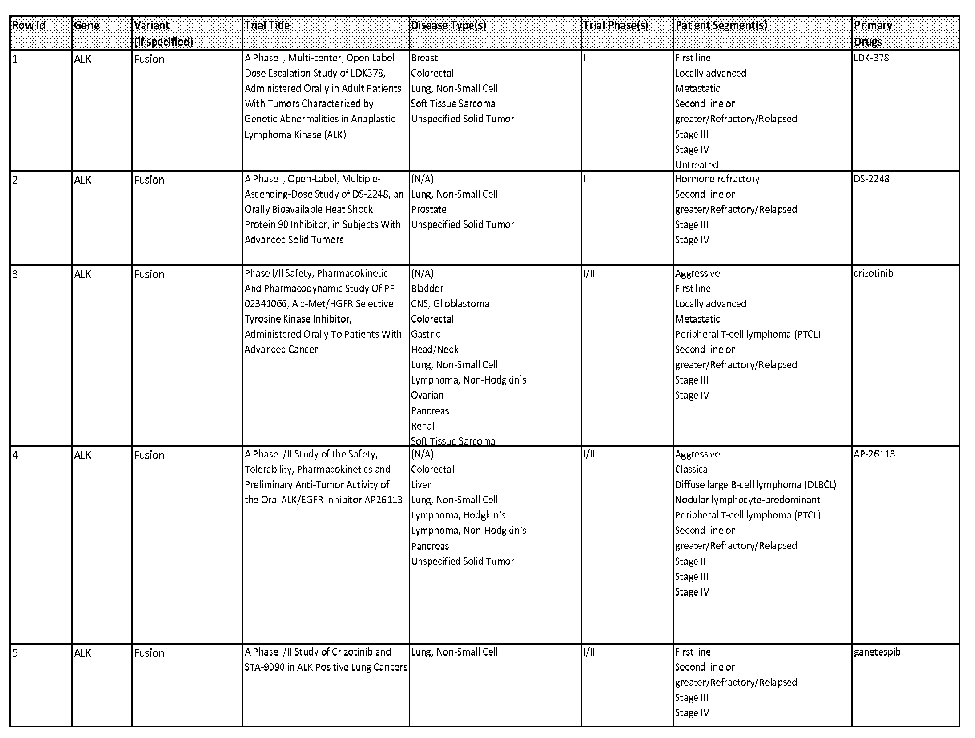

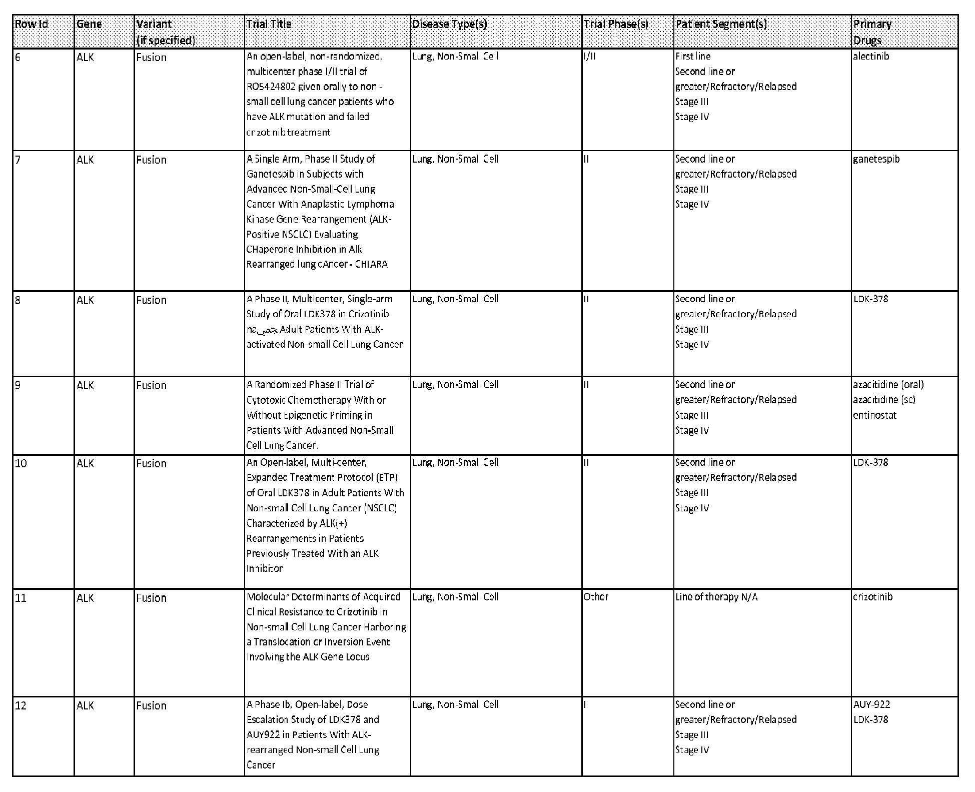

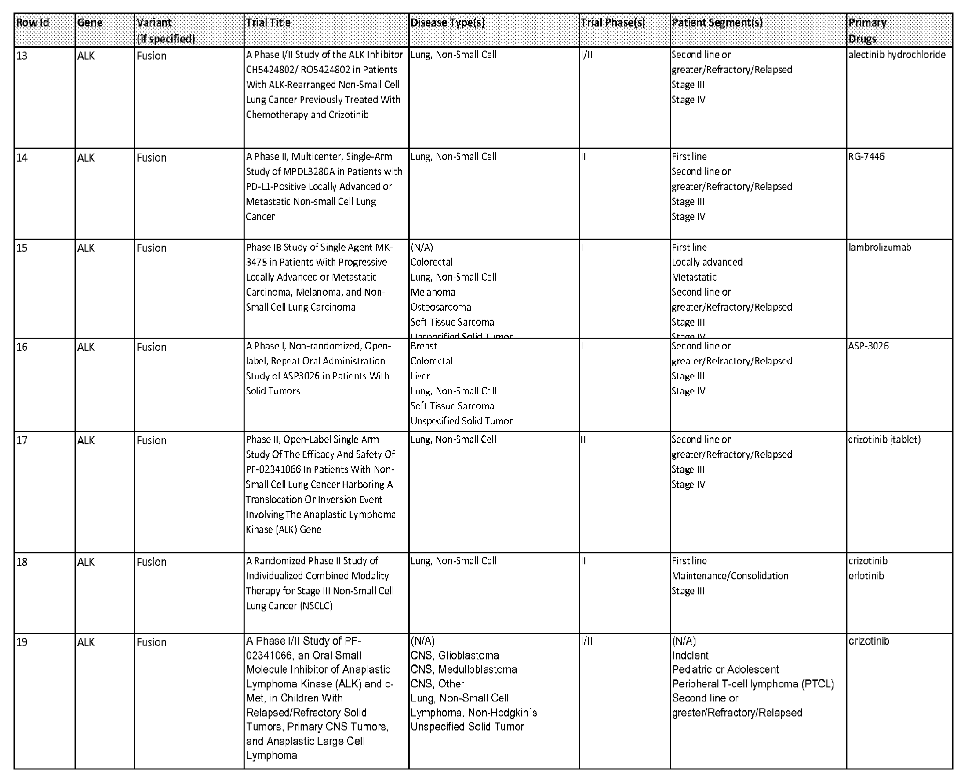

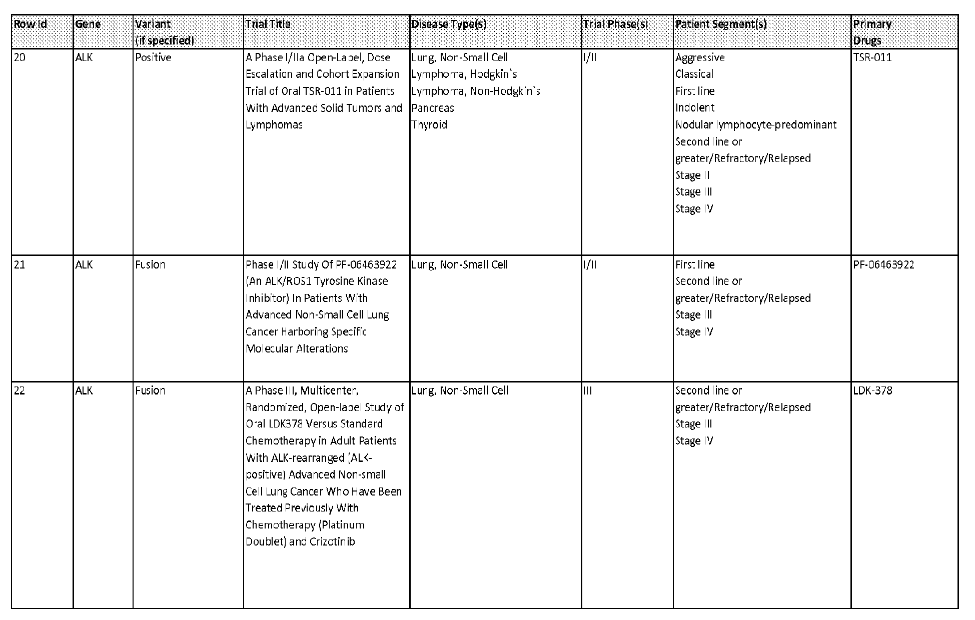

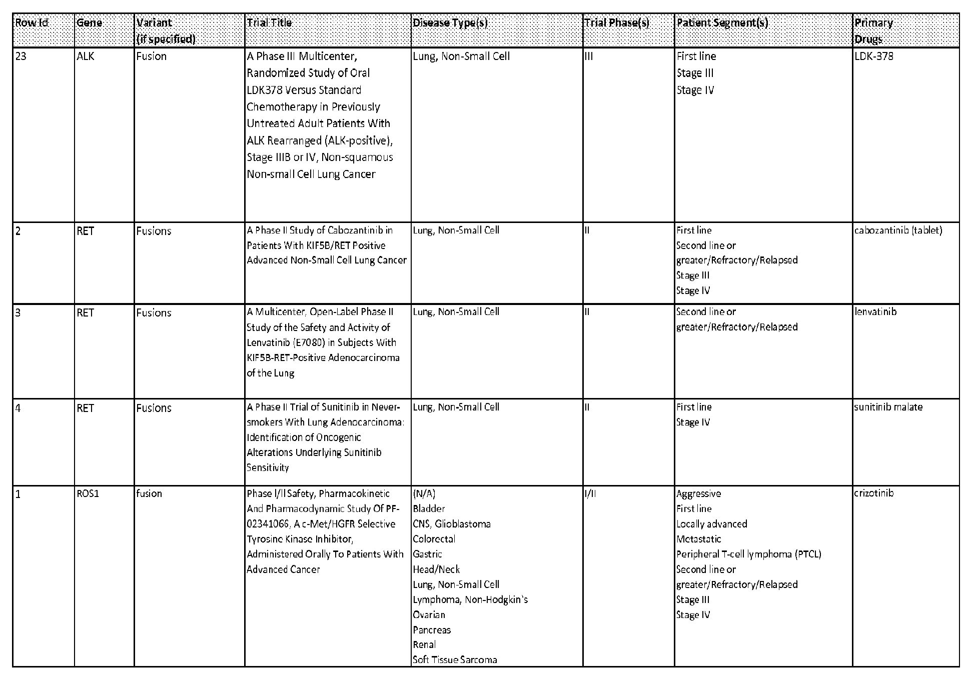

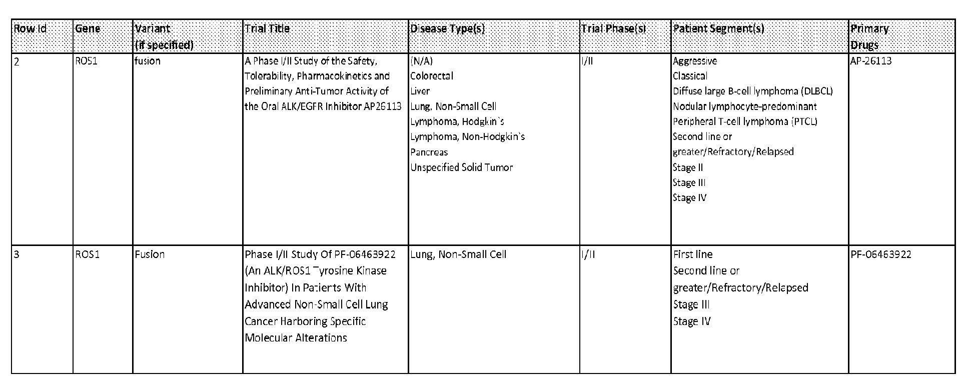

**Table**

| **Row Id** | **Gene Symbol** | **Accesion_number** | **COSMIC_id** | **CDS_mut_syntax** | | **Oncomine Gene Classification** | **Onconomie Variant Classification** |
|---|---|---|---|---|---|---|---|
| 1 | AKT1 | ENST00000349310 | 33765 | c.49G>A | p.E17K | Gain of function | Missense_Mutation |
| 2 | ALK | NM_004304 | 28059 | c.3521T>G | p.F1174C | Gain of function | Missense_Mutation |
| 3 | ALK | NM_004304 | 28491 | c.3520T>A | p.F1174I | Gain of function | Missense_Mutation |
| 4 | ALK | NM_004304 | 28055 | c.3522C>A | P.F1174L | Gain of function | Missense_Mutation |
| 5 | ALK | NM_004304 | 28057 | c.3520T>C | P.F1174L | Gain of function | Missense_Mutation |
| 6 | ALK | NM_004304 | 28061 | c.3522C>G | P.F1174L | Gain of function | Missense_Mutation |
| 7 | ALK | NM_004304 | 28054 | c.3520T>G | p.F1174V | Gain of function | Missense_Mutation |
| 8 | ALK | NM_004304 | 99137 | c.3586C>A | P.L1196M | Gain of function | Missense_Mutation |
| 9 | ALK | N M₋004304 | 98478 | c.3452C>T | p.T1151M | Gain of function | Missense_Mutation |
| 10 | BRAF | NM_004333 | 26625 | c.1794_1795insGTT | p.A598 T599insV | Gain of function | In_Frame_Ins |
| 11 | BRAF | NM_004333 | 21549 | c.1793C>T | p.A598V | Gain of function | Missense_Mutation |
| 12 | BRAF | NM_004333 | 467 | c.1781A>G | p.D594G | Gain of function | Missense_Mutation |
| 13 | BRAF | NM_004333 | 27639 | c.1780G>A | p.D594N | Gain of function | Missense_Mutation |
| 14 | BRAF | NM_004333 | 466 | c.1781A>T | p.D594V | Gain of function | Missense_Mutation |
| 15 | BRAF | NM_004333 | 1118 | c.1758A>G | p.E586E | Gain of function | Synonymous_Mutation |
| 16 | BRAF | NM_004333 | 463 | c.1756G>A | p.E586K | Gain of function | Missense_Mutation |
| 17 | BRAF | NM_004333 | 1116 | c.1749T>C | p.F583F | Gain of function | Synonymous_Mutation |
| 18 | BRAF | NM_004333 | 468 | c.1785T>G | p.F595L | Gain of function | Missense_Mutation |
| 19 | BRAF | NM_004333 | 1123 | c.1784T>C | p.F595S | Gain of function | Missense_Mutation |
| 20 | BRAF | NM_004333 | 449 | c.1391G>A | p.G464E | Gain of function | Missense_Mutation |
| 21 | BRAF | NM_004333 | 450 | c.1391G>T | p.G464V | Gain of function | Missense_Mutation |
| 22 | BRAF | NM_004333 | 26506 | c.1787G>A | p.G596D | Gain of function | Missense_Mutation |
| 23 | BRAF | NM_004333 | 469 | c.1786G>C | p.G596R | Gain of function | Missense_Mutation |
| 24 | BRAF | NM_004333 | 1137 | c.1817G>A | P.G606E | Gain of function | Missense_Mutation |
| 25 | BRAF | NM_004333 | 1138 | c.1823A>G | p.H6D8R | Gain of function | Missense_Mutation |
| 26 | BRAF | NM_004333 | 1115 | c.1746A>G | p.1582M | Gain of function | Missense_Mutation |
| 27 | BRAF | NM_004333 | 1119 | c.1776A>G | p.1592M | Gain of function | Missense_Mutation |
| 28 | BRAF | NM_004333 | 1120 | c.1774A>G | p.1592V | Gain of function | Missense_Mutation |
| 29 | BRAF | NM_004333 | 478 | c.1801A>G | P.K601E | Gain of function | Missense_Mutation |
| 30 | BRAF | NM_004333 | 1132 | c.1803A>C | P.K601N | Gain of function | Missense_Mutation |
| 31 | BRAF | NM_004333 | 6265 | c.1803A>T | P.K601N | Gain of function | Missense_Mutation |
| 32 | BRAF | NM_004333 | 28010 | c.1750C>T | p.L584F | Gain of function | Missense_Mutation |
| 33 | BRAF | NM_004333 | 1125 | c.1790T>A | p.L597Q | Gain of function | Missense_Mutation |
| 34 | BRAF | NM_004333 | 471 | c.1790T>G | p.L597R | Gain of function | Missense_Mutation |
| 35 | BRAF | NM_004333 | 1126 | c.1789_1790CT>TC | p.L597S | Gain of function | Missense_Mutation |
| 36 | BRAF | NM_004333 | 470 | c.1789C>G | p.L597V | Gain of function | Missense_Mutation |
| 37 | BRAF | NM_004333 | 462 | c.1742A>G | p.N581S | Gain of function | Splice_Site |
| 38 | BRAF | NM _004333 | 21492 | c.1357C>A | p.P453T | Gain of function | Missense_Mutation |
| 39 | BRAF | NM _004333 | 6262 | c.1330C>T | p.R444W | Gain of function | Missense_Mutation |
| 40 | BRAF | NM _004333 | 447 | c.1385G>T | p.R462I | Gain of function | Missense_Mutation |
| 41 | BRAF | NM _004333 | 1117 | c.1752T>C | p.L584L | Gain of function | Synonymous_Mutation |
| 42 | BRAF | NM_004333 | 1124 | c.1791A>G | p.LS97L | Gain of function | Synonymous_Mutation |
| 43 | BRAF | NM_004333 | 33729 | c.1807C>T | p.R603* | Gain of function | Nonsense_Mutation |
| 44 | BRAF | NM_004333 | 1135 | c.1813_1814AG>TT | P.S605F | Gain of function | Missense_Mutation |
| 45 | BRAF | NM_004333 | 21542 | c.1813A>G | p.S605G | Gain of function | Missense_Mutation |
| 46 | BRAF | NM_004333 | 1136 | c.1814G>A | P.S605N | Gain of function | Missense_Mutation |
| 47 | BRAF | NM_004333 | 144982 | c.l797_1798insACA | p.T599_V600insT | Gain of function | In_Frame_Ins |
| 48 | BRAF | NM_004333 | 30730 | c1796_1797insTAC | p.T599_V600insT | Gain of function | In_Frame_Ins |
| 49 | BRAF | NM_004333 | 1128 | c.1797 1797A>TACTACG | p.T599_V600insTT | Gain of function | In_Frame_Ins |
| 50 | BRAF | NM_004333 | 472 | c.1796C>T | p.T599I | Gain of function | Missense_Mutation |
| 51 | BRAF | NM_004333 | 1133 | c.1799 1801deITGA | p.V600_K601>E | Gain of function | In_Frame_Del |
| 52 | BRAF | NM_004333 | 18443 | c.1799T>C | p.V600A | Gain of function | Missense_Mutation |
| 53 | BRAF | NM_004333 | 477 | c.1799 1800TG>AT | p.V600D | Gain of function | Missense_Mutation |
| 54 | BRAF | NM_004333 | 6137 | c.1799T>G | p.V600G | Gain of function | Missense_Mutation |
| 55 | BRAF | NM_004333 | 473 | c1798_1799GT>AA | p.V600K | Gain of function | Missense_Mutation |
| 56 | BRAF | NM_004333 | 219798 | c.1798G>C | p.V600L | Gain of function | Missense_Mutation |
| 57 | BRAF | NM_004333 | 33808 | c.1798G>T | p.V600L | Gain of function | Missense_Mutation |
| 58 | BRAF | NM_004333 | 1130 | c.1798G>A | p.V600M | Gain of function | Missense_Mutation |
| 59 | BRAF | NM_004333 | 249889 | c.1798_1799GT>CA | p.V600Q | Gain of function | Missense_Mutation |
| 60 | BRAF | NM_004333 | 474 | c.1798_1799GT,AG | P.V600R | Gain of function | Missense_Mutation |
| 61 | BRAF | NM_004333 | 6267 | c.1808_1810delGAT | p.W604del | Gain of function | In_Frame_Del |
| 62 | BRAF | NM_004333 | 1134 | c.1810T>G | p.W604G | Gain of function | Missense_Mutation |
| 63 | BRAF | NM_004333 | 453 | c.1397G>A | p.G466E | Gain of function | Missense_Mutation |
| 64 | BRAF | NM_004333 | 1112 | c.1396G>C | p.G466R | Gain of function | Missense_Mutation |
| 65 | BRAF | NM_004333 | 451 | c.1397G>T | p.G466V | Gain of function | Missense_Mutation |
| 66 | BRAF | NM_004333 | 460 | c.1406G>C | p.G469L | Gain of function | Missense_Mutation |
| 67 | BRAF | NM_004333 | 460 | c.1406G>C | p.G469A | Gain of function | Missense_Mutation |
| 68 | BRAF | NM_004333 | 461 | c.1406G>A | p.G469E | Gain of function | Missense_Mutation |
| 69 | BRAF | NM_004333 | 457 | c.1405G>A | p.G469R | Gain of function | Missense_Mutation |
| 70 | BRAF | NM_004333 | 458 | c.1405_1406GG>TC | p.G4695 | Gain of function | Missense_Mutation |
| 71 | BRAF | NM_004333 | 459 | c.1406G>T | p.G469V | Gain of function | Missense_Mutation |
| 72 | BRAF | NM_004333 | 475 | c.1799_1800TG>AA | p.V600E | Gain of function | Missense_Mutation |
| 73 | BRAF | NM_004333 | 475 | c.1799_1800TG>AA | p.V600E | Gain of function | Missense_Mutation |
| 74 | BRAF | NM_004333 | 475 | c.1799_1800TG>AA | p.V600E | Gain of function | Missense_Mutation |
| 75 | BRAF | NM_004333 | 476 | c.1799T>A | p.V600E | Gain of function | Missense_Mutation |
| 76 | BRAF | NM_004333 | 476 | c.1799T>A | p.V600E | Gain of function | Missense_Mutation |
| 77 | BRAF | NM_004333 | 476 | c.1799T>A | p.V600E | Gain of function | Missense_Mutation |
| 78 | CTNNB1 | NM_001904 | 5747 | c.37G>A | P.A13T | Gain of function | Missense_Mutation |
| 79 | CTNNB1 | NM_001904 | 5702 | c.59C>T | p.A20V | Gain of function | Missense_Mutation |
| 80 | CTNNB1 | NM_001904 | 5738 | c.61G>A | p.A21T | Gain of function | Missense_Mutation |
| 81 | CTNNB1 | NM_001904 | 5753 | c.116C>G | p.A39G | Gain of function | Missense_Mutation |
| 82 | CTNNB1 | NM_001904 | 5762 | c.115G>A | p.A39T | Gain of function | Missense_Mutation |
| 83 | CTNNB1 | NM_001904 | 5744 | c.127G>C | p.A43P | Gain of function | Missense_Mutation |
| 84 | CTNNB1 | NM_001904 | 5758 | c.127G>A | p.A43T | Gain of function | Missense_Mutation |
| 85 | CTNNB1 | NM_001904 | 5699 | c.128C>T | p.A43V | Gain of function | Missense_Mutation |
| 86 | CTNNB1 | NM_001904 | 5690 | c.95A:C | p.D32A | Gain of function | Missense_Mutation |
| 87 | CTNNB1 | NM_001904 | 5681 | c.95A>G | p.D32G | Gain of function | Missense_Mutation |
| 88 | CTNNB1 | NM_001904 | 5666 | c.94G>C | p.D32H | Gain of function | Missense_Mutation |
| 89 | CTNNB1 | NM_001904 | 5672 | c.94G>A | p.D32N | Gain of function | Missense_Mutation |
| 90 | CTNNB1 | NM_001904 | 5691 | c.95A>T | p.D32V | Gain of function | Missense_Mutation |
| 91 | CTNNB1 | NM_001904 | 5661 | c.94G>T | p.D32Y | Gain of function | Missense_Mutation |
| 92 | CTNNB1 | NM_001904 | 49161 | c.43G>A | p.E15K | Gain of function | Missense_Mutation |
| 93 | CTNNB1 | NM_001904 | 5671 | c.101G>A | p.G34E | Gain of function | Missense_Mutation |
| 94 | CTNNB1 | NM_001904 | 5684 | c.100G>C | p.G34R | Gain of function | Missense_Mutation |
| 95 | CTNNB1 | NM_001904 | 5686 | c.100G>A | p.G34R | Gain of function | Missense_Mutation |
| 96 | CTNNB1 | NM_001904 | 5670 | c.101G>T | p.G34V | Gain of function | Missense_Mutation |
| 97 | CTNNB1 | NM_001904 | 5713 | c.113G>A | p.G38D | Gain of function | Missense_Mutation |
| 98 | CTNNB1 | NM_001904 | 5676 | c.107A>C | p.H36P | Gain of function | Missense_Mutation |
| 99 | CTNNB1 | NM_001904 | 27378 | c.107A>G | p.H36R | Gain of function | Missense_Mutation |
| 100 | CTNNB1 | NM_001904 | 5703 | c.106C>T | p.H36Y | Gain of function | Missense_Mutation |
| 101 | CTNNB1 | NM_001904 | 5674 | c.104T>G | p.1355 | Gain of function | Missense_Mutation |
| 102 | CTNNB1 | NM_001904 | 13168 | c.104T>C | p.I35T | Gain of function | Missense_Mutation |
| 103 | CTNNB1 | NM_001904 | 5721 | c.91C> T | p.L31L | Gain of function | Synonymous_Mutation |
| 104 | CTNNB1 | NM_001904 | 13175 | c.138G>A | p.L46L | Gain of function | Synonymous_Mutation |
| 105 | CTNNB1 | NM_001904 | 17661 | c.130C>G | p.P44A | Gain of function | Missense_Mutation |
| 106 | CTNNB1 | NM_001904 | 5761 | c.131C>T | p.P44L | Gain of function | Missense_Mutation |
| 107 | CTNNB1 | NM_001904 | 5704 | c.130C>T | p.P44S | Gain of function | Missense_Mutation |
| 108 | CTNNB1 | NM_001904 | 6057 | c.67_99del33 | p.S23_S33del | Gain of function | In_Frame_Del |
| 109 | CTNNB1 | NM_001904 | 17941 | c.67A>G | p.S23G | Gain of function | Missense_Mutation |
| 110 | CTNNB1 | NM_001904 | 5714 | c.67A>C | p.523R | Gain of function | Missense_Mutation |
| 111 | CTNNB1 | NM_001904 | 5694 | c.86C>T | p.S29F | Gain of function | Missense_Mutation |
| 112 | CTNNB1 | NM_001904 | 5683 | c.97T>G | p.S33A | Gain of function | Missense_Mutation |
| 113 | CTNNB1 | NM_001904 | 5677 | c.98C>G | p.S33C | Gain of function | Missense_Mutation |
| 114 | CTNNB1 | NM_001904 | 5669 | c.98C>T | p.S33F | Gain of function | Missense_Mutation |
| 115 | CTNNB1 | NM_001904 | 6098 | c.97_98TC>CT | p.S33L | Gain of function | Missense_Mutation |
| 116 | CTNNB1 | NM_001904 | 6099 | c.97 _98TC>AA | p.S33N | Gain of function | Missense_Mutation |
| 117 | CTNNB1 | NM_001904 | 5662 | c.97T>C | p.S33P | Gain of function | Missense_Mutation |
| 118 | CTNNB1 | NM_001904 | 5673 | c.98C>A | p.S33Y | Gain of function | Missense_Mutation |
| 119 | CTNNB1 | NM_001904 | 5675 | c.109T>G | p.S37A | Gain of function | Missense_Mutation |
| 120 | CTNNB1 | NM_001904 | 5679 | c.110C>G | p.537C | Gain of function | Missense_Mutation |
| 121 | CTNNB1 | NM_001904 | 5662 | c.110C>T | p.S37F | Gain of function | Missense_Mutation |
| 122 | CTNNB1 | NM_001904 | 5687 | c.109T>C | p.537P | Gain of function | Missense_Mutation |
| 123 | CTNNB1 | NM_001904 | 5666 | c.110C>A | p.S37Y | Gain of function | Missense_Mutation |
| 124 | CTNNB1 | NM_001904 | 5685 | c.133T>G | p.S45A | Gain of function | Missense_Mutation |
| 125 | CTNNB1 | NM_001904 | 5689 | c.134C>G | p.S45C | Gain of function | Missense_Mutation |
| 126 | CTNNB1 | NM_001904 | 5667 | c.134C>T | p.S4SF | Gain of function | Missense_Mutation |
| 127 | CTNNB1 | NM_001904 | 5663 | c.133T>C | p.545P | Gain of function | Missense_Mutation |
| 128 | CTNNB1 | NM_001904 | 5692 | c.134C>A | p.545Y | Gain of function | Missense_Mutation |
| 129 | CTNNB1 | NM_001904 | 5708 | c.119C>T | p.T40I | Gain of function | Missense_Mutation |
| 130 | CTNNB1 | NM_001904 | 6140 | c.120T>C | p.T40T | Gain of function | Synonymous_Mutation |
| 131 | CTNNB1 | NM_001904 | 5664 | c.121A>G | p.T41A | Gain of function | Missense_Mutation |
| 132 | CTNNB1 | NM_001904 | 5676 | c.122C>T | pT41I | Gain of function | Missense_Mutation |
| 133 | CTNNB1 | NM_001904 | 5730 | c.122C>A | p.T41N | Gain of function | Missense_Mutation |
| 134 | CTNNB1 | NM_001904 | 5688 | c.121A>C | p.T41P | Gain of function | Missense_Mutation |
| 135 | CTNNB1 | NM_001904 | 5701 | c.122C>G | p.T41S | Gain of function | Missense_Mutation |
| 136 | CTNNB1 | NM_001904 | 5716 | c.121A>T | p.T41S | Gain of function | Missense_Mutation |
| 137 | CTNNB1 | NM_001904 | 5717 | c.123C>T | p.T41T | Gain of function | Synonymous_Mutation |
| 138 | CTNNB1 | NM_001904 | 29289 | c.125_126delCA | pT42fs*7 | Gain of function | Frame_Shift_Del |
| 139 | CTNNB1 | NM_001904 | 5696 | c.125C>T | p.T42I | Gain of function | Missense_Mutation |
| 140 | CTNNB1 | NM_001904 | 5732 | c.12SC>G | p.T42R | Gain of function | Missense_Mutation |
| 141 | CTNNB1 | NM_001904 | 6050 | c.64_114del51 | p.V22_G38del | Gain of function | In_Frame_Del |
| 142 | CTNNB1 | NM_001904 | 6052 | c.64 99del36 | p.V22_S33del | Gain of function | In_Frame_Del |
| 143 | CTNNB1 | NM_001904 | 5706 | c.65T>C | p.V22A | Gain of function | Missense_Mutation |
| 144 | CTNNB1 | NM_001904 | 22566 | c.64G>A | p.V22I | Gain of function | Missense_Mutation |
| 145 | CTNNB1 | NM_001904 | 6064 | c.74_97del24 | p.W25 D32del | Gain of function | In_Frame_Del |
| 146 | CTNNB1 | NM_001904 | 5749 | c.74G>T | p.W25L | Gain of function | Missense_Mutation |
| 147 | CTNNB1 | NM_001904 | 14256 | c.73_96del24 | p.WQQQSYLD25? | Gain of function | N/A |
| 148 | CTNNB1 | NM_001904 | 34125 | c.90C>G | p.Y30* | Gain of function | Nonsense_Mutation |
| 149 | CTNNB1 | NM_001904 | 6076 | c.88_99del12 | p.Y30_S33del | Gain of function | In_Frame_Del |
| 150 | DDR2_ENST00000367922 | ENST00000367922 | 173712 | c.390C>T | p.I130I | Unclassified | Synonymous_Mutation |
| 151 | DDR2_ENST00000367922 | ENST00000367922 | 94126 | c.1783C>G | p.L595V | Unclassified | Missense_Mutation |
| 152 | DDR2 | NM_006182 | 48314 | c.1367A>G | P.N456S | Unclassified | Missense_Mutation |
| 153 | DDR2_ENST00000367922 | ENST00000367922 | 140388 | c.691C>A | p.Q231K | Unclassified | Missense_Mutation |
| 154 | DDR2 | NM_006182 | 12821 | c.313C>A | p.R105S | Unclassified | Missense_Mutation |
| 155 | DDR2 | NM_006182 | 140390 | c.1598C>A | p.T533K | Unclassified | Missense_Mutation |
| 156 | DDR2_ENST00000367922 | ENST00000367922 | 140389 | c.1598C>A | p.T533K | Unclassified | Missense_Mutation |
| 157 | EGFR | NM_005228 | 236670 | c.1476C>A | p.5492R | Gain of function | Missense_Mutation |
| 158 | EGFR | NM_005228 | 41905 | c.2092G>A | p.A698T | Gain of function | Missense_Mutation |
| 159 | EGFR | NM_005228 | 28508 | c.2104G>T | p.A702S | Gain of function | Missense_Mutation |
| 160 | EGFR | NM_005228 | 13427 | c.2126A>C | p.E709A | Gain of function | Missense_Mutation |
| 161 | EGFR | NM_005228 | 13009 | c.2126A>G | p.E709G | Gain of function | Missense_Mutation |
| 162 | EGFR | NM_005228 | 12988 | c.2125G>A | p.E709K | Gain of function | Missense_Mutation |
| 163 | EGFR | NM_005228 | 12371 | c.2126A>T | p.E709V | Gain of function | Missense_Mutation |
| 164 | EGFR | NM_005228 | 41603 | c.2134T>C | p.F712L | Gain of function | Missense_Mutation |
| 165 | EGFR | NM_005228 | 28601 | c.2135T>C | p.F712S | Gain of function | Missense_Mutation |
| 166 | EGFR | NM_005228 | 6239 | c.2156G>C | p.G719A | Gain of function | Missense_Mutation |
| 167 | EGFR | NM_005228 | 6239 | c.2156G>C | p.G719A | Gain of function | Missense_Mutation |
| 168 | EGFR | NM_005228 | 6239 | c.2156G>C | p.G719A | Gain of function | Missense_Mutation |
| 169 | EGFR | NM_005228 | 18441 | c.2154_2155GG>π | p.G719C | Gain of function | Missense_Mutation |
| 170 | EGFR | NM_005228 | 18441 | c.2154_2155GG>π | P.G719C | Gain of function | Missense_Mutation |
| 171 | EGFR | NM_005228 | 18441 | c.2154_2155GG>π | P.G719C | Gain of function | Missense_Mutation |
| 172 | EGFR | NM_005228 | 6253 | c.2155G>T | p.G719C | Gain of function | Missense_Mutation |
| 173 | EGFR | NM_005228 | 6253 | c.2155G>T | p.G719C | Gain of function | Missense_Mutation |
| 174 | EGFR | NM_005228 | 6253 | c.2155G>T | p.G719C | Gain of function | Missense_Mutation |
| 175 | EGFR | NM_005228 | 18425 | c.2156G>A | p.G719D | Gain of function | Missense_Mutation |
| 176 | EGFR | NM_005228 | 18425 | c.2156G>A | p.G719D | Gain of function | Missense_Mutation |
| 177 | EGFR | NM_005228 | 18425 | c.2156G>A | p.G719D | Gain of function | Missense_Mutation |
| 178 | EGFR | NM_005228 | 6252 | c.2155G>A | p.G719S | Gain of function | Missense_Mutation |
| 179 | EGFR | NM_005228 | 6252 | c.2155G>A | p.G719S | Gain of function | Missense_Mutation |
| 180 | EGFR | NM_005228 | 6252 | c.2155G>A | p.G719S | Gain of function | Missense_Mutation |
| 181 | EGFR | NM_005228 | 28510 | c.2162G>C | p.G721A | Gain of function | Missense_Mutation |
| 182 | EGFR | NM_005228 | 22992 | c.2161G>A | p.G721S | Gain of function | Missense_Mutation |
| 183 | EGFR | NM_005228 | 13979 | c.2170G>A | p.G724S | Gain of function | Missense_Mutation |
| 184 | EGFR | NM_005228 | 28511 | c.2108T>C | p.L703P | Gain of function | Missense_Mutation |
| 185 | EGFR | NM_005228 | 12373 | c.2159C>T | p.S720F | Gain of function | Missense_Mutation |
| 186 | EGFR | NM_005228 | 26509 | c.2227G>A | p.A743T | Gain of function | Missense_Mutation |
| 187 | EGFR | NM_005228 | 27042 | c.2282A>G | p.D761G | Gain of function | Missense_Mutation |
| 188 | EGFR | NM_005228 | 21984 | c.2281G>T | p.D761Y | Gain of function | Missense_Mutation |
| 189 | EGFR | NM_005228 | 13184 | c.2236G>A | p.E746K | Gain of function | Missense_Mutation |
| 190 | EGFR | NM_005228 | 13182 | c.2203G>A | p.G735S | Gain of function | Missense_Mutation |
| 191 | EGFR | NM_005228 | 85993 | c.2260A>6 | p.K754E | Gain of function | Missense_Mutation |
| 192 | EGFR | NM_005228 | 24267 | c.2239 2240Π>CC | p.L747P | Gain of function | Missense_Mutation |
| 193 | EGFR | NM_005228 | 26704 | c.2240T>C | p.L747S | Gain of function | Missense_Mutation |
| 194 | EGFR | NM_005228 | 13181 | c.2198C>T | p.P733L | Gain of function | Missense_Mutation |
| 195 | EGFR | NM_005228 | 53194 | c.2197C>T | p.P733S | Gain of function | Missense_Mutation |
| 196 | EGFR | NM_005228 | 17570 | c.2222C>T | p.P741L | Gain of function | Missense_Mutation |
| 197 | EGFR | NM_005228 | 6268 | c.2257C>T | p.P753S | Gain of function | Missense_Mutation |
| 198 | EGFR | NM_005228 | 29274 | c.2254T>C | p.5752P | Gain of function | Missense_Mutation |
| 199 | EGFR | NM_005228 | 13185 | c.2252C>T | p.T751I | Gain of function | Missense_Mutation |
| 200 | EGFR | NM_005228 | 27041 | c.2213T>G | p.V738G | Gain of function | Missense_Mutation |
| 201 | EGFR | NM_005228 | 13432 | c.2193G>A | p.W731* | Gain of function | Nonsense_Mutation |
| 202 | EGFR | NM_005228 | 6223 | c.2235_2249del15 | p.E746_A750del | Gain of function | In_Frame_Del |
| 203 | EGFR | NM_005228 | 6223 | c.2235_2249del15 | p.E746_A750del | Gain of function | In_Frame_Del |
| 204 | EGFR | NM_005228 | 6223 | c.2235_2249del15 | p.E746_A750del | Gain of function | In_Frame_Del |
| 205 | EGFR | NM_005228 | 6225 | c.2236_2250del15 | p.E746_A750del | Gain of function | In_Frame_Del |
| 206 | EGFR | NM_005228 | 6225 | c.2236_2250del15 | p.E746_A750del | Gain of function | In_Frame_Del |
| 207 | EGFR | NM_005228 | 6225 | c.2236_2250del15 | p.E746_A750del | Gain of function | In_Frame_Del |
| 208 | EGFR | NM_005228 | 28517 | c.2235_2246del12 | p.E746_E749del | Gain of function | In_Frame_Del |
| 209 | EGFR | NM_005228 | 28517 | c.2235_2246del12 | p.E746_E749del | Gain of function | In_Frame_Del |
| 210 | EGFR | NM_005228 | 28517 | c.2235_2246del12 | p.E746_E749del | Gain of function | In_Frame_Del |
| 211 | EGFR | NM_005228 | 12367 | c.2237_2254del18 | p.E746_S752>A | Gain of function | In_Frame_Del |
| 212 | EGFR | NM_005228 | 12367 | c.2237_2254del18 | p.E746_S752>A | Gain of function | In_Frame_Del |
| 213 | EGFR | NM_005228 | 12367 | c.2237_2254del18 | p.E746_S752>A | Gain of function | In_Frame_Del |
| 214 | EGFR | NM_005228 | 6220 | c.2238_2255del18 | p.E746_S752>D | Gain of function | In_Frame_Del |
| 215 | EGFR | NM_005228 | 6220 | c.2238_2255del18 | p.E746_S752>D | Gain of function | In_Frame_Del |
| 216 | EGFR | NM_005228 | 6220 | c.2238_2255del18 | p.E746_S752>D | Gain of function | In_Frame_Del |
| 217 | EGFR | NM_005228 | 12384 | c.2237_2255>T | p.E746_S752>V | Gain of function | In_Frame_Del |
| 218 | EGFR | NM_005228 | 12384 | c.2237_2255>T | p.E746_S752>V | Gain of function | In_Frame_Del |
| 219 | EGFR | NM_005228 | 12384 | c.2237_2255>T | p.E746_S752>V | Gain of function | In_Frame_Del |
| 220 | EGFR | NM_005228 | 133189 | c.2236_2256del21 | pE746_S752del | Gain of function | In_Frame_Del |
| 221 | EGFR | NM_005228 | 133189 | c.2236_2256del21 | pE746_S752del | Gain of function | In_Frame_Del |
| 222 | EGFR | NM_005228 | 133189 | c.2236_2256del21 | pE746_S752del | Gain of function | In_Frame_Del |
| 223 | EGFR | NM_005228 | 12678 | c.2237_2251del15 | p.E746_T751>A | Gain of function | In_Frame_Del |
| 224 | EGFR | NM_005228 | 12678 | c.2237_2251del15 | p.E746_T751>A | Gain of function | In_Frame_Del |
| 225 | EGFR | NM_005228 | 12678 | c2237_2251del15 | p.E746_T751>A | Gain of function | In_Frame_Del |
| 226 | EGFR | NM_005228 | 12386 | c.2237_2252>T | p.E746_T751>V | Gain of function | In_Frame_Del |
| 227 | EGFR | NM_005228 | 12386 | c.2237_2252>T | p.E746_T751>V | Gain of function | In_Frame_Del |
| 228 | EGFR | NM_005228 | 12386 | c.2237_2252>T | p.E746_T751>V | Gain of function | In_Frame_Del |
| 229 | EGFR | NM_005228 | 12728 | c.2236_2253del18 | p.E746 T7SIdel | Gain of function | In_Frame_Del |
| 230 | EGFR | NM_005228 | 12728 | c.2236_2253del18 | p.E746_T751de! | Gain of function | In_Frame_Del |
| 231 | EGFR | NM_005228 | 12728 | c.2236_2253del18 | p.E746_T751del | Gain of function | In_Frame_Del |
| 232 | EGFR | NM_005228 | 26038 | c.2233_2247del15 | p.K745_E749del | Gain of function | In . Frame . Del |
| 233 | EGFR | NM_005228 | 26038 | c.2233_2247del15 | p.K745_E749del | Gain of function | In_Frame_Del |
| 234 | EGFR | NM_005228 | 26038 | c.2233_2247del15 | p.K745_E749de! | Gain of function | In_Frame_Del |
| 235 | EGFR | NM_005228 | 12382 | c.2239_2248TTAAGAGAAG>C | p.L747 _A750>P | Gain of function | In_Frame_Del |
| 236 | EGFR | NM_005228 | 12382 | c.2239_2248TTAAGAGAAG>C | p.L747 _A750>P | Gain of function | In_Frame_Del |
| 237 | EGFR | NM_005228 | 12382 | c.2239_2248TTAAGAGAAG>C | p.L747 _A750>P | Gain of function | In_Frame_Del |
| 238 | EGFR | NM_005228 | 6218 | c.2239_2247del9 | p.L747_E749del | Gain of function | In_Frame_Del |
| 239 | EGFR | NM_005228 | 6218 | c.2239_2247del9 | p.L747_E749del | Gain of function | In_Frame_Del |
| 240 | EGFR | NM_005228 | 6218 | c.2239 2247del9 | p.L747_E749del | Gain of function | In_Frame_Del |
| 241 | EGFR | NM_005228 | 12370 | c.2240_2257del18 | p.L747_P753>S | Gain of function | In_Frame_Del |
| 242 | EGFR | NM_005228 | 12370 | c.2240_2257del18 | p.L747_P753>S | Gain of function | In_Frame_Del |
| 243 | EGFR | NM_005228 | 12370 | c.2240_2257del18 | p.L747_P753>S | Gain of function | In_Frame_Del |
| 244 | EGFR | NM_005228 | 133197 | c.2239_2257>T | p.L747_P753>S | Gain of function | In_Frame_Del |
| 245 | EGFR | NM_005228 | 133197 | c.2239_2257>T | p.L747_P753>S | Gain of function | In_Frame_Del |
| 246 | EGFR | NM_005228 | 133197 | c.2239_2257>T | p.L747_P753>S | Gain of function | In_Frame_Del |
| 247 | EGFR | NM_005228 | 6255 | c.2239_2256del18 | p.L747_S752del | Gain of function | In_Frame_Del |
| 248 | EGFR | NM_005228 | 6255 | c.2239_2256del18 | p.L747_S752del | Gain of function | In_Frame_Del |
| 249 | EGFR | NM_005228 | 6255 | c.2239_2256del18 | p.L747_S752del | Gain of function | I n Frame Del |
| 250 | EGFR | NM_005228 | 12383 | c.2239_2251>C | p.L747 _T751>P | Gain of function | In_Frame_Del |
| 251 | EGFR | NM_005228 | 12383 | c.2239_2251>C | p.L747 _T751>P | Gain of function | In_Frame_Del |
| 252 | EGFR | NM_005228 | 12383 | c.2239_2251>C | p.L747 _T751>P | Gain of function | In_Frame_Del |
| 253 | EGFR | NM_005228 | 6210 | c.2240_2251del12 | p.L747 _T751>S | Gain of function | In_Frame_Del |
| 254 | EGFR | NM_005228 | 6210 | c.2240_2251del12 | p.L747 _T751>S | Gain of function | In_Frame_Del |
| 255 | EGFR | NM_005228 | 6210 | c.2240_2251del12 | p.L747 _T751>S | Gain of function | In_Frame_Del |
| 256 | EGFR | NM_005228 | 12369 | c.2240_2254del15 | p.L747 _T751del | Gain of function | In_Frame_Del |
| 257 | EGFR | NM_005228 | 12369 | c.2240_2254del15 | p.L747 _T751del | Gain of function | In_Frame_Del |
| 258 | EGFR | NM_005228 | 12369 | c.2240_2254del15 | p.L747 _T751del | Gain of function | In_Frame_Del |
| 259 | EGFR | NM_005228 | 23571 | c.2238_2252del15 | p.L747 _T751del | Gain of function | In_Frame_Del |
| 260 | EGFR | NM_005228 | 23571 | c.2238_2252del15 | p.L747 _T751del | Gain of function | In_Frame_Del |
| 261 | EGFR | NM_005228 | 23571 | c.223S_2252del15 | p.L747 _T751del | Gain of function | In_Frame_Del |
| 262 | EGFR | NM_005228 | 6254 | c2239_2253del15 | pL747 _T751dcl | Gain of function | In_Frame_Del |
| 263 | EGFR | NM_005228 | 6254 | c.2239_2253del15 | p.L747 _T751del | Gain of function | In_Frame_Del |
| 264 | EGFR | NM_005228 | 6254 | c.2239_2253del15 | p.L747 _T751del | Gain of function | In_Frame_Del |
| 265 | EGFR | NM_005228 | 13556 | c.22S3₋2276del24 | p.S752_I759del | Gain of function | In_Frame_Del |
| 266 | EGFR | NM_005228 | 13556 | c.22S3₋2276del24 | p.S752_I759del | Gain of function | In_Frame_Del |
| 267 | EGFR | NM_005228 | 13556 | c.22S3₋2276del24 | p.S752_I759del | Gain of function | In_Frame_Del |
| 268 | EGFR | NM_005228 | 6256 | c.2254_2277del24 | p.S752_I759del | Gain of function | In_Frame_Del |
| 269 | EGFR | NM_005228 | 6256 | c.2254_2277del24 | p.S752_I759del | Gain of function | In_Frame_Del |
| 270 | EGFR | NM_005228 | 6256 | c.2254_2277del24 | p.S752_I759del | Gain of function | In_Frame_Del |
| 271 | EGFR | NM_005228 | 96856 | c.2252_2276>A | p.T751_I759>N | Gain of function | In_Frame_Del |
| 272 | EGFR | NM_005228 | 96856 | c.2252_2276>A | p.T751_I759>N | Gain of function | In_Frame_Del |
| 273 | EGFR | NM_005228 | 96856 | c.2252_2276>A | p.T751_I759>N | Gain of function | In_Frame_Del |
| 274 | EGFR | NM_005228 | 133207 | c.2252_2275del24 | p.T751_I759del | Gain of function | In_Frame_Del |
| 275 | EGFR | NM_005228 | 133207 | c.2252_2275del24 | p.T751_I759del | Gain of function | In_Frame_Del |
| 276 | EGFR | NM_005228 | 133207 | c.2252_2275del24 | p.T751_I759del | Gain of function | In_Frame_Del |
| 277 | EGFR | NM_005228 | 26445 | c.2300C>T | p.A767V | Gain of function | Missense_Mutation |
| 278 | EGFR | NM_005228 | 22954 | c.2324G>A | p.C775Y | Gain of function | Missense_Mutation |
| 279 | EGFR | NM_005228 | 14068 | c.2308G>A | p.D770N | Gain of function | Missense_Mutation |
| 280 | EGFR | NM_005228 | 22951 | c.2384T>C | p.F795S | Gain of function | Missense_Mutation |
| 281 | EGFR | NM_005228 | 13007 | c.2335_2336GG>Π | p.G779F | Gain of function | Missense_Mutation |
| 282 | EGFR | NM_005228 | 27568 | c.2387G>C | p.G796A | Gain of function | Missense_Mutation |
| 283 | EGFR | NM_005228 | 133565 | c.2387G>A | p.G796D | Gain of function | Missense_Mutation |
| 284 | EGFR | NM_005228 | 20891 | c.2386G>A | p.G796S | Gain of function | Missense_Mutation |
| 285 | EGFR | NM_005228 | 13005 | c.2318A>T | p.H773L | Gain of function | Missense_Mutation |
| 286 | EGFR | NM_005228 | 13433 | c.2318A>G | p.H773R | Gain of function | Missense_Mutation |
| 287 | EGFR | NM_005228 | 13190 | c.2375T>C | P.L792P | Gain of function | Missense_Mutation |
| 288 | EGFR | NM_005228 | 6226 | c.2326C>T | p.R776C | Gain of function | Missense_Mutation |
| 289 | EGFR | NM_005228 | 22940 | c.2327G>A | p.R776H | Gain of function | Missense_Mutation |
| 290 | EGFR | NM_005228 | 6241 | c.2303G>T | p.57681 | Gain of function | Missense_Mutation |
| 291 | EGFR | NM_005228 | 13189 | c.2351C>T | p.5784F | Gain of function | Missense_Mutation |
| 292 | EGFR | NM_005228 | 28513 | c.2350T>C | p.5784P | Gain of function | Missense_Mutation |
| 293 | EGFR | NM_005228 | 6240 | c.2369C>T | p.T790M | Gain of function | Missense_Mutation |
| 294 | EGFR | NM_005228 | 6240 | c.2369C>T | p.T790M | Gain of function | Missense_Mutation |
| 295 | EGFR | NM_{_}005228 | 6240 | c.2369C>T | p.T790M | Gain of function | Missense_Mutation |
| 296 | EGFR | NM_{_}005228 | 6240 | c.2369C>T | p.T790M | Gain of function | Missense_Mutation |
| 297 | EGFR | NM_{_}005228 | 6240 | c.2369C>T | p.T790M | Gain of function | Missense_Mutation |
| 298 | EGFR | NM_{_}005228 | 6240 | c.2369C>T | p.T790M | Gain of function | Missense_Mutation |
| 299 | EGFR | NM_005228 | 28603 | c.2293G>A | p.V765M | Gain of function | Missense_Mutation |
| 300 | EGFR | NM_{_}005228 | 6242 | c.2305G>T | p.V769L | Gain of function | Missense_Mutation |
| 301 | EGFR | NM_005228 | 13006 | c.2320G>A | p.V774M | Gain of function | Missense_Mutation |
| 302 | EGFR | NM_005228 | 27110 | c.2356G>A | p.V786M | Gain of function | Missense_Mutation |
| 303 | EGFR | NM_005228 | 12378 | c.2310_2311insGGT | pD770_N771insG | Gain of function | n_Frame_Ins |
| 304 | EGFR | NM_005228 | 12378 | c.2310_2311insGST | p.D770_N771insG | Gain of function | In_Frame_Ins |
| 305 | EGFR | NM_005228 | 12378 | c.2310_2311insGGT | p.D770_N771insG | Gain of function | In_Frame_Ins |
| 306 | EGFR | NM_005228 | 48922 | c.2311_2312insGCGTGGACA | p.D770_N771insSVE | Gain of function | In_Frame_Ins |
| 307 | EGFR | NM_005228 | 48922 | c.2311_2312insGCGTGGACA | p.D770_N771insSVE | Gain of function | In_Frame_Ins |
| 308 | EGFR | NM_005228 | 48922 | c.2311_2312insGCGTGGACA | p.D770_N771insSVC | Gain of function | n_Frame_Ins |
| 309 | EGFR | NM_005228 | 12427 | c.2308_2309insGΠ | p.D770>GY | Gain of function | n_Frame_Ins |
| 310 | EGFR | NM_005228 | 12427 | c.2308_2309insGΠ | p.D770>GY | Gain of function | In Frame Ins |
| 311 | EGFR | NM_005228 | 12427 | c.2308_2309insGΠ | p.D770>GY | Gain of function | In_Frame_Ins |
| 312 | EGFR | NM_005228 | 12377 | c.2319_2320insCAC | p.H773_V774insH | Gain of function | In_Frame_Ins |
| 313 | EGFR | NM_005228 | 12377 | c.2319_2320insCAC | p.H773 V774insH | Gain of function | In_Frame_Ins |
| 314 | EGFR | NM_005228 | 12377 | c.2319_2320insCAC | p.H773_V774insH | Gain of function | In_Frame_Ins |
| 315 | EGFR | NM_005228 | 12675 | c.2575G>A | p.A859T | Gain of function | Missense_Mutation |
| 316 | EGFR | NM_005228 | 13197 | c.2590G>A | p.A864T | Gain of function | Missense_Mutation |
| 317 | EGFR | NM_005228 | 13008 | c.2612C>G | p.A871G | Gain of function | Missense_Mutation |
| 318 | EGFR | NM_005228 | 28605 | c.2611G>A | p.A871T | Gain of function | Missense_Mutation |
| 319 | EGFR | NM_005228 | 28607 | c.2603A>G | p.E868G | Gain of function | Missense_Mutation |
| 320 | EGFR | NM_005228 | 14070 | c.2588G>A | p.G863D | Gain of function | Missense_Mutation |
| 321 | EGFR | NM_005228 | 13199 | c.2618G>A | p.G873E | Gain of function | Missense_Mutation |
| 322 | EGFR | NM_005228 | 26438 | c.2620G>A | p.G874S | Gain of function | Missense_Mutation |
| 323 | EGFR | NM_005228 | 33725 | c.2609A>G | p.H870R | Gain of function | Missense_Mutation |
| 324 | EGFR | NM_005228 | 53292 | c.2608C>T | p.H870Y | Gain of function | Missense_Mutation |
| 325 | EGFR | NM_005228 | 26129 | c.2572C>T | p.L858L | Gain of function | Synonymous_Mutation |
| 326 | EGFR | NM_005228 | 12366 | c.2572C>A | p.L858M | Gain of function | Missense_Mutation |
| 327 | EGFR | NM_005228 | 12429 | c.2573_2574TG>GT | p.L858R | Gain of function | Missense_Mutation |
| 328 | EGFR | NM_005228 | 12429 | c.2573_2574TG>GT | p.L858R | Gain of function | Missense_Mutation |
| 329 | EGFR | NM_005228 | 12429 | c.2573_2574TG>GT | p.L858R | Gain of function | Missense_Mutation |
| 330 | EGFR | NM_005228 | 6224 | c.2573T>G | p.L858R | Gain of function | Missense_Mutation |
| 331 | EGFR | NM_005228 | 6224 | c.2573T>G | p.L858R | Gain of function | Missense_Mutation |
| 332 | EGFR | NM_005228 | 6224 | c.2573T>G | p.L858R | Gain of function | Missense_Mutation |
| 333 | EGFR | NM_005228 | 6213 | c.2582T>A | p.L861Q | Gain of function | Missense_Mutation |
| 334 | EGFR | NM_005228 | 6213 | c.2582T>A | p.L861Q | Gain of function | Missense_Mutation |
| 335 | EGFR | NM_005228 | 12374 | c.2582T>G | p.L861R | Gain of function | Missense_Mutation |
| 336 | EGFR | NM_005228 | 12374 | c.2582T>G | P.L861R | Gain of function | Missense_Mutation |
| 337 | ERBB2 | NM_004448 | 13170 | c.2305G>C | p.D769H | Gain of function | Missense_Mutation |
| 338 | ERBB2 | NM_004448 | 51317 | c.2301C>G | p.I767M | Gain of function | Missense_Mutation |
| 339 | ERBB2 | NM_004448 | 683 | c.2263_2264TT>CC | P.L755P | Gain of function | Missense_Mutation |
| 340 | ERBB2 | NM_004448 | 14060 | c.2264T>C | P.L755S | Gain of function | Missense_Mutation |
| 341 | ERBB2 | NM_004448 | 18609 | c.2327G>T | p.G776V | Gain of function | Missense_Mutation |
| 342 | ERBB2 | NM_004448 | 35496 | c.2330T>C | p.V777A | Gain of function | Missense_Mutation |
| 343 | ERBB2 | NM_004448 | 14062 | c.2329G>T | p.V777L | Gain of function | Missense_Mutation |
| 344 | ERBB2 | NM_004448 | 12552 | c.2326_2327insTTT | p.G776>VC | Gain of function | In_Frame_Ins |
| 345 | ERBB2 | NM_004448 | 12552 | c.2326_2327insTTT | p.6776>VC | Gain of function | In_Frame_Ins |
| 346 | ERBB2 | NM_004448 | 12553 | c.2326_2327insTGT | p.G776>VC | Gain of function | In_Frame_Ins |
| 347 | ERBB2 | NM_004448 | 12553 | c.2326_2327insTGT | p.G776>VC | Gain of function | In_Frame_Ins |
| 348 | ERBB2 | NM_004448 | 26681 | c.2333_2334insGGG | p.G778_S779insG | Gain of function | In_Frame_Ins |
| 349 | ERBB2 | NM_004448 | 26681 | c.2333_2334insGGG | p.G778_S779insG | Gain of function | In Frame Ins |
| 350 | ERBB2 | NM_004448 | 21985 | c.2632C>T | p.H878Y | Gain of function | Missense_Mutation |
| 351 | ERBB2 | NM_004448 | 14065 | c.2524G>A | p.V852I | Gain of function | Missense_Mutation |
| 352 | ERBB4 | NM_005235 | 95705 | c.421+1G>T | p.? | Gain of function. | N/A |
| 353 | ERBB4 | NM_005235 | 48364 | c.1784A>T | P.D595V | Gain of function. | Missense_Mutation |
| 354 | ERBB4 | NM_005235 | 131772 | c.1825G>A | p.D6D9N | Gain of function. | Missense_Mutation |
| 355 | ERBB4 | NM_005235 | 131765 | c.949G>A | p.E317K | Gain of function. | Missense_Mutation |
| 356 | ERBB4 | NM_005235 | 170797 | c.1748T>A | p.F583Y | Gain of function. | Missense_Mutation |
| 357 | ERBB4 | NM_005235 | 108015 | c.2806G>A | p.G936R | Gain of function. | Missense_Mutation |
| 358 | ERBB4 | NM_005235 | 160825 | c.885T>G | p.H29SQ | Gain of function. | Missense_Mutation |
| 359 | ERBB4 | NM_005235 | 48363 | c.1853A>C | p.H618P | Gain of function. | Missense_Mutation |
| 360 | ERBB4 | NM_005235 | 96313 | c.1852C>T | p.H618Y | Gain of function. | Missense_Mutation |
| 361 | ERBB4 | NM_005235 | 131764 | c.939G>A | p.m313I | Gain of function. | Missense_Mutation |
| 362 | ERBB4 | NM_005235 | 48369 | c.542A>G | p.N181S | Gain of function. | Missense_Mutation |
| 363 | ERBB4 | NM_005235 | 138342 | c.1856C>T | p.P619L | Gain of function. | Missense_Mutation |
| 364 | ERBB4 | NM_005235 | 48366 | c.916C>A | p.R306S | Gain of function. | Missense_Mutation |
| 365 | ERBB4 | NM_005235 | 232263 | c.1835G>A | p.R612Q | Gain of function. | Missense_Mutation |
| 366 | ERBB4 | NM_005235 | 12833 | c.908C>A | p.S303Y | Gain of function. | Missense_Mutation |
| 367 | ERBB4 | NM_005235 | 110095 | c.1022C>T | p.S341L | Gain of function. | Missense_Mutation |
| 368 | ERBB4 | NM_005235 | 20392 | c.419C>T | p.T140I | Gain of function. | Missense_Mutation |
| 369 | ERBB4 | NM_005235 | 48368 | c.731C>G | p.T244R | Gain of function. | Missense_Mutation |
| 370 | ERBB4 | NM_005235 | 209862 | c.803A>G | p.Y268C | Gain of function. | Missense_Mutation |
| 371 | ERBB4 | NM_005235 | 48367 | c.854A>G | p.Y285C | Gain of function. | Missense_Mutation |
| 372 | FBXW7 | NM_033632.1 | 22971 | c.832C>T | p.R278* | Loss of Function | Nonsense_Mutation |
| 373 | FBXW7 | NM_033632.1 | 22973 | c.1177C>T | p.R393^{∗} | Loss of Function | Nonsense_Mutation |
| 374 | FBXW7 | NM_033632.1 | 22932 | c.1393C>T | p.R465C | Loss of Function | Missense_Mutation |
| 375 | FBXW7 | NM_033632.1 | 22965 | c.1394G>A | p.R465H | Loss of Function | Missense_Mutation |
| 376 | FBXW7 | NM_033632.1 | 33762 | c.1394G>T | p.R465L | Loss of Function | Missense_Mutation |
| 377 | FBXW7 | NM_033632.1 | 133115 | c.1393_1394CG>TA | p.R465Y | Loss of Function | Missense_Mutation |
| 378 | FBXW7 | NM_033632.1 | 22975 | c.1513C>T | P.R505C | Loss of Function | Missense_Mutation |
| 379 | FBXW7 | NM_033632.1 | 99604 | c.1513C>G | p.R505G | Loss of Function | Missense_Mutation |
| 380 | FBXW7 | NM_033632.1 | 25812 | c.1514G>A | P.R505H | Loss of Function | Missense_Mutation |
| 381 | FBXW7 | NM_033632.1 | 23000 | c.1514G>T | P.R505L | Loss of Function | Missense_Mutation |
| 382 | FBXW7 | NM_033632.1 | 22979 | c.1745C>T | p.5582L | Loss of Function | Missense_Mutation |
| 383 | FBXW7 | NM_033632.1 | 27055 | c.1510G>A | p.V504I | Loss of Function | Missense_Mutation |
| 384 | FBXW7_ENST00000281708 | ENST00000281708 | 170727 | c.1393C>T | p.R465C | Loss of Function | Missense_Mutation |
| 385 | FBXW7_ENST00000281708 | ENST00000281708 | 117310 | c.1394G>A | p.R465H | Loss of Function | Missense_Mutation |
| 386 | FBXW7_ENST00000281708 | ENST00000281708 | 108572 | c.1513C>T | p.R505C | Loss of Function | Missense_Mutation |
| 387 | FBXW7_ENST00000281708 | ENST00000281708 | 99606 | c.1513C>G | P.R505G | Loss of Function | Missense_Mutation |
| 388 | FBXW7_ENST00000534231 | ENST00000534231 | 170726 | c.676C>T | p.R226C | Loss of Function | Missense_Mutation |
| 389 | FBXW7_ENST00000534231 | ENST00000534231 | 117309 | c.677G>A | p.R226H | Loss of Function | Missense_Mutation |
| 390 | FBXW7_ENST00000534231 | ENST00000534231 | 108571 | c.796C>T | p.R266C | Loss of Function | Missense_Mutation |
| 391 | FBXW7_ENST00000534231 | ENST00000534231 | 99605 | c.796C>G | p.R266G | Loss of Function | Missense_Mutation |
| 392 | FBXW7_NM_018315_2 | NM_018315.2 | 170725 | c.1153C>T | p.R385C | Loss of Function | Missense_Mutation |
| 393 | FBXW7_NM_018315_2 | NM_018315.2 | 117308 | c.1154G>A | p.R385H | Loss of Function | Missense_Mutation |
| 394 | FBXW7_NM_018315_2 | NM_018315.2 | 74637 | c.1273C>T | p.R425C | Loss of Function | Missense_Mutation |
| 395 | FBXW7_NM_018315_2 | NM_018315.2 | 99603 | c.1273C>G | p.R425G | Loss of Function | Missense_Mutation |
| 396 | FGFR1 | NM_000604 | 98903 | c.397G>A | p.D133N | Unclassified | Missense_Mutation |
| 397 | FGFR1 | NM_000604 | 187237 | c.448C>T | p.P1505 | Unclassified | Missense_Mutation |
| 398 | FGFR1 | NM_000604 | 12834 | c.754C>A | p.P252T | Unclassified | Missense_Mutation |
| 399 | FGFR1 | NM_000604 | 601 | c.374C>T | P.S125L | Unclassified | Missense_Mutation |
| 400 | FGFR1 | NM_000604 | 48380 | c.422C>G | p.T141R | Unclassified | Missense_Mutation |
| 401 | FGFR1_ENST00000425967 | ENST00000425967 | 98901 | c.130G>A | p.D44N | Unclassified | Missense_Mutation |
| 402 | FGFR1_ENST00000425967 | ENST00000425967 | 187239 | c.181C>T | p.P61S | Unclassified | Spice_Site |
| 403 | FGFR1_ENST00000447712 | ENST00000447712 | 98902 | c.397G>A | p.D133N | Unclassified | Missense_Mutation |
| 404 | FGFR2 | NM_000141.2 | 36906 | c.1144T>C | p.C382R | Gain of function | Missense_Mutation |
| 405 | FGFR2 | NM_000141.2 | 36901 | c.929A>G | p.K310R | Gain of function | Missense_Mutation |
| 406 | FGFR2 | NM_000141.2 | 36902 | c.1647T>G | p.N549K | Gain of function | Missense_Mutation |
| 407 | FGFR2 | NM_000141.2 | 36912 | c.1647T>A | p.N549K | Gain of function | Missense_Mutation |
| 408 | FGFR2 | NM_000141.2 | 49170 | c.758C>G | p.P253R | Gain of function | Missense_Mutation |
| 409 | FGFR2 | NM_000141.2 | 36903 | c.755C>G | p.S252W | Gain of function | Missense_Mutation |
| 410 | FGFR2 | NM_000141.2 | 36904 | c.1124A>G | p.Y375C | Gain of function | Missense_Mutation |
| 411 | FGFR3 | NM_000142 | 722 | c.1107G>T | p.A369A | Gain of function | Synonymous_Mutation |
| 412 | FGFR3 | NM_000142 | 721 | c.1172C>A | p.A391E | Gain of function | Missense_Mutation |
| 413 | FGFR3 | NM_000142 | 29438 | c.1921G>A | p.D641N | Gain of function | Missense_Mutation |
| 414 | FGFR3 | NM_000142 | 27139 | c.2349_2350delAG | p.D785fs*23 | Gain of function | Frame_Shift_Ins |
| 415 | FGFR3 | NM_000142 | 724 | c.1150T>C | p.F384L | Gain of function | Missense_Mutation |
| 416 | FGFR3 | NM_000142 | 716 | c.1108G>T | p.G370C | Gain of function | Missense_Mutation |
| 417 | FGFR3 | NM_000142 | 24842 | c.1138G>A | p.G380R | Gain of function | Missense_Mutation |
| 418 | FGFR3 | NM_000142 | 24802 | c.2089G>T | p.G697C | Gain of function | Missense_Mutation |
| 419 | FGFR3 | NM_000142 | 29446 | c.753C>T | p.H251H | Gain of function | Synonymous_Mutation |
| 420 | FGFR3 | NM_000142 | 719 | c.1948A>G | p.K650E | Gain of function | Missense_Mutation |
| 421 | FGFR3 | NM_000142 | 720 | c.1949A>T | p.K650M | Gain of function | Missense_Mutation |
| 422 | FGFR3 | NM_000142 | 726 | c.1948A>C | p.K650Q | Gain of function | Missense_Mutation |
| 423 | FGFR3 | NM_000142 | 731 | c.1949A>C | p.K650T | Gain of function | Missense_Mutation |
| 424 | FGFR3 | NM_000142 | 729 | c.2381_2381T>GA | p.L794fs*23 | Gain of function | Frame_Shift_Ins |
| 425 | FGFR3 | NM_000142 | 725 | c.2382_2421+2del42 | p.P795fs*139 | Gain of function | Frame_Shift_Ins |
| 426 | FGFR3 | NM_000142 | 714 | c.742C>T | p.R248C | Gain of function | Missense_Mutation |
| 427 | FGFR3 | NM_000142 | 715 | c.746C>G | P.S249C | Gain of function | Missense_Mutation |
| 428 | FGFR3 | NM_000142 | 17461 | c.1111A>T | P.S371C | Gain of function | Missense_Mutation |
| 429 | FGFR3 | NM_000142 | 718 | c.1118A>G | p.Y373C | Gain of function | Missense_Mutation |
| 430 | KRAS | NM_004985 | 87301 | c.33_34insGGAGCT | p.All_G12insGA | Gain of function | In_Frame_Ins |
| 431 | KRAS | NM_004985 | 510 | c.31G>C | p.A11P | Gain of function | Missense_Mutation |
| 432 | KRAS | NM_004985 | 511 | c.32C>T | p.A11V | Gain of function | Missense_Mutation |
| 433 | KRAS | NM_004985 | 19905 | c.436G>C | P.A146P | Gain of function | Missense_Mutation |
| 434 | KRAS | NM_004985 | 19404 | c.436G>A | P.A146T | Gain of function | Missense_Mutation |
| 435 | KRAS | NM_004985 | 19900 | c.437C>T | p.A146V | Gain of function | Missense_Mutation |
| 436 | KRAS | NM_004985 | 542 | c.53C>A | p.A18D | Gain of function | Missense_Mutation |
| 437 | KRAS | NM_004985 | 547 | c.176C>A | p.A59E | Gain of function | Missense_Mutation |
| 438 | KRAS | NM_004985 | 28518 | c.176C>G | p.A59G | Gain of function | Missense_Mutation |
| 439 | KRAS | NM_004985 | 546 | c.175G>A | p.A59T | Gain of function | Missense_Mutation |
| 440 | KRAS | NM_004985 | 12654 | c.30_31insGGA | p.G10_A11inG | Gain of function | In_Frame_Ins |
| 441 | KRAS | NM_004985 | 12655 | c.36_37insGGT | p.G12_G13insG | Gain of function | In_Frame_Ins |
| 442 | KRAS | NM_004985 | 522 | c.35G>C | p.G12A | Gain of function | Missense_Mutation |
| 443 | KRAS | NM_004985 | 522 | c.35G>C | p.G12A | Gain of function | Missense_Mutation |
| 444 | KRAS | NM_004985 | 522 | c.35G>C | p.G12A | Gain of function | Missense_Mutation |
| 445 | KRAS | NM_004985 | 516 | c.34G>T | p.G12C | Gain of function | Missense_Mutation |
| 446 | KRAS | NM_004985 | 516 | c.34G>T | p.G12C | Gain of function | Missense_Mutation |
| 447 | KRAS | NM_004985 | 516 | c.34G>T | P.G12C | Gain of function | Missense_Mutation |
| 448 | KRAS | NM_004985 | 14209 | c.35_36GT>AC | p.G12D | Gain of function | Missense_Mutation |
| 449 | KRAS | NM_004985 | 14209 | c.35_36GT>AC | P.G12D | Gain of function | Missense_Mutation |
| 450 | KRAS | NM_004985 | 14209 | c.35_36GT>AC | p.G12D | Gain of function | Missense_Mutation |
| 451 | KRAS | NM_004985 | 521 | c.35G>A | p.G12D | Gain of function | Missense_Mutation |
| 452 | KRAS | NM_004985 | 521 | c.35G>A | p.G12D | Gain of function | Missense_Mutation |
| 453 | KRAS | NM_004985 | 521 | c.35G>A | p.G12D | Gain of function | Missense_Mutation |
| 454 | KRAS | NM_004985 | 519 | c.35_36GT>AA | p.G12E | Gain of function | Missense_Mutation |
| 455 | KRAS | NM_004985 | 519 | c.35_36GT>AA | p.G12E | Gain of function | Missense_Mutation |
| 456 | KRAS | NM_004985 | 519 | c.35_36GT>AA | p.G12E | Gain of function | Missense_Mutation |
| 457 | KRAS | NM_004985 | 512 | c.34_35GG>TT | p.G12F | Gain of function | Missense_Mutation |
| 458 | KRAS | NM_004985 | 512 | c.34_35GG>TT | p.G12F | Gain of function | Missense_Mutation |
| 459 | KRAS | NM_004985 | 512 | c.34_35GG>TT | p.G12F | Gain of function | Missense_Mutation |
| 460 | KRAS | NM_004985 | 523 | c.36T>C | p.G12G | Gain of function | Synonymous_Mutation |
| 461 | KRAS | NM_004985 | 523 | c.36T>C | p.G12G | Gain of function | Synonymous_Mutation |
| 462 | KRAS | NM_004985 | 523 | c.36T>C | p.G12G | Gain of function | Synonymous_Mutation |
| 463 | KRAS | NM_004985 | 524 | c.36T>A | p.G12G | Gain of function | Synonymous_Mutation |
| 464 | KRAS | NM_004985 | 524 | c.36T>A | p.G12G | Gain of function | Synonymous_Mutation |
| 465 | KRAS | NM_004985 | 524 | c.36T>A | p.G12G | Gain of function | Synonymous_Mutation |
| 466 | KRAS | NM_004985 | 34144 | c.34_35GG>AT | p.G12I | Gain of function | Missense_Mutation |
| 467 | KRAS | NM_004985 | 34144 | c.34_35GG>AT | p.G12I | Gain of function | Missense_Mutation |
| 468 | KRAS | NM_004985 | 34144 | c.34_35GG>AT | p.G12I | Gain of function | Missense_Mutation |
| 469 | KRAS | NM_004985 | 514 | c.34_35GG>CT | p.G12L | Gain of function | Missense_Mutation |
| 470 | KRAS | NM_004985 | 514 | c.34_35GG>CT | p.G12L | Gain of function | Missense_Mutation |
| 471 | KRAS | NM_004985 | 514 | c.34_35GG>CT | p.G12L | Gain of function | Missense_Mutation |
| 472 | KRAS | NM_004985 | 518 | c.34G>C | p.G12R | Gain of function | Missense_Mutation |
| 473 | KRAS | NM_004985 | 518 | c.34G>C | p.G12R | Gain of function | Missense_Mutation |
| 474 | KRAS | NM_004985 | 518 | c.34G>C | p.G12R | Gain of function | Missense_Mutation |
| 475 | KRAS | NM_004985 | 517 | c.34G>A | p.G12S | Gain of function | Missense_Mutation |
| 476 | KRAS | NM_004985 | 517 | c.34G>A | p.G12S | Gain of function | Missense_Mutation |
| 477 | KRAS | NM_004985 | 517 | c.34G>A | p.G12S | Gain of function | Missense_Mutation |
| 478 | KRAS | NM_004985 | 515 | c.35_36GT>TC | p.G12V | Gain of function | Missense_Mutation |
| 479 | KRAS | NM_004985 | 515 | c.35_36GT>TC | p.G12V | Gain of function | Missense_Mutation |
| 480 | KRAS | NM_004985 | 515 | c.35_36GT>TC | p.G12V | Gain of function | Missense_Mutation |
| 481 | KRAS | NM_004985 | 520 | c.35G>T | p.G12V | Gain of function | Missense_Mutation |
| 482 | KRAS | NM_004985 | 520 | c.35G>T | p.G12V | Gain of function | Missense_Mutation |
| 483 | KRAS | NM_004985 | 520 | c.35G>T | p.G12V | Gain of function | Missense_Mutation |
| 484 | KRAS | NM_004985 | 25081 | c.34_35GG>TA | p.G12Y | Gain of function | Missense_Mutation |
| 485 | KRAS | NM_004985 | 25081 | c.34_35GG>TA | p.G12Y | Gain of function | Missense_Mutation |
| 486 | KRAS | NM_004985 | 25081 | c.34_35GG>TA | p.G12Y | Gain of function | Missense_Mutation |
| 487 | KRAS | NM_004985 | 219781 | c.39_40insGGC | p.G13_V14insG | Gain of function | In_Frame_Ins |
| 488 | KRAS | NM_004985 | 533 | c.38G>C | pG13A | Gain of function | Missense_Mutation |
| 489 | KRAS | NM_004985 | 533 | c.38G>C | p.G13A | Gain of function | Missense_Mutation |
| 390 | KRAS | NM_004985 | 533 | c.38G>C | p.G13A | Gain of function | Missense_Mutation |
| 391 | KRAS | NM_004985 | 527 | c.37G>T | p.G13C | Gain of function | Missense_Mutation |
| 492 | KRAS | NM_004985 | 527 | c.37G>T | p.G13C | Gain of function | Missense_Mutation |
| 393 | KRAS | NM_004985 | 527 | c.37G>T | p.G13C | Gain of function | Missense_Mutation |
| 494 | KRAS | NM_004985 | 531 | c.38_39GC>AT | p.G13D | Gain of function | Missense_Mutation |
| 495 | KRAS | NM_004985 | 531 | c.38_39GC>AT | p.G13D | Gain of function | Missense_Mutation |
| 496 | KRAS | NM_004985 | 531 | c.38_39GC>AT | p.G13D | Gain of function | Missense_Mutation |
| 497 | KRAS | NM_004985 | 532 | c.38G>A | p.G13D | Gain of function | Missense_Mutation |
| 498 | KRAS | NM_004985 | 532 | c.38G>A | p.G13D | Gain of function | Missense_Mutation |
| 499 | KRAS | NM_004985 | 532 | c.38G>A | p.G13D | Gain of function | Missense_Mutation |
| 500 | KRAS | NM_004985 | 87280 | c.38_39GC>AA | p.G13E | Gain of function | Missense_Mutation |
| 501 | KRAS | NM_004985 | 87280 | c38_39GC>AA | p.G13E | Gain of function | Missense_Mutation |
| 502 | KRAS | NM_004985 | 87280 | c.38_39GC>AA | p.G13E | Gain of function | Missense_Mutation |
| 503 | KRAS | NM_004985 | 535 | c.39C>G | p.G13G | Gain of function | Synonymous_Mutation |
| 504 | KRAS | NM_004985 | 536 | c.39C>T | p.G13G | Gain of function | Synonymous_Mutation |
| 505 | KRAS | NM_004985 | 537 | c.39C>A | p.G13G | Gain of function | Synonymous_Mutation |
| 506 | KRAS | NM_004985 | 529 | c.37G>C | p.G13R | Gain of function | Missense_Mutation |
| 507 | KRAS | NM_004985 | 529 | c.37G>C | p.G13R | Gain of function | Missense_Mutation |
| 508 | KRAS | NM_004985 | 529 | c.37G>C | p.G13R | Gain of function | Missense_Mutation |
| 509 | KRAS | NM_004985 | 528 | c.37G>A | p.G13S | Gain of function | Missense_Mutation |
| 510 | KRAS | NM_004985 | 528 | c.37G>A | p.G13S | Gain of function | Missense_Mutation |
| 511 | KRAS | NM_004985 | 528 | c.37G>A | p.G13S | Gain of function | Missense_Mutation |
| 512 | KRAS | NM_004985 | 12721 | c.38_39GC>TT | p.G13V | Gain of function | Missense_Mutation |
| 513 | KRAS | NM_004985 | 12721 | c.38_39GC>TT | p.G13V | Gain of function | Missense_Mutation |
| 514 | KRAS | NM_004985 | 12721 | c.38_39GC>TT | p.G13V | Gain of function | Missense_Mutation |
| 515 | KRAS | NM_004985 | 534 | c.38G>T | p.G13V | Gain of function | Missense_Mutation |
| 516 | KRAS | NM_004985 | 534 | c.38G>T | p.G13V | Gain of function | Missense_Mutation |
| 517 | KRAS | NM_004985 | 534 | c.38G>T | p.G13V | Gain of function | Missense_Mutation |
| 518 | KRAS | NM_004985 | 538 | c.43G>A | p.G15S | Gain of function | Missense_Mutation |
| 519 | KRAS | NM_004985 | 19940 | c.351A>C | P.K117N | Gain of function | Missense_Mutation |
| 520 | KRAS | NM_004985 | 28519 | c.351A>T | p.K117N | Gain of function | Missense_Mutation |
| 521 | KRAS | NM_004985 | 12703 | c.57G>C | p.L19F | Gain of function | Missense_Mutation |
| 522 | KRAS | NM_004985 | 20818 | c.57G>T | p.L19F | Gain of function | Missense_Mutation |
| 523 | KRAS | NM_004985 | 543 | c.64C>A | p.Q22K | Gain of function | Missense_Mutation |
| 524 | KRAS | NM_004985 | 550 | c.181C>G | p.Q61E | Gain of function | Missense_Mutation |
| 525 | KRAS | NM_004985 | 550 | c.181C>G | p.Q61E | Gain of function | Missense_Mutation |
| 526 | KRAS | NM_004985 | 550 | c.181C>G | p.Q61E | Gain of function | Missense_Mutation |
| 527 | KRAS | NM_004985 | 554 | c.183A>C | p.Q61H | Gain of function | Missense_Mutation |
| 528 | KRAS | NM_004985 | 554 | c.183A>C | p.Q61H | Gain of function | Missense_Mutation |
| 529 | KRAS | NM_004985 | 554 | c.183A>C | p.Q61H | Gain of function | Missense_Mutation |
| 530 | KRAS | NM_004985 | 555 | c.183A>T | p.Q61H | Gain of function | Missense_Mutation |
| 531 | KRAS | NM_004985 | 555 | c.183A>T | p.Q61H | Gain of function | Missense_Mutation |
| 532 | KRAS | NM_004985 | 555 | c.183A>T | p.Q61H | Gain of function | Missense_Mutation |
| 533 | KRAS | NM_004985 | 549 | c.181C>A | p.Q61K | Gain of function | Missense_Mutation |
| 534 | KRAS | NM_004985 | 549 | c.181C>A | p.Q61K | Gain of function | Missense_Mutation |
| 535 | KRAS | NM_004985 | 549 | c.181C>A | p.Q61K | Gain of function | Missense_Mutation |
| 536 | KRAS | NM_004985 | 87298 | c.180_181TC>AA | p.Q61K | Gain of function | Missense_Mutation |
| 537 | KRAS | NM_004985 | 87298 | c.180_181TC>AA | p.Q61K | Gain of function | Missense_Mutation |
| 538 | KRAS | NM_004985 | 87298 | c.180_181TC>AA | p.Q61K | Gain of function | Missense_Mutation |
| 539 | KRAS | NM_004985 | 553 | c.182A>T | p.Q61L | Gain of function | Missense_Mutation |
| 540 | KRAS | NM_004985 | 553 | c.182A>T | p.Q61L | Gain of function | Missense_Mutation |
| 541 | KRAS | NM_004985 | 553 | c.182A>T | p.Q61L | Gain of function | Missense_Mutation |
| 542 | KRAS | NM_004985 | 551 | c.182A>C | p.Q61P | Gain of function | Missense_Mutation |
| 543 | KRAS | NM_004985 | 551 | c.182A>C | p.Q61P | Gain of function | Missense_Mutation |
| 544 | KRAS | NM_004985 | 551 | c.182A>C | p.Q61P | Gain of function | Missense_Mutation |
| 545 | KRAS | NM_004985 | 552 | c.182A>G | p.Q61R | Gain of function | Missense_Mutation |
| 546 | KRAS | NM_004985 | 552 | c.182A>G | p.Q61R | Gain of function | Missense_Mutation |
| 547 | KRAS | NM_004985 | 552 | c.182A>G | P.Q61R | Gain of function | Missense_Mutation |
| 548 | KRAS | NM_004985 | 87288 | c.173C>T | p.T58I | Gain of function | Missense_Mutation |
| 549 | KRAS | NM_004985 | 12722 | c.40G>A | P.V14I | Gain of function | Missense_Mutation |
| 550 | KRAS | NM_004985 | 507 | c.24A>G | p.V8V | Gain of function | Synonymous_Mutation |
| 551 | MAP2K1_EN ST00000307102 | ENST00000307102 | 236154 | c.171G>T | p.K57N | Gain of function | Missense_Mutation |
| 552 | MET | NM_000245 | 201908 | c.3350A>G | p.D1117G | Gain of function | Missense_Mutation |
| 553 | MET | NM_000245 | 706 | c.504G>T | p.E168D | Gain of function | Missense_Mutation |
| 554 | MET | NM_000245 | 698 | c.3335A>T | p.H1112L | Gain of function | Missense_Mutation |
| 555 | MET | NM_000245 | 703 | c.3335A>G | p.H1112R | Gain of function | Missense_Mutation |
| 556 | MET | NM_000245 | 696 | c.3334C>T | p.H1112Y | Gain of function | Missense_Mutation |
| 557 | MET | NM_000245 | 697 | c.3370C>G | p.H1124D | Gain of function | Missense_Mutation |
| 558 | MET | NM_000245 | 701 | c.3390G>A | p.L1130L | Gain of function | Synonymous_Mutation |
| 559 | MET | NM_000245 | 691 | c.3803T>C | p.M1268T | Gain of function | Missense_Mutation |
| 560 | MET | NM_000245 | 702 | c.3352A>T | p.N1118Y | Gain of function | Missense_Mutation |
| 561 | MET | NM_000245 | 710 | c.1124A>G | P.N375S | Gain of function | Missense_Mutation |
| 562 | MET | NM_000245 | 707 | c.3029C>T | p.T1010I | Gain of function | Missense_Mutation |
| 563 | MET | NM_000245 | 699 | c.3743A>G | p.Y1248C | Gain of function | Missense_Mutation |
| 564 | MET | NM_000245 | 700 | c.3757T>G | p.Y1253D | Gain of function | Missense_Mutation |
| 565 | NOTCH1 | NM_017617.2 | 13050 | c.4778T>C | P.F1593S | Loss of Function | Missense_Mutation |
| 566 | NOTCH1 | NM_017617.2 | 12772 | c.4724T>C | p.L1575P | Loss of Function | Missense_Mutation |
| 567 | NOTCH1 | NM_017617.2 | 24673 | c.4724T>C | P.L1575P | Loss of Function | Missense_Mutation |
| 568 | NOTCH1 | NM_017617.2 | 13046 | c.4757T>C | p.L1586P | Loss of Function | Missense_Mutation |
| 569 | NOTCH1 | NM_017617.2 | 25839 | c.4757T>G | p.L1586R | Loss of Function | Missense_Mutation |
| 570 | NOTCH1 | NM_017617.2 | 13042 | c.4781T>C | P.L1594P | Loss of Function | Missense_Mutation |
| 571 | NOTCH1 | NM_017617.2 | 24888 | c.4790T>A | P.L1597H | Loss of Function | Missense_Mutation |
| 572 | NOTCH1 | NM_017617.2 | 12771 | c.4802T>C | p.L1601P | Loss of Function | Missense_Mutation |
| 573 | NOTCH1 | NM_017617.2 | 28524 | c.4802T>A | p.L1601Q | Loss of Function | Missense_Mutation |
| 574 | NOTCH1 | NM_017617.2 | 13053 | c.4796G>C | p.R1599P | Loss of Function | Missense_Mutation |
| 575 | NOTCH1 | NM_017617.2 | 25836 | c.4730T>A | p.V1577E | Loss of Function | Missense_Mutation |
| 576 | NOTCH1 | NM_017617.2 | 13047 | c.4735_4737delGTG | p.V1579del | Loss of Function | In_Frame_Del |
| 577 | NOTCH1 | NM_017617.2 | 13040 | c.5030T>A | p.V1677D | Loss of Function | Missense_Mutation |
| 578 | NRAS | NM_002524 | 558 | c.31G>A | p.A11T | Gain of function | Missense_Mutation |
| 579 | NRAS | NM_002524 | 577 | c.52G>A | p.A18T | Gain of function | Missense_Mutation |
| 580 | NRAS | NM_002524 | 565 | c.35G>C | p.G12A | Gain of function | Missense_Mutation |
| 581 | NRAS | NM_002524 | 562 | c.34G>T | p.G12C | Gain of function | Missense_Mutation |
| 582 | NRAS | NM_002524 | 564 | c.35G>A | p.G12D | Gain of function | Missense_Mutation |
| 583 | NRAS | NM_002524 | 567 | c.36T>C | p.G12G | Gain of function | Synonymous_Mutation |
| 584 | NRAS | NM_002524 | 12723 | c.34_35GG>AA | p.G12N | Gain of function | Missense_Mutation |
| 585 | NRAS | NM_002524 | 561 | c.34G>C | p.G12R | Gain of function | Missense_Mutation |
| 586 | NRAS | NM_002524 | 563 | c.34G>A | p.G12S | Gain of function | Missense_Mutation |
| 587 | NRAS | NM_002524 | 566 | c.35G>T | p.G12V | Gain of function | Missense_Mutation |
| 588 | NRAS | NM_002524 | 575 | c.38G>C | p.G13A | Gain of function | Missense_Mutation |
| 589 | NRAS | NM_002524 | 570 | c.37G>T | p.G13C | Gain of function | Missense_Mutation |
| 590 | NRAS | NM_002524 | 573 | c.38G>A | p.G13D | Gain of function | Missense_Mutation |
| 591 | NRAS | NM_002524 | 576 | c.39T>C | p.G13G | Gain of function | Synonymous_Mutation |
| 592 | NRAS | NM_002524 | 569 | c.37G>C | p.G13R | Gain of function | Missense_Mutation |
| 593 | NRAS | NM_002524 | 571 | c.37G>A | p.G13S | Gain of function | Missense_Mutation |
| 594 | NRAS | NM_002524 | 574 | c.38G>T | p.G13V | Gain of function | Missense_Mutation |
| 595 | NRAS | NM_002524 | 28673 | c.179G>A | p.G60E | Gain of function | Missense_Mutation |
| 596 | NRAS | NM_002524 | 581 | c.181C>G | p.Q61E | Gain of function | Missense_Mutation |
| 597 | NRAS | NM_002524 | 585 | c.183A>T | p.Q61H | Gain of function | Missense_Mutation |
| 598 | NRAS | NM_002524 | 586 | c.183A>C | p.Q61H | Gain of function | Missense_Mutation |
| 599 | NRAS | NM_002524 | 12730 | c.180_181AC>TA | p.Q61K | Gain of function | Missense_Mutation |
| 600 | NRAS | NM_002524 | 580 | c.181C>A | p.Q61K | Gain of function | Missense_Mutation |
| 601 | NRAS | NM_002524 | 12725 | c.181_182CA>TT | p.Q61L | Gain of function | Missense_Mutation |
| 602 | NRAS | NM_002524 | 30646 | c.182_183AA>TG | p.Q61L | Gain of function | Missense_Mutation |
| 603 | NRAS | NM_002524 | 583 | c.182A>T | p.Q61L | Gain of function | Missense_Mutation |
| 604 | NRAS | NM_002524 | 582 | c.182A>C | p.Q61P | Gain of function | Missense_Mutation |
| 605 | NRAS | NM_002524 | 587 | c.183A>G | p.Q61Q | Gain of function | Synonymous_Mutation |
| 606 | NRAS | NM_002524 | 33693 | c.182_183AA>GG | p.Q61R | Gain of function | Missense_Mutation |
| 607 | NRAS | NM_002524 | 579 | c.181_182CA>AG | p.Q61R | Gain of function | Missense_Mutation |
| 608 | NRAS | NM_002524 | 584 | c.182A>G | p.Q61R | Gain of function | Missense_Mutation |
| 609 | NRAS | NM_002524 | 589 | c.193A>T | p.565C | Gain of function | Missense_Mutation |
| 610 | PIK3CA | NM_0062181 | 17449 | c.3207A>G | p.*1069_*1069insW | Gain of function | N/A |
| 611 | PIK3CA | NM_006218.1 | 249908 | c.3207+29T>C | p.? | Gain of function | N/A |
| 612 | PIK3CA | NM_006218.1 | 28938 | c.3059C>T | p.A1020V | Gain of function | Missense_Mutation |
| 613 | PIK3CA | NM_006218.1 | 17445 | c.3104C>T | p.A1035V | Gain of function | Missense_Mutation |
| 614 | PIK3CA | NM_006218.1 | 27156 | c.3137C>A | p.A1046E | Gain of function | Missense_Mutation |
| 615 | PIK3CA | NM_006218.1 | 27273 | c.3136G>A | p.A1046T | Gain of function | Missense_Mutation |
| 616 | PIK3CA | NM_006218.1 | 36286 | c.3085G>C | p.D1D29H | Gain of function | Missense_Mutation |
| 617 | PIK3CA | NM_006218.1 | 25086 | c.3133G>A | p.D1045N | Gain of function | Missense_Mutation |
| 618 | PIK3CA | NM_006218.1 | 760 | c.1624G>A | p.E542K | Gain of function | Missense_Mutation |
| 619 | PIK3CA | NM_006218.1 | 17442 | c.1624G>C | p.E542Q | Gain of function | Missense_Mutation |
| 620 | PIK3CA | NM_006218.1 | 762 | c.1625A>T | p.E542V | Gain of function | Missense_Mutation |
| 621 | PIK3CA | NM_006218.1 | 12458 | c.1634A>C | p.E545A | Gain of function | Missense_Mutation |
| 622 | PIK3CA | NM_006218.1 | 27374 | c.1635G>C | p.E545D | Gain of function | Missense_Mutation |
| 623 | PIK3CA | NM_006218.1 | 765 | c.1635G>T | p.E545D | Gain of function | Missense_Mutation |
| 624 | PIK3CA | NM_006218.1 | 764 | c.1634A>G | p.E545G | Gain of function | Missense_Mutation |
| 625 | PIK3CA | NM_006218.1 | 763 | c.1633G>A | p.E545K | Gain of function | Missense_Mutation |
| 626 | PIK3CA | NM_006218.1 | 27133 | c.1633G>C | p.E545Q | Gain of function | Missense_Mutation |
| 627 | PIK3CA | NM_006218.1 | 27155 | c.1634A>T | p.E545V | Gain of function | Missense_Mutation |
| 628 | PIK3CA | NM_006218.1 | 29110 | c.3115T>C | p.F1039L | Gain of function | Missense_Mutation |
| 629 | PIK3CA | NM_006218.1 | 27158 | c.3146G>C | p.G1D49A | Gain of function | Missense_Mutation |
| 630 | PIK3CA | NM_006218.1 | 12597 | c.3145G>C | p.G1D49R | Gain of function | Missense_Mutation |
| 631 | PIK3CA | NM_006218.1 | 777 | c.3145G>A | p.G1049S | Gain of function | Missense_Mutation |
| 632 | PIK3CA | NM_006218.1 | 776 | c.3140A>T | p.H1047L | Gain of function | Missense_Mutation |
| 633 | PIK3CA | NM_006218.1 | 24714 | c.3141T>G | p.H1047Q | Gain of function | Missense_Mutation |
| 634 | PIK3CA | NM_006218.1 | 775 | c.3140A>G | p.H1047R | Gain of function | Missense_Mutation |
| 635 | PIK3CA | NM_006218.1 | 774 | c.3139C>T | p.H1D47Y | Gain of function | Missense_Mutation |
| 636 | PIK3CA | NM_006218.1 | 36289 | c.3143A>G | p.H1D48R | Gain of function | Missense_Mutation |
| 637 | PIK3CA | NM_006218.1 | 778 | c.2102A>C | p.H701P | Gain of function | Missense_Mutation |
| 638 | PIK3CA | NM_006218.1 | 25085 | c.3120G>A | p.M1040I | Gain of function | Missense_Mutation |
| 639 | PIK3CA | NM_006218.1 | 29313 | c.3129G>A | p.M1043I | Gain of function | Missense_Mutation |
| 640 | PIK3CA | NM_006218.1 | 773 | c.3129G>T | p.M1043I | Gain of function | Missense_Mutation |
| 641 | PIK3CA | NM_006218.1 | 94984 | c.3129G>C | p.M1043I | Gain of function | Missense_Mutation |
| 642 | PIK3CA | NM_006218.1 | 12463 | c.3128T>C | p.M 1043T | Gain of function | Missense_Mutation |
| 643 | PIK3CA | NM_006218.1 | 12591 | c.3127A>G | p.M 1043V | Gain of function | Missense_Mutation |
| 644 | PIK3CA | NM_006218.1 | 27134 | c.3130A>G | P.N1044D | Gain of function | Missense_Mutation |
| 645 | PIK3CA | NM_006218.1 | 12592 | c.3132T>A | p.N1044K | Gain of function | Missense_Mutation |
| 646 | PIK3CA | NM_006218.1 | 759 | c.1616C>G | p.P539R | Gain of function | Missense_Mutation |
| 647 | PIK3CA | NM_006218.1 | 6147 | c.1636C>G | p.Q546E | Gain of function | Missense_Mutation |
| 648 | PIK3CA | NM_006218.1 | 24712 | c.1638G>T | p.Q546H | Gain of function | Missense_Mutation |
| 649 | PIK3CA | NM_006218.1 | 766 | c.1636C>A | P.Q546K | Gain of function | Missense_Mutation |
| 650 | PIK3CA | NM_006218.1 | 25041 | c.1637A>T | p.Q546L | Gain of function | Missense_Mutation |
| 651 | PIK3CA | NM_006218.1 | 767 | c.1637A>C | P.Q546P | Gain of function | Missense_Mutation |
| 652 | PIK3CA | NM_006218.1 | 12459 | c.1637A>G | p.Q546R | Gain of function | Missense_Mutation |
| 653 | PIK3CA | NM_006218.1 | 13594 | c.3068G>A | p.R1023Q | Gain of function | Missense_Mutation |
| 654 | PIK3CA | NM_006218.1 | 771 | c.3073A>G | p.T1025A | Gain of function | Missense_Mutation |
| 655 | PIK3CA | NM_006218.1 | 36285 | c.3074C>T | p.T1025I | Gain of function | Missense_Mutation |
| 656 | PIK3CA | NM_006218.1 | 772 | c.3074C>A | p.T1025N | Gain of function | Missense_Mutation |
| 657 | PIK3CA | NM_006218.1 | 12590 | c.3073A>T | p.T1025S | Gain of function | Missense_Mutation |
| 658 | PIK3CA | NM_006218.1 | 21451 | c.3075C>T | p.T1025T | Gain of function | Synonymous_Mutation |
| 659 | PIK3CA | NM_006218.1 | 249872 | c.1631C>A | p.T544N | Gain of function | Missense_Mutation |
| 660 | PIK3CA | NM_006218.1 | 12461 | c.3062A>G | p.Y1021C | Gain of function | Missense_Mutation |
| 661 | PIK3CA | NM_006218.1 | 17444 | c.3061T>C | p.Y1021H | Gain of function | Missense_Mutation |
| 662 | PIK3CA_E NST00000263967 | ENST00000263967 | 125370 | c.1633G>A | p.E545K | Gain of function | Missense_Mutation |
| 663 | PIK3CA_E NST00000263967 | ENST00000263967 | 94987 | c.3140A>T | p.H1047L | Gain of function | Missense_Mutation |
| 664 | PIK3CA_E NST00000263967 | ENST00000263967 | 94986 | c.3140A>G | p.H1047R | Gain of function | Missense_Mutation |
| 665 | PIK3CA_E NST00000263967 | ENST00000263967 | 94985 | c.3129G>C | p.M1043I | Gain of function | Missense_Mutation |
| 666 | PTEN | NM_000314.4 | 14087 | c.165_209del45 | p.? | Loss of Function | N/A |
| 667 | PTEN | NM_000314.4 | 19564 | c.1026+1G>T | p.? | Loss of Function | N/A |
| 668 | PTEN | NM_000314.4 | 249834 | c.635-2A>G | p.? | Loss of Function | N/A |
| 669 | PTEN | NM_000314.4 | 27365 | c.635-91G>C | p.? | Loss of Function | N/A |
| 670 | PTEN | NM_000314.4 | 28920 | c.635-1G>A | p.? | Loss of Function | N/A |
| 671 | PTEN | NM_000314.4 | 5907 | c.493-12delT | p.? | Loss of Function | N/A |
| 672 | PTEN | NM_000314.4 | 5915 | c.1-9C>G | p.? | Loss of Function | N/A |
| 673 | PTEN | NM_000314.4 | 5916 | c.209+5G>A | p.? | Loss of Function | N/A |
| 674 | PTEN | NM_000314.4 | 5932 | c.635-9A>G | p.? | Loss of Function | N/A |
| 675 | PTEN | NM_000314.4 | 5950 | c.635-12T>G | p.? | Loss of Function | N/A |
| 676 | PTEN | NM_000314.4 | 5951 | c.635-17T>G | p.? | Loss of Function | N/A |
| 677 | PTEN | NM_000314.4 | 5957 | c.1026+1G>T | | Loss of Function | N/A |
| 678 | PTEN | NM_000314.4 | 5958 | c.165-1G>T | p.? | Loss of Function | N/A |
| 679 | PTEN | NM_000314.4 | 5959 | c.165-2A>C | p.? | Loss of Function | N/A |
| 680 | PTEN | NM_000314.4 | 5960 | c.165-1G>A | p.? | Loss of Function | N/A |
| 681 | PTEN | NM_000314.4 | 5961 | c.493-1G>A | p.? | Loss of Function | N/A |
| 682 | PTEN | NM_000314.4 | 5971 | c.635-1G>T | p.? | Loss of Function | N/A |
| 683 | PTEN | NM_000314.4 | 5974 | c.209+1G>C | p.? | Loss of Function | N/A |
| 684 | PTEN | NM_000314.4 | 5975 | c.209+1deIGT | p.? | Loss of Function | N/A |
| 685 | PTEN | NM_000314.4 | 5976 | c.209+1G>T | p.? | Loss of Function | N/A |
| 686 | PTEN | NM_000314.4 | 5979 | c.209+1deIGTAA | p.? | Loss of Function | N/A |
| 687 | PTEN | NM_000314.4 | 4912 | c.750_751delTG | p.C250fs * 2 | Loss of Function | Frame_Shift_Del |
| 688 | PTEN | NM_000314.4 | 5125 | c.755A>G | p.D252G | Loss of Function | Missense_Mutation |
| 689 | PTEN | NM_000314.4 | 5246 | c.754G>T | p.D252Y | Loss of Function | Missense_Mutation |
| 690 | PTEN | NM_000314.4 | 5814 | c.993delC | p.D331Fs*13 | Loss of Function | Frame_Shift_Del |
| 691 | PTEN | NM_000314.4 | 5093 | c.992A>G | p.D331G | Loss of Function | Missense_Mutation |
| 692 | PTEN | NM_000314.4 | 5292 | c.703G>T | p.E235* | Loss of Function | Nonsense_Mutation |
| 693 | PTEN | NM_000314.4 | 88109 | c.724G>T | p.E242* | Loss of Function | Nonsense_Mutation |
| 694 | PTEN | NM_000314.4 | 26404 | c.723_724insT | p.E242fs*1 | Loss of Function | Frame_Shift_Ins |
| 695 | PTEN | NM_000314.4 | 5888 | c.723_724insTT | p.E242fs*15 | Loss of Function | Frame_Shift_Ins |
| 696 | PTEN | NM_000314.4 | 17564 | c.766G>A | P.E256K | Loss of Function | Missense_Mutation |
| 697 | PTEN | NM_000314.4 | 5314 | c.862G>T | p.E288* | Loss of Function | Nonsense_Mutation |
| 698 | PTEN | NM_000314.4 | 13452 | c.863delA | p.E288fs*3 | Loss of Function | Frame_Shift_Del |
| 699 | PTEN | NM_000314.4 | 28906 | c.871G>T | p.E291* | Loss of Function | Nonsense_Mutation |
| 700 | PTEN | NM_000314.4 | 5298 | c.19G>T | p.E7* | Loss of Function | Nonsense_Mutation |
| 701 | PTEN | NM_000314.4 | 4885 | c.1011_1014delTTCT | pF337fs*6 | Loss of Function | Frame_Shift_Del |
| 702 | PTEN | NM_000314.4 | 5869 | c.1009delT | p.F337fs*7 | Loss of Function | Frame_Shift_Del |
| 703 | PTEN | NM_000314.4 | 5255 | c.1021T>G | p.F341V | Loss of Function | Missense_Mutation |
| 704 | PTEN | NM_000314.4 | 5257 | c.166T>G | p.F56V | Loss of Function | Missense_Mutation |
| 705 | PTEN | NM_000314.4 | 5114 | c.494G>A | p.G165E | Loss of Function | Splice_Site |
| 706 | PTEN | NM_000314.4 | 5091 | c.493G>A | p.G165R | Loss of Function | Splice_Site |
| 707 | PTEN | NM_000314.4 | 5220 | c.751G>T | p.G251C | Loss of Function | Missense_Mutation |
| 708 | PTEN | NM_000314.4 | 13981 | c.752G>A | p.G251D | Loss of Function | Missense_Mutation |
| 709 | PTEN | NM_000314.4 | 28914 | c.878delG | p.G293fs*14 | Loss of Function | Frame_Shift_Del |
| 710 | PTEN | NM_000314.4 | 5042 | c.182A>G | p.H61R | Loss of Function | Missense_Mutation |
| 711 | PTEN | NM_000314.4 | 5230 | c.758T>A | p.I253N | Loss of Function | Missense_Mutation |
| 712 | PTEN | NM_000314.4 | 5037 | c.37A>G | p.K13E | Loss of Function | Missense_Mutation |
| 713 | PTEN | NM_000314.4 | 5887 | c.711_712insAA | p.K237fs*19 | Loss of Function | Frame_Shift_Ins |
| 714 | PTEN | NM_000314.4 | 4908 | c.760_764delAAAGT | p.K254fs*42 | Loss of Function | Frame_Shift_Del |
| 715 | PTEN | NM_000314.4 | 133713 | c.780_780delA | p.K260fs*6 | Loss of Function | Frame_Shift_Del |
| 716 | PTEN | NM_000314.4 | 43075 | c.787A>T | p.K263* | Loss of Function | Nonsense_Mutation |
| 717 | PTEN | NM_000314.4 | 41768 | c.179 179delA | p.K60fs*39 | Loss of Function | Frame_Shift_Del |
| 718 | PTEN | NM_000314.4 | 5048 | c.196A>G | p.K66E | Loss of Function | Missense_Mutation |
| 719 | PTEN | NM_000314.4 | 5191 | c.198G>T | p.K66N | Loss of Function | Missense_Mutation |
| 720 | PTEN | NM_000314.4 | 4929 | c.17_18delAA | p.K6fs*4 | Loss of Function | Frame_Shift_Del |
| 721 | PTEN | NM_000314.4 | 4937 | c.16_17delAA | p.K6fs*4 | Loss of Function | Frame_Shift_Del |
| 722 | PTEN | NM_000314.4 | 39615 | c.950_953delTACT | p.L318fs*2 | Loss of Function | Frame_Shift_Del |
| 723 | PTEN | NM_000314.4 | 4894 | c.952_955delCTTA | p.L318fs*2 | Loss of Function | Frame_Shift_Del |
| 724 | PTEN | NM_000314.4 | 4903 | c.954_957delTACT | p.L318fs*2 | Loss of Function | Frame_Shift_Del |
| 725 | PTEN | NM_000314.4 | 4916 | c.953_956delTTAC | p.L318fs*2 | Loss of Function | Frame_Shift_Del |
| 726 | PTEN | NM_000314.4 | 5000 | c.170_171insT | p.L57fs*6 | Loss of Function | Frame_Shift_Ins |
| 727 | PTEN | NM_000314.4 | 5127 | c.170T>C | p.L57S | Loss of Function | Missense_Mutation |
| 728 | PTEN | NM_000314.4 | 5253 | c.170T>G | p.L57W | Loss of Function | Missense_Mutation |
| 729 | PTEN | NM_000314.4 | 23626 | c.962_963insA | p.N323fs*2 | Loss of Function | Frame_Shift_Ins |
| 730 | PTEN | NM_000314.4 | 4990 | c.968_969insA | p.N323fs*2 | Loss of Function | Frame_Shift_Ins |
| 731 | PTEN | NM_000314.4 | 5801 | c.968delA | p.N323fs*21 | Loss of Function | Frame_Shift_Del |
| 732 | PTEN | NM_000314.4 | 4932 | c.987_990delTAAA | p.N329fs*14 | Loss of Function | Frame_Shift_Del |
| 733 | PTEN | NM_000314.4 | 4942 | c.187_188delAA | p.N63fs*10 | Loss of Function | Frame_Shift_Del |
| 734 | PTEN | NM_000314.4 | 5811 | c.188delA | p.N63fs*36 | Loss of Function | Frame_Shift_Del |
| 735 | PTEN | NM_000314.4 | 17561 | c.638C>A | p.P213H | Loss of Function | Missense_Mutation |
| 736 | PTEN | NM_000314.4 | 241294 | c.638C>G | p.P213R | Loss of Function | Missense_Mutation |
| 737 | PTEN | NM_000314.4 | 30729 | c.637C>T | P.P213S | Loss of Function | Missense_Mutation |
| 738 | PTEN | NM_000314.4 | 5822 | c.738delG | p.P246fs*10 | Loss of Function | Frame_Shift_Del |
| 739 | PTEN | NM_000314.4 | 5111 | c.737C>T | p.P246L | Loss of Function | Missense_Mutation |
| 740 | PTEN | NM_000314.4 | 23644 | c.743C>G | p.P248? | Loss of Function | N/A |
| 741 | PTEN | NM_000314.4 | 4986 | c.741_742insA | p.P248fs*5 | Loss of Function | Frame_Shift_Ins |
| 742 | PTEN | NM_000314.4 | 23657 | c.1015C>T | p.P339S | Loss of Function | Missense_Mutation |
| 743 | PTEN | NM_000314.4 | 5153 | c.49C>T | p.Q17* | Loss of Function | Nonsense_Mutation |
| 744 | PTEN | NM_000314.4 | 5149 | c.511C>T | p.Q171* | Loss of Function | Nonsense_Mutation |
| 745 | PTEN | NM_000314.4 | 5200 | c.511C>G | P.Q171E | Loss of Function | Missense_Mutation |
| 746 | PTEN | NM_000314.4 | 5244 | c.512A>C | p.Q171P | Loss of Function | Missense_Mutation |
| 747 | PTEN | NM_000314.4 | 4976 | c.49_51delCAA | p.Q17del | Loss of Function | In_Frame_Del |
| 748 | PTEN | NM_000314.4 | 5159 | c.733C>T | p.Q245* | Loss of Function | Nonsense_Mutation |
| 749 | PTEN | NM_000314.4 | 5160 | c.781C>T | p.Q261* | Loss of Function | Nonsense_Mutation |
| 750 | PTEN | NM_000314.4 | 5156 | c.892C>T | p.Q298* | Loss of Function | Nonsense_Mutation |
| 751 | PTEN | NM_000314.4 | 5101 | c.40A>G | p.R14G | Loss of Function | Missense_Mutation |
| 752 | PTEN | NM_000314.4 | 5232 | c.44G>T | p.R15I | Loss of Function | Missense_Mutation |
| 753 | PTEN | NM_000314.4 | 5270 | c.45A>T | p.R15S | Loss of Function | Missense_Mutation |
| 754 | PTEN | NM_000314.4 | 5089 | c.517C>T | p.R173C | Loss of Function | Missense_Mutation |
| 755 | PTEN | NM_000314.4 | 5825 | c.517delC | p.R173fs*10 | Loss of Function | Frame_Shift_Del |
| 756 | PTEN | NM_000314.4 | 5039 | c.518G>A | p.R173H | Loss of Function | Missense_Mutation |
| 757 | PTEN | NM_000314.4 | 5151 | c.1003C>T | p.R335* | Loss of Function | Nonsense_Mutation |
| 758 | PTEN | NM_000314.4 | 5775 | c1002_1003CC>TT | p.R335* | Loss of Function | Nonsense_Mutation |
| 759 | PTEN | NM_000314.4 | 5049 | c.29G>A | p.S10N | Loss of Function | Missense_Mutation |
| 760 | PTEN | NM_000314.4 | 5218 | c.509G>T | p.S170I | Loss of Function | Missense_Mutation |
| 761 | PTEN | NM_000314.4 | 5045 | c.509G>A | P.S170N | Loss of Function | Missense_Mutation |
| 762 | PTEN | NM_000314.4 | 4931 | c.881_885delGTCTA | p.S294fs*2 | Loss of Function | Frame_Shift_Del |
| 763 | PTEN | NM_000314.4 | 5313 | c.176C>A | p.S59* | Loss of Function | Nonsense_Mutation |
| 764 | PTEN | NM_000314.4 | 5052 | c.499A>G | p.T167A | Loss of Function | Missense_Mutation |
| 765 | PTEN | NM_000314.4 | 4982 | c.955_957delACT | p.T319del | Loss of Function | Frame_Shift_Del |
| 766 | PTEN | NM_000314.4 | 4958 | c.955_958delACTT | p.T319fs*1 | Loss of Function | Frame_Shift_Del |
| 767 | PTEN | NM_000314.4 | 4896 | c.956_959delCTTT | p.T319fs*24 | Loss of Function | Frame_Shift_Del |
| 768 | PTEN | NM_000314.4 | 5008 | c.955_956insA | p.T319fs*6 | Loss of Function | Frame_Shift_Ins |
| 769 | PTEN | NM_000314.4 | 5823 | c.963delA | p.T321fs*23 | Loss of Function | Frame_Shift_Del |
| 770 | PTEN | NM_000314.4 | 4994 | c.963_964insA | p.T321fs*3 | Loss of Function | Frame_Shift_Ins |
| 771 | PTEN | NM_000314.4 | 5816 | c.867delA | p.V290fs*1 | Loss of Function | Frame_Shift_Del |
| 772 | PTEN | NM_000314.4 | 4898 | c.950_953delTACT | p.V317fs*3 | Loss of Function | Frame_Shift_Del |
| 773 | PTEN | NM_000314.4 | 4899 | c.951_954delACTT | p.V317fs*3 | Loss of Function | Frame_Shift_Del |
| 774 | PTEN | NM_000314.4 | 4943 | c.950_954delTACTT | p.V317fs*6 | Loss of Function | Frame_Shift_Del |
| 775 | PTEN | NM_000314.4 | 5133 | c.47A>G | p.Y16C | Loss of Function | Missense_Mutation |
| 776 | PTEN | NM_000314.4 | 5878 | c.46_47insT | p.Y16fs*28 | Loss of Function | Frame_Shift_Ins |
| 777 | PTEN | NM_000314.4 | 28897 | c.520T>G | p.Y174D | Loss of Function | Missense_Mutation |
| 778 | PTEN | NM_000314.4 | 4969 | c.526_528delTAT | p.Y176del | Loss of Function | In_Frame_Del |
| 779 | PTEN | NM_000314.4 | 33702 | c.530A>G | p.Y177C | Loss of Function | Missense_Mutation |
| 780 | PTEN | NM_000314.4 | 5290 | c.1008C>G | p.Y336* | Loss of Function | Nonsense_Mutation |
| 781 | PTEN | NM_000314.4 | 5296 | c.195C>A | p.Y65* | Loss of Function | Nonsense_Mutation |
| 782 | PTEN | NM_000314.4 | 5317 | c.195C>G | p.Y65* | Loss of Function | Nonsense_Mutation |
| 783 | PTEN | NM_000314.4 | 43077 | c.203A>G | p.Y68C | Loss of Function | Missense_Mutation |
| 784 | PTEN | NM_000314.4 | 4889 | c.202_203delTA | p.Y68fs*5 | Loss of Function | Frame_Shift_Del |
| 785 | PTEN | NM_000314.4 | 5036 | c.202T>C | p.Y68H | Loss of Function | Missense_Mutation |
| 786 | Q7Z2S2_HUMAN | ENST00000341462 | 98900 | c.397G>A | p.D133N | Unclassified | Missense_Mutation |
| 787 | Q7Z2S2_HUMAN | ENST00000341462 | 187238 | c.448C>T | p.P150S | Unclassified | Missense_Mutation |
| 788 | SMAD4 | NM_005359.3 | 14167 | c.955+5G>C | p.? | Loss of Function | Classification N/A |
| 789 | SMAD4 | NM_{.}005359.3 | 14215 | c.353C>T | p.A118V | Loss of Function | Missense_Mutation |
| 790 | SMAD4 | NM_{.}005359.3 | 14105 | c1394_1395insT | pA466fs^{∗}28 | Loss of Function | Frame_Shift_Ins |
| 791 | SMAD4 | NM_{.}005359.3 | 14216 | c.363_364insA | p.C123fs^{∗}2 | Loss of Function | Frame_Shift_Ins |
| 792 | SMAD4 | NM_005359.3 | 25274 | c.366_367insA | p.C123fs^{∗}2 | Loss of Function | Frame_Shift_Ins |
| 793 | SMAD4 | NM_005359.3 | 14221 | c.1496G>A | p.C499Y | Loss of Function | Missense_Mutation |
| 794 | SMAD4 | NM_005359.3 | 14115 | c.1569C>G | p.C523W | Loss of Function | Missense_Mutation |
| 795 | SMAD4 | NM_005359.3 | 14135 | c.1051G>C | p.D351H | Loss of Function | Missense_Mutation |
| 796 | SMAD4 | NM_005359.3 | 14232 | c.1064A>G | p.D355G | Loss of Function | Missense_Mutation |
| 797 | SMAD4 | NM_005359.3 | 14110 | c.989A>C | p.E330A | Loss of Function | Missense_Mutation |
| 798 | SMAD4 | NM_005359.3 | 14134 | c.1576G>T | p.E526^{∗} | Loss of Function | Nonsense_Mutation |
| 799 | SMAD4 | NM_005359.3 | 14121 | c.1015_1029del15 | p.F339_S343del | Loss of Function | In_Frame_Del |
| 800 | SMAD4 | NM_005359.3 | 14118 | c.502G>T | p.G168^{∗} | Loss of Function | Nonsense_Mutation |
| 801 | SMAD4 | NM_005359.3 | 14174 | c.1072G>T | p.G358^{∗} | Loss of Function | Nonsense_Mutation |
| 802 | SMAD4 | NM_005359.3 | 14126 | c.1519A>C | p.K507Q | Loss of Function | Missense_Mutation |
| 803 | SMAD4 | NM_005359.3 | 14057 | c.733C>T | p.Q245^{∗} | Loss of Function | Nonsense_Mutation |
| 804 | SMAD4 | NM_005359.3 | 14163 | c.931C>T | p.Q311^{∗} | Loss of Function | Nonsense_Mutation |
| 805 | SMAD4 | NM_005359.3 | 14223 | c.1229_1230insCA | p.Q410fs^{∗}6 | Loss of Function | Frame_Shift_Ins |
| 806 | SMAD4 | NM_005359.3 | 14124 | c.1341_1365del25 | p.Q448fs^{∗}20 | Loss of Function | Frame_Shift_Del |
| 807 | SMAD4 | NM_005359.3 | 14140 | c.1081C>T | p.R361C | Loss of Function | Missense_Mutation |
| 808 | SMAD4 | NM_005359.3 | 14122 | c.1082G>A | p.R361H | Loss of Function | Missense_Mutation |
| 809 | SMAD4 | NM_005359.3 | 14113 | c.1490G>A | p.R497H | Loss of Function | Missense_Mutation |
| 810 | SMAD4 | NM_005359.3 | 14129 | c.1543A>T | p.R515^{∗} | Loss of Function | Nonsense_Mutation |
| 811 | SMAD4 | NM_005359.3 | 14111 | c.1028C>G | p.S343^{∗} | Loss of Function | Nonsense_Mutation |
| 812 | SMAD4 | NM_005359.3 | 14177 | c.1546_1553delCAGAGCAT | p.S517fs^{∗}7 | Loss of Function | Frame_Shift_Del |
| 813 | SMAD4 | NM_005359.3 | 14217 | c.776_777delCT | p.T259fs^{∗}4 | Loss of Function | Frame_Shift_Del |
| 814 | SMAD4 | NM_005359.3 | 14220 | c.1058A>G | p.Y353C | Loss of Function | Missense_Mutation |
| 815 | SMAD4 | NM_005359.3 | 14175 | c.1236C>G | p.Y412^{∗} | Loss of Function | Nonsense_Mutation |
| 816 | STK11 | NM_000455 | 25847 | c.5806>A | p.D194N | Loss of Function | Missense_Mutation |
| 817 | STK11 | NM_000455 | 20944 | c.580G>T | p.D194Y | Loss of Function | Missense_Mutation |
| 818 | STK11 | NM_000455 | 25229 | c.595G>T | p.E199^{∗} | Loss of Function | Nonsense_Mutation |
| 819 | STK11 | NM_000455 | 21212 | c.169delG | p.E57fs^{∗}7 | Loss of Function | In_Frame_Del |
| 820 | STK11 | NM_000455 | 20857 | c.787_790delTTGT | p.F264fs^{∗}22 | Loss of Function | In_Frame_Del |
| 821 | STK11 | NM_000455 | 21360 | c.1062C>G | p.F354L | Loss of Function | Missense_Mutation |
| 822 | STK11 | NM_000455 | 25851 | c.842_843insC | p.L282fs^{∗}3 | Loss of Function | In_Frame_Ins |
| 323 | STK11 | NM_000455 | 12924 | c.842delC | p.P281fs^{∗}6 | Loss of Function | In_Frame_Del |
| 824 | STK11 | NM_000455 | 20871 | c.837delC | p.P281fs^{∗}6 | Loss of Function | In_Frame_Del |
| 825 | STK11 | NM_000455 | 21355 | c.842C>T | p.P281L | Loss of Function | Missense_Mutation |
| 826 | STK11 | NM_000455 | 12925 | c.109C>T | p.Q37^{∗} | Loss of Function | Nonsense_Mutation |
| 827 | STK11 | NM_000455 | 21378 | c.96C>G | p.T32T | Loss of Function | Synonymous_M utation |
| 828 | STK11 | NM_000455 | 18652 | c.996G>A | p.W332^{∗} | Loss of Function | Nonsense_Mutation |
| 829 | STK11 | NM_000455 | 29005 | c.816C>T | p.Y272Y | Loss of Function | Synonymous_Mutation |
| 830 | STK11 | NM_000455 | 27322 | c.180delC | pY60fs^{∗}1 | Loss of Function | In_Frame_Del |
| 831 | TP53 | NM_000546 | 18657 | c.560-2A>G | p.? | Loss of Function | N/A |
| 832 | TP53 | NM_000546 | 21572 | c.376-1G>A | p.? | Loss of Function | N/A |
| 833 | TP53 | NM_000546 | 22908 | c.376-1G>T | p.? | Loss of Function | N/A |
| 834 | TP53 | NM_000546 | 43541 | c.559+3G>C | p.? | Loss of Function | N/A |
| 835 | TP53 | NM_000546 | 43753 | c.560-1G>A | p.? | Loss of Function | N/A |
| 836 | TP53 | NM_000546 | 43841 | c.560-1G>T | p.? | Loss of Function | N/A |
| 837 | TP53 | NM_000546 | 43872 | c.560-1G>C | p.? | Loss of Function | N/A |
| 838 | TP53 | NM_000546 | 43927 | c.559+9C>T | p.? | Loss of Function | N/A |
| 839 | TP53 | NM_000546 | 44268 | c.559+1G>T | p.? | Loss of Function | N/A |
| 840 | TP53 | NM_000546 | 44297 | c.376-3C>T | p.? | Loss of Function | N/A |
| 841 | TP53 | NM_000546 | 44495 | c.559+2T>A | p.? | Loss of Function | N/A |
| 842 | TP53 | NM_000546 | 44933 | c.376-4A>G | p.? | Loss of Function | N/A |
| 843 | TP53 | NM_000546 | 45026 | c.560-2A>T | p.? | Loss of Function | N/A |
| 844 | TP53 | NM_000546 | 45364 | c.376-1delG | p.? | Loss of Function | N/A |
| 845 | TP53 | NM_000546 | 45672 | c.376-2A>G | p.? | Loss of Function | N/A |
| 846 | TP53 | NM_000546 | 45711 | c.559+2T>G | p.? | Loss of Function | N/A |
| 847 | TP53 | NM_000546 | 45809 | c.376-1G>C | p.? | Loss of Function | N/A |
| 848 | TP53 | NM_000546 | 46049 | c.376-2A>C | p.? | Loss of Function | N/A |
| 849 | TP53 | NM_000546 | 46059 | c.560-3T>G | p.? | Loss of Function | N/A |
| 850 | TP53 | NM_000546 | 6900 | c.376-1G>A | p.? | Loss of Function | N/A |
| 851 | TP53 | NM_000546 | 6901 | c.559+1G>A | p.? | Loss of Function | N/A |
| 852 | TP53 | NM_000546 | 44966 | c.385G>A | p.A129T | Loss of Function | Missense_Mutation |
| 853 | TP53 | NM_000546 | 44550 | c.386C>T | p.A129V | Loss of Function | Missense_Mutation |
| 854 | TP53 | NM_000546 | 44130 | c.477C>T | p.A159A | Loss of Function | Synonymous_Mutation |
| 855 | TP53 | NM_000546 | 11496 | c.476C>A | p.A159D | Loss of Function | Missense_Mutation |
| 856 | TP53 | NM_000546 | 45057 | c.475delG | p.A159fs*11 | Loss of Function | Frame_Shift_Del |
| 857 | TP53 | NM_000546 | 43836 | c.4756>C | p.A159P | Loss of Function | Missense_Mutation |
| 858 | TP53 | NM_000546 | 45286 | c.475G>T | p.A1595 | Loss of Function | Missense_Mutation |
| 859 | TP53 | NM_000546 | 43626 | c.475G>A | p.A159T | Loss of Function | Missense_Mutation |
| 860 | TP53 | NM_000546 | 11148 | c.476C>T | p.A159V | Loss of Function | Missense_Mutation |
| 861 | TP53 | NM_000546 | 44119 | c.483C>T | p.A161A | Loss of Function | Synonymous_Mutation |
| 862 | TP53 | NM_000546 | 11323 | c.482C>A | p.A161D | Loss of Function | Missense_Mutation |
| 863 | TP53 | NM_000546 | 44230 | c.481deIG | p.A161fs*9 | Loss of Function | Frame_Shift_Del |
| 864 | TP53 | NM_000546 | 10739 | c.481G>A | p.A161T | Loss of Function | Missense_Mutation |
| 865 | TP53 | NM_000546 | 43689 | c.482C>T | p.A151V | Loss of Function | Missense_Mutation |
| 866 | TP53 | NM_000546 | 45029 | c.565_591del27 | p.A189_V197delAPP | Loss of Function | In_Frame_Del |
| 867 | TP53 | NM_000546 | 45440 | c.567C>T | p.A189A | Loss of Function | Synonymous_Mutation |
| 868 | TP53 | NM_000546 | 43698 | c.566C>G | p.A189G | Loss of Function | Missense_Mutation |
| 869 | TP53 | NM_000546 | 44923 | c.565G>C | p.A189P | Loss of Function | Missense_Mutation |
| 870 | TP53 | NM_000546 | 43537 | c.565G>A | p.A189T | Loss of Function | Missense_Mutation |
| 871 | TP53 | NM_000546 | 44349 | c.566C>T | p.A189V | Loss of Function | Missense_Mutation |
| 872 | TP53 | NM_000546 | 45268 | c.827C>A | p.A276D | Loss of Function | Missense_Mutation |
| 873 | TP53 | NM_000546 | 45695 | c.827C>G | p.A276G | Loss of Function | Missense_Mutation |
| 874 | TP53 | NM_000546 | 43663 | c.826G>C | p.A276P | Loss of Function | Missense_Mutation |
| 875 | TP53 | NM_000546 | 45467 | c.826G>T | p.A276S | Loss of Function | Missense_Mutation |
| 876 | TP53 | NM_000546 | 44114 | c.826G>A | p.A276T | Loss of Function | Missense_Mutation |
| 877 | TP53 | NM_000546 | 10756 | c.827C>T | p.A276V | Loss of Function | Missense_Mutation |
| 878 | TP53 | NM_000546 | 44019 | c.226_270del45 | p.A76_S90del15 | Loss of Function | In_Frame_Del |
| 879 | TP53 | NM_000546 | 45200 | c.233C>T | p.A78V | Loss of Function | Missense_Mutation |
| 880 | TP53 | NM_000546 | 44075 | c.251C>G | p.A84G | Loss of Function | Missense_Mutation |
| 881 | TP53 | NM_000546 | 44194 | c.251C>T | p.A84V | Loss of Function | Missense_Mutation |
| 882 | TP53 | NM_000546 | 44231 | c.262delG | p.A88fs^{∗}35 | Loss of Function | Frame_Shift_Del |
| 883 | TP53 | NM_000546 | 44319 | c.405C>A | p.C135^{∗} | Loss of Function | Nonsense_Mutation |
| 884 | TP53 | NM_000546 | 43704 | c.405C>T | p.C135C | Loss of Function | Synonymous_Mutation |
| 885 | TP53 | NM_000546 | 10647 | c.404G>T | p.C135F | Loss of Function | Missense_Mutation |
| 886 | TP53 | NM_000546 | 44670 | c.400delT | p.C135fs^{∗}35 | Loss of Function | Frame_Shift_Del |
| 887 | TP53 | NM_000546 | 44829 | c.403T>G | p.C135G | Loss of Function | Missense_Mutation |
| 888 | TP53 | NM_000546 | 10684 | c.403T>C | p.C135R | Loss of Function | Missense_Mutation |
| 889 | TP53 | NM_000546 | 44643 | c.404G>C | P.C135S | Loss of Function | Missense_Mutation |
| 890 | TP53 | NM_000546 | 44910 | c.403T>A | p.C135S | Loss of Function | Missense_Mutation |
| 891 | TP53 | NM_000546 | 44219 | c.405C>G | p.C135W | Loss of Function | Missense_Mutation |
| 892 | TP53 | NM_000546 | 10801 | c.4046>A | p.C135Y | Loss of Function | Missense_Mutation |
| 893 | TP53 | NM_000546 | 43734 | c.528C>A | p.C176^{∗} | Loss of Function | Nonsense_Mutation |
| 894 | TP53 | NM_000546 | 45399 | c.526_543del18 | p.C176_R181delCPb | Loss of Function | In_Frame_Del |
| 895 | TP53 | NM_000546 | 10645 | c.527G>T | p.C176F | Loss of Function | Missense_Mutation |
| 896 | TP53 | NM_000546 | 44759 | c.526delT | p.C176fs^{∗}71 | Loss of Function | Frame_Shift_Del |
| 897 | TP53_ENST00000269305 | ENST00000269305 | 99601 | c.404G>A | p.C135Y | Loss of Function | Missense_Mutation |
| 898 | TP53 | NM_000546 | 44948 | c.526T>C | p.C176R | Loss of Function | Missense_Mutation |
| 899 | TP53 | NM_000546 | 44146 | c.526T>A | p.C1765 | Loss of Function | Missense_Mutation |
| 900 | TP53_ENST00000413465 | ENST00000413465 | 99598 | c.404G>A | p.C135Y | Loss of Function | Missense_Mutation |
| 901 | TP53_ENST00000545858 | ENST00000545858 | 99625 | c.434G>T | p.C145F | Loss of Function | Missense_Mutation |
| 902 | TP53 | NM_000546 | 10687 | c.527G>A | p.C176Y | Loss of Function | Missense_Mutation |
| 903 | TP53 | NM_000546 | 45562 | c.546C>A | p.C182^{∗} | Loss of Function | Nonsense_Mutation |
| 904 | TP53 ENST00000269305 | ENST00000269305 | 117398 | c.527G>T | p.C176F | Loss of Function | Missense_Mutation |
| 905 | TP53_ENST00000413465 | ENST00000413465 | 117395 | c.527G>T | p.C176F | Loss of Function | Missense_Mutation |
| 906 | TP53 | NM_000546 | 44692 | c.526T>G | p.C176G | Loss of Function | Missense_Mutation |
| 907 | TP53 | NM_000546 | 44645 | c.527G>C | p.C1765 | Loss of Function | Missense_Mutation |
| 908 | TP53 | NM_000546 | 45394 | c.687T>A | p.C229^{∗} | Loss of Function | Nonsense_Mutation |
| 909 | TP53 | NM_000546 | 45654 | c.685_699del15 | p.C229_H233delCTT | Loss of Function | In_Frame_Del |
| 910 | TP53 | NM_000546 | 43648 | c.685_686delTG | p.C229fs^{∗}10 | Loss of Function | Frame_Shift_Del |
| 911 | TP53 | NM_000546 | 44360 | c.686_687delGT | p.C229fs^{∗}10 | Loss of Function | Frame_Shift_Del |
| 912 | TP53 | NM_000546 | 11114 | c.528C>G | p.C176W | Loss of Function | Missense_Mutation |
| 913 | TP53 | NM_000546 | 46288 | c.546C>T | P.C182C | Loss of Function | Synonymous_Mutation |
| 914 | TP53 | NM_000546 | 45677 | c.714T>A | p.C238^{∗} | Loss of Function | Nonsense_Mutation |
| 915 | TP53 | NM_000546 | 43778 | c.713G>T | p.C238F | Loss of Function | Missense_Mutation |
| 916 | TP53 | NM_000546 | 44563 | c.544T>C | p.C182R | Loss of Function | Missense_Mutation |
| 917 | TP53 | NM_000546 | 43828 | c.544T>A | p.C182S | Loss of Function | Missense_Mutation |
| 918 | TP53 | NM_000546 | 43700 | c.712T>A | p.C238S | Loss of Function | Missense_Mutation |
| 919 | TP53 | NM_000546 | 44653 | c.713G>C | p.C238S | Loss of Function | Missense_Mutation |
| 920 | TP53 | NM_000546 | 44676 | c.714T>G | p.C238W | Loss of Function | Missense_Mutation |
| 921 | TP53 | NM_000546 | 11059 | c.713G>A | p.C238Y | Loss of Function | Missense_Mutation |
| 922 | TP53 | NM_000546 | 44378 | c.726C>A | p.C242^{∗} | Loss of Function | Nonsense_Mutation |
| 923 | TP53 | NM_000546 | 45691 | c.726C>T | p.C242C | Loss of Function | Synonymous_Mutation |
| 924 | TP53 | NM_000546 | 10810 | c.725G>T | p.C242F | Loss of Function | Missense_Mutation |
| 925 | TP53 | NM_000546 | 44657 | c.722delC | p.C242fs^{∗}5 | Loss of Function | Frame_Shift_Del |
| 926 | TP53 | NM_000546 | 6530 | c.723delC | p.C242fs^{∗}5 | Loss of Function | Frame_Shift_Del |
| 927 | TP53 | NM_000546 | 44546 | c.545G>A | p.C182Y | Loss of Function | Missense_Mutation |
| 928 | TP53 | NM_000546 | 45612 | c.685T>C | p.C229R | Loss of Function | Missense_Mutation |
| 929 | TP53 | NM_000546 | 11133 | c.725G>C | p.C242S | Loss of Function | Missense_Mutation |
| 930 | TP53 | NM_000546 | 44935 | c.724T>A | p.C242S | Loss of Function | Missense_Mutation |
| 931 | TP53 | NM_000546 | 44313 | c.686G>A | p.C229Y | Loss of Function | Missense_Mutation |
| 932 | TP53 | NM_000546 | 10646 | c.725G>A | p.C242Y | Loss of Function | Missense_Mutation |
| 933 | TP53 | NM_000546 | 44735 | c.825T>C | p.C275C | Loss of Function | Synonymous_Mutation |
| 934 | TP53 | NM_000546 | 10701 | c.824G>T | p.C275F | Loss of Function | Missense_Mutation |
| 935 | TP53_ENST00000269305 | ENST00000269305 | 99626 | c.713G>T | p.C238F | Loss of Function | Missense_Mutation |
| 936 | TP53_ENST00000413465 | ENST00000413465 | 99624 | c.713G>T | p.C238F | Loss of Function | Missense_Mutation |
| 937 | TP53 | NM_000546 | 45413 | c.824G>C | p.C275S | Loss of Function | Missense_Mutation |
| 938 | TP53 | NM_000546 | 46336 | c.712T>G | p.C238G | Loss of Function | Missense_Mutation |
| 939 | TP53 | NM_000546 | 10893 | c.824G>A | p.C275Y | Loss of Function | Missense_Mutation |
| 940 | TP53 | NM_000546 | 44972 | c.831T>A | p.C277^{∗} | Loss of Function | Nonsense_Mutation |
| 941 | TP53 | NM_000546 | 45109 | c.831T>C | p.C277C | Loss of Function | Synonymous_Mutation |
| 942 | TP53 | NM_000546 | 10749 | c.830G>T | p.C277F | Loss of Function | Missense_Mutation |
| 943 | TP53 | NM_000546 | 44321 | c.712T>C | p.C238R | Loss of Function | Missense_Mutation |
| 944 | TP53 | NM_000546 | 44135 | c.724T>G | p.C242G | Loss of Function | Missense_Mutation |
| 945 | TP53 | NM_000546 | 11738 | c.724T>C | p.C242R | Loss of Function | Missense_Mutation |
| 946 | TP53 | NM_000546 | 45338 | c.552T>C | p.D184D | Loss of Function | Synonymous_Mutation |
| 947 | TP53 | NM_000546 | 11356 | c.726C>G | p.C242W | Loss of Function | Missense_Mutation |
| 948 | TP53_ENST00000269305 | ENST00000269305 | 99932 | c.824G>T | p.C275F | Loss of Function | Missense_Mutation |
| 949 | TP53 | NM_000546 | 11501 | c.823T>G | p.C275G | Loss of Function | Missense_Mutation |
| 950 | TP53 | NM_000546 | 45823 | c.558T>C | p.D186D | Loss of Function | Synonymous_Mutation |
| 951 | TP53 | NM_000546 | 45838 | c.556delG | p.D186fs^{∗}61 | Loss of Function | Frame_Shift_Del |
| 952 | TP53 | NM_000546 | 43902 | c.823T>C | p.C275R | Loss of Function | Missense_Mutation |
| 953 | TP53 | NM_000546 | 43823 | c.825T>G | p.C275W | Loss of Function | Missense_Mutation |
| 954 | TP53_ENST00000269305 | ENST00000269305 | 165084 | c.824G>A | p.C275Y | Loss of Function | Missense_Mutation |
| 955 | TP53 | NM_000546 | 45074 | c.829T>G | p.C277G | Loss of Function | Missense_Mutation |
| 956 | TP53 | NM_000546 | 45299 | c.831T>G | p.C277W | Loss of Function | Missense_Mutation |
| 957 | TP53 | NM_000546 | 45796 | c.623A>G | p.D208G | Loss of Function | Missense_Mutation |
| 958 | TP53 | NM_000546 | 43737 | c.830G>A | p.C277Y | Loss of Function | Missense_Mutation |
| 959 | TP53 | NM_000546 | 44249 | c.623A>T | p.D208V | Loss of Function | Missense_Mutation |
| 960 | TP53_ENST00000414315 | ENST00000414315 | 99599 | c.8G>A | p.C3Y | Loss of Function | Missense_Mutation |
| 961 | TP53 | NM_000546 | 45028 | c.684C>T | p.D228D | Loss of Function | Synonymous_Mutation |
| 962 | TP53 ENST00000545858 | ENST00000545858 | 99600 | c.125G>A | p.C42Y | Loss of Function | Missense_Mutation |
| 963 | TP53_ENST0000054S858 | ENST00000545858 | 117397 | c.248G>T | p.C83F | Loss of Function | Missense_Mutation |
| 964 | TP53_EN ST00000414315 | ENST00000414315 | 117396 | c.131G>T | p.C44F | Loss of Function | Missense_Mutation |
| 965 | TP53 | NM_000546 | 43797 | c.550G>C | P.D184H | Loss of Function | Missense_Mutation |
| 966 | TP53 | NM_000546 | 44029 | c.550G>A | p.D184N | Loss of Function | Missense_Mutation |
| 967 | TP53 | NM_000546 | 11665 | c.842A>C | p.D281A | Loss of Function | Missense_Mutation |
| 968 | TP53 | NM_000546 | 43958 | c.843C>T | p.D281D | Loss of Function | Synonymous_Mutation |
| 969 | TP53 | NM_000546 | 43837 | c.843C>G | p.D281E | Loss of Function | Missense_Mutation |
| 970 | TP53 | NM_000546 | 43906 | c.843C>A | p.D281E | Loss of Function | Missense_Mutation |
| 971 | TP53 | NM_000546 | 44202 | c.550G>T | p.D184Y | Loss of Function | Missense_Mutation |
| 972 | TP53 | NM_000546 | 10943 | c.841G>C | p.D281H | Loss of Function | Missense_Mutation |
| 973 | TP53 | NM_000546 | 43596 | c.841G>A | p.D281N | Loss of Function | Missense_Mutation |
| 974 | TP53 | NM_000546 | 45729 | c.842A>T | p.D281V | Loss of Function | Missense_Mutation |
| 975 | TP53 | NM_000546 | 11516 | c.841G>T | p.D281Y | Loss of Function | Missense_Mutation |
| 976 | TP53 | NM_000546 | 44837 | c.556G>C | p.D186H | Loss of Function | Missense_Mutation |
| 977 | TP53 | NM_000546 | 10996 | c.511G>T | p.E171* | Loss of Function | Nonsense_Mutation |
| 978 | TP53 | NM_000546 | 46095 | c.511delG | pE171fs*3 | Loss of Function | Frame_Shift_Del |
| 979 | TP53 | NM_000546 | 44700 | c.556G>A | p.D186N | Loss of Function | Missense_Mutation |
| 980 | TP53 | NM_000546 | 44312 | c.511G>A | p.E171K | Loss of Function | Missense_Mutation |
| 981 | TP53 | NM_000546 | 45519 | c.620A>G | p.D2D7G | Loss of Function | Missense_Mutation |
| 982 | TP53 | NM_000546 | 43597 | c.538G>T | p.E180* | Loss of Function | Nonsense_Mutation |
| 983 | TP53 | NM_000546 | 45372 | c.540G>T | p.E180D | Loss of Function | Missense_Mutation |
| 984 | TP53 | NM_000546 | 45707 | c.624C>A | p.D208E | Loss of Function | Missense_Mutation |
| 985 | TP53 | NM_000546 | 44241 | c.592G>T | p.E198* | Loss of Function | Nonsense_Mutation |
| 986 | TP53 | NM_000546 | 45851 | c.624C>G | p.D208E | Loss of Function | Missense_Mutation |
| 987 | TP53 | NM_000546 | 43987 | c.622G>A | p.D208N | Loss of Function | Missense_Mutation |
| 988 | TP53 | NM_000546 | 10804 | c.610G>T | p.E204* | Loss of Function | Nonsense_Mutation |
| 989 | TP53 | NM_000546 | 43761 | c.612G>A | p.E204E | Loss of Function | Synonymous_Mutation |
| 990 | TP53 | NM_000546 | 44011 | c.610delG | p.E204fs*43 | Loss of Function | Frame_Shift_Del |
| 991 | TP53 | NM_000546 | 43862 | c.683A>C | p.D228A | Loss of Function | Missense_Mutation |
| 992 | TP53 | NM_000546 | 43853 | c.684C>G | p.D228E | Loss of Function | Missense_Mutation |
| 993 | TP53 | NM_000546 | 44817 | c.661G>T | p.E221* | Loss of Function | Nonsense_Mutation |
| 994 | TP53 | NM_000546 | 43960 | c.683A>G | p.D228G | Loss of Function | Missense_Mutation |
| 995 | TP53 | NM_000546 | 44398 | c.682G>A | p.D228N | Loss of Function | Missense_Mutation |
| 996 | TP53 | NM_000546 | 43750 | c.811G>T | p.E271* | Loss of Function | Nonsense_Mutation |
| 997 | TP53 | NM_000546 | 45529 | c.683A>T | p.D228V | Loss of Function | Missense_Mutation |
| 998 | TP53 | NM_000546 | 45786 | c.682G>T | p.D228Y | Loss of Function | Missense_Mutation |
| 999 | TP53 | NM_000546 | 11232 | c.842A>G | p.0281G | Loss of Function | Missense_Mutation |
| 1000 | TP53 | NM_000546 | 10719 | c.811G>A | p.E271K | Loss of Function | Missense_Mutation |
| 1001 | TP53 | NM_000546 | 11606 | c.31G>C | p.E11Q | Loss of Function | Missense_Mutation |
| 1002 | TP53 | NM_000546 | 44469 | c.812A>T | p.E271V | Loss of Function | Missense_Mutation |
| 1003 | TP53 | NM_000546 | 44388 | c.853G>T | p.E285^{∗} | Loss of Function | Nonsense_Mutation |
| 1004 | TP53 | NM_000546 | 44732 | c.512A>G | p.E171G | Loss of Function | Missense_Mutation |
| 1005 | TP53 | NM_000546 | 44709 | c.855G>A | p.E285E | Loss of Function | Synonymous_Mutation |
| 1006 | TP53 | NM_000546 | 45751 | c.511G>C | p.E171Q | Loss of Function | Missense_Mutation |
| 1007 | TP53 | NM_000546 | 10722 | c.853G>A | p.E285K | Loss of Function | Missense_Mutation |
| 1008 | TP53 | NM_000546 | 43772 | c.538G>A | p.E180K | Loss of Function | Missense_Mutation |
| 1009 | TP53 | NM_000546 | 43690 | c.592G>A | p.E198K | Loss of Function | Missense_Mutation |
| 1010 | TP53 | NM_000546 | 43919 | c.856G>T | p.E286* | Loss of Function | Nonsense_Mutation |
| 1011 | TP53 | NM_000546 | 44292 | c.858A>G | p.E286E | Loss of Function | Synonymous_Mutation |
| 1012 | TP53 | NM_000546 | 44651 | c.856_863delGAAGAGAA | p.E286fs*17 | Loss of Function | Frame_Shift_Del |
| 1013 | TP53 | NM_000546 | 45277 | c.856delG | p.E286fs*59 | Loss of Function | Frame_Shift_Del |
| 1014 | TP53 | NM_000546 | 43565 | c.857A>G | p.E286G | Loss of Function | Missense_Mutation |
| 1015 | TP53 | NM_000546 | 10726 | c.856G>A | p.E286K | Loss of Function | Missense_Mutation |
| 1016 | TP53 | NM_000546 | 44250 | c.856G>C | p.E286Q | Loss of Function | Missense_Mutation |
| 1017 | TP53 | NM_000546 | 43936 | c.857A>T | p.E286V | Loss of Function | Missense_Mutation |
| 1018 | TP53 | NM_000546 | 44133 | c.859G>T | p.E287* | Loss of Function | Nonsense_Mutation |
| 1019 | TP53 | NM_000546 | 43776 | c.861G>A | p.E287E | Loss of Function | Synonymous_Mutation |
| 1020 | TP53 | NM_000546 | 45449 | c.592G>C | p.E198Q | Loss of Function | Missense_Mutation |
| 1021 | TP53 | NM_000546 | 45253 | c.611A>G | p.E204G | Loss of Function | Missense_Mutation |
| 1022 | TP53 | NM_000546 | 10856 | c.880G>T | p.E294* | Loss of Function | Nonsense_Mutation |
| 1023 | TP53 | NM_000546 | 43990 | c.610G>A | pE204K | Loss of Function | Missense_Mutation |
| 1024 | TP53 | NM_000546 | 45516 | c.662A>G | p.E221G | Loss of Function | Missense_Mutation |
| 1025 | TP53 | NM_000546 | 44207 | c.874delA | p.E294fs*51 | Loss of Function | Frame_Shift_Del |
| 1026 | TP53 | NM_000546 | 45670 | c.877deIG | p.E294fs*51 | Loss of Function | Frame_Shift_Del |
| 1027 | TP53 | NM_000546 | 6621 | c.880delG | p.E294fs*S1 | Loss of Function | Frame_Shift_Del |
| 1028 | TP53 | NM_000546 | 44853 | c.661G>A | p.E221K | Loss of Function | Missense_Mutation |
| 1029 | TP53 | NM_000546 | 44441 | c.813G>C | p.E271D | Loss of Function | Missense_Mutation |
| 1030 | TP53 | NM_000546 | 45284 | c.813G>A | p.E271E | Loss of Function | Synonymous_Mutation |
| 1031 | TP53 | NM_000546 | 10710 | c.892G>T | p.E298* | Loss of Function | Nonsense_Mutation |
| 1032 | TP53 | NM_000546 | 43879 | c.812A>G | p.E271G | Loss of Function | Missense_Mutation |
| 1033 | TP53 | NM_000546 | 11291 | c.1006G>T | p.E336* | Loss of Function | Nonsense_Mutation |
| 1034 | TP53 | NM_000546 | 11286 | c.1015G>T | p.E339* | Loss of Function | Nonsense_Mutation |
| 1035 | TP53 | NM_000546 | 11078 | c.1027G>T | p.E343* | Loss of Function | Nonsense_Mutation |
| 1036 | TP53 | NM_000546 | 10770 | c.1045G>T | p.E349* | Loss of Function | Nonsense_Mutation |
| 1037 | TP53 | NM_000546 | 43706 | c.811G>C | p.E271Q | Loss of Function | Missense_Mutation |
| 1038 | TP53 | NM_000546 | 43614 | c.854A>C | p.E285A | Loss of Function | Missense_Mutation |
| 1039 | TP53 | NM_000546 | 45649 | c.854A>G | p.E285G | Loss of Function | Missense_Mutation |
| 1040 | TP53 | NM_000546 | 44654 | c.400T>C | p.F134L | Loss of Function | Missense_Mutation |
| 1041 | TP53_ENST00000269305 | ENST00000269305 | 137087 | c.853G>A | p.E285K | Loss of Function | Missense_Mutation |
| 1042 | TP53 | NM_000546 | 43941 | c.400T>G | p.F134V | Loss of Function | Missense_Mutation |
| 1043 | TP53 | NM_000546 | 44162 | c.635_636delπ | p.F212fs*3 | Loss of Function | Frame_Shift_Del |
| 1044 | TP53 | NM_000546 | 44695 | c.634_635delπ | p.F212fs*3 | Loss of Function | Frame_Shift_Del |
| 1045 | TP53 | NM_000546 | 45138 | c.853G>C | p.E285Q | Loss of Function | Missense_Mutation |
| 1046 | TP53 | NM_000546 | 44227 | c.854A>T | p.E285V | Loss of Function | Missense_Mutation |
| 1047 | TP53_EN ST00000269305 | ENST00000269305 | 99924 | c.856G>A | p.E286K | Loss of Function | Missense_Mutation |
| 1048 | TP53 | NM_000546 | 43621 | c.809T>G | p.F270C | Loss of Function | Missense_Mutation |
| 1049 | TP53 | NM_000546 | 43809 | c.808T>A | p.F270I | Loss of Function | Missense_Mutation |
| 1050 | TP53 | NM_000546 | 44156 | c.810T>A | p.F270L | Loss of Function | Missense_Mutation |
| 1051 | TP53 | NM_000546 | 44262 | c.808T>C | p.F270L | Loss of Function | Missense_Mutation |
| 1052 | TP53 | NM_000546 | 45297 | c.810T>G | p.F270L | Loss of Function | Missense_Mutation |
| 1053 | TP53 | NM_000546 | 11305 | c.809T>C | P.F270S | Loss of Function | Missense_Mutation |
| 1054 | TP53 | NM_000546 | 44737 | c.860A>G | p.E287G | Loss of Function | Missense_Mutation |
| 1055 | TP53 | NM_000546 | 44225 | c.859G>A | p.E287K | Loss of Function | Missense_Mutation |
| 1056 | TP53 | NM_000546 | 42813 | c.313G>T | p.GIDSC | Loss of Function | Missense_Mutation |
| 1057 | TP53 | NM_000546 | 44481 | c.313G>T | p.GIDSC | Loss of Function | Missense_Mutation |
| 1058 | TP53 | NM_000546 | 45801 | c.312delG | p.G105fs*18 | Loss of Function | Frame_Shift_Del |
| 1059 | TP53 | NM_000546 | 45179 | c.313G>C | ^{p}.G10SR | Loss of Function | Missense_Mutation |
| 1060 | TP53 | NM_000546 | 45534 | c.882G>T | p.E294D | Loss of Function | Missense_Mutation |
| 1061 | TP53 | NM_000546 | 44715 | c.460G>T | p.G154C | Loss of Function | Missense_Mutation |
| 1062 | TP53 | NM_000546 | 44412 | c.882G>A | p.E294E | Loss of Function | Synonymous_Mutation |
| 1063 | TP53 | NM_000546 | 44726 | c.460_466delGGCACCC | p.G154fs*14 | Loss of Function | Frame_Shift_Del |
| 1064 | TP53 | NM_000546 | 43666 | c.462C>T | p.G154G | Loss of Function | Synonymous_Mutation |
| 1065 | TP53 | NM_000546 | 44298 | c.462C>A | p.G154G | Loss of Function | Synonymous_Mutation |
| 1066 | TP53 | NM_000546 | 43746 | c.881A>G | p.E294G | Loss of Function | Missense_Mutation |
| 1067 | TP53 | NM_000546 | 44127 | c.880G>A | p.E294K | Loss of Function | Missense_Mutation |
| 1068 | TP53 | NM_000546 | 6815 | c.461G>T | p.G154V | Loss of Function | Missense_Mutation |
| 1069 | TP53 | NM_000546 | 45824 | c.880G>C | p.E294Q | Loss of Function | Missense_Mutation |
| 1070 | TP53 | NM_000546 | 45820 | c.893A>T | p.E298V | Loss of Function | Missense_Mutation |
| 1071 | TP53 | NM_000546 | 44026 | c.559G>A | p.G187S | Loss of Function | Splice_Site |
| 1072 | TP53 | NM_000546 | 45169 | c.326T>C | p.F109S | Loss of Function | Missense_Mutation |
| 1073 | TP53 | NM_000546 | 44537 | c.595G>T | P.G199* | Loss of Function | Nonsense_Mutation |
| 1074 | TP53 | NM_000546 | 43949 | c.401T>G | p.F134C | Loss of Function | Missense_Mutation |
| 1075 | TP53 | NM_000546 | 45051 | c.597A>G | p.G199G | Loss of Function | Synonymous_Mutation |
| 1076 | TP53 | NM_000546 | 11319 | c.402T>G | P.F134L | Loss of Function | Missense_Mutation |
| 1077 | TP53 | NM_000546 | 44140 | c.596G>T | p.G199V | Loss of Function | Missense_Mutation |
| 1078 | TP53 | NM_000546 | 44506 | c.401T>C | p.F134S | Loss of Function | Missense_Mutation |
| 1079 | TP53 | NM_000546 | 45703 | c.634T>A | p.F212! | Loss of Function | Missense_Mutation |
| 1080 | TP53 | NM_000546 | 45868 | c.678C>T | p.G226G | Loss of Function | Synonymous_Mutation |
| 1081 | TP53 | NM_000546 | 44846 | c.636T>A | p.F212L | Loss of Function | Missense_Mutation |
| 1082 | TP53 | NM_000546 | 46214 | c.635T>C | P.F212S | Loss of Function | Missense_Mutation |
| 1083 | TP53 | NM_000546 | 44956 | c.808T>G | p.F270V | Loss of Function | Missense_Mutation |
| 1084 | TP53 | NM_000546 | 11524 | c.730G>T | p.G244C | Loss of Function | Missense_Mutation |
| 1085 | TP53 | NM_000546 | 10883 | c.731G>A | p.G244D | Loss of Function | Missense_Mutation |
| 1086 | TP53 | NM_000546 | 44940 | c.730delG | p.G244fs*3 | Loss of Function | Frame_Shift_Del |
| 1087 | TP53 | NM_000546 | 43656 | c.732C>G | p.G244G | Loss of Function | Synonymous_Mutation |
| 1088 | TP53 | NM_000546 | 44513 | c.732C>A | p.G244G | Loss of Function | Synonymous_Mutation |
| 1089 | TP53 | NM_000546 | 44787 | c.732C>T | p.G244G | Loss of Function | Synonymous_Mutation |
| 1090 | TP53 | NM_000546 | 44221 | c.730G>C | p.G244R | Loss of Function | Missense_Mutation |
| 1091 | TP53 | NM_000546 | 10941 | c.730G>A | P.G244S | Loss of Function | Missense_Mutation |
| 1092 | TP53 | NM_000546 | 43918 | c.809T>A | p.F270Y | Loss of Function | Missense_Mutation |
| 1093 | TP53 | NM_000546 | 13119 | c.322_324delGGT | p.G108del | Loss of Function | In_Frame_Del |
| 1094 | TP53 | NM_000546 | 11081 | c.733G>T | p.G245C | Loss of Function | Missense_Mutation |
| 1095 | TP53 | NM_000546 | 39293 | c.734G>A | p.G245D | Loss of Function | Missense_Mutation |
| 1096 | TP53 | NM_000546 | 43606 | c.734G>A | p.G245D | Loss of Function | Missense_Mutation |
| 1097 | TP53 | NM_000546 | 44642 | c.733delG | p.G245fs*2 | Loss of Function | Frame_Shift_Del |
| 1098 | TP53 | NM_000546 | 44900 | c.735C>T | p.G245G | Loss of Function | Synonymous_Mutation |
| 1099 | TP53_ENST0000054S858 | ENST00000545858 | 179807 | c.455G>A | P.G152D | Loss of Function | Missense_Mutation |
| 1100 | TP53 | NM_000546 | 10957 | c.733G>C | p.G245R | Loss of Function | Missense_Mutation |
| 1101 | TP53 | NM_000546 | 6932 | c.733G>A | P.G245S | Loss of Function | Missense_Mutation |
| 1102 | TP53 | NM_000546 | 11196 | c.734G>T | p.G245V | Loss of Function | Missense_Mutation |
| 1103 | TP53 | NM_000546 | 44891 | c.796G>T | p.G266* | Loss of Function | Nonsense_Mutation |
| 1104 | TP53_ENST0000054S858 | ENST00000545858 | 121037 | c.454G>A | p.G152S | Loss of Function | Missense_Mutation |
| 1105 | TP53 | NM_000546 | 10867 | c.797G>A | P.G266E | Loss of Function | Missense_Mutation |
| 1106 | TP53 | NM_000546 | 10794 | c.796G>A | p.G266R | Loss of Function | Missense_Mutation |
| 1107 | TP53 | NM_000546 | 11205 | c.796G>C | p.G266R | Loss of Function | Missense_Mutation |
| 1108 | TP53 | NM_000546 | 10958 | c.797G>T | p.G266V | Loss of Function | Missense_Mutation |
| 1109 | TP53 | NM_000546 | 43714 | c.836G>A | p.G279E | Loss of Function | Missense_Mutation |
| 1110 | TP53 | NM_000546 | 44896 | c.835_838delGGGA | p.G279fs*65 | Loss of Function | Frame_Shift_Del |
| 1111 | TP53 | NM_000546 | 46284 | c.837G>A | p.G279G | Loss of Function | Synonymous_Mutation |
| 1112 | TP53 | NM_000546 | 44603 | c.835G>A | p.G279R | Loss of Function | Missense_Mutation |
| 1113 | TP53 | NM_000546 | 45622 | c.461G>A | p.G154D | Loss of Function | Missense_Mutation |
| 1114 | TP53 | NM_000546 | 45506 | c.460_461GG>AT | p.G1541 | Loss of Function | Missense_Mutation |
| 1115 | TP53 | NM_000546 | 44128 | c.879G>A | p.G293G | Loss of Function | Synonymous_Mutation |
| 1116 | TP53 | NM_000546 | 44131 | c.879G>C | p.G293G | Loss of Function | Synonymous_Mutation |
| 1117 | TP53 | NM_000546 | 43692 | c.460G>A | p.S154S | Loss of Function | Missense_Mutation |
| 1118 | TP53 | NM_000546 | 45275 | c.559G>T | p.G187C | Loss of Function | Missense_Mutation |
| 1119 | TP53 | NM_000546 | 87513 | c.902_903insC | p.G302fs*4 | Loss of Function | Frame_Shift_lns |
| 1120 | TP53 | NM_000546 | 45998 | c.906G>C | p.G302G | Loss of Function | Synonymous_Mutation |
| 1121 | TP53 | NM_000546 | 11514 | c.1001G>T | p.G334V | Loss of Function | Missense_Mutation |
| 1122 | TP53 | NM_000546 | 44023 | c.560G>A | p.G187D | Loss of Function | Missense_Mutation |
| 1123 | TP53 | NM_000546 | 45479 | c.504C>T | p.H168H | Loss of Function | Synonymous_Mutation |
| 1124 | TP53 | NM_000546 | 44801 | c.503A>T | p.H168L | Loss of Function | Missense_Mutation |
| 1125 | TP53 | NM_000546 | 44808 | c.503A>C | p.H168P | Loss of Function | Missense_Mutation |
| 1126 | TP53 | NM_000546 | 45240 | c.560G>T | p.G187V | Loss of Function | Missense_Mutation |
| 1127 | TP53 | NM_000546 | 43989 | c.596G>A | p.G199E | Loss of Function | Missense_Mutation |
| 1128 | TP53 | NM_000546 | 44901 | c.532C>G | p.H178D | Loss of Function | Missense_Mutation |
| 1129 | TP53 | NM_000546 | 43978 | c.529delC | p.H178fs*69 | Loss of Function | Frame_Shift_Del |
| 1130 | TP53 | NM_000546 | 44134 | c.528delC | p.H178fs*69 | Loss of Function | Frame_Shift_Del |
| 1131 | TP53 | NM_000546 | 44659 | c.532delC | p.H178fs*69 | Loss of Function | Frame_Shift_Del |
| 1132 | TP53 | NM_000546 | 44971 | c.534C>T | p.H178H | Loss of Function | Synonymous_Mutation |
| 1133 | TP53 | NM_000546 | 43749 | c.595G>A | p.G199R | Loss of Function | Missense _Mutation |
| 1134 | TP53 | NM_000546 | 45739 | c.677G>C | p.G226A | Loss of Function | Missense_Mutation |
| 1135 | TP53 | NM_000546 | 11998 | c.534C>A | p..H178Q | Loss of Function | Missense_Mutation |
| 1136 | TP53 | NM_000546 | 46163 | c.534C>G | p.H178Q | Loss of Function | Missense_Mutation |
| 1137 | TP53 | NM_000546 | 44547 | c.677G>A | p.G226D | Loss of Function | Missense_Mutation |
| 1138 | TP53 | NM_000546 | 44776 | c.535C>G | P.H179D | Loss of Function | Missense_Mutation |
| 1139 | TP53 | NM_000546 | 44793 | c.537T>C | p.H179H | Loss of Function | Synonymous_Mutation |
| 1140 | TP53 | NM_000546 | 43635 | c.536A>T | p.H179L | Loss of Function | Missense_Mutation |
| 1141 | TP53 | NM_000546 | 44151 | c.535C>A | p.H179N | Loss of Function | Missense_Mutation |
| 1142 | TP53 | NM_000546 | 44218 | c.536A>C | p.H179P | Loss of Function | Missense_Mutation |
| 1143 | TP53 | NM_000546 | 11249 | c.537T>G | pH179Q | Loss of Function | Missense_Mutation |
| 1144 | TP53 | NM_000546 | 44214 | c.537T>A | p.H179Q | Loss of Function | Missense_Mutation |
| 1145 | TP53 | NM_000546 | 10889 | c.536A>G | p.H179R | Loss of Function | Missense_Mutation |
| 1146 | TP53 | NM_000546 | 10768 | c.535C>T | p.H179Y | Loss of Function | Missense_Mutation |
| 1147 | TP53 | NM_000546 | 43584 | c.534_535CC>TT | p.H179Y | Loss of Function | Missense_Mutation |
| 1148 | TP53 | NM_000546 | 44002 | c.577C>G | p.H193D | Loss of Function | Missense Mutation |
| 1149 | TP53 | NM_000546 | 44848 | c.579T>C | p.H193H | Loss of Function | Synonymous_Mutation |
| 1150 | TP53 | NM_000546 | 11066 | c.578A>T | p.H193L | Loss of Function | Missense_Mutation |
| 1151 | TP53 | NM_000546 | 45607 | c.676G>A | p.G226S | Loss of Function | Missense_Mutation |
| 1152 | TP53 | NM_000546 | 43833 | c.578A>C | p.H193P | Loss of Function | Missense_Mutation |
| 1153 | TP53 | NM_000546 | 10742 | c.578A>G | p.H193R | Loss of Function | Missense_Mutation |
| 1154 | TP53 | NM_000546 | 10672 | c.577C>T | p.H193Y | Loss of Function | Missense_Mutation |
| 1155 | TP53 | NM_000546 | 44399 | c.677G>T | p.G226V | Loss of Function | Missense_Mutation |
| 1156 | TP53 | NM_000546 | 44372 | c.640delC | p.H214fs*33 | Loss of Function | Frame_Shift_Del |
| 1157 | TP53 | NM_000546 | 44638 | c.640_647delCATAGTGT | p.H214fs*5 | Loss of Function | Frame_Shift_Del |
| 1158 | TP53 | NM_000546 | 12013 | c.731G>C | p.G244A | Loss of Function | Missense_Mutation |
| 1159 | TP53 | NM_000546 | 42811 | c.641A>G | p.H214R | Loss of Function | Missense_Mutation |
| 1160 | TP53 | NM_000546 | 43687 | c.641A>G | p.H214R | Loss of Function | Missense_Mutation |
| 1161 | TP53 | NM_000546 | 43652 | c.731G>T | p.G244V | Loss of Function | Missense_Mutation |
| 1162 | TP53 | NM_000546 | 43965 | c.734G>C | p.G245A | Loss of Function | Missense_Mutation |
| 1163 | TP53_ENST0000026930S | ENST00000269305 | 179806 | c.734G>A | p.G245D | Loss of Function | Missense_Mutation |
| 1164 | TP53_ENST0000041346S | ENST00000413465 | 179805 | c.734G>A | p.G245D | Loss of Function | Missense_Mutation |
| 1165 | TP53 | NM_000546 | 45410 | c.733_734GG>AA | p.G245N | Loss of Function | Missense_Mutation |
| 1166 | TP53 | NM_000546 | 45069 | c.886delC | p.H296fs*49 | Loss of Function | Frame_Shift_Del |
| 1167 | TP53_ENST0000026930S | ENST00000269305 | 121035 | c.733G>A | p.G2455 | Loss of Function | Missense_Mutation |
| 1168 | TP53_ENST0000041346S | ENST00000413465 | 121036 | c.733G>A | p.G245S | Loss of Function | Missense_Mutation |
| 1169 | TP53 | NM_000546 | 45488 | c.797G>C | p.G266A | Loss of Function | Missense_Mutation |
| 1170 | TP53₋ENST00000269305 | ENST00000269305 | 99952 | c.797G>T | p.G266V | Loss of Function | Missense_Mutation |
| 1171 | TP53 | NM_000546 | 46032 | c.836G>T | p.G279V | Loss of Function | Missense_Mutation |
| 1172 | TP53 | NM_000546 | 43674 | c.835G>T | p.G279W | Loss of Function | Missense_Mutation |
| 1173 | TP53 | NM_000546 | 45417 | c.877G>A | p.G293R | Loss of Function | Missense_Mutation |
| 1174 | TP53 | NM_000546 | 43988 | c.905G>A | p.G302E | Loss of Function | Missense_Mutation |
| 1175 | TP53 | NM_000546 | 44830 | c.1066G>T | p.G356W | Loss of Function | Missense_Mutation |
| 1176 | TP53_ENST0000054S858 | ENST00000545858 | 99918 | c.299A>T | p.H100L | Loss of Function | Missense_Mutation |
| 1177 | TP53 | NM_000546 | 43545 | c.503A>G | p.H168R | Loss of Function | Missense _Mutation |
| 1178 | TP53 | NM_000546 | 43861 | c.502C>T | p.H168Y | Loss of Function | Missense_Mutation |
| 1179 | TP53 | NM_000546 | 44633 | c.583A>T | p.I195F | Loss of Function | Missense _Mutation |
| 1180 | TP53 | NM_000546 | 44877 | c.584T>A | p.I195N | Loss of Function | Missense_Mutation |
| 1181 | TP53 | NM_000546 | 44539 | c.584T>G | p.I1955 | Loss of Function | Missense_Mutation |
| 1182 | TP53 | NM_000546 | 11089 | c.584T>C | p.195T | Loss of Function | Missense_Mutation |
| 1183 | TP53 | NM_000546 | 43550 | c.694A>T | p.I232F | Loss of Function | Missense_Mutation |
| 1184 | TP53 | NM_000546 | 10715 | c.695T>A | p.I232N | Loss of Function | Missense_Mutation |
| 1185 | TP53 | NM_000546 | 45045 | c.695T>G | p.I232S | Loss of Function | Missense_Mutation |
| 1186 | TP53 | NM_000546 | 44601 | c.695T>C | p.I232T | Loss of Function | Missense_Mutation |
| 1187 | TP53 | NM_000546 | 44068 | c.532C>A | p.H178N | Loss of Function | Missense_Mutation |
| 1188 | TP53 | NM_000546 | 44457 | c.751_759delATCCTCACC | p.I251 _T253delILT | Loss of Function | In_Frame_Del |
| 1189 | TP53 | NM_000546 | 44215 | c.533A>C | p.H178P | Loss of Function | Missense_Mutation |
| 1190 | TP53 | NM_000546 | 43967 | c.751A>T | p.I251F | Loss of Function | Missense_Mutation |
| 1191 | TP53 | NM_000546 | 44064 | c.748delC | p.I251fs*94 | Loss of Function | Frame_Shift_Del |
| 1192 | TP53 | NM_000546 | 44124 | c.751delA | p.I251fs*94 | Loss of Function | Frame_Shift_Del |
| 1193 | TP53 | NM_000546 | 44511 | c.753C>A | p.I251I | Loss of Function | Synonymous_M utation |
| 1194 | TP53 | NM_000546 | 44120 | c.532C>T | p.H178Y | Loss of Function | Missense_Mutation |
| 1195 | TP53 | NM_000546 | 11374 | c.752T>A | p.I251N | Loss of Function | Missense_Mutation |
| 1196 | TP53 | NM_000546 | 43829 | c.752T>G | p.I251S | Loss of Function | Missense_Mutation |
| 1197 | TP53_ENST0000026930S | ENST00000269305 | 129848 | c.535C>T | p.H179Y | Loss of Function | Missense_Mutation |
| 1198 | TP53_ENST0000041346S | ENST00000413465 | 129849 | c.535C>T | p.H179Y | Loss of Function | Missense_Mutation |
| 1199 | TP53_ENST00000269305 | ENST00000269305 | 99919 | c.578A>T | P.H193L | Loss of Function | Missense_Mutation |
| 1200 | TP53_ENST00000413465 | ENST00000413465 | 99916 | c.578A>T | p.H193L | Loss of Function | Missense_Mutation |
| 1201 | TP53 | NM_000546 | 43935 | c.577C>A | p.H193N | Loss of Function | Missense_Mutation |
| 1202 | TP53 | NM_000546 | 45035 | c.761T>G | p.12545 | Loss of Function | Missense_Mutation |
| 1203 | TP53 | NM_000546 | 45115 | c.640C>G | p.H214D | Loss of Function | Missense_Mutation |
| 1204 | TP53 | NM_000546 | 44407 | c.642T>G | p.H214Q | Loss of Function | Missense_Mutation |
| 1205 | TP53 | NM_000546 | 43651 | c.763A>T | p.I255F | Loss of Function | Missense_Mutation |
| 1206 | TP53 | NM_000546 | 11244 | c.764T>A | p.I255N | Loss of Function | Missense_Mutation |
| 1207 | TP53 | NM_000546 | 10788 | c.764T>G | p.12555 | Loss of Function | Missense_Mutation |
| 1208 | TP53 | NM_000546 | 44112 | c.640C>T | p.H214Y | Loss of Function | Missense_Mutation |
| 1209 | TP53 | NM_000546 | 46031 | c.697C>G | p.H233D | Loss of Function | Missense_Mutation |
| 1210 | TP53 | NM_000546 | 45959 | c.698A>T | p.H233L | Loss of Function | Missense_Mutation |
| 1211 | TP53 | NM_000546 | 44641 | c.394A>T | p.K132* | Loss of Function | Nonsense_Mutation |
| 1212 | TP53 | NM_000546 | 10813 | c.394A>G | p.K132E | Loss of Function | Missense_Mutation |
| 1213 | TP53 | NM_000546 | 43661 | c.394delA | p.K132fs*38 | Loss of Function | Frame_Shift_Del |
| 1214 | TP53 | NM_000546 | 43592 | c.395A>T | p.K132M | Loss of Function | Missense_Mutation |
| 1215 | TP53 | NM_000546 | 10991 | c.396G>T | p.K132N | Loss of Function | Missense_Mutation |
| 1216 | TP53 | NM_000546 | 43963 | c.396G>C | p.K132N | Loss of Function | Missense_Mutation |
| 1217 | TP53 | NM_000546 | 44350 | c.699C>G | p.H233Q | Loss of Function | Missense_Mutation |
| 1218 | TP53 | NM_000546 | 11582 | c.395A>G | p.K132R | Loss of Function | Missense_Mutation |
| 1219 | TP53 | NM_000546 | 43912 | c.395A>C | p.K132T | Loss of Function | Missense_Mutation |
| 1220 | TP53 | NM_000546 | 10750 | c.490A>T | p.K164 * | Loss of Function | Nonsense_Mutation |
| 1221 | TP53 | NM_000546 | 10762 | c.490A>G | p.K164E | Loss of Function | Missense_Mutation |
| 1222 | TP53 | NM_000546 | 45187 | c.490_ 499del10 | p.K164fs*3 | Loss of Function | Frame_Shift_Del |
| 1223 | TP53 | NM_000546 | 44861 | c.490delA | p.K164fs*6 | Loss of Function | Frame_Shift_Del |
| 1224 | TP53 | NM_000546 | 45103 | c.492G>A | p.K164K | Loss of Function | Synonymous_Mutation |
| 1225 | TP53 | NM_000546 | 44705 | c.697C>T | p.H233Y | Loss of Function | Missense_Mutation |
| 1226 | TP53 | NM_000546 | 44522 | c.887A>T | p.H296L | Loss of Function | Missense_Mutation |
| 1227 | TP53 | NM_000546 | 45306 | c.886C>A | p.H296N | Loss of Function | Missense_Mutation |
| 1228 | TP53 | NM_000546 | 43915 | c.886C>T | p.H296Y | Loss of Function | Missense_Mutation |
| 1229 | TP53 | NM_000546 | 44475 | c.871A>T | p.K291* | Loss of Function | Nonsense_Mutation |
| 1230 | TP53 | NM_000546 | 45494 | c.888_889CC>TT | p.H297Y | Loss of Function | Missense_Mutation |
| 1231 | TP53 | NM_000546 | 44897 | c.871_889del19 | p.K291fs*48 | Loss of Function | Frame_Shift_Del |
| 1232 | TP53 | NM_000546 | 46224 | c.873G>A | p.K291K | Loss of Function | Synonymous_Mutation |
| 1233 | TP53 | NM_000546 | 45803 | c.889C>T | p.H297Y | Loss of Function | Missense_Mutation |
| 1234 | TP53 _E NST0000041431S | ENST00000414315 | 129851 | c.139C>T | p.H47Y | Loss of Function | Missense_Mutation |
| 1235 | | ENST00000414315 | 99917 | c.182A>T | p.H61L | Loss of Function | Missense_Mutation |
| 1236 | TP53_ENST0000054S858 | ENST00000545858 | 129850 | c.256C>T | p.H86Y | Loss of Function | Missense_Mutation |
| 1237 | TP53 | NM_000546 | 44320 | c.484A>T | p.1162F | Loss of Function | Missense _Mutation |
| 1238 | TP53 | NM_000546 | 44694 | c.486C>A | p.1162I | Loss of Function | Missense_Mutation |
| 1239 | TP53 | NM_000546 | 45627 | c.486C>T | p.1162I | Loss of Function | Missense_Mutation |
| 1240 | TP53 | NM_000546 | 43773 | c.913A>T | p.K305^{∗} | Loss of Function | Nonsense_Mutation |
| 1241 | TP53 | NM_000546 | 44125 | c.486C>G | p.I162M | Loss of Function | Missense_Mutation |
| 1242 | TP53 | NM_000546 | 11966 | c.485T>A | p.I162N | Loss of Function | Missense_Mutation |
| 1243 | TP53 | NM_000546 | 11449 | c.388C>T | p.L130F | Loss of Function | Missense_Mutation |
| 1244 | TP53 | NM_000546 | 43898 | c.485T>G | p.11625 | Loss of Function | Missense_Mutation |
| 1245 | TP53 | NM_000546 | 45077 | c.390C>T | p.L130L | Loss of Function | Synonymous_M utation |
| 1246 | TP53 | NM_000546 | 44413 | c.484A>G | p.1162V | Loss of Function | Missense_Mutation |
| 1247 | TP53 | NM_000546 | 11462 | c.388C>G | p.L130V | Loss of Function | Missense . Mutation |
| 1248 | TP53 | NM_000546 | 10995 | c.580C>T | p.L194F | Loss of Function | Missense_Mutation |
| 1249 | TP53 | NM_000546 | 43623 | c.581T>A | p.L194H | Loss of Function | Missense_Mutation |
| 1250 | TP53 | NM_000546 | 43929 | c.582T>C | P.L194L | Loss of Function | Synonymous_Mutation |
| 1251 | TP53 | NM_000546 | 43827 | c.581T>C | P.L194P | Loss of Function | Missense_Mutation |
| 1252 | TP53 | NM_000546 | 44571 | c.581T>G | p.L194R | Loss of Function | Missense_Mutation |
| 1253 | TP53 | NM_000546 | 44622 | c.694A>G | p.1232V | Loss of Function | Missense_Mutation |
| 1254 | TP53 | NM_000546 | 44650 | c.751_753delATC | p.l251del | Loss of Function | In_Frame_Del |
| 1255 | TP53 | NM_000546 | 44157 | c.601delT | p.L201fs*46 | Loss of Function | Frame_Shift_Del |
| 1256 | TP53 | NM_000546 | 43793 | c.617T>A | p.L206^{∗} | Loss of Function | Nonsense_Mutation |
| 1257 | TP53 | NM_000546 | 44852 | c.617delT | p.L206fs^{∗}41 | Loss of Function | Frame_Shift_Del |
| 1258 | TP53 | NM_000546 | 10931 | c.751A>C | p.1251L | Loss of Function | Missense_Mutation |
| 1259 | TP53 | NM_000546 | 11213 | c.752T>C | p.1251T | Loss of Function | Missense . Mutation |
| 1260 | TP53 | NM_000546 | 44541 | c.754delC | p.L252fs*93 | Loss of Function | Frame_Shift_Del |
| 1261 | TP53 | NM_000546 | 45407 | c.751A>G | p.1251V | Loss of Function | Missense . Mutation |
| 1262 | TP53 | NM_000546 | 44769 | c.755T>C | p.L252P | Loss of Function | Missense_Mutation |
| 1263 | TP53 | NM_000546 | 11929 | c.760_761AT>GA | p.1254D | Loss of Function | Missense . Mutation |
| 1264 | TP53 | NM_000546 | 11011 | c.794T>C | p.L265P | Loss of Function | Missense_Mutation |
| 1265 | TP53 | NM_000546 | 44092 | c.794T>G | p.L265R | Loss of Function | Missense _Mutation |
| 1266 | TP53 | NM_000546 | 45446 | c.865C>T | p.L289F | Loss of Function | Missense_Mutation |
| 1267 | TP53 | NM_000546 | 45688 | c.867C>T | p.L289L | Loss of Function | Synonymous_Mutation |
| 1268 | TP53 | NM_000546 | 45647 | c.760A>T | p12S4F | Loss of Function | Missense_Mutation |
| 1269 | TP53 | NM_000546 | 44535 | c.761T>A | p.1254N | Loss of Function | Missense_Mutation |
| 1270 | TP53 | NM_000546 | 46015 | c.1043T>A | p.L348* | Loss of Function | Nonsense_Mutation |
| 1271 | TP53 | NM_000546 | 46348 | c.1044G>T | p.L348F | Loss of Function | Missense_Mutation |
| 1272 | TP53 | NM_000546 | 45586 | c.397delA | p.M133fs^{∗}37 | Loss of Function | Frame_Shift_Del |
| 1273 | TP53 | NM_000546 | 44058 | c.761T>C | p.1254T | Loss of Function | Missense_Mutation |
| 1274 | TP53 | NM_000546 | 11781 | c.398T>A | P.M133K | Loss of Function | Missense_Mutation |
| 1275 | TP53 | NM_000546 | 44030 | c.760A>G | p.1254V | Loss of Function | Missense_Mutation |
| 1276 | TP53 | NM_000546 | 11181 | c.764T>C | p.1255T | Loss of Function | Missense_Mutation |
| 1277 | TP53 | NM_000546 | 44290 | c.763A>G | p.1255V | Loss of Function | Missense_Mutation |
| 1278 | TP53 | NM_000546 | 44986 | c.302A>G | p.K101R | Loss of Function | Missense_Mutation |
| 1279 | TP53 | NM_000546 | 11224 | c.394A>C | p.K132Q | Loss of Function | Missense_Mutation |
| 1280 | TP53 | NM_000546 | 44841 | c.491A>T | p.K164M | Loss of Function | Missense_Mutation |
| 1281 | TP53 | NM_000546 | 11369 | c.492G>T | p.K164N | Loss of Function | Missense_Mutation |
| 1282 | TP53 | NM_000546 | 44126 | c.507G>A | p.M169I | Loss of Function | Missense_Mutation |
| 1283 | TP53 | NM_000546 | 45490 | c.507G>T | p.M169I | Loss of Function | Missense_Mutation |
| 1284 | TP53 | NM_000546 | 44521 | c.490A>C | p.K164Q | Loss of Function | Missense_Mutation |
| 1285 | TP53 | NM_000546 | 44397 | c.491A>C | p.K164T | Loss of Function | Missense_Mutation |
| 1286 | TP53 | NM_000546 | 45162 | c.709delA | p.M237fs*10 | Loss of Function | Frame_Shift_Del |
| 1287 | TP53 | NM_000546 | 45862 | c.710delT | p.M237fs*10 | Loss of Function | Frame_Shift_Del |
| 1288 | TP53 | NM_000546 | 10834 | c.711G>A | p.M237I | Loss of Function | Missense_Mutation |
| 1289 | TP53 | NM_000546 | 11063 | c.711G>T | p.M237I | Loss of Function | Missense_Mutation |
| 1290 | TP53 | NM_000546 | 44415 | c.711G>C | p.M2371 | Loss of Function | Missense_Mutation |
| 1291 | TP53 | NM_000546 | 43952 | c.710T>A | p.M237K | Loss of Function | Missense_Mutation |
| 1292 | TP53 | NM_000546 | 45050 | c.871A>G | p.K291E | Loss of Function | Missense_Mutation |
| 1293 | TP53 | NM_000546 | 44446 | c.873G>C | p.K291N | Loss of Function | Missense_Mutation |
| 1294 | TP53 | NM_000546 | 43747 | c.872A>G | p.K291R | Loss of Function | Missense_Mutation |
| 1295 | TP53 | NM_000546 | 44525 | c.709A>G | p.M237V | Loss of Function | Missense_Mutation |
| 1296 | TP53 | NM_000546 | 44433 | c.872A>C | p.K291T | Loss of Function | Missense_Mutation |
| 1297 | TP53 | NM_000546 | 44451 | c.874A>G | p.K292E | Loss of Function | Missense_Mutation |
| 1298 | TP53 | NM_000546 | 45611 | c.876A>C | p.K292N | Loss of Function | Missense_Mutation |
| 1299 | TP53 | NM_000546 | 43624 | c.875A>G | p.K292R | Loss of Function | Missense_Mutation |
| 1300 | TP53 | NM_000546 | 44346 | c.875A>C | p.K292T | Loss of Function | Missense_Mutation |
| 1301 | TP53 | NM_000546 | 44345 | c.915G>T | p.K305N | Loss of Function | Missense_Mutation |
| 1302 | TP53 | NM_000546 | 43743 | c.914A>G | p.K305R | Loss of Function | Missense_Mutation |
| 1303 | TP53 | NM_000546 | 46114 | c.389T>A | p.L130H | Loss of Function | Missense_Mutation |
| 1304 | TP53 | NM_000546 | 44664 | c.736_750del15 | p.M246_P250deIMN | Loss of Function | In_Frame_Del |
| 1305 | TP53 | NM_000546 | 44903 | c.736delA | p.M246fs^{∗}1 | Loss of Function | Frame_Shift_Del |
| 1306 | TP53 | NM_000546 | 10757 | c.738G>C | p.M246I | Loss of Function | Missense_Mutation |
| 1307 | TP53 | NM_000546 | 44310 | c.738G>A | p.M246I | Loss of Function | Missense_Mutation |
| 1308 | TP53 | NM_000546 | 46136 | c.738G>T | p.M246I | Loss of Function | Missense_Mutation |
| 1309 | TP53 | NM_000546 | 44063 | c.389T>G | p.L130R | Loss of Function | Missense_Mutation |
| 1310 | TP53 | NM_000546 | 43777 | c.603G>C | p.L201F | Loss of Function | Missense_Mutation |
| 1311 | TP53 | NM_000546 | 11376 | c.737T>G | p.M246R | Loss of Function | Missense_Mutation |
| 1312 | TP53 | NM_000546 | 45489 | c.603G>T | p.L201F | Loss of Function | Missense_Mutation |
| 1313 | TP53 | NM_000546 | 43555 | c.736A>G | p.M246V | Loss of Function | Missense_Mutation |
| 1314 | TP53 | NM_000546 | 44247 | c.754_756delCTC | p.L252del | Loss of Function | In_Frame_Del |
| 1315 | TP53 | NM_000546 | 44054 | c.754C>T | p.L252F | Loss of Function | Missense_Mutation |
| 1316 | TP53 | NM_000546 | 45882 | c.391delA | p.N131fs*39 | Loss of Function | Frame _ Shift _ Del |
| 1317 | TP53 | NM_000546 | 45091 | c.755T>A | p.L252H | Loss of Function | Missense_Mutation |
| 1318 | TP53 | NM_000546 | 45393 | c.793C>A | p.L265M | Loss of Function | Missense_Mutation |
| 1319 | TP53 | NM_000546 | 43968 | c.866T>C | p.L289P | Loss of Function | Missense_Mutation |
| 1320 | TP53 | NM_000546 | 44070 | c.1031T>C | p.L344P | Loss of Function | Missense_Mutation |
| 1321 | TP53 | NM_000546 | 43859 | c.598delA | p.N200fs*47 | Loss of Function | Frame_Shift_Del |
| 1322 | TP53 | NM_000546 | 44206 | c.399G>T | p.M133I | Loss of Function | Missense_Mutation |
| 1323 | TP53 | NM_000546 | 43730 | c.398T>G | P.M133R | Loss of Function | Missense_Mutation |
| 1324 | TP53 | NM_000546 | 43641 | c.628delA | p.N210fs*37 | Loss of Function | Frame_Shift_Del |
| 1325 | TP53 | NM_000546 | 43723 | c.398T>C | p.M133T | Loss of Function | Missense_Mutation |
| 1326 | TP53_ENST00000545858 | ENST00000545858 | 99647 | c.432G>A | p.M144I | Loss of Function | Missense_Mutation |
| 1327 | TP53 | NM_000546 | 43891 | c.480G>A | p.M160I | Loss of Function | Missense_Mutation |
| 1328 | TP53 | NM_000546 | 45674 | c.480G>T | p.M160I | Loss of Function | Missense_Mutation |
| 1329 | TP53 | NM_000546 | 44305 | c.479T>A | p.M160K | Loss of Function | Missense_Mutation |
| 1330 | TP53 | NM_000546 | 44842 | c.478A>C | p.M160L | Loss of Function | Missense_Mutation |
| 1331 | TP53 | NM_000546 | 44328 | c.478A>G | p.M160V | Loss of Function | Missense_Mutation |
| 1332 | TP53 | NM_000546 | 45134 | c.715_726del12 | p.N239_C242delNSS | Loss of Function | In_Frame_Del |
| 1333 | TP53 | NM_000546 | 43851 | c.506T>C | p.M169T | Loss of Function | Missense_Mutation |
| 1334 | TP53 | NM_000546 | 10777 | c.715A>G | p.N239D | Loss of Function | Missense_Mutation |
| 1335 | TP53 | NM_000546 | 69195 | c.714_715insT | p.N239fs*1 | Loss of Function | Frame_Shift_Ins |
| 1336 | TP53 | NM_000546 | 44183 | c.715delA | p.N239fs*8 | Loss of Function | Frame_Shift_Del |
| 1337 | TP53 | NM_000546 | 44431 | c.505A>G | p.M169V | Loss of Function | Missense_Mutation |
| 1338 | TP53_ENST00000269305 | ENST00000269305 | 99646 | c.711G>A | p.M237I | Loss of Function | Missense_Mutation |
| 1339 | TP53 | NM_000546 | 44094 | c.716A>G | p.N239S | Loss of Function | Missense_Mutation |
| 1340 | TP53_ENST00000413465 | ENST00000413465 | 99646 | c.711G>A | p.M237I | Loss of Function | Missense_Mutation |
| 1341 | TP53 | NM_000546 | 44965 | c.709A>T | p.M237L | Loss of Function | Missense_Mutation |
| 1342 | TP53 | NM_000546 | 6546 | c.741_742CC>AT | p.N247 _ R248>KW | Loss of Function | In_Frame_Del |
| 1343 | TP53 | NM_000546 | 45032 | c.710T>G | p.M237R | Loss of Function | Missense_Mutation |
| 1344 | TP53 | NM_000546 | 43995 | c.740A>T | p.N247I | Loss of Function | Missense_Mutation |
| 1345 | TP53 | NM_000546 | 45329 | c.710T>C | p.M237T | Loss of Function | Missense_Mutation |
| 1346 | TP53 | NM_000546 | 44428 | c.741C>T | p.N247N | Loss of Function | Synonymous_Mutation |
| 1347 | TP53 | NM_000546 | 44129 | c.729G>A | p.M243I | Loss of Function | Missense_Mutation |
| 1348 | TP53 | NM_000546 | 46228 | c.729G>C | p.M243I | Loss of Function | Missense_Mutation |
| 1349 | TP53 | NM_000546 | 44322 | c.728T>A | p.M243K | Loss of Function | Missense_Mutation |
| 1350 | TP53 | NM_000546 | 43726 | c.727A>T | p.M243L | Loss of Function | Missense_Mutation |
| 1351 | TP53 | NM_000546 | 43765 | c.727A>C | p.M243L | Loss of Function | Missense_Mutation |
| 1352 | TP53 | NM_000546 | 46208 | c.59_872del14 | p.N288fs*13 | Loss of Function | Frame_Shift_Del |
| 1353 | TP53 | NM_000546 | 45459 | c.862delA | p.N288fs*57 | Loss of Function | Frame_Shift_Del |
| 1354 | TP53 | NM_000546 | 44514 | c.728T>G | p.M243R | Loss of Function | Missense_Mutation |
| 1355 | TP53 | NM_000546 | 44536 | c.728T>C | p.M243T | Loss of Function | Missense_Mutation |
| 1356 | TP53 | NM_000546 | 44879 | c.1033delA | p.N345fs*25 | Loss of Function | Frame_Shift_Del |
| 1357 | TP53 | NM_000546 | 44396 | c.382delC | p.P128fs*42 | Loss of Function | Frame_Shift_Del |
| 1358 | TP53 | NM_000546 | 46131 | c.380delC | p.P128fs*42 | Loss of Function | Frame_Shift_Del |
| 1359 | TP53 | NM_000546 | 44844 | c.727A>G | p.M243V | Loss of Function | Missense_Mutation |
| 1360 | TP53 | NM_000546 | 44103 | c.737T>A | p.M246K | Loss of Function | Missense_Mutation |
| 1361 | TP53 | NM_000546 | 44749 | c.453C>T | p.P151P | Loss of Function | Synonymous_Mutation |
| 1362 | TP53 | NM_000546 | 45594 | c.453C>G | p.P151P | Loss of Function | Synonymous_Mutation |
| 1363 | TP53 | NM_000546 | 45992 | c.736A>T | p.M246L | Loss of Function | Missense_Mutation |
| 1364 | TP53 | NM_000546 | 10790 | c.455C>T | p.P152L | Loss of Function | Missense_Mutation |
| 1365 | TP53 | NM_000546 | 44061 | c.456G>A | p.P152P | Loss of Function | Synonymous_Mutation |
| 1366 | TP53 | NM_000546 | 44613 | c.4SSC>A | p.P152Q | Loss of Function | Missense_Mutation |
| 1367 | TP53 | NM_000546 | 45505 | c.455C>G | p.P152R | Loss of Function | Missense_Mutation |
| 1368 | TP53 | NM_000546 | 43582 | c.454C>T | P.P152S | Loss of Function | Missense_Mutation |
| 1369 | TP53 | NM_000546 | 11355 | c.737T>C | p.M246T | Loss of Function | Missense_Mutation |
| 1370 | TP53 | NM_000546 | 44212 | c.391_393delAAC | p.N131del | Loss of Function | In_Frame_Del |
| 1371 | TP53 | NM_000546 | 43964 | c.459C>T | P.P153P | Loss of Function | Synonymous_Mutation |
| 1372 | TP53 | NM_000546 | 44416 | c.459C>A | p.P153P | Loss of Function | Synonymous_Mutation |
| 1373 | TP53 | NM_000546 | 44589 | c.393_395delCAA | p.N131del | Loss of Function | In_Frame_Del |
| 1374 | TP53 | NM_000546 | 43535 | c.391A>C | p.N131H | Loss of Function | Missense_Mutation |
| 1375 | TP53 | NM_000546 | 43570 | c.S29_546del18 | p.P177_C182delPHH | Loss of Function | In_Frame_Del |
| 1376 | TP53 | NM_000546 | 44730 | c.529_545del17 | p.P177fs*3 | Loss of Function | Frame_Shift_Del |
| 1377 | TP53 | NM_000546 | 44794 | c.392A>T | P.N131I | Loss of Function | Missense_Mutation |
| 1378 | TP53 | NM_000546 | 44097 | c.530C>T | p.P177L | Loss of Function | Missense_Mutation |
| 1379 | TP53 | NM_000546 | 43679 | c.531C>T | p.P177P | Loss of Function | Synonymous_Mutation |
| 1380 | TP53 | NM_000546 | 44818 | c.531C>G | p.P177P | Loss of Function | Synonymous_Mutation |
| 1381 | TP53 | NM_000546 | 10651 | c.530C>G | p.P177R | Loss of Function | Missense_Mutation |
| 1382 | TP53 | NM_000546 | 44474 | c.392A>G | p.N131S | Loss of Function | Missense_Mutation |
| 1383 | TP53 | NM_000546 | 43533 | c.391A>T | p.N131Y | Loss of Function | Missense_Mutation |
| 1384 | TP53 | NM_000546 | 46107 | c.599A>T | p.N200I | Loss of Function | Missense_Mutation |
| 1385 | TP53 | NM_000546 | 39455 | c.569delC | p.P190fs*57 | Loss of Function | Frame_Shift_Del |
| 1386 | TP53 | NM_000546 | 45320 | c.569delC | p.P190fs*57 | Loss of Function | Frame_Shift_Del |
| 1387 | TP53 | NM_000546 | 43657 | c.569C>T | p.P190L | Loss of Function | Missense_Mutation |
| 1388 | TP53 | NM_000546 | 44502 | c.599A>G | p.N200S | Loss of Function | Missense_Mutation |
| 1389 | TP53 | NM_000546 | 45441 | c.629A>G | p.N210S | Loss of Function | Missense_Mutation |
| 1390 | TP53 | NM_000546 | 11542 | c.703A>G | p.N235D | Loss of Function | Missense_Mutation |
| 1391 | TP53 | NM_000546 | 44784 | c.703_705delAAC | p.N235del | Loss of Function | In_Frame_Del |
| 1392 | TP53 | NM_000546 | 43860 | c.704A>T | p.N235I | Loss of Function | Missense_Mutation |
| 1393 | TP53 | NM_000546 | 45341 | c.571delC | p.P191fs*56 | Loss of Function | Frame_Shift_Del |
| 1394 | TP53 | NM_000546 | 43616 | c.704A>G | p.N235S | Loss of Function | Missense_Mutation |
| 1395 | TP53 | NM_000546 | 45620 | c.704A>C | p.N235T | Loss of Function | Missense . Mutation |
| 1396 | TP53 | NM_000546 | 45172 | c.703A>T | p.N235Y | Loss of Function | Missense . Mutation |
| 1397 | TP53 | NM_000546 | 45055 | c.715_720delAACAGT | p.N239 S240deINS | Loss of Function | In . Frame . Del |
| 1398 | TP53 | NM_000546 | 44689 | c.657C>T | p.P219P | Loss of Function | Synonymous_Mutation |
| 1399 | TP53 | NM_000546 | 44510 | c.717C>G | p.N239K | Loss of Function | Missense_Mutation |
| 1400 | TP53 | NM_000546 | 44647 | c.717C>A | p.N239K | Loss of Function | Missense_Mutation |
| 1401 | TP53 | NM_000546 | 43801 | c.716A>C | p.N239T | Loss of Function | Missense_Mutation |
| 1402 | TP53 | NM_000546 | 45870 | c.715A>T | p.N239Y | Loss of Function | Missense_Mutation |
| 1403 | TP53 | NM_000546 | 45005 | c.739A>G | p.N247D | Loss of Function | Missense_Mutation |
| 1404 | TP53 | NM_000546 | 45632 | c.741C>A | p.N247K | Loss of Function | Missense_Mutation |
| 1405 | TP53 | NM_000546 | 10771 | c.749C>T | p.P250L | Loss of Function | Missense_Mutation |
| 1406 | TP53 | NM_000546 | 44512 | c.740A>G | p.N247S | Loss of Function | Missense_Mutation |
| 1407 | TP53 | NM_000546 | 43588 | c.740A>C | p.N247T | Loss of Function | Missense_Mutation |
| 1408 | TP53 | NM_000546 | 43864 | c.739A>T | p.N247Y | Loss of Function | Missense_Mutation |
| 1409 | TP53 | NM_000546 | 43979 | c.802A>C | p.N268H | Loss of Function | Missense_Mutation |
| 1410 | TP53 | NM_000546 | 10814 | c.832C>G | p.P278A | Loss of Function | Missense_Mutation |
| 1411 | TP53 | NM_000546 | 44868 | c.804C>T | p.N268N | Loss of Function | Synonymous_Mutation |
| 1412 | TP53 | NM_000546 | 44871 | c.833deIC | p.P278fs*67 | Loss of Function | Frame_Shift_Del |
| 1413 | TP53 | NM_000546 | 45178 | c.832deIC | p.P278fs*67 | Loss of Function | Frame_Shift_Del |
| 1414 | TP53 | NM_000546 | 43755 | c.833C>A | p.P278H | Loss of Function | Missense_Mutation |
| 1415 | TP53 | NM_000546 | 10863 | c.833C>T | p.P278L | Loss of Function | Missense_Mutation |
| 1416 | TP53 | NM_000546 | 10887 | c.833C>G | p.P278R | Loss of Function | Missense_Mutation |
| 1417 | TP53 | NM_000546 | 10939 | c.832C>T | P2785 | Loss of Function | Missense_Mutation |
| 1418 | TP53 | NM_000546 | 43697 | c.832C>A | p.P278T | Loss of Function | Missense_Mutation |
| 1419 | TP53 | NM_000546 | 44523 | c.863A>G | P.N2SSS | Loss of Function | Missense_Mutation |
| 1420 | TP53 | NM_000546 | 45332 | c.885T>C | p.P295P | Loss of Function | Synonymous_Mutation |
| 1421 | TP53 | NM_000546 | 43725 | c.862A>T | p.N288Y | Loss of Function | Missense_Mutation |
| 1422 | TP53 | NM_000546 | 45131 | c.383C>T | p.P128L | Loss of Function | Missense_Mutation |
| 1423 | TP53 | NM_000546 | 44397 | c.382C>T | p.P128S | Loss of Function | Missense_Mutation |
| 1424 | TP53 | NM_000546 | 44788 | c.454C>G | p.P152A | Loss of Function | Missense_Mutation |
| 1425 | TP53 | NM_000546 | 45184 | c.902delC | p.P301fs*44 | Loss of Function | Frame_Shift_Del |
| 1426 | TP53 | NM_000546 | 45487 | c.898de1C | p.P301fs*44 | Loss of Function | Frame_Shift_Del |
| 1427 | TP53 | NM_000546 | 45546 | c.901delC | p.P301fs*44 | Loss of Function | Frame_Shift_Del |
| 1428 | TP53 | NM_000546 | 44165 | c.903A>G | p.P301P | Loss of Function | Synonymous_Mutation |
| 1429 | TP53_ENST0000026930S | ENST00000269305 | 129856 | c.455C>T | p.P152L | Loss of Function | Missense_Mutation |
| 1430 | TP53_ENST00000413465 | ENST00000413465 | 129857 | c.455C>T | p.P152L | Loss of Function | Missense_Mutation |
| 1431 | TP53 | NM_000546 | 44561 | c.454C>A | p.P152T | Loss of Function | Missense_Mutation |
| 1432 | TP53 | NM_000546 | 44367 | c.458C>T | p.P153L | Loss of Function | Missense_Mutation |
| 1433 | TP53 | NM_000546 | 43675 | c.457C>T | p.P153S | Loss of Function | Missense_Mutation |
| 1434 | TP53 | NM_000546 | 45660 | c.457C>A | p.P153T | Loss of Function | Missense_Mutation |
| 1435 | TP53 | NM_000546 | 45326 | c.530C>A | p.P177H | Loss of Function | Missense_Mutation |
| 1436 | TP53 | NM_000546 | 10650 | c.529C>T | p.P1775 | Loss of Function | Missense_Mutation |
| 1437 | TP53 | NM_000546 | 44426 | c.568C>G | p.P190A | Loss of Function | Missense_Mutation |
| 1438 | TP53 | NM_000546 | 44032 | c.298C>T | p.Q100* | Loss of Function | Nonsense_Mutation |
| 1439 | TP53 | NM_000546 | 10886 | c.310C>T | p.Q104* | Loss of Function | Nonsense_Mutation |
| 1440 | TP53 | NM_000546 | 11166 | c.406C>T | p.Q136* | Loss of Function | Nonsense_Mutation |
| 1441 | TP53 | NM_000546 | 43767 | c.406C>G | p.Q136E | Loss of Function | Missense_Mutation |
| 1442 | TP53 | NM_000546 | 45089 | c.407A>C | p.Q136P | Loss of Function | Missense_Mutation |
| 1443 | TP53 | NM_000546 | 44665 | c.568 570deICCT | p.P190del | Loss of Function | In_Frame_Del |
| 1444 | TP53 | NM_000546 | 43632 | c.493C>T | p.Q165* | Loss of Function | Nonsense_Mutation |
| 1445 | TP53 | NM_000546 | 44004 | c.569C>G | p.P190R | Loss of Function | Missense_Mutation |
| 1446 | TP53 | NM_000546 | 44682 | c.568C>T | p.P190S | Loss of Function | Missense_Mutation |
| 1447 | TP53 | NM_000546 | 44438 | c.568C>A | P.P190T | Loss of Function | Missense_Mutation |
| 1448 | TP53 | NM_000546 | 11333 | c.499C>T | p.Q167* | Loss of Function | Nonsense_Mutation |
| 1449 | TP53 | NM_000546 | 44275 | c.499_500delCA | p.Q167fs*13 | Loss of Function | Frame_Shift_Del |
| 1450 | TP53 | NM_000546 | 51646 | c.498_499insC | p.Q167fs*14 | Loss of Function | Frame_Shift_Ins |
| 1451 | TP53 | NM_000546 | 44336 | c.499delC | p.Q167fs*3 | Loss of Function | Frame_Shift_Del |
| 1452 | TP53 | NM_000546 | 44234 | c.571_573deICCT | p.P191del | Loss of Function | In_Frame_Del |
| 1453 | TP53 | NM_000546 | 45140 | c.572_574delCTC | p.P191del | Loss of Function | In_Frame_Del |
| 1454 | TP53 | NM_000546 | 44299 | c.501G>A | p.Q167Q | Loss of Function | Synonymous_Mutation |
| 1455 | TP53_ENST00000269305 | ENST00000269305 | 111724 | c.572_574deICTC | p.P191delP | Loss of Function | In_Frame_Del |
| 1456 | TP53 | NM_000546 | 10733 | c.574C>T | p.Q192* | Loss of Function | Nonsense_Mutation |
| 1457 | TP53_ENST0000041346S | ENST00000413465 | 111721 | c.S72_S74delCTC | p.P191delP | Loss of Function | In_Frame_Del |
| 1458 | TP53 | NM_000546 | 43782 | c.576G>A | p.Q192Q | Loss of Function | Synonymous_Mutation |
| 1459 | TP53 | NM_000546 | 44351 | c.572C>T | p.P191L | Loss of Function | Missense_Mutation |
| 1460 | TP53 | NM_000546 | 44172 | c.572C>G | p.P191R | Loss of Function | Missense_Mutation |
| 1461 | TP53 | NM_000546 | 43682 | c.328C>T | p.R110C | Loss of Function | Missense_Mutation |
| 1462 | TP53 | NM_000546 | 43702 | c.571C>T | p.P191S | Loss of Function | Missense_Mutation |
| 1463 | TP53 | NM_000546 | 10716 | c.329G>T | p.R110L | Loss of Function | Missense_Mutation |
| 1464 | TP53 | NM_000546 | 11250 | c.329G>C | p.R110P | Loss of Function | Missense_Mutation |
| 1465 | TP53_ENST0000041431S | ENST00000414315 | 129859 | c.59C>T | p.P20L | Loss of Function | Missense_Mutation |
| 1466 | TP53 | NM_000546 | 46124 | c.466C>T | p.R156C | Loss of Function | Missense_Mutation |
| 1467 | TP53 | NM_000546 | 45896 | c.466delC | p.R156fs*14 | Loss of Function | Frame_Shift_Del |
| 1468 | TP53 | NM_000546 | 44439 | c.656C>T | p.P219L | Loss of Function | Missense_Mutation |
| 1469 | TP53 | NM_000546 | 43739 | c.467G>A | p.R156H | Loss of Function | Missense_Mutation |
| 1470 | TP53 | NM_000546 | 44076 | c.655C>T | p.P219S | Loss of Function | Missense_Mutation |
| 1471 | TP53 | NM_000546 | 10760 | c.4676>C | p.R156P | Loss of Function | Missense_Mutation |
| 1472 | TP53 | NM_000546 | 44301 | c.468C>G | p.R156R | Loss of Function | Synonymous_Mutation |
| 1473 | TP53 | NM_000546 | 44854 | c.655C>A | p.P219T | Loss of Function | Missense_Mutation |
| 1474 | TP53 | NM_000546 | 44921 | c.748_756delCCCATCCTC | p.P250_L252delPIL | Loss of Function | In_Frame_Del |
| 1475 | TP53 | NM_000546 | 43848 | c.472C>T | p.R158C | Loss of Function | Missense_Mutation |
| 1476 | TP53 | NM_000546 | 45019 | c.471_472CC>TT | p.R158C | Loss of Function | Missense_Mutation |
| 1477 | TP53 | NM_000546 | 43831 | c.472₋475delCGCG | p.R158fs*11 | Loss of Function | Frame_Shift_Del |
| 1478 | TP53 | NM_000546 | 43761 | c.472delC | p.R158fs*12 | Loss of Function | Frame_Shift_Del |
| 1479 | TP53 | NM_000546 | 11087 | c.472C>G | p.R158G | Loss of Function | Missense_Mutation |
| 1480 | TP53 | NM_000546 | 10690 | c.473G>A | p.R158H | Loss of Function | Missense_Mutation |
| 1481 | TP53 | NM_000546 | 10714 | c.473G>T | p.R158L | Loss of Function | Missense_Mutation |
| 1482 | TP53 | NM_000546 | 44096 | c.748C>G | p.P250A | Loss of Function | Missense_Mutation |
| 1483 | TP53 | NM_000546 | 43940 | c.474C>T | P.R158R | Loss of Function | Synonymous_Mutation |
| 1484 | TP53 | NM_000546 | 46393 | c.520 536de117 | pR174fs*1 | Loss of Function | Frame_Shift_Del |
| 1485 | TP53 | NM_000546 | 44725 | c.522deIG | p.R174fs*73 | Loss of Function | Frame_Shift_Del |
| 1486 | TP53 | NM_000546 | 44609 | c.748_749CC>TT | P.P250F | Loss of Function | Missense_Mutation |
| 1487 | TP53 | NM_000546 | 44476 | c.749C>A | p.P250H | Loss of Function | Missense_Mutation |
| 1488 | TP53 | NM_000546 | 45034 | c.748_749CC>AA | P.P250N | Loss of Function | Missense_Mutation |
| 1489 | TP53 | NM_000546 | 43957 | c.750C>T | P.P250P | Loss of Function | Synonymous_Mutation |
| 1490 | TP53 | NM_000546 | 44742 | c.523 540de118 | p.R175_E180delRCP | Loss of Function | Frame_Shift_Del |
| 1491 | TP53 | NM_000546 | 43680 | c.523C>T | p.R175C | Loss of Function | Missense_Mutation |
| 1492 | TP53 | NM_000546 | 10870 | c.523C>G | p.R175G | Loss of Function | Missense_Mutation |
| 1493 | TP53 | NM_000546 | 10648 | c.524G>A | p.R175H | Loss of Function | Missense_Mutation |
| 1494 | TP53 | NM_000546 | 44464 | c.749_750CC>AG | p.P250Q | Loss of Function | Missense_Mutation |
| 1495 | TP53 | NM_000546 | 43695 | c.748C>T | p.P250S | Loss of Function | Missense_Mutation |
| 1496 | TP53 | NM_000546 | 44566 | c.525C>G | p.R175R | Loss of Function | Synonymous_Mutation |
| 1497 | TP53 | NM_000546 | 45515 | c.525C>T | p.R175R | Loss of Function | Synonymous_Mutation |
| 1498 | TP53_ENST0000026930S | ENST0000026930S | 99725 | c.832C>G | p.P278A | Loss of Function | Missense_Mutation |
| 1499 | TP53 | NM_000546 | 11090 | c.541C>T | p.R181C | Loss of Function | Missense_Mutation |
| 1500 | TP53 | NM_000546 | 43587 | c.832_833CC>TT | p.P278F | Loss of Function | Missense_Mutation |
| 1501 | TP53_ENST0000026930S | ENST0000026930S | 129831 | c.833C>T | p.P278L | Loss of Function | Missense_Mutation |
| 1502 | TP53 | NM_000546 | 45046 | c.542G>C | p.R181P | Loss of Function | Missense_Mutation |
| 1503 | TP53 | NM_000546 | 43728 | c.543C>T | p.R181R | Loss of Function | Synonymous_Mutation |
| 1504 | TP53 | NM_000546 | 10705 | c.586C>T | p.R196* | Loss of Function | Nonsense_Mutation |
| 1505 | TP53 | NM_000546 | 45021 | c.585_586CC>TT | p.R196* | Loss of Function | Nonsense_Mutation |
| 1506 | TP53 | NM_000546 | 44757 | c.586delC | p.R196fs*51 | Loss of Function | Frame_Shift_Del |
| 1507 | TP53 | NM_000546 | 43814 | c.587G>C | p.R196P | Loss of Function | Missense_Mutation |
| 1508 | TP53_ENST00000269305 | ENST0000026930S | 139044 | c.832C>T | p.P278S | Loss of Function | Missense_Mutation |
| 1509 | TP53 | NM_000546 | 44569 | c.588A>G | p.R196R | Loss of Function | Synonymous_Mutation |
| 1510 | TP53 | NM_000546 | 44615 | c.586C>A | p.R196R | Loss of Function | Synonymous_Mutation |
| 1511 | TP53 | NM_000546 | 45233 | c.884C>T | p.P295L | Loss of Function | Missense_Mutation |
| 1512 | TP53 | NM_000546 | 44750 | c.883C>T | p.P295S | Loss of Function | Missense_Mutation |
| 1513 | TP53 | NM_000546 | 45311 | c.898C>G | p.P300A | Loss of Function | Missense_Mutation |
| 1514 | TP53 | NM_000546 | 43766 | c.899C>T | p.P300L | Loss of Function | Missense_Mutation |
| 1515 | TP53 | NM_000546 | 44729 | c.898C>T | p.P300S | Loss of Function | Missense_Mutation |
| 1516 | TP53 | NM_000546 | 44753 | c.901C>T | p.P301S | Loss of Function | Missense_Mutation |
| 1517 | TP53 | NM_000546 | 11290 | c.625A>T | p.R209* | Loss of Function | Nonsense_Mutation |
| 1518 | TP53 | NM_000546 | 96575 | c.625_634del10 | p.R209fs*35 | Loss of Function | Frame_Shift_Del |
| 1519 | TP53 | NM_000546 | 45438 | c.626delG | p.R209fs*38 | Loss of Function | Frame_Shift_Del |
| 1520 | TP53 | NM_000546 | 13120 | c.626_627delGA | p.R209fs*6 | Loss of Function | Frame_Shift_Del |
| 1521 | TP53 | NM_000546 | 6482 | c.625_626delAG | p.R209fs*6 | Loss of Function | Frame_Shift_Del |
| 1522 | TP53_ENST00000414315 | ENST0000041431S | 111722 | c.176_178delCTC | p.P59delP | Loss of Function | In_Frame_Del |
| 1523 | TP53_ENST00000545858 | ENST00000545853 | 129858 | c.176C>T | p.P59L | Loss of Function | Missense_Mutation |
| 1524 | TP53 | NM_000546 | 10654 | c.637C>T | p.R213" | Loss of Function | Nonsense_Mutation |
| 1525 | TP53 | NM_000546 | 43607 | c.637delC | p.R213fs*34 | Loss of Function | Frame_Shift_Del |
| 1526 | TP53 | NM_000546 | 44358 | c.634delT | p.R213fs*34 | Loss of Function | Frame_Shift_Del |
| 1527 | TP53 | NM_000546 | 45777 | c.633delT | p.R213fs*34 | Loss of Function | Frame_Shift_Del |
| 1528 | TP53 | NM_000546 | 44102 | c.637C>G | p.R213G | Loss of Function | Missense_Mutation |
| 1529 | TP53 | NM_000546 | 43650 | c.638G>T | p.R213L | Loss of Function | Missense_Mutation |
| 1530 | TP53 | NM_000546 | 11860 | c.638G>C | p.R213P | Loss of Function | Missense_Mutation |
| 1531 | TP53 | NM_000546 | 10735 | c.638G>A | p.R213Q | Loss of Function | Missense_Mutation |
| 1532 | TP53 | NM_000546 | 45116 | c.742delC | p.R248fs*97 | Loss of Function | Frame_Shift_Del |
| 1533 | TP53 | NM_000546 | 11564 | c.742C>G | P.R248G | Loss of Function | Missense_Mutation |
| 1534 | TP53 | NM_000546 | 45543 | c.743_744GG>TT | p.R248L | Loss of Function | Missense_Mutation |
| 1535 | TP53 | NM_000546 | 6549 | c.743G>T | p.R248L | Loss of Function | Missense_Mutation |
| 1536 | TP53 | NM_000546 | 11491 | c.743G>C | p.R248P | Loss of Function | Missense_Mutation |
| 1537 | TP53 | NM_000546 | 10662 | c.743G>A | p.R248Q | Loss of Function | Missense_Mutation |
| 1538 | TP53 | NM_000546 | 44908 | c.743_744GG>AA | p.R248Q | Loss of Function | Missense_Mutation |
| 1539 | TP53 | NM_000546 | 46265 | c.224C>G | p.P75R | Loss of Function | Missense_Mutation |
| 1540 | TP53 | NM_000546 | 44287 | c.229C>G | p.P77A | Loss of Function | Missense_Mutation |
| 1541 | TP53 | NM_000546 | 43910 | c.245C>T | p.P82L | Loss of Function | Missense_Mutation |
| 1542 | TP53 | NM_000546 | 10656 | c.742C>T | p.R248W | Loss of Function | Missense_Mutation |
| 1543 | TP53 | NM_000546 | 6545 | c.741_742CC>TT | p.R248W | Loss of Function | Missense_Mutation |
| 1544 | TP53 | NM_000546 | 44916 | c.746deIG | p.R249fs*96 | Loss of Function | Frame_Shift_Del |
| 1545 | TP53 | NM_000546 | 10668 | c.745A>G | p.R249G | Loss of Function | Missense_Mutation |
| 1546 | | NM_000546 | 44091 | c.746G>A | p.R249K | Loss of Function | Missense_Mutation |
| 1547 | TP53 | NM_000546 | 43871 | c.746G>T | p.R249M | Loss of Function | Missense_Mutation |
| 1548 | TP53 | NM_000546 | 45918 | c.253C>T | p.P85S | Loss of Function | Missense_Mutation |
| 1549 | TP53 | NM_000546 | 10785 | c.747G>C | p.R249S | Loss of Function | Missense_Mutation |
| 1550 | TP53 | NM_000546 | 10817 | c.747G>T | p.R249S | Loss of Function | Missense_Mutation |
| 1551 | TP53 | NM_000546 | 43665 | c.746G>C | p.R249T | Loss of Function | Missense_Mutation |
| 1552 | TP53 | NM_000546 | 43629 | c.745A>T | p.R249W | Loss of Function | Missense_Mutation |
| 1553 | TP53 | NM_000546 | 11392 | c.800G>C | p.R267P | Loss of Function | Missense_Mutation |
| 1554 | TP53 | NM_000546 | 43923 | c.800G>A | p.R267Q | Loss of Function | Missense_Mutation |
| 1555 | TP53 | NM_000546 | 43544 | c.260C>A | p.P87Q | Loss of Function | Missense_Mutation |
| 1556 | TP53 | NM_000546 | 11183 | c.799C>T | p.R267W | Loss of Function | Missense_Mutation |
| 1557 | TP53 | NM_000546 | 10659 | c.817C>T | p.R273C | Loss of Function | Missense_Mutation |
| 1558 | TP53 | NM_000546 | 44701 | c.817delC | p.R273fs*72 | Loss of Function | Frame_Shift_Del |
| 1559 | TP53 | NM_000546 | 43688 | c.265C>T | p.P89S | Loss of Function | Missense_Mutation |
| 1560 | TP53 | NM_000546 | 10660 | c.818G>A | p.R273H | Loss of Function | Missense_Mutation |
| 1561 | TP53 | NM_000546 | 10779 | c.818G>T | p.R273L | Loss of Function | Missense_Mutation |
| 1562 | TP53 | NM_000546 | 43896 | c.818G>C | p.R273P | Loss of Function | Missense_Mutation |
| 1563 | TP53 | NM_000546 | 43909 | c.817C>A | p.R273S | Loss of Function | Missense_Mutation |
| 1564 | TP53 | NM_000546 | 44390 | c.838A>T | p.R280* | Loss of Function | Nonsense_Mutation |
| 1565 | TP53_ENST00000545858 | ENST00000545853 | 111723 | c.293_295delCTC | p.P98delP | Loss of Function | In_Frame_Del |
| 1566 | TP53 | NM_000546 | 44005 | c.835delG | p.R280fs*65 | Loss of Function | Frame_Shift_Del |
| 1567 | TP53 | NM_000546 | 11123 | c.838A>G | p.R280G | Loss of Function | Missense_Mutation |
| 1568 | TP53 | NM_000546 | 11287 | c.839G>T | p.R280I | Loss of Function | Missense_Mutation |
| 1569 | TP53 | NM_000546 | 10728 | c.839G>A | p.R280K | Loss of Function | Missense_Mutation |
| 1570 | TP53 | NM_000546 | 44568 | c.840A>G | p.R280R | Loss of Function | Synonymous_Mutation |
| 1571 | TP53 | NM_000546 | 44171 | c.840A>T | P.R280S | Loss of Function | Missense_Mutation |
| 1572 | TP53 | NM_000546 | 44233 | c.840A>C | P.R280S | Loss of Function | Missense_Mutation |
| 1573 | TP53 | NM_000546 | 10724 | c.839G>C | p.R280T | Loss of Function | Missense_Mutation |
| 1574 | TP53 | NM_000546 | 10992 | c.844C>G | p.R282G | Loss of Function | Missense_Mutation |
| 1575 | TP53 | NM_000546 | 44681 | c.293C>T | p.P98L | Loss of Function | Missense_Mutation |
| 1576 | TP53 | NM_000546 | 12296 | c.292C>T | p.P98S | Loss of Function | Missense_Mutation |
| 1577 | TP53 | NM_000546 | 44338 | c.845G>A | p.R282Q | Loss of Function | Missense_Mutation |
| 1578 | TP53 | NM_000546 | 44724 | c.846G>A | p.R282R | Loss of Function | Synonymous_Mutation |
| 1579 | TP53 | NM_000546 | 44918 | c.844C>A | p.R282R | Loss of Function | Synonymous_Mutation |
| 1580 | TP53 | NM_000546 | 10704 | c.844C>T | p.R282W | Loss of Function | Missense_Mutation |
| 1581 | TP53 | NM_000546 | 43585 | c.843_844CC>TT | p.R282W | Loss of Function | Missense_Mutation |
| 1582 | TP53 | NM_000546 | 45293 | c.407A>G | p.Q136R | Loss of Function | Missense_Mutation |
| 1583 | TP53 | NM_000546 | 45891 | c.847_S66de!20 | p.R283fs*16 | Loss of Function | Frame_Shift_Del |
| 1584 | TP53 | NM_000546 | 45188 | c.847delC | p.R283fs*62 | Loss of Function | Frame_Shift_Del |
| 1585 | TP53 | NM_000546 | 44850 | c.494A>T | P.Q165L | Loss of Function | Missense_Mutation |
| 1586 | TP53 | NM_000546 | 44851 | c.494A>C | p.Q165P | Loss of Function | Missense_Mutation |
| 1587 | TP53 | NM_000546 | 44308 | c.494A>G | p.Q165R | Loss of Function | Missense_Mutation |
| 1588 | TP53 | NM_000546 | 10743 | c.848G>C | p.R283P | Loss of Function | Missense_Mutation |
| 1589 | TP53 | NM_000546 | 43977 | c.849C>T | p.R283R | Loss of Function | Synonymous_Mutation |
| 1590 | TP53 | NM_000546 | 45679 | c.868C>T | p.R290C | Loss of Function | Missense_Mutation |
| 1591 | TP53 | NM_000546 | 45626 | c.501G>T | P.Q167H | Loss of Function | Missense_Mutation |
| 1592 | TP53 | NM_000546 | 45342 | c.500A>T | p.Q167L | Loss of Function | Missense_Mutation |
| 1593 | TP53 | NM_000546 | 10663 | c.916C>T | p.R306* | Loss of Function | Nonsense_Mutation |
| 1594 | TP53 | NM_000546 | 11071 | c.1009C>T | p.R337C | Loss of Function | Missense_Mutation |
| 1595 | TP53 | NM_000546 | 43882 | c.1010G>A | p.R337H | Loss of Function | Missense . Mutation |
| 1596 | TP53 | NM_000546 | 11411 | c.1010G>T | p.R337L | Loss of Function | Missense_Mutation |
| 1597 | TP53 | NM_000546 | 11073 | c.1024C>T | p.R342* | Loss of Function | Nonsense_Mutation |
| 1598 | TP53 | NM_000546 | 18597 | c.1024delC | p.R342fs*3 | Loss of Function | Frame_Shift_Del |
| 1599 | TP53 | NM_000546 | 43795 | c.1023delC | p.R342fs*3 | Loss of Function | Frame_Shift_Del |
| 1600 | TP53 | NM_000546 | 45639 | c.1024delC | p.R342fs*3 | Loss of Function | Frame_Shift_Del |
| 1601 | TP53 | NM_000546 | 45276 | c.1025G>C | p.R342P | Loss of Function | Missense_Mutation |
| 1602 | TP53 | NM_000546 | 43709 | c.500A>G | p.Q167R | Loss of Function | Missense_Mutation |
| 1603 | TP53 | NM_000546 | 45044 | c.576G>T | P.Q192H | Loss of Function | Missense_Mutation |
| 1604 | TP53 | NM_000546 | 44849 | c.575A>G | p.Q192R | Loss of Function | Missense_Mutation |
| 1605 | TP53 | NM_000546 | 45944 | c.318C>G | p.5106R | Loss of Function | Missense_Mutation |
| 1606 | TP53 | NM_000546 | 40942 | c.380C>T | P.S127F | Loss of Function | Missense_Mutation |
| 1607 | TP53 | NM_000546 | 45536 | c.1061A>G | p.Q354R | Loss of Function | Missense_Mutation |
| 1608 | TP53 | NM_000546 | 46115 | c.329G>A | p.R110H | Loss of Function | Missense_Mutation |
| 1609 | TP53 | NM_000546 | 44687 | c.379T>C | p.5127P | Loss of Function | Missense_Mutation |
| 1610 | TP53_ENST00000269305 | ENST00000269305 | 99929 | c.329G>T | p.R110L | Loss of Function | Missense_Mutation |
| 1611 | TP53 | NM_000546 | 43970 | c.380C>A | p.5127Y | Loss of Function | Missense . Mutation |
| 1612 | TP53 | NM_000546 | 11508 | c.497C>A | p.S166* | Loss of Function | Nonsense_Mutation |
| 1613 | TP53 | NM_000546 | 44467 | c.497C>G | p.5166* | Loss of Function | Nonsense_Mutation |
| 1614 | TP53_ENST00000413465 | ENST00000413465 | 99928 | c.329G>T | p.R110L | Loss of Function | Missense_Mutation |
| 1615 | TP53_ENST00000545858 | ENST00000545858 | 242000 | c.359G>T | p.R120L | Loss of Function | Missense . Mutation |
| 1616 | TP53_ENST00000545858 | ENST00000545858 | 99021 | c.464G>A | p.R155Q | Loss of Function | Missense_Mutation |
| 1617 | TP53 | NM_000546 | 10706 | c.548C>G | p.5183* | Loss of Function | Nonsense_Mutation |
| 1618 | TP53 | NM_000546 | 11717 | c.548C>A | p.S183* | Loss of Function | Nonsense_Mutation |
| 1619 | TP53 ENST00000545858 | ENST00000545858 | 120006 | c.463C>T | p.R155W | Loss of Function | Missense_Mutation |
| 1620 | TP53 | NM_000546 | 46001 | c.466_486del21 | p.R156_1162deIRVR, | Loss of Function | In_Frame_Del |
| 1621 | TP53 | NM_000546 | 45314 | c.553delA | p.S185fs*62 | Loss of Function | Frame_Shift_Del |
| 1622 | TP53 | NM_000546 | 45154 | c.466C>G | p.R156G | Loss of Function | Missense_Mutation |
| 1623 | TP53 | NM_000546 | 43548 | c.467G>T | p.R156L | Loss of Function | Missense_Mutation |
| 1624 | TP53 | NM_000546 | 43744 | c.466C>A | p.R156S | Loss of Function | Missense_Mutation |
| 1625 | TP53 | NM_000546 | 44267 | c.472_477delCGCGCC | p.R158_A159delRA | Loss of Function | In_Frame_Del |
| 1626 | TP53 | NM_000546 | 44887 | c.644delG | p.S215fs*32 | Loss of Function | Frame_Shift_Del |
| 1627 | TP53 | NM_000546 | 43951 | c.643A>G | P.S215G | Loss of Function | Missense_Mutation |
| 1628 | TP53 | NM_000546 | 11450 | c.644G>T | p.5215I | Loss of Function | Missense_Mutation |
| 1629 | TP53_ENST00000269305 | ENST00000269305 | 220779 | c.473G>A | p.R158H | Loss of Function | Missense_Mutation |
| 1630 | TP53 | NM_000546 | 44979 | c.645T>G | P.S215R | Loss of Function | Missense_Mutation |
| 1631 | TP53 | NM_000546 | 45122 | c.645T>A | P.S215R | Loss of Function | Missense_Mutation |
| 1632 | TP53 | NM_000546 | 46000 | c.643A>C | p.5215R | Loss of Function | Missense_Mutation |
| 1633 | TP53_ENST00000413465 | ENST00000413465 | 220778 | c.473G>A | p.R158H | Loss of Function | Missense_Mutation |
| 1634 | TP53 | NM_000546 | 43615 | c.473G>C | p.R158P | Loss of Function | Missense_Mutation |
| 1635 | TP53 | NM_000546 | 44524 | c.521G>A | p.R174K | Loss of Function | Missense_Mutation |
| 1636 | TP53 | NM_000546 | 45671 | c.521G>T | p.R174M | Loss of Function | Missense_Mutation |
| 1637 | TP53 | NM_000546 | 44217 | c.718delA | p.S240fs*7 | Loss of Function | Frame_Shift_Del |
| 1638 | TP53 | NM_000546 | 43973 | c.718A>G | p.5240G | Loss of Function | Missense_Mutation |
| 1639 | TP53 | NM_000546 | 44518 | c.522G>A | p.R174R | Loss of Function | Synonymous_Mutation |
| 1640 | TP53 | NM_000546 | 44782 | c.520A>T | p.R174W | Loss of Function | Missense_Mutation |
| 1641 | TP53_ENST00000269305 | ENST00000269305 | 99914 | c.524G>A | p.R175H | Loss of Function | Missense_Mutation |
| 1642 | TP53 | NM_000546 | 44838 | c.720T>C | p.S240S | Loss of Function | Synonymous_Mutation |
| 1643 | TP53_ENST00000413465 | ENST00000413465 | 99022 | c.524G>A | p.R175H | Loss of Function | Missense_Mutation |
| 1644 | TP53 | NM_000546 | 10718 | c.524G>T | p.R175L | Loss of Function | Missense_Mutation |
| 1645 | TP53 | NM_000546 | 10709 | c.722C>G | p.S241C | Loss of Function | Missense_Mutation |
| 1646 | TP53 | NM_000546 | 45416 | c.524G>C | p.R175P | Loss of Function | Missense_Mutation |
| 1647 | TP53 | NM_000546 | 43931 | c.523C>A | p.R175S | Loss of Function | Missense_Mutation |
| 1648 | TP53 | NM_000546 | 10812 | c.722C>T | p.S241F | Loss of Function | Missense_Mutation |
| 1649 | TP53 | NM_000546 | 45017 | c.722_723CC>TT | p.S241F | Loss of Function | Missense_Mutation |
| 1650 | TP53 | NM_000546 | 43645 | c.721delT | p.S241fs*6 | Loss of Function | Frame_Shift_Del |
| 1651 | TP53 | NM_000546 | 44578 | c.721T>C | p.S241P | Loss of Function | Missense . Mutation |
| 1652 | TP53 | NM_000546 | 10738 | c.542G>A | p.R181H | Loss of Function | Missense_Mutation |
| 1653 | TP53 | NM_000546 | 10935 | c.722C>A | p.S241Y | Loss of Function | Missense_Mutation |
| 1654 | TP53 | NM_000546 | 44152 | c.542G>T | p.R181L | Loss of Function | Missense_Mutation |
| 1655 | TP53 | NM_000546 | 44599 | c.587G>A | p.R196q | Loss of Function | Missense_Mutation |
| 1656 | TP53 | NM_000546 | 46074 | c.604C>T | p.R202C | Loss of Function | Missense_Mutation |
| 1657 | TP53 | NM_000546 | 43594 | c.605G>A | p.R202H | Loss of Function | Missense_Mutation |
| 1658 | TP53 | NM_000546 | 44925 | c.605G>T | p.R202L | Loss of Function | Missense_Mutation |
| 1659 | TP53 | NM_000546 | 43608 | c.605G>C | p.R202P | Loss of Function | Missense_Mutation |
| 1660 | TP53 | NM_000546 | 44074 | c.605_606GT>CG | p.R202P | Loss of Function | Missense Mutation |
| 1661 | TP53 | NM_000546 | 44237 | c.904delG | p.5303f5*42 | Loss of Function | Frame_Shift_Del |
| 1662 | TP53 | NM_000546 | 44174 | c.604C>A | P.R202S | Loss of Function | Missense_Mutation |
| 1663 | TP53 | NM_000546 | 45995 | c.626G>A | p.R209K | Loss of Function | Missense_Mutation |
| 1664 | TP53 | NM_000546 | 45257 | c.626G>C | p.R209T | Loss of Function | Missense_Mutation |
| 1665 | TP53 | NM_000546 | 18610 | c.267delC | p.S90fs*33 | Loss of Function | Frame_Shift_Del |
| 1666 | TP53 | NM_000546 | 45500 | c.281C>A | p.S94* | Loss of Function | Nonsense_Mutation |
| 1667 | TP53_ENST00000269305 | ENST00000269305 | 241998 | c.638G>T | p.R213L | Loss of Function | Missense_Mutation |
| 1668 | TP53_EN ST00000413465 | ENST00000413465 | 241997 | c.638G>T | p.R213L | Loss of Function | Missense_Mutation |
| 1669 | TP53_ENST00000269305 | ENST00000269305 | 99602 | c.743G>A | p.R248Q | Loss of Function | Missense_Mutation |
| 1670 | TP53_ENST00000413465 | ENST00000413465 | 99020 | c.743G>A | p.R248Q | Loss of Function | Missense_Mutation |
| 1671 | TP53 | NM_000546 | 85574 | c.291_295delCCCTT | p.S99fs*48 | Loss of Function | Frame_Shift_Del |
| 1672 | TP53 | NM_000546 | 44257 | c.301delA | pT102fs*21 | Loss of Function | Frame_Shift_Del |
| 1673 | TP53 | NM_000546 | 44920 | c.742C>A | p.R248R | Loss of Function | Synonymous_Mutation |
| 1674 | TP53 | NM_000546 | 44303 | c.463A>G | p.T155A | Loss of Function | Missense_Mutation |
| 1675 | TP53 | NM_000546 | 44009 | c.463_470delACCCGCGT | p.T155fs*23 | Loss of Function | Frame_Shift_Del |
| 1676 | TP53 | NM_000546 | 44033 | c.464C>T | p.T155I | Loss of Function | Missense_Mutation |
| 1677 | TP53 | NM_000546 | 11218 | c.464C>A | p.T155N | Loss of Function | Missense_Mutation |
| 1678 | TP53 | NM_000546 | 10912 | c.463A>C | p.T155P | Loss of Function | Missense_Mutation |
| 1679 | TP53 | NM_000546 | 43670 | c.465C>T | p.T155T | Loss of Function | Synonymous_Mutation |
| 1680 | TP53 | NM_000546 | 45084 | c.744G>A | p.R248R | Loss of Function | Synonymous_Mutation |
| 1681 | TP53 | NM_000546 | 44384 | c.510G>A | p.T170T | Loss of Function | Synonymous_Mutation |
| 1682 | TP53 | NM_000546 | 45541 | c.510G>T | p.T170T | Loss of Function | Synonymous_Mutation |
| 1683 | TP53 | NM_000546 | 45735 | c.744G>C | p.R248R | Loss of Function | Synonymous_Mutation |
| 1684 | TP53 | NM_000546 | 44371 | c.631delA | p.T211fs*36 | Loss of Function | Frame_Shift_Del |
| 1685 | TP53 | NM_000546 | 43939 | c.632C>T | p.T211I | Loss of Function | Missense_Mutation |
| 1686 | TP53_ENST00000269305 | ENST00000269305 | 120007 | c.742C>T | p.R248W | Loss of Function | Missense_Mutation |
| 1687 | TP53 | NM_000546 | 46211 | c.633T>C | p.T211T | Loss of Function | Synonymous_Mutation |
| 1688 | TP53 | NM_000546 | 45157 | c.688delA | p.T230fs*17 | Loss of Function | Frame_Shift_Del |
| 1689 | TP53 | NM_000546 | 44458 | c.688_698del11 | p.T230fs*6 | Loss of Function | Frame_Shift_Del |
| 1690 | TP53_ENST00000413465 | ENST00000413465 | 120005 | c.742C>T | p.R248W | Loss of Function | Missense_Mutation |
| 1691 | TP53 | NM_000546 | 44625 | c.747G>A | p.R249R | Loss of Function | Synonymous_Mutation |
| 1692 | TP53 | NM_000546 | 44271 | c.688A>C | p.T230P | Loss of Function | Missense_Mutation |
| 1693 | TP53_ENST00000269305 | ENST00000269305 | 131478 | c.747G>T | p.R249S | Loss of Function | Missense_Mutation |
| 1694 | TP53_ENST00000413465 | ENST00000413465 | 131479 | c.747G>T | p.R249S | Loss of Function | Missense_Mutation |
| 1695 | TP53 | NM_000546 | 45784 | c.691delA | p.T231fs*16 | Loss of Function | Frame_Shift_Del |
| 1696 | TP53 | NM_000546 | 45706 | c.801G>T | p.R267R | Loss of Function | Synonymous_Mutation |
| 1697 | TP53_ENST00000269305 | ENST00000269305 | 179804 | c.799C>T | p.R267W | Loss of Function | Missense_Mutation |
| 1698 | TP53 | NM_000546 | 44113 | c.693C>T | p.T231T | Loss of Function | Synonymous_Mutation |
| 1699 | TP53_ENST00000414315 | ENST00000414315 | 220780 | c.77G>A | p.R26H | Loss of Function | Missense_Mutation |
| 1700 | TP53 | NM_000546 | 44460 | c.757_760delACCA | p.T253fs*91 | Loss of Function | Frame_Shift_Del |
| 1701 | TP53_ENST00000269305 | ENST00000269305 | 99933 | c.817C>T | p.R273C | Loss of Function | Missense_Mutation |
| 1702 | TP53 | NM_000546 | 43843 | c.817C>G | p.R273G | Loss of Function | Missense_Mutation |
| 1703 | TP53_ENST00000269305 | ENST00000269305 | 99729 | c.818G>A | p.R273H | Loss of Function | Missense_Mutation |
| 1704 | TP53 | NM_000546 | 44843 | c.759C>T | p.T253T | Loss of Function | Synonymous_Mutation |
| 1705 | TP53 | NM_000546 | 45843 | c.838_843delAGAGAC | p.R280_D281delRD | Loss of Function | In_Frame_Del |
| 1706 | TP53_ENST00000269305 | ENST00000269305 | 129830 | c.839G>A | p.R280K | Loss of Function | Missense_Mutation |
| 1707 | TP53 | NM_000546 | 44470 | c.845G>T | p.R282L | Loss of Function | Missense_Mutation |
| 1708 | TP53 | NM_000546 | 44352 | c.850A>C | p.T284P | Loss of Function | Missense_Mutation |
| 1709 | TP53 | NM_000546 | 44835 | c.852A>T | p.T284T | Loss of Function | Synonymous_Mutation |
| 1710 | TP53 | NM_000546 | 44306 | c.845G>C | p.R282P | Loss of Function | Missense_Mutation |
| 1711 | TP53 | NM_000546 | 44417 | c.910delA | p.T304fs*41 | Loss of Function | Frame_Shift_Del |
| 1712 | TP53_ENST00000269305 | ENST00000269305 | 99925 | c.844C>T | p.R282W | Loss of Function | Missense_Mutation |
| 1713 | TP53 | NM_000546 | 10911 | c.847C>T | p.R283C | Loss of Function | Missense_Mutation |
| 1714 | TP53 | NM_000546 | 46035 | c.847C>G | p.R283G | Loss of Function | Missense_Mutation |
| 1715 | TP53 | NM_000546 | 11483 | c.848G>A | p.R283H | Loss of Function | Missense_Mutation |
| 1716 | TP53 | NM_000546 | 10670 | c.469G>T | p.V157F | Loss of Function | Missense_Mutation |
| 1717 | TP53 | NM_000546 | 43710 | c.468delC | p.V157fs*13 | Loss of Function | Frame_Shift_Del |
| 1718 | TP53 | NM_000546 | 45111 | c.469_473delGTCCG | p.V157fs*22 | Loss of Function | Frame_Shift_Del |
| 1719 | TP53 | NM_000546 | 43903 | c.470T>G | p.V157G | Loss of Function | Missense_Mutation |
| 1720 | TP53 | NM_000546 | 44463 | c.848G>T | p.R283L | Loss of Function | Missense_Mutation |
| 1721 | TP53 | NM_000546 | 44017 | c.869G>A | p.R290H | Loss of Function | Missense_Mutation |
| 1722 | TP53 | NM_000546 | 43934 | c.471C>A | p.V157V | Loss of Function | Synonymous_Mutation |
| 1723 | TP53 | NM_000546 | 44526 | c.471C>T | p.V157V | Loss of Function | Synonymous_Mutation |
| 1724 | TP53 | NM_000546 | 44639 | c.869G>T | p.R290L | Loss of Function | Missense_Mutation |
| 1725 | TP53 | NM_000546 | 45278 | c.1025G>A | p.R342Q | Loss of Function | MissenseMutation |
| 1726 | TP53 | NM_000546 | 44240 | c.514G>T | p.V172F | Loss of Function | Missense_Mutation |
| 1727 | TP53 | NM_000546 | 45906 | c.514delG | p.V172fs*2 | Loss of Function | Frame_Shift_Del |
| 1728 | TP53 | NM_000546 | 45047 | c.515T>G | p.V172G | Loss of Function | Missense_Mutation |
| 1729 | TP53_ENST00000414315 | ENST00000414315 | 99023 | c.128G>A | p.R43H | Loss of Function | Missense_Mutation |
| 1730 | TP53 | NM_000546 | 44973 | c.516T>C | p.V172V | Loss of Function | Synonymous_Mutation |
| 1731 | TP53_ENST00000545858 | ENST00000545858 | 220781 | c.194G>A | p.R65H | Loss of Function | Missense_Mutation |
| 1732 | TP53 | NM_000546 | 43732 | c.517delG | p.V173fs*1 | Loss of Function | Frame_Shift_Del |
| 1733 | TP53 | NM_000546 | 45583 | c.514_559del46 | p.V173fs*59 | Loss of Function | Frame_Shift_Del |
| 1734 | TP53 | NM_000546 | 43054 | c.518T>G | p.V173G | Loss of Function | Missense_Mutation |
| 1735 | TP53 | NM_000546 | 44383 | c.518T>G | p.V173G | Loss of Function | Missense_Mutation |
| 1736 | TP53 | NM_000546 | 43559 | c.517G>T | p.V173L | Loss of Function | Missense_Mutation |
| 1737 | TP53 | NM_000546 | 44057 | c.517G>C | p.V173L | Loss of Function | Missense_Mutation |
| 1738 | TP53 | NM_000546 | 11084 | c.517G>A | p.V173M | Loss of Function | Missense_Mutation |
| 1739 | TP53 | NM_000546 | 44517 | c.519G>A | p.V173V | Loss of Function | Synonymous_Mutation |
| 1740 | TP53 | NM_000546 | 44018 | c.214C>T | p.R72C | Loss of Function | Missense_Mutation |
| 1741 | TP53 | NM_000546 | 43905 | c.590T>G | p.V197G | Loss of Function | Missense_Mutation |
| 1742 | TP53 | NM_000546 | 45985 | c.215G>A | p.R72H | Loss of Function | Missense_Mutation |
| 1743 | TP53_ENST00000414315 | ENST00000414315 | 241999 | c.242G>T | P.R81L | Loss of Function | Missense_Mutation |
| 1744 | TP53 | NM_000546 | 44845 | c.591G>A | p.V197V | Loss of Function | Synonymous_Mutation |
| 1745 | TP53_ENST00000545858 | ENST00000545858 | 99024 | c.245G>A | P.R82H | Loss of Function | Missense_Mutation |
| 1746 | TP53 | NM_000546 | 45308 | c.607delG | p.V203fs*44 | Loss of Function | Frame_Shift_Del |
| 1747 | TP53 | NM_000546 | 44226 | c.380C>T | P.S127F | Loss of Function | Missense_Mutation |
| 1748 | TP53 | NM_000546 | 45015 | c380_381CC>TT | P.S127F | Loss of Function | Missense Mutation |
| 1749 | TP53 | NM_000546 | 44707 | c.609G>A | p.V203V | Loss of Function | SynonymousMutation |
| 1750 | TP53 | NM_000546 | 53285 | c.379T>A | p.5127T | Loss of Function | Missense_Mutation |
| 1751 | TP53 | NM_000546 | 44282 | c.496T>G | p.5166A | Loss of Function | Missense_Mutation |
| 1752 | TP53 | NM_000546 | 44274 | c.647T>A | p.V216E | Loss of Function | Missense_Mutation |
| 1753 | TP53 | NM_000546 | 44239 | c.647dclT | p.V216fs*31 | Loss of Function | Frame_Shift_Del |
| 1754 | TP53 | NM_000546 | 43681 | c.647T>G | p.V216G | Loss of Function | Missense_Mutation |
| 1755 | TP53 | NM_000546 | 11210 | c.646G>T | p.V216L | Loss of Function | Missense_Mutation |
| 1756 | TP53 | NM_000546 | 10667 | c.646G>A | p.V216M | Loss of Function | Missense_Mutation |
| 1757 | TP53 | NM_000546 | 44289 | c.497C>T | p.S166L | Loss of Function | Missense_Mutation |
| 1758 | TP53 | NM_000546 | 44035 | c.496T>C | p.5166P | Loss of Function | Missense_Mutation |
| 1759 | TP53 | NM_000546 | 44300 | c.548C>T | p.5183L | Loss of Function | Missense_Mutation |
| 1760 | TP53 | NM_000546 | 44343 | c.547T>C | p.5183P | Loss of Function | Missense_Mutation |
| 1761 | TP53 | NM_000546 | 44714 | c.553A>G | p.5185G | Loss of Function | Missense_Mutation |
| 1762 | TP53 | NM_000546 | 44185 | c.555C>A | p.S185R | Loss of Function | Missense_Mutation |
| 1763 | TP53 | NM_000546 | 45198 | c.555C>T | p.S185S | Loss of Function | Missense_Mutation |
| 1764 | TP53 | NM_000546 | 44198 | c.653T>G | p.V218G | Loss of Function | Missense_Mutation |
| 1765 | TP53 | NM_000546 | 11307 | c.643A>T | p.S215C | Loss of Function | Missense_Mutation |
| 1766 | TP53 | NM_000546 | 44093 | c.644G>A | p.5215N | Loss of Function | Missense_Mutation |
| 1767 | TP53 | NM_000546 | 45511 | c.645T>C | p.52155 | Loss of Function | Missense_Mutation |
| 1768 | TP53 | NM_000546 | 13421 | c.814delG | p.V272fs*73 | Loss of Function | Frame_Shift_Del |
| 1769 | TP53 | NM_000546 | 44870 | c.815T>G | p.V272G | Loss of Function | Missense_Mutation |
| 1770 | TP53 | NM_000546 | 44175 | c.644G>C | p.S215T | Loss of Function | Missense_Mutation |
| 1771 | TP53 | NM_000546 | 43920 | c.680C>T | p.5227F | Loss of Function | Missense_Mutation |
| 1772 | TP53 | NM_000546 | 10891 | c.814G>A | p.V272M | Loss of Function | Missense_Mutation |
| 1773 | TP53 | NM_000546 | 44393 | c.821T>C | p.V274A | Loss of Function | Missense_Mutation |
| 1774 | TP53 | NM_000546 | 44448 | c.821T>A | p.V274D | Loss of Function | Missense_Mutation |
| 1775 | TP53 | NM_000546 | 10769 | c.820G>T | p.V274F | Loss of Function | Missense_Mutation |
| 1776 | TP53 | NM_000546 | 43945 | c.821T>G | p.V274G | Loss of Function | Missense_Mutation |
| 1777 | TP53 | NM_000546 | 44621 | c.718A>T | p.S240C | Loss of Function | Missense_Mutation |
| 1778 | TP53 | NM_000546 | 44443 | c.820G>C | p.V274L | Loss of Function | Missense_Mutation |
| 1779 | TP53 | NM_000546 | 45491 | c.822T>G | p.V274V | Loss of Function | Synonymous_Mutation |
| 1780 | TP53 | NM_000546 | 43660 | c.719G>T | p.52401 | Loss of Function | Missense_Mutation |
| 1781 | TP53 | NM_000546 | 43684 | c.720T>G | p.5240R | Loss of Function | Missense_Mutation |
| 1782 | TP53 | NM_000546 | 44192 | c.272G>A | p.W91* | Loss of Function | Nonsense_Mutation |
| 1783 | TP53 | NM_000546 | 44492 | c.273G>A | p.W91* | Loss of Function | Nonsense_Mutation |
| 1784 | TP53 | NM_000546 | 44453 | c.309C>G | p.Y103* | Loss of Function | Nonsense_Mutation |
| 1785 | TP53 | NM_000546 | 11448 | c.321C>G | p.Y107* | Loss of Function | Nonsense_Mutation |
| 1786 | TP53 | NM_000546 | 45040 | c.321C>A | p.Y107* | Loss of Function | Nonsense_Mutation |
| 1787 | TP53 | NM_000546 | 46103 | c.319T>G | p.Y107D | Loss of Function | Missense_Mutation |
| 1788 | TP53 | NM_000546 | 45509 | c.321C>T | p.Y107Y | Loss of Function | Synonymous_Mutation |
| 1789 | TP53 | NM_000546 | 10862 | c.378C>G | p.Y126* | Loss of Function | Nonsense_Mutation |
| 1790 | TP53 | NM_000546 | 10888 | c.378C>A | p.Y126* | Loss of Function | Nonsense_Mutation |
| 1791 | TP53 | NM_000546 | 45261 | c.720T>A | p.5240R | Loss of Function | Missense_Mutation |
| 1792 | TP53 | NM_000546 | 44964 | c.719G>C | p.5240T | Loss of Function | Missense_Mutation |
| 1793 | TP53 | NM_000546 | 11517 | c.377A>G | p.Y126C | Loss of Function | Splice_Site |
| 1794 | TP53 | NM_000546 | 43900 | c.376T>G | p.Y126D | Loss of Function | Splice_Site |
| 1795 | TP53 | NM_000546 | 249845 | c.377_377delA | p.Y126fs*44 | Loss of Function | Frame_Shift_Del |
| 1796 | TP53 | NM_000546 | 44380 | c.376T>A | p.Y126N | Loss of Function | Splice_Site |
| 1797 | TP53 | NM_000546 | 44142 | c.377A>C | p.Y126S | Loss of Function | Splice_Site |
| 1798 | TP53 | NM_000546 | 43820 | c.489C>G | p.Y163* | Loss of Function | Nonsense_Mutation |
| 1799 | TP53 | NM_000546 | 45411 | c.489C>A | p.Y163* | Loss of Function | Nonsense_Mutation |
| 1800 | TP53 | NM_000546 | 10808 | c.488A>G | p.Y163C | Loss of Function | Missense_Mutation |
| 1801 | TP53 | NM_000546 | 44216 | c.487T>G | p.Y163D | Loss of Function | Missense_Mutation |
| 1802 | TP53 | NM_000546 | 45194 | c.487delT | p.Y163fs*7 | Loss of Function | Frame_Shift_Del |
| 1803 | TP53 | NM_000546 | 43846 | c.487T>C | p.Y163H | Loss of Function | Missense_Mutation |
| 1804 | TP53 | NM_000546 | 44623 | c.487T>A | p.Y163N | Loss of Function | Missense_Mutation |
| 1805 | TP53 | NM_000546 | 44224 | c.721T>G | p.S241A | Loss of Function | Missense_Mutation |
| 1806 | TP53 | NM_000546 | 44391 | c.489C>T | p.Y163Y | Loss of Function | Synonymous_Mutation |
| 1807 | TP53 | NM_000546 | 43928 | c.615T>A | p.Y205* | Loss of Function | Nonsense_Mutation |
| 1808 | TP53 | NM_000546 | 44924 | c.615T>G | p.Y205* | Loss of Function | Nonsense_Mutation |
| 1809 | TP53 | NM_000546 | 43947 | c.614A>G | p.Y205C | Loss of Function | Missense_Mutation |
| 1810 | TP53 | NM_000546 | 45168 | c.722_724delCCT | p.S241del | Loss of Function | In_Frame_Del |
| 1811 | TP53 | NM_000546 | 45548 | c.721_723delTCC | p.S241del | Loss of Function | In_Frame_Del |
| 1812 | TP53 | NM_000546 | 44067 | c.721T>A | p.S241T | Loss of Function | Missense_Mutation |
| 1813 | TP53 | NM_000546 | 45695 | c.613T>A | p.Y205N | Loss of Function | Missense_Mutation |
| 1814 | TP53 | NM_000546 | 146240 | c.806_808delGCT | p.S269_F270>I | Loss of Function | In_Frame_Del |
| 1815 | TP53 | NM_000546 | 44505 | c.660T>G | p.Y220* | Loss of Function | Nonsense_Mutation |
| 1816 | TP53 | NM_000546 | 10758 | c.659A>G | p.Y220C | Loss of Function | Missense_Mutation |
| 1817 | TP53 | NM_000546 | 45248 | c.805A>T | p.S269C | Loss of Function | Missense_Mutation |
| 1818 | TP53 | NM_000546 | 44585 | c.655delC | p.Y220fs*27 | Loss of Function | Frame_Shift_Del |
| 1819 | TP53 | NM_000546 | 44637 | c.658T>C | p.Y220H | Loss of Function | Missense_Mutation |
| 1820 | TP53 | NM_000546 | 43962 | c.805A>G | p.S269G | Loss of Function | Missense_Mutation |
| 1821 | TP53 | NM_000546 | 43850 | c.659A>C | p.Y220S | Loss of Function | Missense_Mutation |
| 1822 | TP53 | NM_000546 | 45114 | c.702C>A | p.Y234* | Loss of Function | Nonsense_Mutation |
| 1823 | TP53 | NM_000546 | 10725 | c.701A>G | p.Y234C | Loss of Function | Missense_Mutation |
| 1824 | TP53 | NM_000546 | 44236 | c.806G>A | p.5269N | Loss of Function | Missense_Mutation |
| 1825 | TP53 | NM_000546 | 44886 | c.807C>T | p.S269S | Loss of Function | Missense_Mutation |
| 1826 | TP53 | NM_000546 | 45507 | c.806G>C | p.S269T | Loss of Function | Missense_Mutation |
| 1827 | TP53 | NM_000546 | 43956 | c.700T>A | p.Y234N | Loss of Function | Missense . Mutation |
| 1828 | TP53 | NM_000546 | 43865 | c.701A>C | p.Y234S | Loss of Function | Missense_Mutation |
| 1829 | TP53 | NM_000546 | 43564 | c.708C>A | p.Y236* | Loss of Function | Nonsense_Mutation |
| 1830 | TP53 | NM_000546 | 44960 | c.708C>G | p.Y236* | Loss of Function | Nonsense_Mutation |
| 1831 | TP53 | NM_000546 | 10731 | c.707A>G | p.Y236C | Loss of Function | Missense_Mutation |
| 1832 | TP53 | NM_000546 | 43602 | c.706T>G | p.Y236D | Loss of Function | Missense_Mutation |
| 1833 | TP53 | NM_000546 | 44565 | c.907A>T | p.5303C | Loss of Function | Missense_Mutation |
| 1834 | TP53 | NM_000546 | 43986 | c.908G>A | p.S303N | Loss of Function | Missense_Mutation |
| 1835 | TP53 | NM_000546 | 43826 | c.706T>A | p.Y236N | Loss of Function | Missense_Mutation |
| 1836 | TP53 | NM_000546 | 44167 | c.908G>C | p.S303T | Loss of Function | Missense_Mutation |
| 1837 | TP53 | NM_000546 | 44132 | c.708C>T | p.Y236Y | Loss of Function | Synonymous_Mutation |
| 1838 | TP53_ENST00000269305 | ENST00000269305 | 131534 | c.559+1G>A | p.? | Loss of Function | N/A |
| 1839 | TP53 | NM_000546 | 44832 | c.1096T>G | p.5366A | Loss of Function | Missense_Mutation |
| 1840 | TP53_ENST00000269305 | ENST00000269305 | 179823 | c.528C>A | p.C176* | Loss of Function | Nonsense_Mutation |
| 1841 | TP53 | NM_000546 | 44048 | c.280T>A | p.S94T | Loss of Function | Missense_Mutation |
| 1842 | TP53 | NM_000546 | 44673 | c.284C>T | p.S95F | Loss of Function | Missense_Mutation |
| 1843 | TP53 | NM_000546 | 44447 | c.287C>T | p.S96F | Loss of Function | Missense_Mutation |
| 1844 | TP53 | NM_000546 | 44036 | c.296C>T | p.S99F | Loss of Function | Missense_Mutation |
| 1845 | TP53_ENST00000269305 | ENST00000269305 | 118013 | c.592G>T | p.E198* | Loss of Function | Nonsense_Mutation |
| 1846 | TP53 | NM_000546 | 43678 | c.305C>T | p.T1021 | Loss of Function | Missense_Mutation |
| 1847 | TP53 | NM_000546 | 44552 | c.509C>T | p.T170M | Loss of Function | Missense_Mutation |
| 1848 | TP53_ENST00000269305 | ENST00000269305 | 126981 | c.880G>T | p.E294* | Loss of Function | Nonsense_Mutation |
| 1849 | TP53 | NM_000546 | 44238 | c.631A>G | p.T211A | Loss of Function | Missense_Mutation |
| 1850 | TP53 | NM_000546 | 44661 | c.632C>A | p.T211N | Loss of Function | Missense_Mutation |
| 1851 | TP53 | NM_000546 | 43868 | c.689C>T | p.T230I | Loss of Function | Missense_Mutation |
| 1852 | TP53_ENST00000269305 | ENST00000269305 | 111498 | c.532delC | p.H178fs*69 | Loss of Function | Frame_Shift_Del |
| 1853 | TP53 | NM_000546 | 43806 | c.689C>A | p.T230N | Loss of Function | Missense_Mutation |
| 1854 | TP53 | NM_000546 | 45631 | c.688A>T | P.T230S | Loss of Function | Missense_Mutation |
| 1855 | TP53 | NM_000546 | 43980 | c.691A>G | p.T231A | Loss of Function | Missense_Mutation |
| 1856 | TP53 | NM_000546 | 44820 | c.692C>T | p.T231I | Loss of Function | Missense_Mutation |
| 1857 | TP53 | NM_000546 | 43889 | c.691A>T | p.T231S | Loss of Function | Missense_Mutation |
| 1858 | TP53 | NM_000546 | 45322 | c.757A>G | p.T253A | Loss of Function | Missense_Mutation |
| 1859 | TP53 | NM_000546 | 43683 | c.758C>T | p.T253I | Loss of Function | Missense_Mutation |
| 1860 | TP53 | NM_000546 | 45980 | c.757A>C | p.T253P | Loss of Function | Missense_Mutation |
| 1861 | TP53_ENST00000269305 | ENST00000269305 | 117949 | c.574C>T | p.Q192* | Loss of Function | Nonsense_Mutation |
| 1862 | TP53 | NM_000546 | 43881 | c.757A>T | p.T253S | Loss of Function | Missense_Mutation |
| 1863 | TP53 | NM_000546 | 44544 | c.766A>G | p.T256A | Loss of Function | Missense_Mutation |
| 1864 | TP53 | NM_000546 | 44662 | c.766A>T | p.T256S | Loss of Function | Missense_Mutation |
| 1865 | TP53_ENST00000269305 | ENST00000269305 | 99668 | c.586C>T | p.R196* | Loss of Function | Nonsense_Mutation |
| 1866 | TP53_ENST00000269305 | ENST00000269305 | 99618 | c.637C>T | p.R213* | Loss of Function | Nonsense_Mutation |
| 1867 | TP53 | NM_000546 | 45728 | c.850A>G | p.T284A | Loss of Function | Missense_Mutation |
| 1868 | TP53 | NM_000546 | 46207 | c.910A>G | p.T304A | Loss of Function | Missense_Mutation |
| 1869 | TP53 | NM_000546 | 45128 | c.911C>T | p.T304I | Loss of Function | Missense_Mutation |
| 1870 | TP53 | NM_000546 | 44200 | c.242C>T | p.T81I | Loss of Function | Missense_Mutation |
| 1871 | TP53 | NM_000546 | 44329 | c.470T>A | p.V157D | Loss of Function | Missense_Mutation |
| 1872 | TP53 | NM_000546 | 45551 | c.469_471delGTC | p.V157del | Loss of Function | In_Frame_Del |
| 1873 | TP53_ENST00000269305 | ENST00000269305 | 131480 | c.469G>T | p.V157F | Loss of Function | Missense_Mutation |
| 1874 | TP53_ENST00000413465 | ENST00000413465 | 131481 | c.469G>T | p.V157F | Loss of Function | Missense_Mutation |
| 1875 | TP53 | NM_000546 | 43625 | c.469G>A | p.V157I | Loss of Function | Missense_Mutation |
| 1876 | TP53_ENST00000269305 | ENST00000269305 | 99947 | c.916C>T | p.R306* | Loss of Function | Nonsense_Mutation |
| 1877 | TP53_ENST00000269305 | ENST00000269305 | 99721 | c.1024C>T | p.R342* | Loss of Function | Nonsense_Mutation |
| 1878 | TP53 | NM_000546 | 45120 | c.469G>C | p.V157L | Loss of Function | Missense_Mutation |
| 1879 | TP53 | NM_000546 | 44996 | c.515T>C | p.V172A | Loss of Function | Missense_Mutation |
| 1880 | TP53 | NM_000546 | 44229 | c.515T>A | p.V172D | Loss of Function | Missense_Mutation |
| 1881 | TP53 | NM_000546 | 43955 | c.514G>A | p.V172I | Loss of Function | Missense_Mutation |
| 1882 | TP53 | NM_000546 | 44327 | c.518T>C | p.V173A | Loss of Function | Missense_Mutation |
| 1883 | TP53_ENST00000269305 | ENST00000269305 | 121042 | c.517G>C | p.V173L | Loss of Function | Missense_Mutation |
| 1884 | TP53_ENST00000269305 | ENST00000269305 | 99946 | c.378C>G | p.Y126* | Loss of Function | Nonsense_Mutation |
| 1885 | TP53_ENST00000269305 | ENST00000269305 | 99641 | c.517G>T | p.V173L | Loss of Function | Missense_Mutation |
| 1886 | TP53_ENST00000413465 | ENST00000413465 | 121043 | c.517G>C | p.V173L | Loss of Function | Missense_Mutation |
| 1887 | TP53_ENST00000413465 | ENST00000413465 | 99638 | c.517G>T | p.V173L | Loss of Function | Missense_Mutation |
| 1888 | TP53_ENST00000413465 | ENST00000413465 | 98964 | c.517G>A | p.V173M | Loss of Function | Missense_Mutation |
| 1889 | TP53 | NM_000546 | 44424 | c.590T>A | p.V197E | Loss of Function | Missense_Mutation |
| 1890 | TP53_ENST00000413465 | ENST00000413465 | 131535 | c.559+1G>A | p.? | Loss of Function | N/A |
| 1891 | TP53 | NM_000546 | 46212 | c.589G>T | p.V197L | Loss of Function | Missense_Mutation |
| 1892 | TP53_ENST00000413465 | ENST00000413465 | 179822 | c.528C>A | p.C176* | Loss of Function | Nonsense_Mutation |
| 1893 | TP53 | NM_000546 | 43779 | c.589G>A | p.V197M | Loss of Function | Missense_Mutation |
| 1894 | TP53 | NM_000546 | 44411 | c.608T>A | p.V203E | Loss of Function | Missense_Mutation |
| 1895 | TP53_ENST00000413465 | ENST00000413465 | 118010 | c.592G>T | p.E198* | Loss of Function | Nonsense_Mutation |
| 1896 | TP53 | NM_000546 | 44365 | c.607G>T | p.V203L | Loss of Function | Missense_Mutation |
| 1897 | TP53 | NM_000546 | 43599 | c.607G>A | pV203M | Loss of Function | Missense_Mutation |
| 1898 | TP53_ENST00000413465 | ENST00000413465 | 111495 | c.532delC | p.H178fs*69 | Loss of Function | Frame_Shift_Del |
| 1899 | TP53 | NM_000546 | 44567 | c.647T>C | p.V216A | Loss of Function | Missense_Mutation |
| 1900 | TP53 | NM_000546 | 44607 | c.646_648delGTG | p.V216del | Loss of Function | In_Frame_Del |
| 1901 | TP53 | NM_000546 | 45110 | c.650T>C | p.V217A | Loss of Function | Missense_Mutation |
| 1902 | TP53 | NM_000546 | 44929 | c.650T>A | p.V217E | Loss of Function | Missense_Mutation |
| 1903 | TP53 | NM_000546 | 44375 | c.650T>G | p.V217G | Loss of Function | Missense_Mutation |
| 1904 | TP53_ENST00000413465 | ENST00000413465 | 117946 | c.574C>T | p.Q192* | Loss of Function | Nonsense_Mutation |
| 1905 | TP53 | NM_000546 | 44334 | c.649G>T | p.V217L | Loss of Function | Missense_Mutation |
| 1906 | TP53 | NM_000546 | 44930 | c.653T>C | p.V218A | Loss of Function | Missense_Mutation |
| 1907 | TP53 | NM_000546 | 6496 | c.652_654delGTG | p.V218del | Loss of Function | In_Frame_Del |
| 1908 | TP53_ENST00000413465 | ENST00000413465 | 99665 | c.586C>T | p.R196* | Loss of Function | Nonsense_Mutation |
| 1909 | TP53_ENST00000413465 | ENST00000413465 | 99615 | c.637C>T | p.R213* | Loss of Function | Nonsense_Mutation |
| 1910 | TP53 | NM_000546 | 44317 | c.653T>A | p.V218E | Loss of Function | Missense_Mutation |
| 1911 | TP53 | NM_000546 | 44683 | c.652G>A | p.V218M | Loss of Function | Missense_Mutation |
| 1912 | TP53_ENST00000414315 | ENST00000414315 | 131483 | c.73G>T | p.V25F | Loss of Function | Missense_Mutation |
| 1913 | TP53 | NM_000546 | 44294 | c.815T>C | p.V272A | Loss of Function | Missense_Mutation |
| 1914 | TP53 | NM_000546 | 44580 | c.815T>A | p.V272E | Loss of Function | Missense_Mutation |
| 1915 | TP53 | NM_000546 | 10859 | c.814G>T | p.V272L | Loss of Function | Missense_Mutation |
| 1916 | TP53 | NM_000546 | 45898 | c.814G>C | p.V272L | Loss of Function | Missense_Mutation |
| 1917 | TP53_ENST00000269305 | ENST00000269305 | 99950 | c.814G>A | p.V272M | Loss of Function | Missense_Mutation |
| 1918 | TP53_ENST00000269305 | ENST00000269305 | 165075 | c.820G>T | p.V274F | Loss of Function | Missense_Mutation |
| 1919 | TP53_ENST00000413465 | ENST00000413465 | 99944 | c.378C>G | p.Y126* | Loss of Function | Nonsense_Mutation |
| 1920 | TP53 | NM_000546 | 43667 | c.820G>A | p.V274I | Loss of Function | Missense_Mutation |
| 1921 | TP53_ENST00000414315 | ENST00000414315 | 121045 | c.121G>C | p.V41L | Loss of Function | Missense_Mutation |
| 1922 | TP53_ENST00000414315 | ENST00000414315 | 99639 | c.121G>T | p.V41L | Loss of Function | Missense_Mutation |
| 1923 | TP53_ENST00000414315 | ENST00000414315 | 98965 | c.121G>A | p.V41M | Loss of Function | Missense_Mutation |
| 1924 | TP53_ENST00000545858 | ENST00000545858 | 131482 | c.190G>T | p.V64F | Loss of Function | Missense_Mutation |
| 1925 | TP53_ENST00000414315 | ENST00000414315 | 131537 | c.163+1G>A | p.? | Loss of Function | N/A |
| 1926 | TP53 | NM_000546 | 45288 | c.217G>C | p.V73L | Loss of Function | Missense_Mutation |
| 1927 | TP53_ENST00000414315 | ENST00000414315 | 179824 | c.132C>A | p.C44* | Loss of Function | Nonsense_Mutation |
| 1928 | TP53 | NM_000546 | 43787 | c.217G>A | p.V73M | Loss of Function | Missense_Mutation |
| 1929 | TP53_ENST00000414315 | ENST00000414315 | 118011 | c.196G>T | p.E66* | Loss of Function | Nonsense_Mutation |
| 1930 | TP53_ENST00000414315 | ENST00000414315 | 111496 | c.136delC | pH46fs*>45 | Loss of Function | Frame_Shift_Del |
| 1931 | TP53_ENST00000545858 | ENST00000545858 | 121044 | c.238G>C | p.V80L | Loss of Function | Missense_Mutation |
| 1932 | TP53.ENST00000545858 | ENST00000545858 | 99640 | c.238G>T | p.V80L | Loss of Function | Missense_Mutation |
| 1933 | TP53_ENST00000545858 | ENST00000545858 | 98966 | c.238G>A | p.V80M | Loss of Function | Missense_Mutation |
| 1934 | TP53_ENST00000269305 | ENST00000269305 | 220766 | c.319T>G | p.Y107D | Loss of Function | Missense_Mutation |
| 1935 | TP53_ENST00000414315 | ENST00000414315 | 117947 | c.178C>T | p.Q60^{∗} | Loss of Function | Nonsense_Mutation |
| 1936 | TP53_ENST00000413465 | ENST00000413465 | 220765 | c.319T>G | p.Y107D | Loss of Function | Missense_Mutation |
| 1937 | TP53 | NM_000546 | 44405 | c.376_396del21 | p.Y126_K132delYSP | Loss of Function | In_Frame_Del |
| 1938 | TP53_ENST00000414315 | ENST00000414315 | 99666 | c.190C>T | p.R64* | Loss of Function | Nonsense_Mutation |
| 1939 | TP53_ENST00000414315 | ENST00000414315 | 99616 | c.241C>T | p.R81* | Loss of Function | Nonsense_Mutation |
| 1940 | TP53 | NM_000546 | 44774 | c.376_393del18 | p.Y126_N131delYSP | Loss of Function | In_Frame_Del |
| 1941 | TP53_ENST00000269305 | ENST00000269305 | 220783 | c.376T>G | p.Y126D | Loss of Function | Missense_Mutation |
| 1942 | TP53_ENST00000413465 | ENST00000413465 | 220782 | c.376T>G | p.Y126D | Loss of Function | Missense_Mutation |
| 1943 | TP53_ENST00000545858 | ENST00000545858 | 99719 | c.380A>G | p.Y127C | Loss of Function | Missense_Mutation |
| 1944 | TP53_ENST00000545858 | ENST00000545858 | 165074 | c.422A>G | p.Y141C | Loss of Function | Missense_Mutation |
| 1945 | TP53_ENST00000545858 | ENST00000545858 | 116673 | c.428A>G | p.Y143C | Loss of Function | Missense_Mutation |
| 1946 | TP53_ENST00000545858 | ENST00000545858 | 131536 | c.280+1G>A | p.? | Loss of Function | N/A |
| 1947 | TP53_ENST00000269305 | ENST00000269305 | 129852 | c.488A>G | p.Y163C | Loss of Function | Missense_Mutation |
| 1948 | TP53_ENST00000413465 | ENST00000413465 | 129853 | c.488A>G | p.Y163C | Loss of Function | Missense_Mutation |
| 1949 | TP53_ENST00000545858 | ENST00000545858 | 179825 | c.249C>A | p.C83* | Loss of Function | Nonsense_Mutation |
| 1950 | TP53 | NM_000546 | 45025 | c.488A>C | p.Y163S | Loss of Function | Missense_Mutation |
| 1951 | TP53_ENST00000545858 | ENST00000545858 | 118012 | c.313G>T | p.E105* | Loss of Function | Nonsense_Mutation |
| 1952 | TP53 | NM_000546 | 43844 | c.613T>G | p.Y205D | Loss of Function | Missense_Mutation |
| 1953 | TP53 | NM_000546 | 11351 | c.614A>T | p.Y205F | Loss of Function | Missense_Mutation |
| 1954 | TP53 | NM_000546 | 43642 | c.613T>C | p.Y205H | Loss of Function | Missense_Mutation |
| 1955 | TP53_ENST00000545858 | ENST00000545858 | 111497 | c.253delC | p.H85fs*69 | Loss of Function | Frame_Shift_Del |
| 1956 | TP53 | NM_000546 | 44169 | c.614A>C | p.Y205S | Loss of Function | Missense_Mutation |
| 1957 | TP53_ENST00000269305 | ENST00000269305 | 99720 | c.659A>G | p.Y220C | Loss of Function | Missense_Mutation |
| 1958 | TP53_ENST00000413465 | ENST00000413465 | 99718 | c.659A>G | p.Y220C | Loss of Function | Missense_Mutation |
| 1959 | TP53 | NM_000546 | 11847 | c.658T>G | p.Y220D | Loss of Function | Missense_Mutation |
| 1960 | TP53_ENST00000545858 | ENST00000545858 | 117948 | c.295C>T | p.Q99* | Loss of Function | Nonsense_Mutation |
| 1961 | TP53_ENST00000545858 | ENST00000545858 | 99667 | c.307C>T | p.R103* | Loss of Function | Nonsense_Mutation |
| 1962 | TP53_ENST00000545858 | ENST00000545858 | 99617 | c.358C>T | p.R120* | Loss of Function | Nonsense_Mutation |
| 1963 | TP53 | NM_000546 | 44672 | c.658T>A | p.Y220N | Loss of Function | Missense_Mutation |
| 1964 | TP53_ENST00000269305 | ENST00000269305 | 165073 | c.701A>G | p.Y234C | Loss of Function | Missense_Mutation |
| 1965 | TP53_ENST00000413465 | ENST00000413465 | 165072 | c.701A>G | p.Y234C | Loss of Function | Missense_Mutation |
| 1966 | TP53 | NM_000546 | 43768 | c.700T>G | p.Y234D | Loss of Function | Missense_Mutation |
| 1967 | TP53 | NM_000546 | 44953 | c.700_702delTAC | p.Y234del | Loss of Function | In_Frame_Del |
| 1968 | TP53 | NM_000546 | 11152 | c.700T>C | p.Y234H | Loss of Function | Missense_Mutation |
| 1969 | TP53_ENST00000269305 | ENST00000269305 | 116674 | c.707A>G | p.Y236C | Loss of Function | Missense_Mutation |
| 1970 | TP53_ENST00000413465 | ENST00000413465 | 116672 | c.707A>G | p.Y236C | Loss of Function | Missense_Mutation |
| 1971 | TP53 | NM_000546 | 44072 | c.706_708delTAC | p.Y236del | Loss of Function | In_Frame_Del |
| 1972 | TP53 | NM_000546 | 44326 | c.706T>C | p.Y236H | Loss of Function | Missense_Mutation |
| 1973 | TP53 | NM_000546 | 44693 | c.707A>C | p.Y236S | Loss of Function | Missense_Mutation |
| 1974 | TP53_ENST00000414315 | ENST00000414315 | 129855 | c.92A>G | p.Y31C | Loss of Function | Missense_Mutation |
| 1975 | TP53_ENST00000545858 | ENST00000545858 | 99945 | c.99C>G | p.Y33* | Loss of Function | Nonsense_Mutation |
| 1976 | TP53_ENST00000545858 | ENST00000545858 | 220784 | c.97T>G | p.Y33D | Loss of Function | Missense_Mutation |
| 1977 | TP53_ENST00000545858 | ENST00000545858 | 129854 | c.209A>G | p.Y70C | Loss of Function | Missense_Mutation |

## Claims

1. A method to determine an actionable treatment recommendation for a subject diagnosed with lung cancer, comprising:
in a single biological sample obtained from the subject, using a set of amplification primer pairs that hybridize to and amplify ALK, ROS1, KRAS, BRAF, ERBB2, MET, RET, FGFR1 and KIT/PDGFRA genes to generate amplicons in a single assay, wherein the set of amplification primer pairs can amplify nucleic acid of one or more lung cancer-associated allelic variants of said genes; and
detecting by nucleic acid sequencing at least one variant among ALK, ROS1, KRAS, BRAF, ERBB2, MET, RET, FGFR1, and KIT/PDGFRA, genes from the sample, and
determining, based on the at least one variant detected, an actionable treatment recommendation for the subject.

2. The method of claim 1, wherein the variant additionally includes EGFR ERRBB4, FGFR2, FGFR3, DDR2, NRAS, PTEN, MAP2K1, TP53, STK11, CTNNB1, SMAD4, FBXW7, PIK3CA, AKT1, HRAS and NOTCH 1 genes.

3. The method of claim 1, wherein the lung cancer is an adenocarcinoma or a squamous cell carcinoma.

4. The method of claim 1, wherein the sample is tumor tissue, optionally FFPE tumor tissue, a blood sample comprising circulating tumor cells or cell-free DNA.

5. The method of claim 1, wherein the at least one variant is selected from ALK fusion, ROS1 fusion (EZR, SLC34A2, CD74, and/or SDC4), or MET gene amplification, EGFR (L858R, Exon 19 del, G719X and/or T790M), KRAS (G12C/V/D/A/S/R/F, G13C, G13D and/or G12F), BRAF (L597R, D594H/N, V600E), ERBB2 exon 20 ins, PIK3CA (E545K, E545G, E545a, H1047R, E542K, and/or H1047L), and a combination thereof.

6. The method of claim 1, wherein the actionable treatment recommendation is a course of treatment, refraining from a treatment, enrollment in a clinical trial, or a combination thereof.

7. The method of claim 1 or claim 2, wherein the actionable treatment recommendation is selected from
a. Crizotinib when the variant detected is an ALK fusion, ROS1 fusion (EZR, SLC34A2, CD74, and/or SDC4), or MET gene amplification;
b. EGFR tyrosine kinase inhibitor (TKI) when the variant detected is EGFR (L858R, Exon 19 del, and/or G719X);
c. A non EGFR TKI when the variant detected is EGFR T790M;
d. A MEK inhibitor when the variant detected is KRAS G12C/V/D/A/S/R/F, G13C, G13D and/or G12F;
e. Vermurafenib when the variant detected is BRAF V600E;
f. An irreversible pan-erb inhibitor when the variant detected is ERBB2 exon 20 ins; and
g. A PIC3CA inhibitor when the variant detected is PIK3CA (E545K, E545G, E545a, H1047R, and/or H1047L).

8. Use of the method of claim 7 to determine the likelihood of a response to a treatment in an individual afflicted with lung cancer, wherein the presence of at least one variant indicates the individual is likely or unlikely to respond to the treatment.

9. The method of claim 7, wherein the actionable treatment recommendation is selected from Crizotinib and the variant detected is an ALK fusion, and wherein the ALK gene fusion isoform is an EML4-ALK gene fusion isoform.

10. The method of any one of the previous claims, wherein the nucleic acid sequencing is by next generation sequencing technology.

11. A kit comprising a set of amplification primer pairs in a single panel, wherein the set of amplification primer pairs specifically recognize the genes, ALK, ROS1, KRAS, BRAF, ERBB2, MET, RET, FGFR1, and KIT/PDGFRA and wherein the set of amplification primer pairs can amplify nucleic acid of one or more lung cancer-associated allelic variants of said genes.

12. The kit of claim 11, additionally comprising amplification primer pairs that specifically recognize the genes EGFR ERRBB4, FGFR2, FGFR3, DDR2, NRAS, PTEN, MAP2K1, TP53, STK11, CTNNB1, SMAD4, FBXW7, PIK3CA, AKT1, HRAS and NOTCH 1 and wherein the amplification primer pairs can amplify nucleic acid of one or more lung cancer-associated allelic variants of said genes

13. The kit of claim 11, wherein the allelic variants include one or more of the polynucleotides encoding AKT1 (E17K), BRAF (L597R, D594H/N, V600E), EGFR (L858R, G719X, T790M), HRAS (Q61L/K/R, G12C/D), KRAS G12A/C/D/F/R/V) and PIK3CA (E545A/G/K, H1047L/R), preferably EGFR (L858R, G719X, T790M) and KRAS G12A/C/D/F/R/V).

14. A composition comprising a set of amplification primer pairs, wherein the set of amplification primer pairs specifically recognize the genes ALK, ROS1, KRAS, BRAF, ERBB2, MET, RET, FGFR1, and KIT/PDGFRA, and wherein the set of amplification primer pairs can amplify nucleic acid of one or more lung cancer-associated allelic variants of the genes ALK, ROS1, KRAS, BRAF, ERBB2, MET, RET, FGFR1, and KIT/PDGFRA and optionally further comprising a sample, wherein the sample comprises nucleic acids from lung cancer tumor cells.

15. The method, kit or composition of any one of the previous claims, wherein the gene variants are selected from an AI and prevalence selected from AI1 + Prevalence > 1%, A12 + Prevalence > 1%, AI3 + Prevalence > 1%, AI1 + Prevalence 0.1%-1%, AI2 + Prevalence 0.1%-1%, AI3 + Prevalence 0.1%-1%, and combinations thereof.

## Patentansprüche

1. Verfahren zum Bestimmen einer umsetzbaren Behandlungsempfehlung für einen Probanden, der/die mit Lungenkrebs diagnostiziert wurde, umfassend:
in einer einzigen biologischen Probe, die von dem Probanden erhalten wird, unter Verwendung eines Satzes von Amplifikationsprimerpaaren, die an ALK, ROS1, KRAS, BRAF, ERBB2, MET, RET, FGFR1 und KIT/PDGFRA-Gene hybridisieren und diese amplifizieren, um Amplikons in einem einzigen Assay zu erzeugen, wobei der Satz von Amplifikationsprimerpaaren Nukleinsäure einer oder mehrerer Lungenkrebs-assoziierter Allelvarianten der Gene amplifizieren kann; und
Detektieren, durch Nukleinsäuresequenzierung, von mindestens einer Variante unter ALK, ROS1, KRAS, BRAF, ERBB2, MET, RET, FGFR1 und KIT/PDGFRA-Genen aus der Probe, und Bestimmen, basierend auf der mindestens einen detektierten Variante, einer umsetzbaren Behandlungsempfehlung für den Probanden.

2. Verfahren nach Anspruch 1, wobei die Variante zusätzlich EGFR ERRBB4, FGFR2, FGFR3, DDR2, NRAS, PTEN, MAP2K1, TP53, STK11, CTNNB1, SMAD4, FBXW7, PIK3CA, AKT1, HRAS und NOTCH-1 Gene einschließt.

3. Verfahren nach Anspruch 1, wobei der Lungenkrebs ein Adenokarzinom oder ein Plattenepithelkarzinom ist.

4. Verfahren nach Anspruch 1, wobei die Probe Tumorgewebe, optional FFPE-Tumorgewebe, eine Blutprobe umfassend zirkulierende Tumorzellen oder zellfreie DNA ist.

5. Verfahren nach Anspruch 1, wobei die mindestens eine Variante ausgewählt ist aus ALK-Fusion, ROS1-Fusion (EZR, SLC34A2, CD74 und/oder SDC4) oder MET-Genamplifikation, EGFR (L858R, Exon-19-del, G719X und/oder T790M), KRAS (G12C/V/D/A/S/R/F, G13C, G13D und/oder G12F), BRAF (L597R, D594H/N, V600E), ERBB2-Exon-20-ins, PIK3CA (E545K, E545G, E545a, H1047R, E542K und/oder H1047L) und einer Kombination davon.

6. Verfahren nach Anspruch 1, wobei die umsetzbare Behandlungsempfehlung eine Behandlung, ein Verzicht auf eine Behandlung, die Anmeldung zu einer klinischen Studie oder eine Kombination davon ist.

7. Verfahren nach Anspruch 1 oder Anspruch 2, wobei die umsetzbare Behandlungsempfehlung ausgewählt ist aus
a. Crizotinib, wenn die detektierte Variante eine ALK-Fusion, ROS1-Fusion (EZR, SLC34A2, CD74 und/oder SDC4) oder MET-Genamplifikation ist;
b. EGFR-Tyrosinkinase-Inhibitor (TKI), wenn die detektierte Variante EGFR (L858R, Exon-19-del und/oder G719X) ist;
c. einem Nicht-EGFR-TKI, wenn die detektierte Variante EGFR-T790M ist;
d. einem MEK-Inhibitor, wenn die detektierte Variante KRAS-G12C/V/D/A/S/R/F, G13C, G13D und/oder G12F ist;
e. Vermurafenib, wenn die detektierte Variante BRAF-V600E ist;
f. einem irreversiblen pan-erb-Inhibitor, wenn die detektierte Variante ERBB2-Exon-20-ins ist; und
g. einem PIC3CA-Inhibitor, wenn die detektierte Variante PIK3CA (E545K, E545G, E545a, H1047R, und/oder H1047L) ist.

8. Verwendung des Verfahrens nach Anspruch 7, um die Wahrscheinlichkeit eines Ansprechens einer Behandlung bei einer an Lungenkrebs leidenden Person zu bestimmen, wobei das Vorhandensein mindestens einer Variante darauf hinweist, dass die Person wahrscheinlich oder unwahrscheinlich auf die Behandlung anspricht.

9. Verfahren nach Anspruch 7, wobei die umsetzbare Behandlungsempfehlung aus Crizotinib ausgewählt ist und die detektierte Variante eine ALK-Genfusion ist, und wobei die ALK-Genfusionsisoform eine EML4-ALK-Genfusionsisoform ist.

10. Verfahren nach einem der vorhergehenden Ansprüche, wobei die Nukleinsäuresequenzierung durch Sequenzierungstechnologie der nächsten Generation erfolgt.

11. Kit, das einen Satz Amplifikationsprimerpaare in einem einzigen Panel umfasst, wobei der Satz Amplifikationsprimerpaare spezifisch die Gene ALK, ROS1, KRAS, BRAF, ERBB2, MET, RET, FGFR1 und KIT/PDGFRA erkennt und wobei der Satz Amplifikationsprimerpaare Nukleinsäure einer oder mehrerer Lungenkrebs-assoziierter Allelvarianten dieser Gene amplifizieren können.

12. Kit nach Anspruch 11, zusätzlich umfassend Amplifikationsprimerpaare, die spezifisch die Gene EGFR ERRBB4, FGFR2, FGFR3, DDR2, NRAS, PTEN, MAP2K1, TP53, STK11, CTNNB1, SMAD4, FBXW7, PIK3CA, AKT1, HRAS und NOTCH-1 erkennen, und wobei die Amplifikationsprimerpaare eine Nukleinsäure einer oder mehrerer Lungenkrebs-assoziierter Allelvarianten dieser Gene amplifizieren können

13. Kit nach Anspruch 11, wobei die Allelvarianten ein oder mehrere der Polynukleotide einschließen, die für AKT1 (E17K), BRAF (L597R, D594H/N, V600E), EGFR (L858R, G719X, T790M), HRAS (Q61L/K/R, G12C/D), KRAS G12A/C/D/F/R/V) und PIK3CA (E545A/G/K, H1047L/R), vorzugsweise EGFR (L858R, G719X, T790M) und KRAS G12A/C/D/
F/R/V) kodieren.

14. Zusammensetzung, umfassend einen Satz Amplifikationsprimerpaare, wobei der Satz Amplifikationsprimerpaare spezifisch die Gene ALK, ROS1, KRAS, BRAF, ERBB2, MET, RET, FGFR1 und KIT/PDGFRA erkennt und wobei der Satz Amplifikationsprimerpaare Nukleinsäure einer oder mehrerer Lungenkrebs-assoziierter allelischer Varianten der Gene ALK, ROS1, KRAS, BRAF, ERBB2, MET, RET, FGFR1 und KIT/PDGFRA amplifizieren kann und optional weiterhin eine Probe umfasst, wobei die Probe Nukleinsäuren aus Lungenkrebstumorzellen umfasst.

15. Verfahren, Kit oder Zusammensetzung nach einem der vorherigen Ansprüche, wobei die Genvarianten aus einem AI und einer Prävalenz ausgewählt sind, ausgewählt aus AI1 + Prävalenz > 1 %, AI2 + Prävalenz > 1 %, AI3 + Prävalenz > 1 %, AI1 + Prävalenz 0,1 % - 1 %, AI2 + Prävalenz 0,1 % - 1 %, AI3 + Prävalenz 0,1 % - 1 % und Kombinationen davon.

## Revendications

1. Procédé permettant de déterminer une recommandation de traitement appropriée pour un sujet atteint d'un cancer du poumon, comprenant :
dans un échantillon biologique unique obtenu à partir du sujet, l'utilisation d'un ensemble de paires d'amorces d'amplification qui s'hybrident à ALK, ROS1, KRAS, BRAF, ERBB2, MET, RET, FGFR1 et KIT/PDGFRA et amplifient ceux-ci pour générer des amplicons dans un essai unique, dans lequel l'ensemble de paires d'amorces d'amplification peut amplifier l'acide nucléique d'un ou plusieurs variants alléliques associés à un cancer du poumon desdits gènes ; et
La détection par séquençage d'acide nucléique d'au moins un variant parmi les gènes ALK, ROS1, KRAS, BRAF, ERBB2, MET, RET, FGFR1, et KIT/PDGFRA de l'échantillon, et la détermination, sur la base de l'au moins un variant détecté, d'une recommandation de traitement appropriée pour le sujet.

2. Procédé selon la revendication 1, dans lequel le variant comporte en outre les gènes EGFR ERRBB4, FGFR2, FGFR3, DDR2, NRAS, PTEN, MAP2K1, TP53, STK11, CTNNB1, SMAD4, FBXW7, PIK3CA, AKT1, HRAS et NOTCH 1.

3. Procédé selon la revendication 1, dans lequel le cancer du poumon est un adénocarcinome ou un carcinome squameux.

4. Procédé selon la revendication 1, dans lequel l'échantillon est un tissu tumoral, éventuellement un tissu tumoral FFPE, un échantillon sanguin comprenant des cellules tumorales circulantes ou un ADN acellulaire.

5. Procédé selon la revendication 1, dans lequel l'au moins un variant est choisi parmi la fusion ALK, la fusion ROS1 (EZR, SLC34A2, CD74, et/ou SDC4), ou l'amplification de gène MET, EGFR (L858R, Exon 19 del, G719X et/ou T790M), KRAS (G12C/V/D/A/S/R/F, G13C, G13D et/ou G12F), BRAF (L597R, D594H/N, V600E), ERBB2 exon 20 ins, PIK3CA (E545K, E545G, E545a, H1047R, E542K, et/ou H1047L), et une combinaison de ceux-ci.

6. Procédé selon la revendication 1, dans lequel la recommandation de traitement appropriée est un cours de traitement, l'abstinence vis-à-vis d'un traitement, d'un enrôlement dans un essai clinique, ou d'une combinaison de ceux-ci.

7. Procédé selon la revendication 1 ou 2, dans lequel la recommandation de traitement appropriée est choisie parmi
a. le crizotinib lorsque le variant détecté est une fusion ALK, une fusion ROS1 (EZR, SLC34A2, CD74, et/ou SDC4), ou une amplification génique MET ;
b. l'inhibiteur de tyrosine kinase EGFR (TKI) lorsque le variant détecté est EGFR (L858R, Exon 19 del, et/ou G719X) ;
c. un non-EGFR TKI lorsque le variant détecté est EGFR T790M ;
d. un inhibiteur de MEK lorsque le variant détecté est KRAS G12C/V/D/A/S/R/F, G13C, G13D et/ou G12F ;
e. le vermurafenib lorsque le variant détecté est BRAF V600E ;
f. l'inhibiteur de pan-erb irréversible lorsque le variant détecté est l'exon 20 ins ERBB2 ; et
g. un inhibiteur de PIC3CA lorsque le variant détecté est PIK3CA (E545K, E545G, E545a, H1047R, et/ou HI047L).

8. Utilisation du procédé selon la revendication 7 pour déterminer la probabilité d'une réponse à un traitement chez un sujet atteint d'un cancer du poumon, dans laquelle la présence d'au moins un variant indique que l'individu est susceptible de répondre au traitement.

9. Procédé selon la revendication 7, dans lequel la recommandation de traitement appropriée est choisie parmi le crizotinib et le variant détecté est une fusion ALK, et dans lequel l'isoforme de fusion de gène ALK est une isoforme de fusion génique EML4-ALK.

10. Procédé selon l'une quelconque des revendications précédentes, dans lequel le séquençage d'acide nucléique est réalisée au moyen de la technologie de séquençage de nouvelle génération.

11. Kit comprenant un ensemble de paires d'amorces d'amplification dans un panneau unique, dans lequel l'ensemble de paires d'amorces d'amplification reconnaît spécifiquement les gènes, ALK, ROS1, KRAS, BRAF, ERBB2, MET, RET, FGFR1, et KIT/PDGFRA, et dans lequel l'ensemble de paires d'amorces d'amplification peut amplifier l'acide nucléique d'un ou de plusieurs variants alléliques associés à un cancer du poumon desdits gènes.

12. Kit selon la revendication 11, comprenant en outre des paires d'amorces d'amplification qui reconnaissent spécifiquement les gènes EGFR ERRBB4, FGFR2, FGFR3, DDR2, NRAS, PTEN, MAP2K1, TP53, STK11, CTNNB1, SMAD4, FBXW7, PIK3CA, AKT1, HRAS et NOTCH 1, et dans lequel les paires d'amorces d'amplification peuvent amplifier l'acide nucléique d'un ou de plusieurs variants alléliques associés à un cancer du poumon desdits gènes

13. Kit selon la revendication 11, dans lequel les variants alléliques comportent un ou plusieurs des polynucléotides codant pour AKT1 (E17K), BRAF (L597R, D594H/N, V600E), EGFR (L858R, G719X, T790M), HRAS (Q61L/K/R, G12C/D), KRAS G12A/C/D/F/R/V) et PIK3CA (E545A/G/K, H1047L/R), de préférence EGFR (L858R, G719X, T790M) et KRAS G12A/C/D/F/R/V).

14. Composition comprenant un ensemble de paires d'amorces d'amplification, dans laquelle l'ensemble de paires d'amorces d'amplification reconnaît spécifiquement les gènes ALK, ROS1, KRAS, BRAF, ERBB2, MET, RET, FGFR1, et KIT/PDGFRA, et dans laquelle l'ensemble de paires d'amorces d'amplification peut amplifier l'acide nucléique d'un ou de plusieurs variants alléliques associés à un cancer du poumon des gènes ALK, ROS1, KRAS, BRAF, ERBB2, MET, RET, FGFR1, et KIT/PDGFRA, et éventuellement comprenant en outre un échantillon, dans laquelle l'échantillon comprend des acides nucléiques provenant de cellules tumorales du cancer du poumon.

15. Procédé, kit ou composition selon l'une quelconque des revendications précédentes, dans lesquels les variants de gène sont choisis parmi un AI et une prévalence choisie parmi AI1 + prévalence > 1 %, AI2 + prévalence > 1 %, AI3 + prévalence > 1 %, AI1 + prévalence 0,1 % -1 %, AI2 + prévalence 0,1 % - 1 %, AI3 + prévalence 0,1 %-1 %, et des combinaisons de ceux-ci.
